(19) Europäisches Patentamt
European Patent Office
Office européen des brevets

(11) EP 3 211 003 A1

(12) EUROPEAN PATENT APPLICATION

(43) Date of publication:
**30.08.2017 Bulletin 2017/35**

(21) Application number: **16305218.6**

(22) Date of filing: **24.02.2016**

(51) Int Cl.:
***C07K 14/725*** *(2006.01)*
***A61K 35/17*** *(2015.01)*
***C07K 14/16*** *(2006.01)*
***C12N 15/867*** *(2006.01)*
***A61K 48/00*** *(2006.01)*
***C07K 16/10*** *(2006.01)*
***C12N 5/0783*** *(2010.01)*
***A61P 31/18*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Institut Pasteur**
**75724 Paris Cedex 15 (FR)**

(72) Inventors:
- **CHAKRABARTI, Lisa, Amita**
**75013 Paris (FR)**
- **BENATI, Daniela**
**42124 Reggio Emilia (IT)**
- **GALPERIN, Moran**
**92200 Neuilly-sur-Seine (FR)**

(74) Representative: **Desaix, Anne et al**
**Ernest Gutmann - Yves Plasseraud SAS**
**3, rue Auber**
**75009 Paris (FR)**

# (54) T CELL RECEPTORS FROM THE HIV-SPECIFIC REPERTOIRE, MEANS FOR THEIR PRODUCTION AND THERAPEUTIC USES THEREOF

(57) The present invention pertains to the field of T Cell receptors (TCR) identification and clonotyping, and especially concerns particular TCRs identified by clonotyping of a HIV-specific TCR repertoire, or fragments thereof. the invention relates especially to TCRs recognizing Gag peptide located between positions 293-312 in the GAG protein of HIV-1.

The present invention further relates to nucleic acid constructs suitable as means for cloning or expressing nucleic acid molecules or TCRs of the invention, such as plasmids, vectors, especially lentiviral transfer vectors.

The invention is of particular interest in the context of therapeutic treatment of human beings seropositive for HIV.

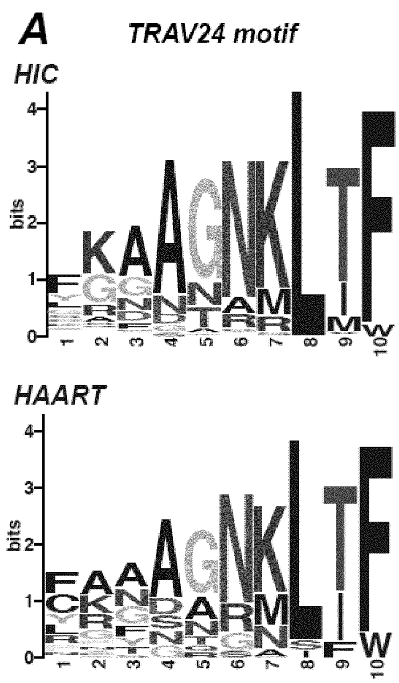


Fig. 9A

EP 3 211 003 A1

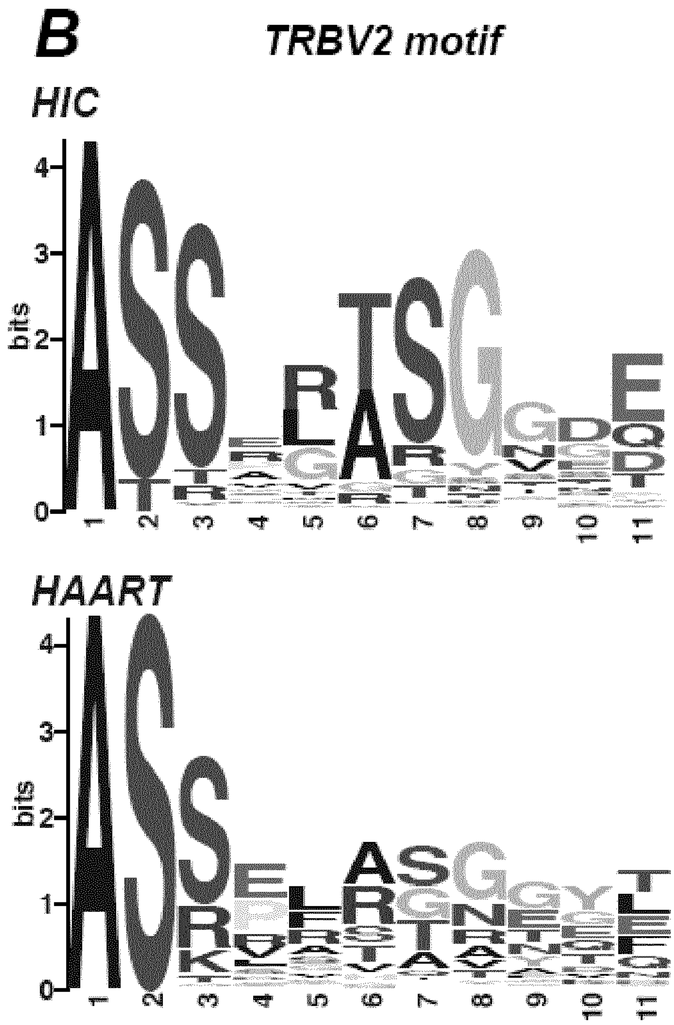


Fig. 9B

## Description

The present invention pertains to the field of T Cell receptors (TCR) identification and clonotyping, and especially concerns particular TCRs identified by clonotyping of a HIV-specific TCR repertoire, or fragments thereof.

The present invention further relates to chimeric TCRs derived from the TCRs of the invention or fragments thereof, and to nucleic acid molecules encoding the TCRs or fragments thereof of the invention.

The present invention further relates to nucleic acid constructs suitable as means for cloning or expressing nucleic acid molecules or TCRs of the invention, such as plasmids, vectors, especially lentiviral transfer vectors.

The present invention also provides recombinant lentiviral vector particles and methods and means for producing recombinant lentiviral vector particles, as well as a collection of recombinant human cells presenting TCRs of the invention or fragments thereof and methods for obtaining the same.

The invention is of particular interest in the context of therapeutic treatment of human beings.

The means of the invention can be especially formulated for administration to a human host, more particularly used as a medicament in the immunotherapeutic treatment of a human patient seropositive for HIV, in particular HIV-1.

The invention also relates to methods for the treatment of a patient infected with HIV, in particular HIV-1, especially for active control of HIV infection.

Such patients may be under antiretroviral therapy following a HIV infection, in particular a HIV-1 infection, especially undergoing HAART therapy (highly active antiretroviral therapy).

HIV relentlessly destroys CD4+ T cells in the course of infection and causes functional impairment in the remaining CD4+ T cell population. This leads to the progressive loss of adaptive responses to opportunistic pathogens and ultimately to the collapse of the immune system characteristic of AIDS. Chronic immune activation is thought to drive dysfunction of the remaining CD4+ T cell population, with both persistent viral antigenic stimulation and microbial translocation conspiring to exhaust T cell responses (1). Another parameter contributing to the loss of helper function may be the poor quality of CD4+ T cells that escape depletion. Early studies of the repertoire of T cell receptors (TCRs) documented a general loss of CD4+ T cell diversity in HIV infected patients (2, 3), while further studies highlighted a preferential depletion of HIV-specific CD4+ T cells (4-6), suggesting that the HIV-specific repertoire was especially prone to diversity contraction. To date, the HIV-specific CD4 repertoire remains mostly uncharacterized at the molecular level, even though this information would be critical to define the potential for immune reconstitution in treated patients.

Rare cases of spontaneously controlled HIV-1 infection provide a unique opportunity to study the molecular characteristics of a fully functional CD4+ T cell response directed at HIV. Patients who maintain an undetectable viral load in standard assays (<50 copies HIV-1 RNA/mL) represent fewer than 0.5% of seropositive individuals but have a remarkably low risk of progressing to AIDS (7). These rare patients, called HIV Controllers, or alternatively Elite Controllers, show signs of particularly efficient cellular responses that actively control the infected cell population (8). Controller CD8+ T cells have the capacity to potently inhibit HIV replication when added to cultures of infected autologous CD4+ T cells, and are thought to play a key role in HIV control (9, 10). Recent evidence suggest that particular TCR clonotypes expressed by Controller CD8+ T cells are responsible for their efficient cytotoxic responses, while HLA-matched non-Controller patients show clonotypes of lower efficacy (11, 12). The dominant Gag-specific clonotypes from Controllers appear more efficient at lyzing HIV-infected target cells through the rapid release of cytolytic granule content (12-14). In addition, clonotypes from Controllers are able to maintain cross-recognition of dominant epitope variants, thus preventing the emergence viral escape mutants (11, 12, 15).

However, the role of the CD4 response in HIV control remains more debated. Controllers maintain a population of HIV-specific CD4+ T cells endowed with a strong proliferative capacity, which has been linked to autocrine IL-2 production and the upregulation of anti-apoptotic molecules (16-18). A significant fraction of IL-2 producing CD4+ T cells in Controllers retain a central memory phenotype, while the HIV-specific central memory population is depleted in progressor patients (16, 19). The presence of long-lived central memory T cells in Controllers allows the persistence of immunological memory, but does not appear sufficient to ensure HIV control. Indeed, patients treated early in the course of primary HIV infection also maintain central memory CD4+ T cells with strong proliferative capacity, but in most cases fail to control HIV replication upon treatment interruption (20). A contributing factor may be the preferential infection and depletion of HIV-specific CD4+ T cells upon viral rebound, as these cells activate upon HIV antigen recognition and become highly susceptible target cells for viral replication (5). How HIV-specific CD4+ T cells escape depletion in Controllers is not entirely understood, though some studies suggest a lower susceptibility of Controller CD4+ T cells to HIV replication (21). In addition, multiple lines of evidence indicate that the antiviral CD4 response in Controllers is qualitatively different from that of efficiently treated patients, and is not just a consequence of a very low viremia. In particular, Controller CD4+ T cells preferentially target Gag rather than Env epitopes, suggesting differences in the repertoire of specific CD4+ T cells (22). Controller CD4+ T cells are more polyfunctional, as indicated by the capacity to produce multiple cytokines and chemokines simultaneously upon antigenic stimulation (23). A key difference lies in the persistence of specific CD4+ T cells with an effector differentiation status in controlled HIV infection, even though the amount of HIV antigens available to drive these responses is minimal. We reported that Gag-specific CD4+ T cells in Controllers maintain a Th1 effector profile with IFN-γ production and degranulation capacity, while such effectors disappear in patients treated in the long term (24). Controller CD4+ T cells express very low levels of negative costimulatory molecules such as CTLA-4 and PD-1, compatible with preserved effector functions (25, 26). Taken together, these findings suggest that Th1 effectors escape depletion and retain optimal functions to sustain the antiviral response in Controlled HIV infection.

An explanation for the remarkable properties of HIV-specific CD4 responses in Controllers may lie in the nature of the TCRs that mediate these responses, which remains unexplored so far. The inventors previously identified a population of CD4+ T cells with a high antigen sensitivity for immunoprevalent Gag peptides in HIV Controllers, while this population was absent in treated patients (27). The sensitive detection of Gag antigens depended on the TCR expressed by Controller CD4+ T cells, as indicated by a high TCR avidity measured in MHC II tetramer dilution assays. These experiments revealed intrinsic differences in the set of TCRs expressed by Controller CD4+ T cells as compared to those of treated patients. High TCR avidity is a hallmark of viral control in several models of chronic viral infections (28), and has been associated with antiviral efficacy in HIV-specific CD8+ T cells (10, 14, 29). In contrast, there are little data available on the impact of TCR avidity on human CD4+ T cell antiviral responses. The inventors reasoned that the presence of high avidity TCRs may explain why Controllers maintained HIV-specific CD4+ T cells with an effector differentiation status in spite of their very low viremia, and they set to characterize these TCRs at the molecular and functional level. The study reported herein was focused on TCRs specific for the most immunoprevalent CD4 epitope in Gag, located at position 293-312 in the capsid major homology region (MHR). This epitope, designated Gag293, is exceptionally immunoprevalent, as it induces an ELISPOT response in close to half of HIV-1 infected patients irrespective of genetic background (22, 30), and gives a >70% response rate in Controllers of the ANRS CO21 CODEX cohort (27). The inventors could thus compare the repertoire of Gag293-specific clonotypes in patients of varied HLA backgrounds, who controlled HIV infection either naturally or pharmacologically, through antiretroviral therapy. Clonotypes corresponding to both the TCRα and TCRβ chains were analyzed, so that patient-derived TCRs could be engineered and functionally tested.

The CD4+ T cell response in HIV control appears to remain an important field of investigation, at the source of therapeutic achievements. In this respect, there is a need for effective therapeutic strategies, which would be applicable to human patients having HIV infection, especially, but not only, those under long-term antiretroviral therapy, in order to circumvent or alleviate the drawbacks associated with such a treatment.

The present invention emanates from experiments, whose conclusions are that particular public TCRs may confer high-avidity CD4+ T cell responses to HIV Controllers.

Therefore, the invention concerns a T-cell receptor (TCR) which is specific for the epitope located between positions 293-312 in the GAG protein of HIV-1 (Gag293 epitope herein), said TCR comprising an alpha chain and a beta chain, wherein the sequence of the alpha variable domain of the alpha chain comprises a gene product from the human TRAV24 gene and the sequence of the beta variable domain of the beta chain comprises a gene product from the human TRBV2 gene.

By "Gag293 epitope", it is meant the most immunoprevalent CD4 epitope in Gag, located at position 293-312 in the capsid major homology region (MHR) of the HIV-1, especially the epitope encoded by the nucleic acid sequence TTTAGAGACTATGTAGACCGGTTCTATAAAACTCTAAGAGCCGAGCAAGCTTCACAGGAG (SEQ ID NO: 54), corresponding to nucleotides 1212-1271 from HIV-1 HXB2 sequence with the Genebank accession number AF033819.

The nucleotide sequence encoding the Gag 293-312 epitope according to the present disclosure is variable, given HIV extensive genetic diversity. The skilled person in the art is however aware of these variations.

TCRs alpha and beta chains each consist of a variable domain and a constant domain. The variable domain is at the amino-terminal end of the TCR molecule. Within a TCR variable domain, there are six complementarity determining regions (CDRs) that mediate recognition with an epitope, i.e. three CDRs found in the sequence of the alpha variable domain of the alpha chain, and three CDRs found in the sequence of the beta variable domain of the beta chain. The CDR1 and CDR2 regions found in the variable regions are encoded by germline TRAV and TRBV gene segments.

The diversity of the TCR repertoire produced by an individual relies on two phenomena known as 1) combinatorial diversity and 2) junctional diversity.

Combinatorial diversity results from the many possible different permutations and combinations of variable (V - TRAV or TRBV), diversity (D - TRBD) and joining (J - TRAJ or TRBJ) gene segments that can be chosen amongst the numerous V, D and J gene segments available in the genome of an individual, which can be recruited when TCRs are generated by the human body.

Junctional diversity results from the further rearrangement, known as V(D)J recombination or V(D)J rearrangement, within the selected V and J gene segments, and also the selected D gene segment for the beta chain, during which germline-encoded nucleotide(s) may be deleted and random non-contemplated nucleotide(s) may be added.

The CDR3 regions on each variable chain of a TCR emanates from rearranged junction(s) of different V(D)J gene segments. The amino-acid sequence or corresponding nucleotide sequence of a CDR3 region (also termed CDR3 junction herein) is therefore particularly hypervariable, especially at the level of the rearranged junction(s), where junctional diversity events (rearrangement events or V(D)J recombination events) occurred.

A TCR of the invention, which is specific for the Gag293 epitope, therefore comprises three CDRs on its alpha variable domain and three CDRs on its variable beta domain, that are the result of a gene product based on the human TRAV24 and TRBV2 genes, wherein these genes are rearranged with TRAJ or TRBJ genes respectively for the constitution of the CDR3 domains.

By "gene product based on the human TRAV24 and TRBV2 genes", it is meant a protein or polypeptide the amino acid sequence of which is produced by the combinatorial selection of these particular gene segments, which defines the amino-acid sequence of the alpha and beta chains, respectively, including the CDR1 and CDR2 regions. This selection also further set the basis for the definition of the amino-acid sequence of the CDR3 region, which is then submitted to V(D)J recombination. According to a particular embodiment, the sequences of the alpha variable domain and the beta variable domains of a TCR of the invention comprise a rearranged gene product at the level of their respective CDR3 regions, which is at least partly based on the human TRAV24 and TRBV2 genes, respectively.

TRAV24 gene is located on chromosome 14 at 14q11.2.

TRBV2 gene is located on chromosome 7 at 7q34.

Part of the present disclosure, sequences for germline TRAV24 and TRBV2 genes are respectively disclosed under:

a. SEQ ID NO: 55 for the TRAV24-01 allelic sequence corresponding to the accession number AE000660 in the IMGT database at www.imgt.org

ATGGAGAAGAATCCTTTGGCAGCCCCATTACTAATCCTCTGGTTTCATCTTGACTGCGTGAG
CAGCATACTGAACGTGGAACAAAGTCCTCAGTCACTGCATGTTCAGGAGGGAGACAGCACC
AATTTCACCTGCAGCTTCCCTTCCAGCAATTTTTATGCCTTACACTGGTACAGATGGGAAACT
GCAAAAAGCCCCGAGGCCTTGTTTGTAATGACTTTAAATGGGGATGAAAAGAAGAAAGGAC
GAATAAGTGCCACTCTTAATACCAAGGAGGGTTACAGCTATTTGTACATCAAAGGATCCCAG
CCTGAAGACTCAGCCACATACCTCTGTGCCTTTA

b. SEQ ID NO: 56 for the TRBV2-01 allelic sequence corresponding to the accession number L36092 in the IMGT;

GAACCTGAAGTCACCCAGACTCCCAGCCATCAGGTCACACAGATGGGACAGGAAGTGATCT
TGCGCTGTGTCCCCATCTCTAATCACTTATACTTCTATTGGTACAGACAAATCTTGGGGCAGA
AAGTCGAGTTTCTGGTTTCCTTTTATAATAATGAAATCTCAGAGAAGTCTGAAATATTCGAT
GATCAATTCTCAGTTGAAAGGCCTGATGGATCAAATTTCACTCTGAAGATCCGGTCCACAAA
GCTGGAGGACTCAGCCATGTACTTCTGTGCCAGCAGTGAAGC

The present disclosure also encompasses other alleles which have been reported for these genes. For example one allelic variant has been reported for the TRAV24 gene: TRAV24-02, accession number M17661 in the IMGT database. Two allelic variants have been reported for the TRBV2 gene: TRBV2-02 (accession M62379) and TRBV2-03 (accession M64351) in the IMGT database. These allelic variants are also part of the present disclosure in order to define the TCR rearranged coding sequences.

According to a particular embodiment, the CDR1 and CDR2 amino-acid sequences of a TCR of the invention are those encoded by the corresponding coding regions of the TRAV24 and TRBV2 germline genes defined herein, or a variant thereof.

In the following part of the description, in the absence of a different definition or meaning, reference to a "gene" is equivalent to a reference to a "germline gene", when the context indicates that the CDR3 region of a TCR is not involved or considered. By definition, the sequence of the CDR3 region of a TCR (amino-acid sequence or nucleotide sequence) results from a rearrangement providing structural diversity with respect to the information directly derivable from germline genes.

The skilled person knows how to identify the amino-acid sequence (and corresponding encoding nucleotide sequence) of a CDR1 or CDR2 of a TCR. The nomenclature used herein is that of the IMGT database (www.imgt.org). In this respect, reference is made to Front Genet. 2012 May 23;3:79. doi: 10.3389/fgene.2012.00079. eCollection 2012.,

"IMGT-ONTOLOGY 2012", Giudicelli V, Lefranc MP which indicates that the numbering nomenclature used in the IMGT database provides a standardized delimitation of the complementarity determining regions (CDR), and therefore allows to correlate each position (amino-acid or codon) with the structure and the function of a variable domain of a TCR. Reference is also made to Cold Spring Harb Protoc. 2011 Jun 1;2011(6):633-42. doi: 10.1101/pdb.ip85. "IMGT unique numbering for the variable (V), constant (C), and groove (G) domains of IG, TR, MH, IgSF, and MhSF." Lefranc MP, which, in particular, discloses in Figure 2 an "IMGT Protein display for V domain" providing a delimitation of CDR1 and CDR2 domains with respect to the IMGT numbering. IMGT numbering allows the inclusion of gaps and additional positions. Using these references and possibly IMGT tools, the skilled in the art can therefore identify the amino-acid sequence (and corresponding encoding nucleotide sequence) of a CDR1 or CDR2 of a TCR upon visualization of a given sequence or sequences alignments.By "variant", it is meant a polypeptide resulting from limited variations in the sequence of the polypeptide of reference, variant polypeptides encompassing polypeptides having at least 80% identity with the sequence of reference. According to a particular embodiment, identity percentages reach 85%, 90% or more. In a particular embodiment, identity percentages are at least of 95%.

By "% identity" it is meant that result is obtained when sequences of amino-acids or nucleotides are compared over the entire length of the considered reference sequence through a local alignment algorithm, for example the Smith and Waterman algorithm, allowing amino-acids or nucleotides substitution(s), addition(s) or deletion(s). According to another particular embodiment, the identity percentage is calculated through a global alignment, for example the Needleman and Wunsch algorithm. In this case, the modifications of amino-acids or nucleotides are especially substitutions.

More specifically, the invention relates to a TCR which is specific for the epitope located between positions 293-312 in the GAG protein of HIV-1 having the amino-acid sequence FRDYVDRF[Y/F]KTLRAEQA[S/T]QE (SEQ ID NO: 1), comprising an alpha chain and a beta chain whose variable domains each comprise three complementarity determining regions (CDR1, CDR2, CDR3), wherein

a. the amino-acid sequence of the CDR1 and CDR2 on the alpha variable chain is encoded by the human TRAV24 gene, and

b. the amino-acid sequence of the CDR1 and CDR2 on the beta variable chain is encoded by the human TRBV2 gene, and

c. the TCR has high sensitivity for the epitope located between positions 293-312 of the GAG protein of HIV-1 as defined herein, in particular a Gag293 epitope that is presented by a MHC molecule, in particular a class II MHC molecule, as disclosed herein, that may be measured as the capability of CD4+ T cells which express the TCR, to proliferate and to differentiate into TNF-alpha and/or IFN-gamma secreting effectors upon stimulation with said epitope with half-maximal responses for TNF-alpha or IFN-gamma production (EC50) being achieved in particular with less than $10^{-7}$ M of Gag293 epitope concentration.

Alternatively the antigen sensitivity may be assessed in transduced J76 cells wherein $EC_{50}$ for the Gag293 peptide concentration able to induce half maximal CD69 is observed.

High antigen sensitivity may be assayed according to the protocols disclosed in the Examples and the EC50 may be in the range of the values disclosed in the Examples.

In a particular embodiment, antigen sensitivity of the TCR may be assessed in CD4+ T cell expressing an assayed TCR by determining the $EC_{50}$ value, e.g., measured by ICS. Accordingly the minimal Gag293 concentration required to achieve half-maximal expression of the assayed cytokine(s) provides the $EC_{50}$ value. According to a particular embodiment, the TCR of the invention has $EC_{50}$ value in the range of 10E-9 to 10E-8 M when measuring one of TNF-alpha, MIP-1beta and degranulation marker CD107a production and has $EC_{50}$ value in the range of 10E-8 to 10E-7 M when measuring IFN-gamma production and/or has intermediate $EC_{50}$ value when measuring IL-2 production.

In a particular embodiment, antigen affinity of a TCR of the invention may be considered to define such TCR in addition or alternatively to the sensitivity.

High affinity of the TCR for the epitope defined herein may be measured by a Kd value that is equal or less than 20 μM measured by SPR (Surface Plasmon Resonance) analysis.

In a particular embodiment, the affinity of the TCR for said a Gag293 epitope is equal or less than 19, 18, 17, 16, 15, 14, 13, 12, 11, 10, 9, 8, 7, 6, 5, 4, 3, 2 or 1 μM, more particularly in the ranges of: 0.5 to 7 μM, 0.5 to 4 μM, 0.5 to 3 μM or 0.5 to 1 μM, by SPR analysis. According to a particular embodiment, the affinity is less than 1 μM, in particular is 0.86 μM.

These values define Gag293-specific TCRs i.e., TCR which have high-affinity for the epitope, being observed that such affinity values are not commonly encountered for TCRs recognizing epitopes presented by class II MHC molecules.

The affinity can be defined by a $Kd_{eq}$ value (also designated Kd) and can be measured by methods conventional in the art, in particular methods reported in the experimental section below, especially by SPR analysis.

According to the nomenclature used herein, when reference is made to motif(s), the indication [Z1/Z2] means that either amino-acid identified as Z1 or amino-acid identified as Z2 can alternatively be found at that position in the sequence. Motif(s) cover(s) several possibilities of amino-acid sequences, according to all possible combinations of amino-acid residues encompassed by the alternatives suggested between brackets "[" and "]" over the whole length of the motif(s).

In a further aspect of the invention the TCR is specific for the epitope located between positions 293-312 in the GAG protein of HIV-1 having the amino-acid sequence FRDYVDRF[Y/F]KTLRAEQA[S/T]QE (SEQ ID NO: 1, comprising an alpha chain and a beta chain whose variable domains each comprise three complementarity determining regions (CDR1, CDR2, CDR3), wherein

a. the amino-acid sequence of the CDR1 and CDR2 on the alpha variable chain is encoded by the human TRAV24 gene, and
b. the amino-acid sequence of the CDR1 and CDR2 on the beta variable chain is encoded by the human TRBV2 gene, and
c. the amino-acid sequence of the CDR3 on the alpha chain comprises a motif selected amongst: [A/S]X[K/R]AAGNKLT (SEQ ID NO: 2) (motif AV24-1 herein), AXYGGATNKLI (SEQ ID NO: 3) (motif AV24-2), AX[R/N][R/N]AGNMLTF (SEQ ID NO: 4) (motif AV24-3), AXD[N/D]RKLI (SEQ ID NO: 5) (motif AV24-4) or AXE[S/G]X[G/A][A/S][Q/E]KLV (SEQ ID NO: 6) (motif AV24-5), X being any amino-acid, and/or
d. the amino-acid sequence of the CDR3 on the beta chain comprises a motif selected amongst: ASSX[R/G/L][T/A][S/G]GXX[E/D/T][Q/T][F/Y]) (SEQ ID NO: 7) (motif BV2-1),ASSX[R/G/L][T/A][S/G/A]GXX[E/D/T/P][Q/T][F/Y/H] (SEQ ID NO: 8) (motif BV2-2-b), ASSGXXNTEAF (SEQ ID NO: 9) (motif BV2-3) or ASVLMRT[N/R]NEQF (SEQ ID NO: 10) (motif BV2-4), X being any amino-acid.

According to a particular embodiment, the amino-acid sequence of the CDR3 on the alpha chain comprises either the motif [A/S]X[K/R]AAGNKLT (SEQ ID NO: 2) or AXYGGATNKLI (SEQ ID NO: 3).

According to a particular embodiment, the amino-acid sequence of the CDR3 on the beta chain comprises either the motif ASSX[R/G/L][T/A][S/G]GXX[E/D/T][Q/T][F/Y]) (SEQ ID NO: 7) or ASSX[R/G/L][T/A][S/G/A]GXX[E/D/T/P][Q/T][F/Y/H] (SEQ ID NO: 8)

According to a specific .embodiment, the CDR3 on the alpha chain comprises one of the above motifs of SEQ ID NO: 2 or SEQ ID NO: 3 and the CDR3 on the beta chain comprises one of the above motifs of SEQ ID NO: 7 or SEQ ID NO: 8.

According to a particular embodiment, the length of the amino-acid sequence of the CDR3 on the alpha chain of a TCR of the invention is from 9 and 16 amino-acid residues, in particular 12 amino-acid residues, and/or the length of the amino-acid sequence of the CDR3 on the beta chain of a TCR of the invention is from 11 and 18 amino-acid residues, in particular 15 amino-acid residues. CDR3 junctions lengths indicated herein are computed by including the conserved C104 and F/W118 residues in the length of the CDR3 junction, according to the numbering scheme of the International ImMunoGeneTics Information System (IMGT) (34), which excludes these two residues from the CDR3 length calculation. Indeed, according to the IMGT-ONTOLOGY unique numbering system, "CDR3 lengths" correspond to the number of amino-acids comprised between, but not including, the two conserved residues C104 and F/W118 defined by the IMGT-ONTOLOGY numbering system. In contrast, the "CDR3 junctions" referred to herein and the lengths of said CDR3 junctions provided herein include said conserved C104 and F/W118 residues. The IMGT-ONTOLOGY numbering system and "CDR3 lengths" calculation according to this system is however used in the experimental section.

When CDR3 is referred to in the present invention, the definition or feature may be adapted (in terms of amino acid residues) to read on CDR3 junction as an alternative unless technically irrelevant or unless specified otherwise.

The inventors have identified particular relevant motifs for the amino-acid sequence of the CDR3s of a TCR of the invention:

| Motifs TRAV24 | (SEQ ID NO: 2 to 6) |
|---|---|
| AV24-1 | [A/S]X[K/R]AAGNKLT |
| AV24-2 | AXYGGATNKLI |
| AV24-3 | AX[R/N][R/N]AGNMLTF |
| AV24-4 | AXD[N/D]RKLI |
| AV24-5 | AXE[S/G]X[G/A][A/S][Q/E]KLV |

| Motifs TRBV2 | (SEQ ID NO: 7 to 10) |
|---|---|
| BV2-1 | ASSX[R/G/L][T/A]SGGXX[E/D/T][Q/T][F/Y] |
| BV2-2-b | ASSX[R/G/L][T/A][S/G/A]GXX[E/D/T/P][Q/T][F/Y/H] |
| BV2-3 | ASSGXXNTEAF |
| BV2-4 | ASVLMRT[N/R]NEQF |

In a particular embodiment, the TCR of the invention is an isolated TCR. By "isolated" reference is made to a TCR that has been separated from at least some of the components with which it was associated when initially produced in nature or through a preparation process and/or produced and/or prepared through dedicated non-naturally occurring settings. The TCR can be a non-naturally occurring TCR and/or a recombinant and/or engineered TCR. In a particular embodiment, the TCR of the invention is modified with respect to a naturally occurring TCR so as to differ from the structure of the naturally occurring TCR while retaining the functional properties discussed herein.

In a particular embodiment, the TCR is a human TCR. In another particular embodiment, the TCR is a chimeric TCR having constant regions domains from another species than human, especially murine constant regions domains.

According to a particular embodiment, a TCR of the invention recognizes the Gag293 epitope defined herein when presented by a major histocompatibility complex (MHC) molecule. According to a more particular embodiment, such a MHC molecule is a MHC Class II molecule. MHC Class II molecules are normally found only on antigen-presenting cells such as dendritic cells, mononuclear phagocytes, some endothelial cells, thymic epithelial cells, and B cells.

According to a particular embodiment the Gag293 epitope is a peptide from the GAG protein of HIV-1 having the amino-acid sequence FRDYVDRF[Y/F]KTLRAEQA[S/T]QE (SEQ ID NO: 1), optionally presented by a MHC molecule as defined herein, in particular presented by a MHC-II molecule present on an Antigen Presenting Cell.

According to a particular embodiment, a TCR of the invention specifically recognizes a peptide from the GAG protein of HIV1 having the amino-acid sequence FRDYVDRF[Y/F]KTLRAEQA[S/T]QE (SEQ ID NO: 1) when presented by a major histocompatibility complex (MHC) molecule, in particular when said peptide is presented by a peptide-MHC II complex.

A TCR of the invention may recognize all or part of the Gag293 epitope defined herein.

According to a particular embodiment, the antigenic peptide of the Gag293 epitope as defined herein is recognized by a TCR of the invention when presented as a peptide-MHC II complex by antigen-presenting cells.

According to a particular embodiment, the MHC class II molecule is a HLA-DR molecule, especially a HLA-DR molecule selected from the group consisting of: HLA-DR11, HLA-DR15, HLA-DRB5, HLA-DR1, HLA-DR7, or any subcombination thereof. According to a particular embodiment, the MHC-II molecule is a HLA-DR11, HLA-DR15, HLA-DRB5 or HLA-DR1 molecule.

The invention also relates to an antigen-TCR complex formed by the interaction between a TCR of the invention as disclosed herein and peptide of the Gag293 epitope as disclosed herein, said antigenic peptide being presented by a MHC molecule, in particular a class II MHC molecule, as disclosed herein.

According to a particular embodiment, the invention relates to a TCR as defined herein, when presented on a cell, in particular on a primary T cell, such as a PBMC, with HLA-DR restriction, in particular with a restriction to at least one of HLA-DR11, HLA-DR15, HLA-DRB5, HLA-DR1 and/or HLA-DR7, or several of them, according to all combinations thereof, more particularly with a restriction to at least one of HLA-DR11, HLA-DR15, HLA-DRB5 or HLA-DR1 or several of them, according to all combinations thereof.

The specificity of the TRC for its epitope may be assessed by assaying the recognition of Gag293-loaded HLA-DR tetramers, as illustrated in the Examples in tetramer titration experiments. Such specificity may also be inferred from the binding affinity of the obtained TCR or from their sensitivity.

According to a particular embodiment, the TCRs of the invention are characterized as being functional TCRs, especially polyfunctional TCRs.

In a particular embodiment, these TCRs confer high antigen sensitivity to gag-specific CD4+ T cells which express them, as measured by the capacity of these cells to proliferate and to differentiate into TNF-alpha and/or IFN-gamma secreting effectors upon stimulation with half-maximal responses for TNF-alpha or IFN-gamma production (EC50) that is equal to less than $10^{-7}$ M of Gag293 peptide.

According to a particular embodiment, antigen sensitivity of cells may be assessed by monitoring the induction of the early activation marker CD69 by cells expressing the TCRs.

According to a particular embodiment TCR function according to the invention may be determined assessing markers production including at least one, preferably at least 2 or at least 3 of the following markers: cytokines such as TNF-alpha, IL-2, IFN-gamma, chemokines such as MIP-Ibeta/CCL4, degranulation marker such as CD107a. TCRs of the invention are in particular very active for the purpose of the invention when they are capable of eliciting the secretion of IFN-gamma in addition to TNF-alpha.

In a particular embodiment of the invention, the TCR is polyfunctional as measured by assaying induction of at least 3 cytokines, in particular 5 cytokines in the group of TNF-alpha, IL-2, INF-gamma, MIP-1beta and degranulation marker CD107a, and preferably cytokines including IFN-gamma and TNF-alpha by primary CD4+T cells expressing said TCR upon limiting antigenic stimulation using the Gag293 peptide. Alternatively induction of the activation marker CD69 in J76 cell lines may be used to assess functionality of the TCRs instead of CD4+ T cells.

If assessed *in vitro,* production of these markers by cells expressing the TCRs of the invention may involve intracellular cytokine staining (ICS) after stimulating the cells with Gag293 peptide.

In a particular embodiment, it has been observed that the TCRs of the invention induce a functional response and in particular a polyfunctional response when expressed on CD4+ T cells, especially CD4+ T cells obtained after transduction with vector particles disclosed herein. Interestingly some TCRs of the invention (such as F24 disclosed hereafter) may also be expressed on transduced CD8+ T cells (by transduction with vector particles as disclosed herein) and induce the secretion of the above cited markers thereby providing CD8+ T cells more effective against HIV replication.

As defined herein, a "public clonotype" corresponds to identical CDR3 amino-acid sequences found in at least two individuals analyzed during the study reported herein.

By extension, a "public clonotype" can also be defined as a corresponding nucleotide sequence encoding such a CDR3 amino-acid sequence, or a set of corresponding nucleotide sequences, given the degeneracy of the genetic code.

According to a particular embodiment, a TCR of the invention has variable domains of the alpha and beta chains each comprising three complementarity determining regions (CDRs), wherein:

a. the amino-acid sequence of the CDR3 on the alpha chain is or comprises a sequence as disclosed in any one of SEQ ID NO: 11 to 27 (as disclosed and individualized in Table 3A herein), and/or
b. the amino-acid sequence of the CDR3 on the beta chain is or comprises a sequence as disclosed in any one of SEQ ID NO: 28 to 46 (as disclosed and individualized in Table 3B herein), or
c. the amino-acid sequence of the CDR3 on the alpha and/or beta chain comprises a variant having at least 80 % amino-acids sequence identity with the sequences disclosed in a. and b. respectively,

the length of the amino-acid sequence of the CDR3 on the alpha chain being from 9 and 16 amino-acid residues, in particular 12 amino-acid residues, the length of the amino-acid sequence of the CDR3 on the beta chain being from 11 and 18 amino-acid residues, in particular 15 amino-acid residues.

Identity percentages for variants are calculated as disclosed above. The length of the amino-acid sequence of the CDR3 is calculated as indicated above.

According to a particular embodiment, a TCR of the invention has variable domains of the alpha and beta chains each comprising three complementarity determining regions (CDRs), wherein:

a. the amino-acid sequence of the CDR3 on the alpha chain is or comprises a sequence selected from : CAFKAAGNKLTF (SEQ ID NO: 47) (public clonotype TRAV24-F herein), CASKAAGNKLTF (SEQ ID NO: 48) (TRAV24-S), and CSRRAAGNKLTF (SEQ ID NO: 49) (TRAV24-RR), and/or
b. the amino-acid sequence of the CDR3 on the beta chain is or comprises a sequence selected from : CASSRLAGGMDEQF (SEQ ID NO: 50) (TRBV2-24), CATTPGASGISEQF (SEQ ID NO: 51) (TRBV2-25), CASSPGTSGVEQFF (SEQ ID NO: 52) (TRBV2-4), and CASSRRTSGGTDTQYF (SEQ ID NO: 53) (TRBV2-13), or
c. the amino-acid sequence of the CDR3 on the alpha and/or beta chain comprises a variant having at least 80 % amino-acids sequence identity with the sequences disclosed in a. and b. respectively,

wherein the length of the amino-acid sequence of the CDR3 on the alpha chain is from 9 and 16 amino-acid residues, in particular 12 amino-acid residues, the length of the amino-acid sequence of the CDR3 on the beta chain is from 11 and 18 amino-acid residues, in particular 15 amino-acid residues.

Identity percentages for variants are calculated as disclosed above. The length of the amino-acid sequence of the CDR3 is calculated as indicated above.

Other particular embodiments of a TCR of the invention are described with respect to the sequence of the alpha and beta chains of the TCR identified as TCR "F24" herein, as disclosed in Table S8, which have been recovered by the inventors upon sequencing of the nucleotide sequence (SEQ ID NO: 57) of the plasmid termed "pCDH-F24-TCR" herein (8241 nucleotides), disclosed in the experimental section and part of the present disclosure.

Translation in amino-acids of the nucleotide sequence found in this plasmid provides the following sequences for the expressed chains of the TCR:

- amino-acid sequence (SEQ ID NO: 58 - 274 aa) of the alpha chain of a TCR of the invention, translated from its encoding nucleic acid sequence:

**MEKNPLVAPLL**ILWFHLDCVSSILNVEQSPQSLHVQEGDSTNFTCSFP**SSNFYA**LHWYRWETAKSPEALFV***MTLNGD***EKKKGRISATLNTKEGYSYLYIKGSQPEDSATYL***CAFKAAGNKLTF***GGGTRVLVKP*NIQKPDPAVYQLRDSKSSDKSVCLFTDFDSQTNVSQSKDSDVYITDKTVLDMRSMDFKSNSAVAWSNKSDFACANAFNNSIIPADTFFPSPESSCDVKLVEKSFETDTNLNFQNLSVIGFRILLLKVAGFNLLMTLRLWSS*

- amino-acid sequence (SEQ ID NO: 59 - 313 aa) of the beta chain of a TCR of the invention, translated from its encoding nucleic acid sequence:

**MDTWLVCWAIFSLLKAGLT**EPEVTQTPSHQVTQMGQEVILRCVPI**SNHLY**FYWYRQILGQKVEFLVS***FYNNEI***SEKSEIFDDQFSVERPDGSNFTLKIRSTKLEDSAMYF***CASSRLAGGMDEQFF***GPGTRLTVL*EDLKNVFPPEVAVFEPSEAEISHTQKATLVCLATGFYPDHVELSWWVNGKEVHSGVSTDPQPLKEQPALNDSRYCLSSRLRVSATFWQNPRNHFRCQVQFYGLSENDEWTQDRAKPVTQIVSAEAWGRADCGFTSESYQQGVLSATILYEILLGKATLYAVLVSALVLMAMVKRKDSRG*

Legend for SEQ ID NO: 58 and SEQ ID NO: 59:

| Alpha chain domains | Beta chain domains |
|---|---|
| **Leader**<br>**CDR1**<br>***CDR2*** | |
| TRAV24 sequence is the sequence from amino acid 1 to the amino acid before the CDR3 junction | TRBV2 sequence is the sequence from amino acid 1 to the amino acid before the CDR3 junction |
| ***CDR3-JUNCTION*** | |
| from 3'TRAV24 and 3'-TRAJ17 | from 3'TRBV2 and 3'-TRBJ2-1 |
| *TRAC* | *$T_{RBC2}$* |

The positions of the corresponding regions of interest in SEQ ID NO: 58 and 59 are therefore:
- Alpha chain (SEQ ID NO: 58):

| Region | Start | End |
|---|---|---|
| TRAV24 | 1 | 111 |
| CDR1-IMGT | 49 | 54 |
| CDR2-IMGT | 72 | 77 |
| CDR3-long | 112 | 123 |
| 3'-TRAJ17 | 124 | 133 |
| TRAC | 134 | 274 |

- Beta chain (SEQ ID NO: 59):

| Region | Start | End |
|---|---|---|
| TRBV2 | 1 | 110 |

(continued)

| Region | Start | End |
|---|---|---|
| CDR1-IMGT | 46 | 50 |
| CDR2-IMGT | 68 | 73 |
| CDR3-long | 111 | 125 |
| 3'-TRBJ2-1 | 126 | 134 |
| TRBC2 | 135 | 313 |

A leader sequence is a sequence at the N-terminus of some eukaryotic proteins that determines their ultimate destination. Rearranged TCR genes contain a short leader sequence upstream of the joined VJ and VDJ sequences. As the corresponding nascent polypeptide enters the endoplasmic reticulum, the amino acid sequences encoded by the leader sequence are cleaved. Mature TCR chains do not encompass amino acids corresponding to a leader sequence.

According to a particular embodiment, a TCR of the invention has an amino acid sequence that is devoid of leader sequences.

Are also part of the present disclosure the amino acid sequences SEQ ID NO: 60 and SEQ ID NO: 61, which correspond respectively to the amino acid sequences SEQ ID NO: 58 and SEQ ID NO: 59 minus the amino acid sequences corresponding to the leader sequences discussed above.

Accordingly, according to a particular embodiment, a TCR of the invention has:

- the amino-acid sequence of the CDR1 and CDR2 on the alpha variable chain encoded by the human TRAV24 gene, in particular has an amino-acid sequence for the CDR1 alpha corresponding to the positions 49 to 54 in SEQ ID NO: 58 and has an amino-acid sequence for the CDR2alpha corresponding to the positions 72 to 77 in SEQ ID NO: 58 and/or
- the amino-acid sequence of the CDR1 and CDR2 on the beta variable chain encoded by the human TRBV2 gene, in particular has an amino-acid sequence for the CDR1 beta corresponding to the positions 46 to 50 in SEQ ID NO: 59 and has an amino-acid sequence for the CDR2beta corresponding to the positions 68 to 73 in SEQ ID NO: 59 and/or
- the amino-acid sequence of the CDR3 on the alpha chain that is: CAFKAAGNKLTF (SEQ ID NO: 11), and the amino-acid sequence of the CDR3 on the beta chain that is: CASSRLAGGMDEQFF (SEQ ID NO: 512), or
- the amino-acid sequence of the CDR3 on the alpha and/or beta chain comprises a variant having at least 80 % amino-acids sequence identity with the sequences disclosed above for the CDR3 found on the alpha and beta chains, respectively, the length of the amino-acid sequence of the CDR3 on the alpha chain being from 9 and 16 amino-acid residues, in particular 12 amino-acid residues, the length of the amino-acid sequence of the CDR3 on the beta chain being from 11 and 18 amino-acid residues, in particular 15 amino-acid residues.

Identity percentages for variants are calculated as disclosed above. The length of the amino-acid sequence of the CDR3 is calculated as indicated above.

According to a particular embodiment, a TCR of the invention has constant domains TRAC and/or TRBC2 as defined in the Table above.

According to a more particular embodiment, a TCR of the invention has:

- the amino-acid sequence of its alpha chain is as disclosed in SEQ ID NO: 58 or SEQ ID NO: 60, and/or
- the amino-acid sequence of its beta chain is as disclosed in SEQ ID NO: 59 or SEQ ID NO: 61, or
- the amino-acid sequence of its alpha and/or beta chain is a variant having at least 80 % amino-acids sequence identity with the sequences disclosed above.

Identity percentages for variants are calculated as disclosed above.

According to a particular embodiment, a TCR of the invention has alpha and/or beta chains and/or variable chains as disclosed herein, especially above, in which the amino-acid sequence of the CDR3 on the alpha and/or beta chains is replaced by any CDR3 junction sequence or motif as disclosed herein, or a variant thereof having at least 80 % amino-acids sequence identity according to the definitions provided herein, in which case the length of the amino-acid sequence of the variant CDR3 on the alpha chain is from 9 and 16 amino-acid residues, in particular 12 amino-acid residues, the length of the amino-acid sequence of the variant CDR3 on the beta chain is from 11 and 18 amino-acid residues, in particular 15 amino-acid residues.

According to a particular embodiment, the obtained TCR remains polyfunctional as disclosed herein, and/or retains an affinity for the epitope located between positions 293-312 of the GAG protein of HIV-1 as defined herein.

Identity percentages for variants are calculated as disclosed above. The length of the amino-acid sequence of the CDR3 is calculated as indicated above.

The skilled persons knows how to substitute an amino-acid sequence (and corresponding encoding nucleotide sequence) of a CDR3 of a TCR, or any part of a segment of a TCR as identified herein. Reference is made to the publications regarding the IMGT nomenclature disclosed above.

According to a particular embodiment, the TCR of the invention as disclosed with respect to its amino acid sequences is a human TCR. According to another particular embodiment the TCR of the invention as disclosed by its amino acid sequences is a chimeric TCR, in particular a human-murine TCR.

According to a particular embodiment, a TCR of the invention has constant domains encoded by the TRAC and TRBC genes for the alpha and beta chain respectively.

Part of the present disclosure, sequences for germline TRAC and TRBC genes are respectively disclosed under:

a. SEQ ID NO: 64 for the TRAC01 allelic sequence corresponding to the accession number X02883 in the IMGT database at www.imgt.org

NATATCCAGAACCCTGACCCTGCCGTGTACCAGCTGAGAGACTCTAAATCCAGTGACAAGTC
TGTCTGCCTATTCACCGATTTTGATTCTCAAACAAATGTGTCACAAAGTAAGGATTCTGATGT
GTATATCACAGACAAAACTGTGCTAGACATGAGGTCTATGGACTTCAAGAGCAACAGTGCT
GTGGCCTGGAGCAACAAATCTGACTTTGCATGTGCAAACGCCTTCAACAACAGCATTATTCC
AGAAGACACCTTCTTCCCCAGCCCAGAAAGTTCCTGTGATGTCAAGCTGGTCGAGAAAAGCT
TTGAAACAGATACGAACCTAAACTTTCAAAACCTGTCAGTGATTGGGTTCCGAATCCTCCTC
CTGAAAGTGGCCGGGTTTAATCTGCTCATGACGCTGCGGCTGTGGTCCAGC

b. SEQ ID NO: 65 for the TRBC02_2 allelic sequence corresponding to the accession number L36092 in the IMGT;

GAGGACCTGAAAAACGTGTTCCCACCCGAGGTCGCTGTGTTTGAGCCATCAGAAGCAGAGA
TCTCCCACACCCAAAAGGCCACACTGGTATGCCTGGCCACAGGCTTCTACCCCGACCACGTG
GAGCTGAGCTGGTGGGTGAATGGGAAGGAGGTGCACAGTGGGGTCAGCACAGACCCGCAG
CCCCTCAAGGAGCAGCCCGCCCTCAATGACTCCAGATACTGCCTGAGCAGCCGCCTGAGGG
TCTCGGCCACCTTCTGGCAGAACCCCCGCAACCACTTCCGCTGTCAAGTCCAGTTCTACGGG
CTCTCGGAGAATGACGAGTGGACCCAGGATAGGGCCAAACCCGTCACCCAGATCGTCAGCG
CCGAGGCCTGGGGTAGAGCAGACTGTGGCTTCACCTCCGAGTCTTACCAGCAAGGGGTCCT
GTCTGCCACCATCCTCTATGAGATCTTGCTAGGGAAGGCCACCTTGTATGCCGTGCTGGTCA
GTGCCCTCGTGCTGATGGCCATGGTCAAGAGAAAGGATTCCAGAGGC

The present disclosure also encompasses other alleles which have been reported for these genes or sequences varying in 1, 2, 3, 4 or 5 nucleotides positions. Slight variations in constant sequences do not affect or do not substantially affect TCR function.

According to a particular embodiment, a TCR of the invention is a recombinant TCR.

According to the invention such recombinant TCR may be obtained by recombination, especially by PCR cloning of the sequences defined herein, in particular by recombination of the sequences encoding the CDR1end CDR2 together with any of the CDR3 where the combination in the respective resulting alpha and beta chains and the assembly of the alpha and beta chains provides a functional, in particular TCR with high antigen affinity or sensitivity. The positions of the domains of interest of the TCR as disclosed herein, provide in particular the features to design a TCR pattern.

The invention further relates to a TCR having the features disclosed, which is an heterodimeric TCR. By "heterodimeric TCR", it is meant a TCR composed of two different disulfide-linked chains. Differently said, an heterodimeric TCR as defined herein consists of both an alpha chain and a beta chain, which are associated through disulphide bond(s) at the level of their respective constant domains. TCR constant domains comprise connecting sequences having at least one cysteine residue capable of engaging in disulfide bonds, enabling the formation of a link between the two chains and an heterodimeric structure.

The invention also relates to a chimeric TCR that is a single chain TCR (scTCR) comprising the variable domains of an alpha and a beta chain of the invention ($V\alpha$ and $V\beta$) as defined herein, which are connected through a linker (L), an alpha constant domain and/or a beta constant domain fused to their respective variable alpha or beta chain, according to the following scheme $V\alpha C\alpha$-L-$V\beta C\beta$.

The invention also relates to a chimeric TCR that is a soluble TCR, meaning a TCR that has lost its capacity to be membrane-bound. The constant domain of an alpha or beta chain of a TCR is encoded by the Trac and Trbc genes, respectively, typically include the following elements: a constant domain sequence $C\alpha$ or $C\beta$, a connection sequence (H), a transmembrane region (Tm), and a cytoplasmic tail (CT). A soluble TCR lacks at least the transmembrane region (Tm) and the cytoplasmic tail (CT) region. A soluble TCR may be an heterodimeric TCR or a scTCR, provided it has lost its capacity to be membrane-bound. According to a particular embodiment, the chains of an heterodimeric soluble TCR are further engineered to add additional disulphide bond(s) between the two chains forming the heterodimeric structure.

The invention also relates to a chimeric TCR that is a single chain TCR fragment (scTv) consisting of the variable domains of an alpha and a beta chain of the invention ($V\alpha$ and $V\beta$) as defined herein, which are connected through a linker (L), according to any one of the following schemes: $V\alpha$-L-$V\beta$ or $V\beta$-L-$V\alpha$. Being devoid of at least a transmembrane region, a scTv is a soluble TCR according to the definitions provided herein. A scTv does not comprise TCR constant domains.

The chimeric TCR of the invention include the features of the alpha and/or beta TCR chains, or fragments thereof, disclosed herein. In particular, a chimeric TCR comprises a variable alpha chain and/or a variable beta chain region of the invention as defined herein.

The invention also relates to a chimeric and/or further engineered TCR, such as a TCR that has undergone murinization its constant region(s), i.e.,a TCR having murine constant regions domain(s) instead of human constant regions domain(s).

The invention also relates to a chimeric and/or further engineered TCR, such as a TCR that has undergone cysteine(s) modification(s) of its TCR chains, especially at the level of its constant regions, i.e., a TCR, the chains of which have been modified to have more cysteine residues than the non-engineered TCR counterpart, that are capable of engaging in additional disulfide bonds linking the alpha and beta chains of, in particular, an heterodimeric structure

The invention also encompasses a TCR as disclosed herein, or a fragment thereof, which is associated, optionally through a linker, with a detectable group such as a fluorescent group, a radiolabelled group, an absorbing group, or an enzyme with properties that generate detectable products.

By "rearranged gene product", it is meant an amino-acid sequence resulting from combinatorial diversity events and junctional diversity events applied to the nucleotide sequence of germline gene segments. The amino-acid sequence of a "rearranged gene product" is therefore defined by the amino-acid sequence produced by the combinatorial selection of particular gene segments, to which junctional diversity is added by V(D)J recombination in the **junctional sequence** comprised by the CDR3 region.

According to a particular embodiment, a TCR of the invention has:

a. an amino-acid sequence of the CDR3 on the alpha chain comprising a **rearranged gene product** obtained upon rearrangement of the human TRAV24 gene with a human TRAJ gene selected from : TRAJ17, TRAJ39, TRAJ32, TRAJ38, TRAJ54 or TRAJ57, and/or

b. the amino-acid sequence of the CDR3 on the beta chain comprises a **rearranged gene product** obtained upon rearrangement of the human TRBV2 gene with a human TRBJ gene selected from : TRBJ2-1, TRBJJ1-2, TRBJJ1-1, TRBJ1-5, TRBJ2-3, TRBJ2-7, and with a human TRBD gene selected from : TRBD1 or TRBD2.

Further to the rearrangement, some nucleotides of the TRAJ or the TRB germline genes may be present in the nucleic acid encoding the resulting alpha, respectively beta chains of the TCR, corresponding to amino residues found at the C-terminal end of the CDR3 domains.

Part of the present disclosure, sequences for germline TRAJ17, TRAJ39, TRAJ32, TRAJ38, TRAJ54, TRAJ57 TRBJ2-1, TRBJ1-2, TRBJ1-1, TRBJ1-5, TRBJ2-3, TRBJ2-7, TRBD1 and TRBD2 are respectively disclosed under:

a. SEQ ID NO: 66 for the TRAJ17-01 allelic sequence corresponding to the accession number X05773 in the IMGT database at www.imgt.org

TGATCAAAGCTGCAGGCAACAAGCTAACTTTTGGAGGAGGAACCAGGGTGCTAGTTAAACC
AA

b. SEQ ID NO: 67 for the TRAJ32-01 allelic sequence corresponding to the accession number M94081 in the IMGT;

TGAATTATGGCGGTGCTACAAACAAGCTCATCTTTGGAACTGGCACTCTGCTTGCTGTCCAGCCAA

c. SEQ ID NO: 68 for the TRAJ39-01 allelic sequence corresponding to the accession number M94081 in the IMGT;

TGAATAATAATGCAGGCAACATGCTCACCTTTGGAGGGGGAACAAGGTTAATGGTCAAACCCC

d. SEQ ID NO: 69 for the TRAJ54-01 allelic sequence corresponding to the accession number M94081 in the IMGT;

TAATTCAGGGAGCCCAGAAGCTGGTATTTGGCCAAGGAACCAGGCTGACTATCAACCCAA

e. SEQ ID NO: 70 for the TRAJ57-01 allelic sequence corresponding to the accession number M94081 in the IMGT;

TAACTCAGGGCGGATCTGAAAAGCTGGTCTTTGGAAAGGGAACGAAACTGACAGTAAACCCAT

f. SEQ ID NO: 71 for the TRBJ1-1-01allelic sequence corresponding to the accession number K02545 in the IMGT;

TGAACACTGAAGCTTTCTTTGGACAAGGCACCAGACTCACAGTTGTAG

g. SEQ ID NO: 72 for the TRBJ1-2-01 allelic sequence corresponding to the accession number K02545 in the IMGT;

CTAACTATGGCTACACCTTCGGTTCGGGGACCAGGTTAACCGTTGTAG

h. SEQ ID NO: 73.for the TRBJ1-5-01 allelic sequence corresponding to the accession number M14158 in the IMGT;

TAGCAATCAGCCCCAGCATTTTGGTGATGGGACTCGACTCTCCATCCTAG

i. SEQ ID NO: 74 for the TRBJ2-1-01 allelic sequence corresponding to the accession number X02987 in the IMGT;

CTCCTACAATGAGCAGTTCTTCGGGCCAGGGACACGGCTCACCGTGCTAG

j. SEQ ID NO: 75 for the TRBJ2-3-01 allelic sequence corresponding to the accession number X02987 in the IMGT;

AGCACAGATACGCAGTATTTTGGCCCAGGCACCCGGCTGACAGTGCTCG

k. SEQ ID NO: 76 for the TRBJ2-7-01 allelic sequence corresponding to the accession number M14159 in the IMGT;

CTCCTACGAGCAGTACTTCGGGCCGGGCACCAGGCTCACGGTCACAG

l. SEQ ID NO: 77 for the TRBD1-01 allelic sequence corresponding to the accession number K02545 in the IMGT;

GGGACAGGGGGC

m. SEQ ID NO: 78 for the TRBD2-01allelic sequence corresponding to the accession number X02987 in the IMGT;

GGGACTAGCGGGGGGG

The present disclosure also encompasses other alleles which have been reported for these genes.

Accordingly, the invention also relates to a method for identifying a TCR specific for the epitope located between positions 293-312 of the GAG protein of HIV-1 as defined herein, comprising the steps of:

a. Selecting a cell bearing TCRs specific for the epitope located between positions 293-312 of the GAG protein of HIV-1 as defined herein, and

b. Sequencing the full length or part of the alpha and beta chains of the TCRs of the selected cells, and

c. Expressing TCRs having the sequence determined in step b. in cells, and

d. Measuring an EC50 value for TNF-alpha or IFN-gamma production by CD4+ T cells expressing the said TCR that is equal to less than $10^{-7}$ M of Gag293 peptide, or measuring an EC50 value for TNF-alpha or IFN-gamma production by J76 cells expressing the said TCR that is equal to less than $10^{-6}$ M of Gag293 peptide.

According to a particular embodiment, a method for identifying a TCR specific for the epitope located between positions 293-312 of the GAG protein of HIV-1 as defined herein, comprising the steps of

a. Selecting a cell bearing TCRs specific for the epitope located between positions 293-312 of the GAG protein of HIV-1 as defined herein, and

b. Sequencing the full length or part of the alpha and beta chains of the TCRs of the selected cells, and

c. Producing soluble TCRs having the sequence determined in step b. in cells, and

d. Measuring a Kd value for the soluble TCRs and identifying hit TCR(s) displaying a Kd value that is equal or less than 20 μM, in particular equal or less than 19, 18, 17, 16, 15, 14, 13, 12, 11, 10, 9, 8, 7, 6, 5, 4, 3, 2 or 1 μM, more particularly between the ranges of: 0.5 to 7 μM, 0.5 to 4 μM, 0.5 to 3 μM or 0.5 to 1 μM.

According to a particular embodiment, TCR(s) expressed or produced in steps c) above have variable domains of the alpha and beta chains each comprising three complementarity determining regions (CDRs), in which:

i. the amino-acid sequence of the CDR3 on the alpha chain comprises a **rearranged gene product** obtained upon rearrangement of the human TRAV24 gene with a human TRAJ gene selected from : TRAJ17, TRAJ39, TRAJ32, TRAJ38, TRAJ54 or TRAJ57, and/or

ii. the amino-acid sequence of the CDR3 on the beta chain comprises a rearranged gene product obtained upon rearrangement of the human TRBV2 gene with a human TRBJ gene selected from : TRBJ2-1, TRBJJ1-2, TRBJJ1-1, TRBJ1-5, TRBJ2-3, TRBJ2-7, and with a human TRBD gene selected from : TRBD1 or TRBD2.

Cell selection in steps a) above can be performed according to conventional methods in the art. The experimental section provides an example of sorting of Gag293-specific CD4+ T cells with HLA-DR tetramers.

The invention also relates to a nucleic acid molecule encoding at least one chain of TCR as defined herein, or a fragment thereof, or both alpha and beta chains. The nucleic acid molecule of the invention may encode several TCRs as defined herein, or fragments thereof.

Such a nucleic acid molecule may be obtained by cloning or by synthesis, optionally including steps of recombination of various nucleic acid segments in accordance with well-known methods for the skilled person.

A nucleic acid molecule of the invention may encode the full length of either the alpha chain or the beta chain of a TCR as defined herein, or both chains, or fragment(s) thereof, especially fragments covering the variable domains of said chains. A nucleic acid molecule of the invention may encode the sequence of the alpha variable domain of the alpha chain and/or the sequence of the beta variable domain of the beta chain of a TCR as defined herein, or fragment(s) thereof. A nucleic acid molecule of the invention may comprise a nucleic acid sequence encoding a constant domain. The nucleotide sequence for a constant domain of a TCR, as comprised in TRAC and TRBC genes for respectively the alpha chain and the beta chain, typically include the following elements: a constant domain nucleotide sequence Cα, a nucleotide sequence coding for a connection sequence (H), a nucleotide sequence coding for a transmembrane region (Tm), and a nucleotide sequence coding for a cytoplasmic tail (CT). A nucleic acid molecule encoding the full length of either the alpha chain or the beta chain of a TCR also generally comprises a leader exon nucleotide sequence (L).

According to a particular embodiment, a nucleic acid molecule of the invention encodes at least the amino-acid sequence of a CDR3 domain as defined herein, or a part thereof, or all or part of the variable domain of an alpha and/or a beta chain, comprising all of the three CDRs (CDR1, CDR2, CDR3).

According to a particular embodiment, a nucleic acid molecule of the invention encodes the alpha chain of a TCR as defined herein, or a fragment thereof, and especially comprises the nucleotide sequence of a public clonotype as disclosed in any one of SEQ ID NO: 79 to 120 (as disclosed and individualized in Table 3A herein) encoding the CDR3 region of the alpha chain, or a sequence having at least 80 % nucleotide sequence identity with those sequences, identity being as defined herein.

According to a particular embodiment, a nucleic acid molecule of the invention encodes the beta chain of a TCR as defined herein, or a fragment thereof and especially comprises the nucleotide sequence of a public clonotype as disclosed in any one of SEQ ID NO: 121 to 161 (as disclosed and individualized in Table 3B herein) encoding the CDR3 region of the beta chain, or a sequence having at least 80 % nucleotide sequence identity with those sequences, identity being as defined herein.

According to a more particular embodiment, such nucleic acid molecules comprise a sequence encoding at least a part of a constant domain, as defined herein.

According to a particular embodiment, the nucleic acid molecule encoding a TCR as defined herein, or fragment thereof, comprises the nucleic acid sequence of both the alpha chain and the beta chain of a TCR as defined herein, or fragment thereof.

The invention thus also relates to a nucleic acid molecule encoding the full length of an alpha chain or the variable domain of the alpha chain of a TCR at least partly encoded by the human TRAV24 gene, as obtained after (i) amplification performed on cDNA obtained from RNA transcripts of cells expressing TCRs of the invention, said amplification, especially PCR amplification, being carried out according to conventional methods accessible to the skilled person with:

a. a forward primer containing the TRAV24 leader sequence with an NheI restriction site and a Kosak sequence added in 5': 5'-CGG CTA GCC GCC ACC ATG GAG AAG AAT CCT TTG GCA GCC-3' (SEQ ID NO: 162), and
b. a reverse primer containing the 3' of TRAC and a NotI site :5'-TTA GCG GCC GCG CTG GAC CAC AGC CGC AGC G-3' (SEQ ID NO: 163),

(ii) recovery of amplified DNA and possibly cloning the recovered DNA.

The invention also relates to a nucleic acid molecule encoding the full length of a beta chain or the variable domain of the beta chain of a TCR at least partly encoded by the human TRBV2 gene, as obtained after (i) amplification performed on cDNA obtained from RNA transcripts of cells expressing TCRs of the invention, said amplification, especially PCR amplification, being carried out with:

a. a forward primer containing the TRBV2 leader sequence and a BspEI site in 5': 5'- GGT CCG GAA TGG ATA CCT GGC TCG TAT GCT GGG C-3' (SEQ ID NO: 164), and
b. a reverse primer containing the 3' of TRBC and a SalI site: 5'- CCG GTC GAC CTA GCC TCT GGA ATC CTT TCT CTT GAC C-3' (SEQ ID NO: 165),

(ii) recovery of amplified DNA and possibly cloning the recovered DNA.

Alternatively, the primers used for amplification of the full length of an alpha chain or beta chain or their variable domain(s) of a TCR at least partly encoded by the human TRAV24 or TRBV2 genes respectively, can be chosen amongst:

a. TRAV24 forward primer: 5'-CCG AGG CCT TGT TTG TAA TG-3' (SEQ ID NO: 166);
b. TRAC reverse primer: 5'GTG AAT AGG CAG ACA GAC TTG T-3' (SEQ ID NO: 167);
c. TRBV2 forward primer: 5'-GGT CCG GAA TGG ATA CCT GGC TCG TAT GCT GGG C-3' (SEQ ID NO: 164);
d. TRBC reverse primer: 5'-CCG GTC GAC CTA GCC TCT GGA ATC CTT TCT CTT GAC C-3' (SEQ ID NO: 165).

These primers do in particular not encompass a Kosak sequence. The skilled person readily knows how to define suitable primers aimed at amplifying nucleotide target sequences of interest.

The invention also relates to a nucleic acid molecule encoding at least a part of the alpha chain of a TCR and/or at least a part of the beta chain of a TCR, in which:

a. the nucleic acid molecule encoding at least a part of the alpha chain of a TCR, especially the variable domain of the alpha chain, comprises at least the nucleotide sequence of the human TRAV24 gene coding the CDR1 and the CDR2 of a TCR and further comprises the junctional rearranged nucleotide sequence disclosed any one of SEQ ID NO: 79 to 120 (as disclosed and individualized in Table 3A herein) or a variant thereof having at least 80 % sequence identity with these sequences, and/or
b. the nucleic acid molecule encoding at least a part of the beta chain of a TCR, especially the variable domain of the beta chain, comprises at least the nucleotide sequence of the human TRBV2 gene coding the CDR1 and the CDR2 of a TCR and further comprises the junctional rearranged nucleotide sequence disclosed any one of SEQ ID NO: 121 to 161 (as disclosed and individualized in Table 3B herein) or a variant thereof having at least 80 % sequence identity with these sequences.

As an example, Figure 3G illustrates a junctional rearranged nucleotide sequence corresponding to the CDR3 junction of the most prevalent clonotype CAFKAAGNKLTF (SEQ ID NO: 11) obtained by rearrangements that occurred between the 3' end of the TRAV24*01 germline sequence and TRAJ17*01 germline sequence, said rearrangements comprising mutations (P and/or N), i.e., random non-contemplated nucleotide(s) addition(s), and/or trimmed nucleotides (trim), i.e., germline-encoded nucleotide(s) deletion(s).

Differently said, according to the particular embodiment defined in the paragraphs above, a nucleic acid molecule of the invention encodes at least a part of either an alpha chain or a beta chain or their variable domain(s) of a TCR, which correspond to gene products based on the human TRAV24 and TRBV2 genes when rearranged with TRAJ and TRBJ genes, said nucleic acid molecule matching one of the clonotypes defined by an amino-acid or nucleotide sequence as set forth in Table 3A or 3B, respectively, or a variant thereof having at least 80 % sequence identity with one of the nucleotide sequences set forth in Table 3A or 3B, respectively.

The length and/or boundaries of a junctional rearranged nucleotide sequence corresponding to a CDR3 junction may also be defined by correspondence with the length of an amino-acid sequence of a CDR3 as defined above, when they are defined and computed according to the numbering scheme of the International ImMunoGeneTics Information System (IMGT) (34).

Accordingly, according to a particular embodiment, a junctional rearranged nucleotide sequence corresponding to a CDR3 junction, which therefore encodes the CDR3 of a TCR, has a length from 25 to 60 nucleotides, more particularly from 27 to 54 nucleotides.

According to particular embodiments, rearranged nucleotide junction(s) of a CDR3 (junctional rearranged nucleotide sequence above) may result from:

- addition of 0 to 10 random non-contemplated nucleotide(s), and/or
- deletion of 0 to 15 germline-encoded nucleotide(s).

Other particular embodiments of nucleic acid molecule encoding at least a part of the alpha chain of a TCR and/or at least a part of the beta chain of a TCR of the invention are described with respect to the sequence of the alpha and beta chains of the TCR identified as TCR "F24" herein, as disclosed in Table S8, which have been recovered by the inventors upon sequencing of the nucleotide sequence (SEQ ID NO: 57) of the plasmid termed "pCDH-F24-TCR" herein (8241 nucleotides), disclosed in the experimental section and part of the present disclosure.

The nucleotide sequence of this plasmid provides the following sequences:

- nucleotide sequence (SEQ ID NO: 62 - 822 nt) of the alpha chain of a TCR of the invention:

ATGGAGAAGAATCCTTTGGTAGCCCCATTACTAATCCTCTGGTTTCATCTTGACTGCGTGAG
CAGCATACTGAACGTGGAACAAAGTCCTCAGTCACTGCATGTTCAGGAGGGAGACAGCACC
AATTTCACCTGCAGCTTCCCTTCCAGCAATTTTTATGCCTTACACTGGTACAGATGGGAAACT
GCAAAAAGCCCCGAGGCCTTGTTTGTAATGACTTTAAATGGGGATGAAAAGAAGAAAGGAC
GAATAAGTGCCACTCTTAATACCAAGGAGGGTTACAGCTATTTGTACATCAAAGGATCCCAG
CCTGAAGACTCAGCCACATACCTCTGTGCCTTTAAAGCTGCAGGCAACAAGCTAACTTTTGG
AGGAGGAACCAGGGTGCTAGTTAAACCAAATATCCAGAAGCCTGACCCTGCCGTGTACCAG
CTGAGAGACTCTAAATCCAGTGACAAGTCTGTCTGCCTATTCACCGATTTTGATTCTCAAAC
AAATGTGTCACAAAGTAAGGATTCTGATGTGTATATCACAGACAAAACTGTGCTAGACATG
AGGTCTATGGACTTCAAGAGCAACAGTGCTGTGGCCTGGAGCAACAAATCTGACTTTGCATG
TGCAAACGCCTTCAACAACAGCATTATTCCAGCAGACACCTTCTTCCCCAGCCCAGAAAGTT
CCTGTGATGTCAAGCTGGTCGAGAAAAGCTTTGAAACAGATACGAACCTAAACTTTCAAAA
CCTGTCAGTGATTGGGTTCCGAATCCTCCTCCTGAAAGTGGCCGGGTTTAATCTGCTCATGA
CGCTGCGGCTGTGGTCCAGC

SEQ ID NO: 62 can be found from positions 2497 to 3318 of the "pCDH-F24-TCR" sequence provided under SEQ ID NO: 57.

- nucleotide sequence (SEQ ID NO: 63 - 939 nt) of the beta chain of a TCR of the invention:

ATGGATACCTGGCTCGTATGCTGGGCAATTTTTAGTCTCTTGAAAGCAGGACTCACAGAACC
TGAAGTCACCCAGACTCCCAGCCATCAGGTCACACAGATGGGACAGGAAGTGATCTTGCGC
TGTGTCCCCATCTCTAATCACTTATACTTCTATTGGTACAGACAAATCTTGGGGCAGAAAGT
CGAGTTTCTGGTTTCCTTTTATAATAATGAAATCTCAGAGAAGTCTGAAATATTCGATGATC
AATTCTCAGTTGAAAGGCCTGATGGATCAAATTTCACTCTGAAGATCCGGTCCACAAAGCTG
GAGGACTCAGCCATGTACTTCTGTGCCAGCAGCCGACTAGCGGGAGGGATGGATGAGCAGT
TCTTCGGGCCAGGGACACGGCTCACCGTGCTAGAGGACCTGAAAAACGTGTTCCCACCCGA
GGTCGCTGTGTTTGAGCCATCAGAAGCAGAGATCTCCCACACCCAAAAGGCCACACTGGTG
TGCCTGGCCACAGGCTTCTACCCCGACCACGTGGAGCTGAGCTGGTGGGTGAATGGGAAGG
AGGTGCACAGTGGGGTCAGCACAGACCCGCAGCCCCTCAAGGAGCAGCCCGCCCTCAATGA
CTCCAGATACTGCCTGAGCAGCCGCCTGAGGGTCTCGGCCACCTTCTGGCAGAACCCCCGCA
ACCACTTCCGCTGTCAAGTCCAGTTCTACGGGCTCTCGGAGAATGACGAGTGGACCCAGGAT
AGGGCCAAACCTGTCACCCAGATCGTCAGCGCCGAGGCCTGGGGTAGAGCAGACTGTGGCT
TCACCTCCGAGTCTTACCAGCAAGGGGTCCTGTCTGCCACCATCCTCTATGAGATCTTGCTA
GGGAAGGCCACCTTGTATGCCGTGCTGGTCAGTGCCCTCGTGCTGATGGCCATGGTCAAGAG
AAAGGATTCCAGAGGC

SEQ ID NO: 63 can be found from positions 3388 to 4326 of the "pCDH-F24-TCR" sequence provided under SEQ ID NO: 57.

The positions of the corresponding regions of interest in SEQ ID NO: 62 and 63 are therefore:
- Alpha chain (SEQ ID NO: 62):

| Region | Start | End |
|---|---|---|
| TRAV24 | 1 | 333 |
| CDR1-IMGT | 145 | 162 |
| CDR2-IMGT | 214 | 231 |
| CDR3-JUNCTION | 334 | 369 |
| 3'-TRAJ17 | 370 | 399 |
| TRAC | 400 | 822 |

- Beta chain (SEQ ID NO: 63):

| Region | Start | End |
|---|---|---|
| TRBV2 | 1 | 330 |
| CDR1-IMGT | 136 | 150 |
| CDR2-IMGT | 202 | 219 |
| CDR3-JUNCTION | 331 | 375 |
| 3'-TRBJ2-1 | 376 | 402 |
| TRBC2 | 403 | 939 |

Accordingly, according to a particular embodiment, a nucleic acid molecule of the invention encodes at least a part of the alpha chain of a TCR, especially the variable domain of the alpha chain, and comprises:

a. at least the nucleotide sequence of the human TRAV24 gene coding the CDR1 and the CDR2 of a TCR, in particular has the nucleotide sequence for the CDR1 alpha corresponding to the positions 145 to 162 in SEQ ID NO: 62 and has the nucleotide sequence for the CDR2alpha corresponding to the positions 214 to 231 in SEQ ID NO: 62 and

b. a junctional rearranged nucleotide sequence (CDR3 junction) corresponding to the positions 334 to 369 in SEQ ID NO: 62, or a variant having at least 80 % nucleotide sequence identity thereof, the length of the nucleotide sequence of the CDR3 junction on the alpha chain being from 25 to 60 nucleotides, more particularly from 27 to 54 nucleotides.

According to a particular embodiment, a nucleic acid molecule of the invention encodes at least a part of the beta chain of a TCR, especially the variable domain of the beta chain, and comprises:

a. at least the nucleotide sequence of the human TRBV2 gene coding the CDR1 and the CDR2 of a TCR, in particular has the nucleotide sequence for the CDR1beta corresponding to the positions 136 to 150 in SEQ ID NO: 63 and has the nucleotide sequence for the CDR2beta corresponding to the positions 202 to 219 in SEQ ID NO: 63 and

b. a junctional rearranged nucleotide sequence (CDR3 junction) corresponding to the positions 331 to 375 in SEQ ID NO: 63, or a variant having at least 80 % nucleotide sequence identity thereof, the length of the nucleotide sequence of the CDR3 junction on the alpha chain being from 25 to 60 nucleotides, more particularly from 27 to 54 nucleotides.

According to a particular embodiment, a nucleic acid molecule of the invention encompasses at least a constant domain TRAC and/or TRBC2 as defined in the Table above, or a part thereof.

According to a particular embodiment, a nucleic acid molecule of the invention encodes at least a part of the alpha chain of a TCR, especially the variable domain of the alpha chain, and at least a part of the beta chain of a TCR, especially the variable domain of the beta chain, as defined herein.

According to a more particular embodiment, a nucleic acid molecule of the invention comprises or consists of the sequence disclosed in SEQ ID NO: 62 from position 1 to 399, and/or comprises or consists of the sequence disclosed in SEQ ID NO: 63 from position 1 to 402, or comprises or consists of a variant thereof having at least 80 % nucleotide sequence identity with the sequences.

According to a more particular embodiment, a nucleic acid molecule of the invention comprises or consists of the sequence disclosed in SEQ ID NO: 62, and/or comprises or consists of the sequence disclosed in SEQ ID NO: 63, or variant thereof having at least 80 % nucleotide sequence identity with the sequences.

According to a particular embodiment however, a nucleic acid molecule of the invention is devoid of a leader sequence, and its size and boundaries are adapted accordingly.

According to a particular embodiment, a nucleic acid molecule of the invention comprises or consists of a sequence encoding alpha and/or beta chains and/or the variable parts of these chains as disclosed herein, especially above, in which the nucleotide sequence of the CDR3 on the alpha and/or beta chains is replaced by any nucleotide CDR3 junction sequence as disclosed herein, or a variant thereof having at least 80 % nucleotide sequence identity according to the definitions provided herein, in which case the length of the nucleotide sequence of the CDR3 junction is from 25 to 60 nucleotides, more particularly from 27 to 54 nucleotides.

The skilled person knows how to substitute a nucleotide sequence encoding for the CDR3 of a TCR, or any part of a nucleotide sequence encoding for a segment of a TCR as identified herein, especially in the Tables above. Reference is made to the publications regarding the IMGT nomenclature disclosed above.

Identity percentages for variants are calculated as disclosed above.

According to a particular embodiment, a nucleic acid molecule as defined herein comprises or further comprises a nucleotide sequence encoding for a constant region of an alpha and/or beta chain, as defined herein.

According to a particular embodiment, a nucleic acid molecule of the invention as defined herein is a fragment of a nucleic acid molecule of the invention, especially a fragment encoding the variable domain of an alpha and/or beta chain of a TCR, in particular at least encoding the CDR1, CDR2 and CDR3 regions on said chains, optionally with other nucleic acid sequences of interest.

The invention also relates to a nucleic acid molecule encoding at least a part of the full length of the alpha chain or the variable domain of the alpha chain of a TCR as defined herein, and further encoding a part of the full length of the beta chain or the variable domain of the beta chain of a TCR as defined herein.

According to a particular embodiment, a nucleic acid molecule of the invention is a variant nucleic acid molecule, or a chimeric acid molecule.

Variants of the nucleic acid molecules defined herein may result from genetic code degeneracy. Definition for a "variant" based on percentage identity with respect to a reference sequence is provided above. Variants of the nucleic acid molecules of the invention may also encompass nucleic acid molecules encoding and/or chimeric engineered TCRs of the invention as disclosed herein.

According to a particular embodiment, the variants generally defined herein with respect to the nucleotide sequences, or the nucleic acid molecules defined herein retain the capacity to encode all or part of a an alpha and/or beta chain of a TCR that retain the functional capacity to recognize a Gag293 epitope as defined herein, when comprising the appropriate alpha and beta chains, in particular with the affinity defined herein.

According to a particular embodiment, a nucleic acid molecule of the invention is an isolated nucleic acid molecule. The nucleic acid molecule of the invention can be a non-naturally occurring nucleic acid molecule and/or a recombinant and/or engineered nucleic acid molecule. In a particular embodiment, the nucleic acid molecule of the invention is a human nucleic acid molecule. In another particular embodiment, the nucleic acid molecule of the invention is a chimeric nucleic acid molecule having constant regions domains from another species than human, especially murine constant regions domains. The nucleic acid molecule of the invention can be a single-stranded or double-stranded nucleic acid molecule. The nucleic acid molecule of the invention can be a DNA or a corresponding RNA molecule.

The invention also concerns a vector, in particular a plasmid comprising at least one nucleic acid molecule as defined herein.

A plasmid or vector can be used either for cloning, for transfer or for expression purposes.

According to a particular embodiment, a plasmid of the invention is suitable for the cloning of the nucleic acid molecule it contains. Such a cloning plasmid may be a bacterial plasmid, an origin of replication and multiple restriction enzyme cleavage sites allowing the insertion of a transgene insert (transcription unit), e.g., a nucleic acid molecule as defined according to the invention, or a fragment thereof.

According to another particular embodiment, a plasmid of the invention is suitable for the expression of the nucleic acid molecule it contains. Such an expression plasmid, also termed expression vector herein, or expression construct, generally contains a promoter sequence, a transcription terminator sequence, and a transgene insert (transcription unit), e.g., a nucleic acid molecule as defined herein, or a fragment thereof. An expression vector may also contain an enhancer sequence which increases the amount of protein or RNA produced.

According to a particular embodiment, a vector of the invention comprises all or a part of the sequence of the plasmid termed "pCDH-F24-TCR" herein, encoding the alpha and beta chains of the "F24" TCR reported in Table S8 is provided under SEQ ID NO: 57 (8241 nucleotides).

The invention is accordingly also directed to a transfer vector, in particular a viral vector, especially a lentiviral transfer vector, which comprises at least one nucleic acid molecule as defined herein, or a fragment thereof, contained in a transcription unit.

According to a particular embodiment, such a lentiviral transfer vector, is used for the preparation of lentiviral vector particles and accordingly is also termed lentiviral vector genome herein.

Given its nature, such a lentiviral transfer vector or lentiviral vector genome is a recombinant construct.

The invention therefore also relates to a lentiviral transfer vector which comprises lentiviral cis-active elements including long terminal repeats (LTRs) or modified LTRs including partially deleted 3'LTR, psi (Ψ) packaging signal, optionally Rev responsive element (RRE), together with a transcription unit comprising a nucleic acid molecule encoding a TCR as defined herein, or a fragment thereof, or comprising a nucleic acid molecule as defined herein, or a fragment thereof.

According to particular embodiment, the lentiviral transfer vector does not comprise any protein from the parental lentivirus, meaning that the sequences of the original lentivirus genome encoding the lentiviral proteins are essentially deleted in the lentiviral transfer vector, resulting in a lack of expression of any viral protein from the parental lentivirus.

As used herein, the term *"encoding"* defines the ability of a nucleic acid molecule to be transcribed and where appropriate translated for product expression into selected cells or cell lines, when said molecule is placed under transcription and expression control sequences including promoter for transcription. Accordingly a *"polynucleotide or a nucleic acid molecule encoding"* according to the invention designates the nucleic acid molecule having its sequence translated into the amino acid sequence and that may be cloned or placed under the control of expression control sequences, especially a heterologous promoter to provide a transcription unit.

In a particular embodiment, the present invention relates to a recombinant lentiviral transfer vector, which can be derived from an Human Immunodeficiency Virus (HIV), for example HIV-1 or HIV-2, Caprine Arthritis Encephalitis Virus (CAEV), Equine Infectious Anaemia Virus (EIAV), Visna/Maedi Virus (VMV)" Simian Immunodeficiency Virus (SIV), Feline Immunodeficiency Virus (FIV) or Bovine Immunodeficiency Virus (BIV).

In a preferred embodiment, the lentiviral transfer vector is derived from the genome of HIV, especially of HIV-1 and is accordingly a HIV-based vector, in particular a HIV-1 based vector.

As indicated above and according to a particular embodiment, the recombinant lentiviral transfer vector is replication-incompetent as a result of lack of expression of any lentiviral protein, *i.e.* as a result of deletion of all or part of the gag and pol genes of the lentiviral genome or mutation in the *gag* and *pol* genes of the lentiviral genome, so that the *gag* and *pol* genes are not capable of encoding functional GAG and POL proteins.

The lentiviral transfer vector comprises the psi (ψ) packaging signal. The packaging signal includes a sequence coding the N-terminal fragment (about 15-30 amino-acids) of the *gag* ORF. In a particular embodiment, its sequence could be modified by frameshift mutation(s) or a mutation in ATG initiation codon.

In a particular embodiment, in said lentiviral transfer vector genome, the 3' LTR sequence of the lentiviral transfer vector is devoid of at least the activator (enhancer) and of the promoter of the U3 region. In another particular embodiment, in the lentiviral transfer vector, the U3 region of the LTR 5' is replaced by a non-lentiviral U3. In another particular preferred embodiment, the 3' LTR region is devoid of the U3 region (delta U3). In this respect, reference is made to the corresponding description in WO 01/27300 and WO 01/27304.

In a particular embodiment, in the lentiviral transfer vector, the U3 region of the LTR 5' is replaced by a promoter suitable to drive tat-independent primary transcription. In such a case, the vector is independent of *tat* transactivator.

According to a particular embodiment, the lentiviral transfer vector further comprises a DNA flap encompassing a DNA sequence of a lentivirus in particular of a HIV-1 genomic sequence which is framed by a central polypurine tract (cPPT) and a central termination sequence (CTS). The DNA flap possibly enhances nuclear import of the transcription unit of the transfer vector and increases transduction efficiency of lentiviral vector particles comprising the transfer vector.

Accordingly, a lentiviral transfer vector may be regarded as a replacement vector in which all the lentiviral protein coding sequences between the 2 LTRs have been deleted and replaced by transcription unit(s) as defined herein, and wherein the DNA flap element has been re-inserted in association with the required cis-acting sequences described herein. Further features relating to the composition of the lentiviral transfer vector are disclosed in relation to the preparation of the lentiviral vector particles.

Nucleotide sequence of a DNA flap of lentiviral origin comprises two essential regions, *i.e.,* the cPPT and the CTS regions, wherein the cPPT and CTS regions induce a three-stranded DNA structure during replication of DNA containing them (previously defined in Zennou et al., Cell, 2000, 101, 173-185*;* and in the international patent applications WO99/55892 and WO01/27300).

In a particular embodiment, the DNA flap is inserted upstream of the transcription unit(s) of interest, advantageously but not necessarily to be located in an approximate central position in the lentiviral transfer vector. A DNA flap suitable for the invention may be obtained from a lentivirus, in particular a human lentivirus such as HIV-1.

It may be alternatively obtained from the CAEV (Caprine Arthritis Encephalitis Virus) virus, the EIAV (Equine Infectious Anaemia Virus) virus, the VISNA virus, the SIV (Simian Immunodeficiency Virus) virus or the FIV (Feline Immunodeficiency Virus) virus. The DNA flap may be either prepared synthetically (chemical synthesis) or by amplification of the DNA providing the DNA flap from the appropriate source as defined above such as by Polymerase chain reaction (PCR). In a more preferred embodiment, the DNA flap is obtained from an HIV lentivirus, especially HIV-1 including from any isolate or consensus sequence..

The recombinant lentiviral transfer vector further comprises transcription unit(s) as defined herein which is placed under the control of a heterologous promoter (*i.e.* a promoter which does not derive from the lentiviral genome providing the cis-active sequences), thereby providing a transcription unit. A particular promoter is the cytomegalovirus (CMV) promoter. Other promoters may in particular be selected for their properties as constitutive promoters, tissue-specific promoters, or inducible promoters. Examples of suitable promoters comprise the promoters of the following genes: EF1$\alpha$, human PGK, PPI (preproinsulin), thiodextrin, Ferritin L chain or Ferritin H chain, Chymosin beta 4, Chymosin beta 10, Cystatin Ribosomal Protein L41, CAG, SV40 or MND.

Accordingly, in another more particular embodiment, the present invention relates to a recombinant lentiviral transfer vector as defined herein which comprises a 3'-LTR in which the promoter and the activator of the U3 region have been deleted (delta U3) and transcription unit(s) are placed under the control of a heterologous promoter, a list of which is provided above.

According to a particular embodiment, the lentiviral transfer vector of the invention enables co-expression of several transcription units, especially two transcription units respectively coding for an alpha chain of a TCR and the beta chain of a TCR, or fragments thereof as defined herein, and is a bicistronic or a multicistronic vector. Such a lentiviral transfer vector accordingly comprises several transcription units, especially two transcriptions units comprising nucleotide sequences encoding a TCR alpha chain or a fragment thereof as defined herein on one end, and a TCR beta chain or a fragment thereof as defined herein on the other end, according to the nucleotide sequences disclosed herein. Multicistronic vectors simultaneously express two or more separate proteins from the same mRNA.

According to a preferred embodiment, the lentiviral transfer vector of the invention enables stoechiometrically equivalent levels of expression of the several nucleic acid molecules it contains, especially stoechiometrically equivalent levels of expression of an alpha chain of a TCR and the beta chain of a TCR of the invention as defined herein, the co-expression of which is sought.

According to a particular preferred embodiment, a lentiviral transfer vector of the invention comprises a polynucleotide encoding a self-cleaving 2A peptide sequence inserted between the sequences encoding said alpha and beta TCR chains.

Self-cleaving 2A peptides are short peptides of about 20 amino acids that enable production of equimolar levels of multiple nucleic acid molecules from the same mRNA.

Sequences of common 2A peptides are provided below.

| Peptide | Amino acid sequence | SEQ ID NO: |
|---|---|---|
| T2A | E G R G S L L T C G D V E E N P G P | 168 |
| P2A | A T N F S L L K Q A G D V E E N P G P | 169 |
| E2A | Q C T N Y A L L K L A G D V E S N P G P | 170 |
| F2A | V K Q T L N F D L L K L A G D V E S N P G P | 171 |

GSG residues can be added to the 5' end of the sequences coding for the above-peptides to improve cleavage efficiency.

A nucleotide sequence encoding a 2A peptide can be either PCR-cloned between the nucleic acid molecules to be co-expressed or a multicistronic cassette can be inserted into a backbone as a single unit.

2A peptides permit stoechiometrically equivalent levels of expression of the nucleic acid molecules between which they are inserted.

According to a particular embodiment, the nucleotide sequence encoding the 2A peptide is a nucleotide sequence encoding T2A peptide.

The present invention also relates to a host cell either transfected or genetically transformed with a recombinant lentiviral transfer vector of the invention as disclosed herein.

The present invention also relates to a host cell either transfected or genetically transformed with the recombinant lentiviral transfer vector of the invention and with additional plasmid vectors for helper functions, including for packaging and for expression of the envelope for vector particles pseudotyping. According to a particular embodiment, more than two additional plasmid vectors may be used for co-transfection or genetic transformation, including for example a packaging plasmid vector (construct) containing only Gag and Pol genes of a lentivirus, a plasmid (construct) expressing the envelope and optionally a separate plasmid (construct) expressing the Rev gene of a lentivirus. The design of these vectors and plasmids is commonly known by the skilled person in the art.

Such a production cell for the expression of lentiviral vector particles may be either transfected or genetically transformed with a lentiviral transfer vector according to the invention and with plasmids providing helper functions, including a plasmid expressing the envelope which encodes a heterologous (i.e., non lentiviral) envelope protein, in particular an envelope protein of a VSV such as a VSV-G protein selected among VSV-G of Indiana strain, of VSV-G of New Jersey strain and including packaging construct(s) as DNA plasmid(s) encoding the GAG and POL proteins of a lentivirus, in particular of the lentivirus providing the sequences of the transfer plasmid.

The host cell of the invention is transfected with these vectors and plasmids by methods well known to the person skilled in the art, *i.e.* by chemical transfection (calcium phospate, lipofectamine), lipid-based techniques (liposome), electroporation, photoporation.

As used herein, the term "*transfected"* refers to a cell comprising a recombinant lentiviral transfer vector of the invention (transient expression), whereas the term "*genetically transformed*" refers to a cell whose genome has been definitively modified by a polynucleotide of the invention (permanent expression).
Said transitory or stably transformed cells can be prokaryotic (bacteria) or eukaryotic (yeast, insect or animal including mammal especially human) cells. In an embodiment, cells are non-human cells. In a particular embodiment, cells of the invention are isolated human cells, "*isolated*" meaning outside of their natural environment.

In a particular embodiment of the invention, the cell is from the HEK 293T (human embryonic kidney) cell line, in particular as disclosed in Zennou et al. (Cell, 2000, 101, 173-185).

The invention also relates to a method to produce lentiviral vector particles, which are recombinant lentiviral vector particles, comprising or consisting of:

a) transfecting the recombinant lentiviral transfer vector according to the invention, into a host cell suitable for packaging, for example a HEK-293T cell line or a cell line derived therefrom such as HEK-293T/17 cell line;
b) co-transfecting the cell of step a) with at least (i) a plasmid expressing the envelope, especially a plasmid vector encoding the envelope glycoprotein G of a VSV, in particular the VSV-G of Indiana or of New Jersey VSV strains and (ii) with a packaging plasmid vector encoding the lentiviral GAG and POL or mutated non integrative POL proteins of a lentivirus, in particular of a HIV-1 lentivirus;
c) recovering the recombinant lentiviral particles expressing recombinant TCR.

The transfection steps with the particular plasmid constructs disclosed above may be carried out according to a different sequence with respect to the one described above.

The present invention also relates to recombinant lentiviral vector particles obtained using a lentiviral transfer vector disclosed herein.

According to a particular embodiment, the recombinant lentiviral vector particles comprise a genome consisting of the recombinant lentiviral transfer vector as disclosed herein, and are pseudotyped with a vesicular stomatitis virus glycoprotein G (VSV-G) protein, in particular the VSV-G protein of the VSV Indiana strain or the VSV protein of the New Jersey strain.

The expression "*recombinant lentiviral vector particles"* defines the obtained particles expressed in a host cell or production cells following transfection by the several plasmid vectors comprising at least the lentiviral transfer vector, the envelope vector encoding the selected envelope protein and the packaging vector providing lentiviral proteins *in trans* (such as lentiviral GAG and POL proteins, in particular mutated POL protein for the avoidance of integration) according to methods well-known in the art.

The terms "*recombinant lentiviral vector particles"* encompass recombinant viral particles, and recombinant virus-like particles.

Virus-like particles result from incomplete assembly of the proteins present for encapsidation of the recombinant lentiviral transfer vector in a way that does not enable the formation of true viral particles.

According to the invention, lentiviral vector particles are the product recovered from co-transfection of host cells, for example from a HEK-293T cell line, with at least:

- a recombinant lentiviral transfer vector according to the invention, as described herein;
- a plasmid expressing an envelope protein, especially a plasmid vector encoding the envelope glycoprotein G of a VSV, in particular the VSV-G of Indiana or of New Jersey VSV strains;
- a packaging plasmid vector encoding the lentiviral GAG and POL proteins or mutated non integrative POL protein of a lentivirus, in particular of a HIV-1 lentivirus.

According to a particular embodiment of the invention, the recombinant lentiviral vector particles are integration defective (or non-integrative) as a result of mutation or deletion in the *pol* gene of the lentivirus present on the packaging plasmid vector. Suitable mutations enabling formation of integration defective particles are well-known in the art and illustrated in WO 2009/019612.

According to a particular embodiment, the lentiviral vector particles encompass a recombinant lentiviral transfer vector which is multicistronic, especially biscistronic, in that it encodes at least a TCR alpha chain and a TCR beta chain of the invention or fragment(s) thereof, as disclosed herein. Accordingly, the lentiviral vector particles can include lentiviral transfer vector plasmids that may comprise bicistronic or multicistronic expression cassettes where the polynucleotides encoding the various polypeptides are separated by a nucleotide sequence encoding a 2A peptide, and/or may also further comprise several expression cassettes for the expression of various polypeptides, where the polynucleotides encoding the additional various polypeptides and distinct from the polynucleotides separated by a polynucleotide encoding a 2A peptide, are separated either by another polynucleotide encoding a 2A peptide, or by an IRES sequence of viral origin (Internal Ribosome Entry Site). Conversely, lentiviral transfer vector plasmids may encode fusion protein(s).

The invention also relates to a method for *ex vivo* obtaining a collection also designated as a population of recombinant cells expressing a TCR or a recombinant TCR as defined herein, comprising the steps of:

a.Transducing cells capable of expressing a functional TCR, with recombinant lentiviral vector particles of the invention, as disclosed herein, and

b. Culturing the transduced cells in conditions that permit the TCR of the invention as defined herein to be expressed, and

c.Obtaining and/or recovering a recombinant cell collection or population expressing said recombinant TCR and optionally further isolating said expressed recombinant TCR.

"Cells capable of expressing a TCR" are preferably mammalian cells, particularly human cells, especially human non dividing cells.

They can be **peripheral blood mononuclear cell (PBMC),** especially appropriately preactivated according to methods known by the skilled person. They encompass T cells in particular primary T-cells, in particular primary CD4+ T cells or CD8+ cells and in particular primary CD8+ T cells or cells amplified from such cells.

According to a particular embodiment, the recovered recombinant cell collection expressing said recombinant TCR comprises or consists of or consists essentially of lymphocyte T cells, in particular primary or mature immunocompetent T cells, especially CD4+T cells or CD8+ T cells.

The invention further relates to a collection or a population of recombinant human cells expressing a TCR of the invention as defined herein at their surface, or a collection or a population of cells obtained after transduction with the lentiviral particles of the invention or by the above method for obtaining a collection of recombinant cells, in particular a collection or a population of *ex vivo* transduced T-cells, which is formulated for administration to a human host. The transduced T cell may have integrated in their genome the nucleic acid molecule encoding the TCR, together with remaining nucleotides sequences of the LTR of the lentiviral transfer vector such as the R segment or part thereof.

The invention also relates to a collection of cells that comprises distinct populations of cells wherein the difference in the cells lies in the expression of distinct TCRs of the invention, i.e.,TCRs having various sequences among those disclosed herein and/or different affinities for the Gag293 epitope of HIV-1.

The invention further relates to a composition, especially a composition, comprising recombinant lentiviral vector particles of the invention as defined herein together with one or more pharmaceutically acceptable carrier(s).

The invention also relates to a composition comprising a population of *ex vivo* transduced cells of the invention as defined herein together with one or more pharmaceutically acceptable carrier(s).

The invention further relates to the use of a recombinant lentiviral transfer vector of the invention as defined herein or recombinant lentiviral vector particles of the invention as defined herein, or population of cells of the invention as defined herein, for the *ex vivo* preparation of an immunotherapeutically active composition, in particular a composition suitable for the treatment of patients seropositive for HIV infection.

The invention also concerns recombinant lentiviral vector particles of the invention as defined herein, or collection of cells of the invention as defined herein, for use as a medicament in a human patient infected with HIV , in particular in an immunotherapeutic treatment (therapeutic vaccine) against HIV-related disease in patients infected with HIV, in particular HIV-1.

The invention also concerns recombinant lentiviral vector particles of the invention as defined herein or collection of cells of the invention as defined herein, for use as a medicament in a human patient infected with HIV, for eliciting a CD4+ and/or CD8+ T cell response in a patient infected with HIV, in particular HIV-1, especially a patient having developed a HIV-related disease.

The human patient treated according to the invention is in particular a patient seropositive for HIV, in particular for HIV-1, who has been treated by antiretroviral therapy and possibly is still undergoing such treatment, especially by HAART, in particular such a patient who is pharmacologically controlling the HIV infection, especially who is controlling viral load under antiretroviral therapy.

Antiretroviral therapy (ART) is treatment of patients infected with human immunodeficiency virus (HIV) using anti-HIV drugs. The standard treatment consists of a combination of at least three drugs (often called "highly active antiretroviral therapy" or HAART) that suppress HIV replication. Three drugs are used in order to reduce the likelihood of the virus developing resistance. ART and HAART have the potential both to reduce mortality and morbidity rates among HIV-infected people, and to improve their quality of life.

According to a particular embodiment, the human patient has been under antiretroviral therapy, including highly active antiretroviral therapy.

According to a particular embodiment, the human patient has been under antiretroviral therapy, including highly active antiretroviral therapy.for at least 6 or 7 or 8, or 9 or 10 months. According to another particular embodiment, the human patient has been under antiretroviral therapy, including highly active antiretroviral therapy for at least 1 year.

In a particular embodiment of the invention, the treatment of a patient seropositive for HIV, in particular HV-1 enables active control of HIV infection. Such active control may involve immune reconstitution in treated patient, sustained antiviral response and in particular maintenance of an undetectable viral load according to standard assay. Such response may in particular be designed to involve one of the following pathways: inducing CD4+ T cell expressing TCRs of the invention in particular CD4+ T cell having a central memory phenotype allowing persistence of immunological memory, or CD4+ T cell having an effector differentiation status (in particular Th1) even when associated with a low viremia, eliciting a cytotoxic response toward HIV infected cells or eliciting a helper response that will enable said cytotoxic response to take place, preventing HIV replication,

In a particular embodiment of the invention, the treatment of a patient seropositive for HIV, in particular HV-1 is designed to enable preventing risk of progressing to AIDS in the treated patient.

In a particular embodiment the patient receiving the disclosed treatment is HLA-DR matched and especially is HLA-DR11, HLA-DR15, HLA-DRB5, HLA-DR1, HLA-DR7, HLA-DR4 and/or HLA-DR3 restricted, more particularly with HLA-DR11, HLA-DR15, HLA-DRB5 or HLA-DR1 restricted.

Other examples and features of the invention will be apparent when reading the examples and the figures, which illustrate the experiments conducted by the inventors, in complement to the features and definitions given in the present description.

## LEGEND OF THE FIGURES

**Figure 1: Immunoscope analysis of Gag293-specific CD4+ T cells**

(A) Sorting of Gag293-specific CD4+ T cells with HLA-DR tetramers. Examples of primary CD4+ T cell lines labeled with DRB5 (top) and DR1 (bottom) tetramers. The percentage of tetramer positive cells (Tet+) in the total CD4+ T cell population (middle plots) and in the sorted Tet+ population (right plots) is reported in the top right corner of the graph. Samples labeled with tetramers loaded with an irrelevant peptide (CLIP or Annexin II) were used as negative controls (left plots).
(B, C) The percentages of Tet+ cells expressing the TRAV24 (B) or TRBV2 family (C) were analyzed by qPCR in the HIC and the HAART groups and compared by the Mann-Whitney test.
(D, E) CDR3 length profiles for the TRAV24 (D) and TRBV2 families (E) are shown for each patient analyzed. Healthy donor PBMC were used as control (bottom left). The percentage of the TRAV24 or TRBV2 family in the total TRAV or TRBV PCR product is reported above each profile.

**Figure 2: Clonotypic diversity of Gag293-specific TCRs**

(A) The number of unique CDR3 amino acid sequences (clonotype AA) obtained per 100 TRAV24 (left) or TRBV2 (right) nucleotide sequences were compared in the HIC and HAART groups with the Mann-Whitney test.
(B) Simpson's diversity indexes computed for TRAV24 (left) and TRBV2 (right) clonotypes AA obtained in each patient were compared in the HIC and HAART groups with the Mann-Whitney test.
(C to E) Frequencies of TRAJ genes (D), TRBJ genes (E), and TRBD genes (F) in Gag293-specific TRA or TRB sequences. Frequencies were compared between the HIC (ligh grey bars) and HAART groups (dark bars).
(F): the distribution of CDR3 lengths were compared for TRAV24 between the sets of HIV Controller sequences (HIC, n=584) and treated patient sequences (HAART, n=496). CDR3 lengths are reported in number of a.a.
(G): the distribution of CDR3 lengths were compared for TRBV2 between the sets of HIV Controller sequences (HIC, n=716) and treated patient sequences (HAART, n=566).

**Figure 3: Quantification of public motifs and clonotypes in the Gag293-specific TCR repertoire**

(A, B) The Meme motif discovery program was used to identify a.a. motifs enriched in Controller TRAV24 sequences (A) and TRBV2 sequences (B) compared to corresponding sequences in treated patients. The Meme program was used in discriminative mode, which highlights differences between sequence datasets. The relative size of each a.a. symbol is proportional to its frequency in the HIC dataset, while the total height of a.a. symbols indicates the information content of the position, in bits.
(C, D) Frequency of public clonotypes per 100 TRAV24 sequences (C) or per 100 TRBV2 sequences (D) for each of the 8 Controllers (HIC) and 8 treated patients (HAART) studied.
(E, F) Frequency of nucleotide sequences coding for a public clonotype per 100 TRAV24 sequences (E) and per 100 TRBV2 sequences (F). (C to F) Significant differences ($P<0.05$) obtained by the Mann-Whitney test are reported.
(G) Structure of the CDR3 junction for the most prevalent TRAV24 public clonotype AFKAAGNKLT. The number of N mutations (N), of trimmed nucleotides (trim), and the frequency (%AFKAAGNKLT) of the 4 nucleotide sequences coding for this clonotype are reported (SEQ ID NO: 172 to 174 on Fig. 3G).

**Figure 4: Ex vivo analysis of the Gag293-specific TCR repertoire**

(A) Gating strategy for tetramer analysis in Controller PBMC. An example of PBMC staining with a control tetramer (AnnII; left plot) and a Gag293-loaded DRB5 tetramer (right plot) is shown.
(B) Frequency of TRAV24 and TRBV2 families in Gag293-Tet+ cells sorted ex vivo. The percentages of TRAV24 expression in total TRAV products (left) and TRBV2 expression in total TRBV products (right) are reported. Dotted lines indicate the mean percentage of TRAV24 and TRBV2 families in CD4+ T cell from 7 healthy donors.
(C) Representation of Gag293-specific clonotypes found ex vivo in the cell line obtained from the same patient. The percentage of sequences matching a TRAV24 (left) or TRBV2 ex vivo clonotype (right) in the corresponding cell line is reported, with medians indicated by horizontal lines.
(D, E) The percentages of public motifs are compared in sequences obtained ex vivo (left) and in the matched cell line (right) for TRAV24 (D) and TRBV2 (E), using the paired Student t test.

**Figure 5: Public TCRs confer MHC II cross-restriction and high-affinity Gag293-MHC binding**

(A) Expression of TCR$\beta$ and CD3 in J76 cells transduced with the F24, F25, and F5 TCRs. Solid grey histograms correspond to mock-transduced J76 cells.
(B) Staining of F24-transduced J76 cells with CLIP-loaded tetramers (top) and Gag293-loaded tetramers (bottom). The percentage of Tet+ cells is reported on each plot.

(C) The percentage of Tet+ cells after transfer of F24 (black), F25 (medium grey), and F5 (light grey) is reported for each of the 4 tetramers tested after subtraction of CLIP-tetramer background (mean of 2 experiments).

(D) Example of SPR sensorgrams. The soluble F24 TCR (concentrations 0.3 to 100 μM) was flown over immobilized DR11-Gag293 monomers to measure the SPR response. RU, response units.

(E) Affinity measurement of the F24, F25, and F5 TCRs for Gag293 complexed to DR11, DRB5, or DR1 monomers. Each soluble TCR was flown over Gag293-DR complexes at different concentrations (x axis) to measure binding RU (y axis).

(F) Summary of affinities (Kdeq) of the F24, F25 or F5 TCRs for Gag293-loaded HLA-DRB monomers. Each Kdeq value represent the mean ± SEM from at least two independent experiments performed in duplicate.

(G) Correlation between TCR affinity and tetramer binding. TCR affinities (log Kdeq) of the F24, F25 and F5 TCRs for the 3 Gag293-DR complexes are plotted in function of the percentage of Gag293-Tet+ cells (log %Tet+) for the corresponding TCR/HLA-DR combination (y axis). R: Spearman correlation coefficient.

**Figure 6: Public TCR transfer confers high antigen sensitivity to J76 cells**

(A, B) Antigen sensitivity assay in TCR-transduced J76 cells. Percentages of CD69 expression in J76 cells transduced with TCRs F24, F25, F5 (A) or F4 and F13 (B), after coculture with L-cells expressing different HLA-DR alleles (DR11, DR15, DRB5, or DR11) and loaded with decreasing Gag293 concentrations. Experiments were conducted in triplicate, with curves corresponding to one experiment shown for clarity.

(C) Antigen sensitivity assay of TCRs HD5 and HY9 from HAART patients.

(D) Correlation between binding affinity (log Kdeq) for Gag293-loaded HLA-DR monomers (DR11, DRB5, and DR1) and antigen sensitivity (log EC50 for CD69 induction) of the F24, F25 and F5 TCRs.

(E) Correlation between the antigen sensitivity (log EC50) measured for 8 TCRs in the presence of DR11 APC and the number of HLA-DR alleles restricting these TCRs. (E, F). R: Spearman correlation coefficient.

(F) TCR reactivity to native HIV-1 capsid antigens. CD69 induction was quantified in TCR-transduced J76 cells cocultured with dendritic cells infected with the VSV-pseudotyped virus HIV-1 (+) or left uninfected (-). One representative experiment out of three is shown.

**Figure 7: Public TCR transfer confers high avidity responses and polyfunctionality to primary T cells**

(A) Cytokine production in primary CD4+ T cells mock-transduced (1st row) or transduced with the F24, F25, or F5 TCR (rows 2-4) and stimulated with 10-5M Gag293. CD4+ T cells were analyzed by ICS for expression of TNF-α, MIP-1β, IL-2, IFN-γ, and CD107a. One representative experiment out of three is shown.

(B) ICS analysis of CD4+ T cells transduced with F24 and stimulated with decreasing Gag293 doses. Expression of the analyzed markers (%marker+) is reported in function of peptide dose, after subtraction of background measured in unstimulated cells.

(C) Summary of EC50 values measured by ICS in CD4+ T cells after TCR transduction. For each TCR, the Gag293 concentration required to achieve half-maximal expression of the 5 markers studied is reported. Mean + SEM of EC50 values obtained for 3 independent experiments are reported.

(D) Cytokine production in CD8+ T cells that were mock-transduced (1st row) or transduced with the F24 TCR (2nd row) and analyzed as in A.

(E) ICS analysis of CD8+ T cells transduced with F24 and stimulated with decreasing Gag293 doses.

(F) Polyfunctionality of CD4+ T cells transduced with the F24, F25, and F5 TCRs and stimulated with decreasing Gag293 doses. The number of co-expressed markers out of the 5 studied (TNF-α, MIP-1β, IL-2, IFN-γ, CD107a) defines the number of functions reported in legend. Stimulation with PMA/ionomycin is used as a positive control.

**Figure 8: Analysis of nucleotide insertions and deletions in Gag293-specific clonotypes**

(A) The number of mutations (P + N) inserted in Gag293-specific clonotypes compared to TRAV24-containing (left) or TRBV2-containing (right) germline sequences is reported.

(B) The number of germline nucleotides trimmed during V(D)J recombination to generate the observed Gag293-speific TRAV24-containing (left) and TRBV2-containing (right) clonotypes is reported.

(A and B): The numbers of mutations and trimmed nucleotides were computed in the IMGT/HighV-QUEST program, using the IMGT database of human germline TRAV and TRBV alleles as a reference (www.imgt.org). Significant differences between means (P<0.05) obtained by the unpaired student t-test are reported.

**Figure 9: Identification of prevalent amino acid motifs in Gag293-specific clonotypes**

The Meme motif discovery program (meme-suite.org) was used to identify the most prevalent a.a. motifs in HIV Controller (HIC) and treated patients (HAART) clonotypes. The Meme program was used in normal mode with the «one ocurrence per sequence>> option, to take all clonotypes into account. The relative sizes of the letters in the logo are proportional to their frequencies, while the total height of the letters indicates the information content of the position, in bits.

(A) Most prevalent motif in TRAV24 clonotypes from the HIC (top; n=584) and HAART (bottom; n=496) groups.
(B) Most prevalent motif in TRBV2 clonotypes from the HIC (top; n=716) and HAART (bottom; n=566) groups.

**Figure 10: Analysis of TCR avidity by MHC II tetramer titration**

(A) Gag293-DR11 tetramer titration: J76 cells transduced with the TCRs F24, F25, or F5 were incubated with decreasing concentrations of HLA-DR11 tetramer loaded with the Gag293 peptide. The percentage of Gag293-specific tetramer+ (Tet+) cells minus the percentage of cells labeled with a contol CLIP-loaded tetramer is reported. (B and C)
Gag293-DRB5 tetramer (B) and Gag293-DR1 tetramer (C) titration on F24-transduced cells. The dip in binding curves at high tetramer concentrations likely reflects competition effects between multivalent ligands. EC50 computation was based on the sigmoidal part of the response curve. (D) Linear correlation between the maximum % of tetramer+ cells and TCR affinity determined by SPR. (E) The half-maximal tetramer binding values (EC50) are reported. ND: not detectable, i.e. tetramer binding was too low to evaluate the EC50 value.

**Figure 11: Analysis of Gag293-specific CD4+ T cell responses in DR11 patients**

(A) Comparison of antigen sensitivity in HIV Controllers (HIC) and treated patients (HAART) carrying at least one DR11 allele. Antigen sensitivity was measured by the last Gag293 peptide dilution (in M) that yielded a specific CD4+ T cell line.
(B) Comparison of the maximal ELIPOT response to Gag293 in DR11 patients. CD4+ T cell lines generated with a 10-5 M Gag293 peptide dose were restimulated with the same high peptide dose and analyzed by IFN-γ ELISpot assay. The number of spot forming cells (SFC) per 106 cells is reported. Values >104 SFC / 106 cells reached saturation and are reported as equal to 104 SFC / 106 cells.
(A and B) P values obtained by the Mann-Whitney U test are reported.
(C and D) Comparison of TRAV24 (C) and TRBV2 (D) expression in Gag293-specific cells of DR11 patients.

The percentage of TRAV24 or TRBV2 expression among tetramer-positive (Tet+) cells is reported.

**Figure 12: TCR transfer in primary T cells from healthy donors confers Gag293-MHC II tetramer recognition**

(A) Example of TCR transduction in primary CD4+ T cells from a healthy donor. PBMCs were mock transduced, or transduced to express the F24, F25, or F5 TCRs, and stained with anti-TRBV2 mAb.
(B) MHC II tetramer staining in CD4+ T cells transduced with the F24 TCR (second row), or mock transduced (first row). The percentage of CD4+ T cells stained with Gag293-loaded HLA-DR tetramers (DR11, DR15, DRB5, and DR1) is reported in the top right corner. For these experiments, the DR15 tetramer used corresponded to the HLA DRB1*1502 rather than the HLA DRB1*1501 allele.
(C) Quantification of MHC II tetramer staining in TCR-transduced primary CD4+ T cells. For each tetramer, the mean percentage of tetramer-positive (Tet+) CD4+ T cells obtained from 4 independent experiments is shown. The percentage of Gag293-specific Tet+ cells was computed by subtracting the percentage of CLIP-Tet+ cells from that of Gag293-Tet+ cells. Light grey bars: mock-transduced; black bars: F24-transduced; grey bars: F25-transduced; light grey bars: F5-transduced.

**Figure 13: Analysis of cytokine production in CD8+ T cells transduced with the F24 TCR.**

(A) Blocking of the cytokine response in CD8+ T cells with anti-HLA antibodies.
Cytokine induction was measured in CD8+ T cells transduced with the F24 TCR after stimulation with 10-5 M Gag293 peptide or in non-stimulated cells (NS). Cells were pretreated with an isotypic IgG2a control antibody (black bars), an HLA-DR blocking antibody (grey bars) or a pan-MHC I blocking antibody (light grey bars) at 10 μg/ml prior to peptide stimulation. >75% of the response was blocked by HLA-DR antibody treatment for each cytokine tested, indicating that F24-expressing CD8+ T cells were predominantly restricted by MHC II.
(B) Analysis of the polyfunctionality of CD8+ T cells transduced with the Gag293-specific TCR F24. Polyfunctionality was defined as the capacity for specific cells to co-express at least 3 markers among the 5 studied (TNF-α, MIP-1β, IL-2, IFN-γ, and CD107a) after Gag293 peptide stimulation. The number of markers co-expressed defines the number of functions reported in legend. Polyfunctionality is visualized with pie charts in which each slice represents a functional category: white, 5 functions;light grey, 4 functions;middle grey, 3 functions;dark grey, 2 functions; andblack, 1 function. Polyfunctionality was assessed after stimulation at different peptide doses ranging from 10-5 M to 10-8 M. Stimulation with PMA and ionomycin was used as a positive control to induce a highly polyfunctional response (right pie).

As a summary of the experiments carried out, it has been observed that the rare patients who spontaneously control HIV replication in the absence of therapy show signs of a particularly efficient cellular immune response. To identify the molecular determinants underlying this response, the inventors characterized the TCR repertoire directed at the most immunoprevalent CD4 epitope in HIV-1 capsid, Gag293. HIV Controllers from the ANRS CODEX cohort showed a highly skewed TCR repertoire characterized by a predominance of TRAV24 and TRBV2 variable genes, shared CDR3 motifs, and a high frequency of public clonotypes. The most prevalent public clonotypes generated TCRs with affinities at the higher end of values reported for naturally occurring TCRs. These high-affinity Gag293-specific TCRs were cross-restricted by up to 5 distinct HLA-DR alleles, accounting for their expression in HIV Controllers of diverse genetic backgrounds. Transfer of these TCRs to healthy donor CD4+ T cells conferred high antigen sensitivity and polyfunctionality, thus recapitulating key features of the Controller CD4 response. Transfer of a high-affinity Gag293-specific TCR could also redirect CD8+ T cells to target HIV-1 capsid via nonconventional MHC II restriction. These findings indicate that TCR clonotypes with superior functions are associated with HIV control. Amplifying or transferring such clonotypes may contribute to immunotherapeutic approaches that aim at a functional HIV cure.

## RESULTS

We set to compare Gag293-specific CD4 responses in HIV Controllers and efficiently treated patients, two groups characterized by long-term viral control. The clinical and immunological characteristics of the studied patients are reported in **Table 1.** A stringent definition of HIV control was applied, based on an undetectable viral load in standard assays (<50 copies HIV-1 RNA/ml) for over 5 years. The duration of control was actually longer, as patients included in the Controller group (HIC group, n=14) had been infected for a median duration of 19 years. They were compared to HIV-1 infected patients (HAART group, n=15) who had received long-term antiretroviral therapy, with an undetectable viral load for at least 5 years, and a median duration of treatment of 11 years. Thus, both groups were characterized by long-term viral suppression, which ensured that potential differences in CD4 responses were not primarily determined by levels of residual HIV viremia.

***Table 1. Clinical and immunological characteristics of patients***

| ***Clinical parameters***[a] | HIV controllers (HIC, n=14) | Treated patients (HAART, n=15) | P value[b] |
|---|---|---|---|
| Age, years | 50 [34-61] | 48 [39 - 56] | N.S. |
| Duration of HIV-1 infection, years | 19.6 [9.8 - 26.0] | 11.9 [6.8 - 25.3] | 0,015 |
| Duration of antiretroviral treatment, years | N/A | 11.1 [5.6 - 19.3] | - |
| Virus load, HIV-1 RNA copies/ml plasma | < 50 | < 50 | N.S. |
| CD4+ T cells / mm3 | 875 [648 - 1400] | 570 [266 - 1534] | 0,008 |
| Nadir of CD4+ T cells /mm3 | N/A | 216 [21 - 589] | - |
| ***Frequency of Gag293-specific CD4+ T cell lines generated at different peptide doses***[c] | HIV controllers (HIC, n=14) | Treated patients (HAART, n=15) | P value[d] |
| Gag293 dose: $10^{-5}$ M | 14/14 | 15/15 | N.S. |

| | | | |
|---|---|---|---|
| Gag293 dose: $10^{-7}$ M | 14/14 | 8/15 | 0.006 |
| Gag293 dose: $10^{-9}$ M | 8/14 | 1/15 | 0.005 |
| Gag293 dose: $10^{-11}$ M | 2/14 | 0/15 | N.S. |

(a) Median values and ranges are reported.
(b) P values were estimated with the Mann-Whitney test.
(c) Frequency of viable cell lines that achieved doubling of input cells and gave a specific response by IFN-g ELISPOT assay.
(d) Differences in frequencies were evaluated with Fisher's exact test. N.S.: not significant ($P \geq 0.05$); N/A: not applicable.

***High antigen sensitivity of Gag293-specific CD4 responses in HIV Controllers***

TCR repertoire studies of specific CD4+ T cells have remained scarce in humans due to the limited clonal amplification of CD4+ T cells as compared to CD8+ T cells, and to the generally lower affinity of TCR expressed by CD4+ T cells, which limits MHC II tetramer detection (31). In the case of HIV infection, these factors are compounded by the general decrease of the CD4+ T cell population, the preferential depletion of HIV-specific CD4+ T cells, and their incomplete restoration under antiretroviral therapy (5, 6). To address these issues and analyze the TCR repertoire of CD4+ T cells from treated patients as well as Controllers, we devised a system of short-term primary CD4+ T cell line cultures that allowed the amplification of MHC II tetramer-positive cells prior to sorting. Using this system, we previously reported the presence Gag293-specific CD4+ T cells with high antigen sensitivity and high MHC II tetramer binding capacity in Controllers, while such cells were absent from treated patients (27). To extend these results, we generated primary CD4+ T cells lines by stimulation with decreasing doses of Gag293 peptide. The specificity of CD4+ T cell lines was evaluated at equivalent growth stages (doubling time), by restimulation with Gag293 and analysis by IFN-γ ELISPOT assay. As reported in Table 1, viable Gag293-specific cell lines were obtained for all patients of the HIC and HAART groups when initially stimulated with the highest peptide dose of $10^{-5}$ M. However, a marked difference was observed for cell lines generated at $10^{-7}$ M peptide, with only 8 out of 15 treated patients responding, versus all of the 14 Controllers (P=0.006). The difference was also marked at the $10^{-9}$ M peptide dose (P=0.005). Moreover, 2 of the Controllers, but none of the treated patients responded at the $10^{-11}$ M peptide dose. These experiments established that Gag293-specific CD4+ T cells had a higher antigen sensitivity in the Controller group, as indicated by the capacity to proliferate and differentiate into IFN-γ-secreting effectors upon stimulation with minimal peptide doses.

***Biased TRA V and TRBV gene usage in Gag293-specific CD4+ T cells***

To characterize the Gag93-specific TCR repertoire, we first genotyped patients for the HLA-DRB1 gene. Eight Controllers and eight treated patients who shared at least one of four HLA-DR alleles (DR1, DR11, DR15, or DRB5) were included in the TCR study (**Table 5**). The frequencies of these four alleles did not differ significantly between the HIC and the HAART groups ($P \geq 0.05$ by Fisher's exact test). CD4+ T cell lines from these patients were labeled with HLA-DR matched Gag293-loaded tetramers, and evaluated for the proportion of Tet+ cells in the CD4+ CD8- T cell population (**Figure 1A, middle panels**). Samples labeled with matched tetramers loaded with an irrelevant peptide (CLIP or Annexin II) were used as negative controls (**Figure 1A, left**). The purity of the Tet+ population was controlled post-sorting, as shown in representative examples (**Figure 1A, right**). TCR diversity of the sorted cells was evaluated through CDR3 length polymorphism analysis, using the Immunoscope technique (32, 33). The expression of 34 TCRα variable gene (TRAV) families and of 24 TCRβ variable gene (TRBV) families was quantified by real-time RT-PCR, followed by an analysis of the length distribution of the amplified CDR3 products on a capillary sequencer. TRAV gene expression in Controller Tet+ cells proved highly skewed, with a median of 44% of Gag293-specific cells expressing the TRAV24 gene family (**Figure 1B**), while this family was amplified at lower levels in specific cells of treated patients (median value: 13%; P=0.037). In comparison the TRAV24 family represented 1 ± 0.2% of T cells in a control group of 7 healthy donors (not shown). Analysis of CDR3 length distribution showed a Gaussian pattern for control PBMC, as expected (**Figure 1D**). In contrast, Gag293-specific cells appeared oligoclonal, with a dominant peak corresponding to a CDR3 of 10 aa, according to the numbering scheme of the International ImMunoGeneTics Information System (IMGT) (34). This peak was more prominent in cell lines from Controllers than those of treated patients, suggesting a stronger TCR bias in controlled HIV infection.

Analysis of TRBV distribution also revealed a major bias in Gag293-specific cells, with a marked predominance of the TRBV2 family, expressed at a median value of 82% in the Controller group (**Figure 1C**). TRBV2 was amplified in Tet+ cells of 7 out of 8 Controllers in the present study, while this amplification was seen in only 1 out of 4 Controllers in a first study where we tested Gag293-specific TCR profiles with a panel of Vβ-specific antibodies (27). The lower frequency of TRBV2 (previously noted Vβ22) amplification detected in the initial study may have resulted from suboptimal Vβ antibody binding due to competition with the MHC II tetramer for binding to the TCR. The TRBV2 family also appeared amplified at high levels in some of the treated patient samples (median value = 28%), with a difference between the HIC and HAART groups that did not reach significance (P=0.13; **Figure 1C**). In comparison, T cells from healthy donors expressed TRBV2 at low levels (3.7 ± 1.0%, not shown). CDR3 length distribution appeared more variable for the TRBV2 than the TRAV24 chain (**Figure 1E**), though a trend for a predominant CDR3 of 13 aa was apparent in Controllers Tet+ cells. Taken together, these data provide evidence for a highly skewed Gag293-specific TCR repertoire characterized by the preferential usage of the TRAV24 and TRBV2 variable gene segments. The fact that the bias is more marked in Gag293-specific cells from Controllers than treated patients suggests a contribution of the TRAV24 and TRBV2 variable regions to the high-avidity recognition of Gag293.

***High clonotypic diversity of Gag293-specific cells in Controllers***

Clonotypic repertoire analysis was carried out for the two variable gene families amplified in Gag293-specific cells, TRAV24 and TRBV2. PCR products corresponding to these two families were cloned, sequenced, and analyzed with IMGT tools (34). A minimum of 50 productive CDR3 sequences were analyzed for each sample, with the full list of CDR3 sequences provided in **Tables 6** and **7.** Productive TRAV24 sequences (n=584 for the HIC group; n=496 for the HAART group - HAART group results not shown) were evaluated for diversity by counting the number of distinct clonotypes, i.e. the number of unique CDR3 aa sequences, present in each patient sample. The normalized number of clonotypes proved significantly higher in the HIC than the HAART group (P=0.0011; **Figure 2A**). Similarly, analysis of TRBV2 sequences (n=716 for the HIC group; n=566 for the HAART group) showed higher clonotypic diversity in HIC samples (P=0.0047; **Figure 2A**). Of note, the number of TRBV2 clonotypes per 100 sequences was remarkably high in the HIC group (median=36), indicating the presence of a diverse TCR repertoire in spite of the pronounced bias for TRBV2. Computation of Simpson's diversity index confirmed a trend for higher clonotypic diversity in the HIC group, which reached significance for TRAV24 but not TRBV2 sequences (**Figure 2B**). The number of mutations needed to generate the observed CDR3 sequences from their germline counterparts was also significantly higher in the HIC group, for both the TRAV24 and TRBV2 datasets (**Figure 8**). Thus, both the number of clonotypes and that of CDR3 insertions and deletions were consistent with the persistence of a more diverse Gag293-specific repertoire in controlled HIV infection.

***Biased J and D gene usage in Gag293-specific TCRs from Controllers***

The Gag293-specific CDR3 sequence dataset was analyzed for the distribution of Junction (J) and Diversity (D) gene segments. TRAJ gene usage was restricted, with 15 and 11 distinct TRAJ genes detected in TRAV24 sequences for the HIC and HAART groups, respectively, out of 61 TRAJ genes reported in the IMGT database (**Figure 2C**). The TRAJ17 gene predominated in the HIC group (48%), while the next most abundant genes, TRAJ39 and TRAJ32, were present at 16 and 12%, respectively. TRAJ gene usage in the HAART group appeared more evenly distributed among TRAJ17 (29%), TRAJ39 (21%), and TRAJ38 (13%). Analysis of the TRBJ distribution in TRBV2 sequences showed again a more biased repertoire in the HIC group, with a predominant TRBJ2-1 gene present in 43% of sequences, while the most abundant TRBJ gene in the HAART group, TRBJ1-2, represented only 22% of sequences (**Figure 2D**). Of note, TRBJ1-2 was only minimally represented in HIC sequences (4%), emphasizing differences between the HIC and HAART Gag293-specific repertoires. In addition, the TRBD2 gene segment was more prevalent in HIC sequences, while the two TRBD genes were equally represented in HAART sequences (**Figure 2E**). The CDR3 length was then computed by counting the number of residues comprised between but not including the conserved cysteine (C104) and the conserved phenylalanine or tryptophan (F/W 118) that define the boundaries of this hypervariable TCR region (34). For TRAV24 sequences, CDR3 length peaked at 10 a.a., consistent with the Immunoscope data (**Figure 2F**). The 10 a.a. peak represented 68% and 48% of HIC and HAART TRAV24 sequences, respectively, pointing to more homogenous CDR3 lengths in Controller sequences (P=5.61e-11; kurtosis: 7.35 for HIC vs 2.91 for HAART). For TRBV2 sequences (**Figure 2G**), CDR3 lengths showed a peak at 13 a.a. in HIC sequences, while CDR3 lengths were more evenly distributed in HAART sequences (P<2.2e-16; kurtosis: 4.82 for HIC vs -2.16 for HAART). These analyses indicated that Gag293-specific CDR3 sequences from Controllers were biased in genetic composition, with a predominant use of TRAJ17, TRBJ2-1, and TRBD2, as well as narrowly distributed CDR3 lengths, while sequences from treated patients appeared more heterogeneous. Thus, Controller TCRs showed restricted V(D)J gene usage, indicative of a repertoire shaped under strong selective pressure, while maintaining a high clonotypic diversity.

***High prevalence of public motifs in Gag293-specific TCRs from Controllers***

Given the observed biases in V(D)J gene usage, we next evaluated whether Gag293-specific CDR3 sequences shared common aa motifs. The Meme motif discovery software was used in discriminative mode to identify motifs enriched in HIC as compared to HAART TRAV24 sequences. This approach revealed a highly prevalent but complex motif shared by 81% of HIC CDR3 sequences (**Figure 3A**). Comparison of the predominant motif in the HIC and HAART datasets highlighted that sequence differences mostly concentrated in the N-terminal part of the CDR3 (**Figure 9A**). More detailed analyses identified two simpler motifs of 10 and 11 aa that were significantly more prevalent in HIC than HAART CDR3 sequences (**Table 2**). CDR3α motif AV24-1, [A/S]x[K/R]AAGNKLT (with x meaning any aa) encompassed the highly conserved AGNKLT sequence derived from the TRAJ17 gene, while motif AV24-2, AxYGGATNKLI, contained a related ATNKLI sequence derived from the TRAJ32 gene. Taken together, these two motifs were found in 49% of HIC versus 29% of HAART TRAV24 sequences (P<0.0001).

*Table 2: Prevalence of public motifs in Gag293-specific clonotypes*

| | TRAV24 Motif Sequence | CDR3 length (AA) | HIC group (% seq.) | HAART group (% seq.) | P value HIC vs HAART | Public clonotypes (% seq.) |
|---|---|---|---|---|---|---|
| Motif AV24-1 | [A/S]x[K/R]AAGNKLT | 10 | 37,16 | 22,38 | <0.0001 | 33,33 |
| Motif AV24-2 | AxYGGATNKLI | 11 | 11,64 | 6,65 | 0,0062 | 27,78 |
| Motifs AV24-1 + AV24-2 | | - | **48,80** | **29,03** | <0.0001 | **61,11** |
| | **TRBV2 Motif Sequence SEQ ID NO: 196 and 197** | **CDR3 length** (AA) | **HIC group** (% seq.) | **HAART group** (% seq.) | **P value** HIC vs HAART | **Public clonotypes** (% seq.) |
| Motif BV2-1 | ASSx[R/G/L][T/A][S/G]Gxx[E/T]Q[F/Y] | 13 | 38,55 | 6,36 | <0.0001 | 50,00 |
| Motif BV2-2 | ASSx[R/G/L][T/A]SGGxx[E/T]Q[F/Y] | 14 | 11,87 | 3,89 | <0.0001 | 33,33 |
| Motifs BV2-1 + BV2-2 | | - | **50,42** | **10,25** | <0.0001 | **83,33** |

The percentage of motif occurrence is reported in total sequences from HIC and HAART patients as well as in the set of public clonotypes.

P values for differences between the HIC and HAART groups were computed with Fisher's exact test.

x = any amino acid. Seq. = Sequences.

Meme analysis of TRBV2 sequences in discriminative mode identified a complex motif with interspersed highly conserved positions in 79% of HIC sequences (**Figure 3B**). A predominant motif was apparent in HIC TRBV2 sequences, while HAART TRBV2 sequences appeared too diverse for motif identification (**Figure 9B**). Further analyses of HIC sequences revealed the overlap of two simpler motifs of 13 and 14 aa, respectively (**Table 2**). CDR3β motifs BV2-1 (ASSx[R/G/L][T/A][S/G]Gxx[E/T]Q[F/Y]) and BV2-2 (ASSx[R/G/L][T/A]SGGxx[E/T]Q[F/Y]) were highly similar and differed only by the presence of an additional G in the latter. Taken together, these two motifs were found in 50% of HIC versus 10% of HAART TRBV2 sequences (P<0.0001), indicating a predominance of conserved CDR3 residues in HIC clonotypes. This bias suggested a requirement of particular CDR3 residues for efficient recognition of the Gag293-MHC II complex (35). It was noteworthy that half of the TRAV24 and TRBV2 clonotypes identified in the HIC group shared the identified public motifs, indicative of a highly constrained Gag293-specific repertoire in controlled HIV infection.

***High frequency of TCR sharing in HIV Controllers***

Public clonotypes were defined as identical CDR3 aa sequences found in at least two individuals, without any mismatch tolerated. A total of 18 public clonotypes were identified in the TRAV24 sequence dataset obtained from the 16 patients studied (**Table 3A**). The most prevalent public clonotype, AFKAAGNKLT (called TRAV24-F), was found in 6 Controllers and 2 treated patients (75% and 25%, respectively), which are remarkably high frequencies in humans with diverse HLA II backgrounds. Groups of highly related public clonotypes were apparent, with a first group sharing the TRAJ17 chain and motif AxKAAGNKLT (AV24-1), and a second group sharing the TRAJ32 chain and motif AxYGGATNKLI (AV24-2). The 18 public clonotypes showed a high degree of motif sharing (61% positive for motifs AV24-1 or AV24-2, **Table 2**), confirming their similarity. Of note, public clonotypes were more frequent in the HIC than in the HAART group (P=0.03; **Figure 3C**). Counting the number of nucleotide sequences encoding public clonotypes, normalized to 100 sequences, confirmed the higher frequency of TCR sharing in the HIC group (P=0.01; **Figure 3E**).

Interestingly, a majority of TRAV24 public clonotypes (67%) were encoded by multiple nucleotide sequences. The mean number of unique nucleotide sequences encoding a TRAV24 public clonotype was 2.33, versus 1.05 for private (i.e. non-public) TRAV24 clonotypes. Detailed analysis of the 4 sequences coding for the most prevalent TRAV24 public clonotype, AFKAAGNKLT, showed that P and N mutations inserted between the TRAV24 and the TRAJ17 genes were few, ranging from 0 to 4 nucleotides (**Figure 3G**). The numbers of nucleotides trimmed from germline sequences were also in the low range (6 to 10) compared to the ensemble of HIC sequences (mean of 9.9 trimmed nucleotides). The two predominant sequences, representing 64.9% and 33.8% of AFKAAGNKLT coding sequences, respectively, contained no mutation, and were simply obtained by trimming 6 nucleotides from joined TRAV24-TRAJ17 genes. Thus, the most prevalent TRAV24 public clonotype could be easily generated from germline sequences, suggestive of convergent V(D)J recombination (36).

***Table 3: Distribution of Gag293-specific public clonotypes***

The number of nucleotide sequences coding for a TRAV24 (A) or TRBV2 (B) public clonotype is reported for each patient studied. The number of HIC and HAART patients sharing a given public clonotype is reported in the last two columns. HIC: HIV Controler; HAART or HAA: patient receiving highly active antiretroviral therapy. The tetramer used for sorting (DR1, DR11, DR15, or DRB5) is reported below each patient code.

| *A- TRAV24 Public Clonotypes* | | | HIC1 DR15 | HIC2 DRB5 | HIC3 DR11 | HIC4 DR15 | HIC5 DR1 | HIC6 DR11 | HIC7 DRB5 | HIC8 DR1 | HAA1 DRB5 | HAA2 DRB5 | HAA3 DR1 | HAA4 DR11 | HAA5 DR1 | HAA6 DR1 | HAA7 DR11 | HAA8 DRB5 | **Nb of HIC** | **Nb of HAART** |
|---|---|---|---|---|---|---|---|---|---|---|---|---|---|---|---|---|---|---|---|---|
| TRAJ17 | **CAFKAAGNKLTF** | tgtgcctttaaagctgcaggcaacaagctaactttt | 2 | 35 | 8 | | | 11 | | | | | | 59 | | | 37 | | **6** | **2** |
| | | tgtgcctttaaggctgcaggcaacaagctaactttt | 1 | | | | 1 | | | | | | | | | | | | | |
| | | tgtgccttcaaagctgcaggcaacaagctaactttt | | 2 | 64 | | | 2 | 10 | | | | | 1 | | | | | | |
| | | tgtgcatttaaagctgcaggcaacaagctaactttt | | | | | | 1 | | | | | | | | | | | | |
| | **CAFRAAGNKLTF** | tgtgccttcagggctgcaggcaacaagctaactttt | | | 2 | | | | | | | | | | | | | | **4** | **1** |
| | | tgtgcctttagagctgcaggcaacaagctaactttt | | | | 1 | | | | | | | | | | | | | | |
| | | tgtgcctttcgagctgcaggcaacaagctaactttt | | | | | 2 | | | | | | | | | 3 | | | | |
| | | tgtgcctttagggctgcaggcaacaagctaactttt | | | | | | 1 | | | | | | | | | | | | |
| | **CAHKAAGNKLTF** | tgtgcccacaaagctgcaggcaacaagctaactttt | | 2 | | | | | 3 | | | | | | | | | | **2** | **0** |
| | | tgtgcccataaagctgcaggcaacaagctaactttt | | 1 | | | | | | | | | | | | | | | | |
| | **CALKAAGNKLTF** | tgtgccttgaaagctgcaggcaacaagctaactttt | | | 1 | | | | | | | | | | | | | | **2** | **1** |
| | | tgtgccttaaaagctgcaggcaacaagctaactttt | | | | | | | | | | | | 1 | | | | | | |
| | | tgtgctctaaaagctgcaggcaacaagctaactttt | | | | | | 6 | | | | | | | | | | | | |
| | **CASKAAGNKLTF** | tgtgcctctaaagctgcaggcaacaagctaactttt | | 13 | | | | | | | | | | | | | | | **2** | **0** |
| | | tgtgcctccaaagctgcaggcaacaagctaactttt | | | 4 | | | | | | | | | | | | | | | |
| | **CSRRAAGNKLTF** | tgtagtcggagggctgcaggcaacaagctaactttt | | | 18 | | | | | | | 10 | | | | | | | **1** | **1** |
| TRAJ32 | **CAEYGGATNKLIF** | tgtgccgaatatggtggtgctacaaacaagctcatcttt | | | | 2 | 1 | | 3 | | | | | | | | | | **3** | **0** |
| | **CANYGGATNKLIF** | tgtgccaactatggcggtgctacaaacaagctcatcttt | | | | | | | 2 | 5 | | | | | | | | | **2** | **0** |
| | **CAPYGGATNKLIF** | tgtgccccttatggtggtgctacaaacaagctcatcttt | | | | 2 | | | 1 | | | | | | | | | | **2** | **1** |
| | | tgtgctccttatggcggtgctacaaacaagctcatcttt | | | | | | | 1 | | | | | | | | | | | |

| | | | | | | | | | | | | | | | | | | | | |
|---|---|---|---|---|---|---|---|---|---|---|---|---|---|---|---|---|---|---|---|---|
| | | tgtgccccgtatggtggtgctacaaacaagctcatcttt | | | | | | | | | | | | | | | | 1 | | |
| | **CARYGGATNKLIF** | tgtgcccgttatggtggtgctacaaacaagctcatcttt | | | | 10 | | | | | | | | | | | | | **1** | **1** |
| | | tgtgctcgttatggtggtgctacaaacaagctcatcttt | | | | 1 | | | | | | | | | | | | | | |
| | | tgtgctcggtatggtggtgctacaaacaagctcatcttt | | | | | | | | | | | | | | | | 26 | | |
| | **CASYGGATNKLIF** | tgtgcctcttatggtggtgctacaaacaagctcatcttt | 8 | | | 1 | | | 1 | | | | | | | | | | **3** | **0** |
| | | tgtgcctcctatggtggtgctacaaacaagctcatcttt | | | | 25 | | | 1 | | | | | | | | | | | |
| | | tgtgcctcgtatggtggtgctacaaacaagctcatcttt | | | | 2 | | | | | | | | | | | | | | |
| | | tgtgcctcctatggcggtgctacaaacaagctcatcttt | | | | | | | 2 | | | | | | | | | | | |
| TRAJ38 | **CAFDNRKLIW** | tgtgccttcgacaaccgtaagctgatttgg | | | | | 31 | | | | 37 | | | | | | | | **1** | **1** |
| | | tgtgcctttgacaaccgtaagctgatttgg | | | | | 2 | | | | | | | | | | | | | |
| TRAJ39 | **CAFRNAGNMLTF** | tgtgcctttcgtaatgcaggcaacatgctcaccttt | | 1 | | | | | | | | | | | | | | | **2** | **2** |
| | | tgtgcctttcggaatgcaggcaacatgctcaccttt | | | | | | 1 | | | | | | | | | | | | |
| | | tgtgcctttaggaatgcaggcaacatgctcaccttt | | | | | | | | | | | | 4 | | | | | | |
| | | tgtgcctttagaaatgcaggcaacatgctcaccttt | | | | | | | | | | | | | | | 10 | | | |
| | **CALNNAGNMLTF** | tgtgccctcaataatgcaggcaacatgctcaccttt | | | | | | 1 | | | | | | | | | | | **1** | **1** |
| | | tgtgccttaaataatgcaggcaacatgctcaccttt | | | | | | | | | | | | | | 24 | | | | |
| | **CALRNAGNMLTF** | tgtgccctgcggaatgcaggcaacatgctcaccttt | | | | | | 3 | | | | | | | | | | | **1** | **1** |
| | | tgtgccttaagaaatgcaggcaacatgctcaccttt | | | | | | | | | | | | 1 | | | | | | |
| | | tgtgccttacgaaatgcaggcaacatgctcaccttt | | | | | | | | | | | | 12 | | | | | | |
| | **CATRRAGNMLTF** | tgtgcgacgcgccgtgcaggcaacatgctcaccttt | | | | 6 | 5 | | | | | | | | | | | | **2** | **0** |
| TRAJ54 | **CASESTGAQKLVF** | tgtgcctccgagtccacgggagcccagaagctggtattt | | | | 3 | 1 | | | | | | | | | | | | **2** | **0** |
| TRAJ57 | **CAYEGASEKLVF** | tgtgcctacgagggggcatctgaaaagctggtcttt | | | | 3 | 1 | | | | | | | | | | | | **2** | **0** |
| | | | | | | | | | | | | | | | | | | | | |
| ***B- TRBV2 Public Clonotypes*** | | | HIC 1 DR 15 | HIC 2 DR B5 | HIC 3 DR 11 | HIC 4 DR 15 | HI C5 DR 1 | HIC 6 DR 11 | HIC 7 DR B5 | HI C8 DR 1 | HA A1 DR B5 | HA A2 DR B5 | HA A3 DR 1 | HA A4 DR 11 | HA A5 DR 1 | HA A6 DR 1 | HA A7 DR 11 | HA A8 DR B5 | **N b of HI C** | **Nb of HAA RT** |
| TRBJ1-1 | **CASSGQTNTEAFF** | tgtgccagctcaggacagacgaacactgaagctttcttt | | | 10 | | | | | | | | | | | | 2 | | **1** | **1** |
| TRBJ1-5 | **CASSEGAAGNQPQHF** | tgtgccagcagtgaaggggcggctggcaatcagccccagcatttt | | | | | | 1 | | | | | | | | | | | **1** | **1** |
| | | tgtgccagcagcgagggggcggcgggcaatcagccccagcatttt | | | | | | | | | | | | 1 | | | | | | |

| | | | | | | | | | | | | | | | | | | | | |
|---|---|---|---|---|---|---|---|---|---|---|---|---|---|---|---|---|---|---|---|---|
| TRBJ2-1 | **CASSELTSGGDEQFF** | tgtgccagcagtgagttgactagcgggggggatgagcagttcttc | | | | | | | 1 | 63 | | | | | | | | | **2** | **0** |
| | **CASSPGTSGVGEQFF** | tgtgcaagcagccccgggactagcggagttggtgagcagttcttc | | | 15 | | | | | | | | | | | | | | **2** | **0** |
| | | tgtgcaagcagccccgggactagcggagttggtgagcagtttttc | | | 1 | | | | | | | | | | | | | | | |
| | | tgtgccagcagtcccgggactagcggggttggtgagcagttcttc | | | | | | 2 | | | | | | | | | | | | |
| | **CASVLMRTNNEQFF** | tgtgccagtgtattaatgaggacgaacaatgagcagttcttc | | | | 9 | 4 | | | | | | | | | | | | **2** | **0** |
| TRBJ2-3 | **CASSALASGTDTQYF** | tgtgccagcagtgcgttagctagcggtacagatacgcagtatttt | 1 | | | | | | | | | | | | | | | | **2** | **1** |
| | | tgtgccagcagtgctttggctagcggcacagatacgcagtatttt | | | 1 | | | | | | | | | | | | | | | |
| | | tgtgccagcagtgccctggctagtggcacagatacgcagtatttt | | | | | | | | | | | | 2 | | | | | | |
| | **CASSARTSGGADTQYF** | tgtgccagcagtgcaaggactagcgggggggccgatacgcagtatttt | | | | 1 | | | | | | | | | | | | | **2** | **0** |
| | | tgtgccagcagtgccaggactagcgggggggcagatacgcagtatttt | | | | | | | 1 | | | | | | | | | | | |
| | | tgtgccagcagtgctaggactagcgggggggcagatacgcagtatttt | | | | | | | 1 | | | | | | | | | | | |
| | **CASSARTSGGSDTQYF** | tgtgccagcagtgccaggactagcgggggctcggatacgcagtatttt | 1 | | | | | | | | | | | | | | | | **3** | **0** |
| | | tgtgccagcagtgctcggactagcggggggtcagatacgcagtatttt | | | | 3 | | | | | | | | | | | | | | |
| | | tgtgccagcagcgcccggactagcggggggtcagatacgcagtatttt | | | | | | | 1 | | | | | | | | | | | |
| | | tgtgccagcagtgccaggactagcggggggtcagatacgcagtatttt | | | | | | | 2 | | | | | | | | | | | |
| | **CASSARTSGGTDTQYF** | tgtgccagcagtgccaggactagcggaggcacagatacgcagtatttt | | | | | | | 2 | 1 | | | | | | | | | **2** | **0** |
| | | tgtgccagcagtgctaggactagcgggggcacagatacgcagtatttt | | | | | | | 1 | | | | | | | | | | | |
| | **CASSHRASGGDTQYF** | tgtgccagcagtcacagggcctcaggcggggatacgcagtatttt | | | | 31 | 1 | | | | | | | | | | | | **2** | **0** |
| | **CASSLRTSGGSDTQYF** | tgtgccagcagcctaaggactagcgggggttcagatacgcagtatttt | 1 | | | | | | | | | | | | | | | | **1** | **1** |
| | | tgtgccagcagccttaggactagcgggggttcagatacgcagtatttt | | | | | | | | | | | | | | | | 1 | | |
| | **CASSLRTSGGTDTQYF** | tgtgccagcagtcttaggactagcgggggcacagatacgcagtatttt | | | | 1 | | | | | | | | | | | | | **1** | **1** |
| | | tgtgccagcagtctccggactagcgggggcacagatacgcagtatttt | | | | | | | | | | | | | | | | 2 | | |
| | | tgtgccagcagtcttcggactagcgggggaacagatacgcagtatttt | | | | | | | | | | | | | | | | 1 | | |
| | **CASSPLTSGTDTQYF** | tgtgccagcagtccgttgactagcggaacagatacgcagtatttt | | 1 | | | | | | | | | | | | | | | **1** | **1** |
| | | tgtgccagcagtccattgactagcggcacagatacgcagtatttt | | | | | | | | | | | | 1 | | | | | | |
| | **CASSRRTSGGTDTQYF** | tgtgccagcagtcgtaggactagcgggggcacagatacgcagtatttt | | 6 | | | | | | | | | | | | | | | **4** | **0** |
| | | tgtgccagcagtcgacggactagcgggggcacagatacgcagtatttt | | | | 1 | | | | | | | | | | | | | | |
| | | tgtgccagctcacgacggactagcgggggcacagatacgcagtatttt | | | | 1 | | | | | | | | | | | | | | |
| | | tgtgccagcagccgccggactagcgggggcacagatacgcagtatttt | | | | | | 12 | | | | | | | | | | | | |

| | | | | | | | | | | | | | | | | | | | | |
|---|---|---|---|---|---|---|---|---|---|---|---|---|---|---|---|---|---|---|---|---|
| | | tgtgccagcagtcggaggactagcgggggcacagatacgcagtatttt | | | | | | | 1 | | | | | | | | | | | |
| TRBJ2-7 | CASSKLASGADEQYF | tgtgccagctcaaaactggctagcggggccgacgagcagtacttc | | | | 6 | 1 | | | | | | | | | | | | 2 | 0 |
| | | tgtgccagctcaaaactggctagcggggccgacgagcagtatttc | | | | 1 | | | | | | | | | | | | | | |
| | CASSPRTSGGDEQYF | tgtgccagcagtccccggactagcgggggcgacgagcagtacttc | | | | | | | 1 | | | | | | | | | | 1 | 1 |
| | | tgtgccagcagcccccggactagcggggggacgagcagtacttc | | | | | | | | | | | | | | | | 1 | | |
| | CASSPRTSGTYEQYF | tgtgccagcagtccccggactagcggtacctacgagcagtacttc | | | | 1 | | | | | | | | | | | | | 1 | 1 |
| | | tgtgccagcagtcctaggactagcggaacctacgagcagtacttc | | | | | | | | | | | | | | | | 1 | | |
| | CASSRRTSGTYEQYF | tgtgccagcagccgtaggactagcgggacttacgagcagtacttc | 1 | | | | | | | | | | | | | | | | 2 | 0 |
| | | tgtgccagcagtagacggactagcgggacctacgagcagtacttc | | | | 1 | | | | | | | | | | | | | | |

Public clonotypes were also abundant in Gag293-specific TCRβ chains, with a total of 18 found in the TRBV2 sequence dataset (**Table 3B**). Public clonotype sequences derived predominantly from the TRBJ2-3, TRBJ2-1, and TRBJ2-7 genes, which were all more represented in HIC than HAART sequences. TRBV2 public clonotypes showed a remarkably high degree of motif sharing (83%, **Table 2**), emphasizing the presence of conserved features in spite of relatively high sequence diversity. Again, TRBV2 public clonotypes were clearly more frequent in the HIC than the HAART group, in terms of aa sequences (P=0.006; **Figure 3D**) and of nucleotide sequences (P=0.009; **Figure 3F**). The most frequent public clonotype, ASSRRTSGGTDTQY (called TRBV2-13), was found in 4/8 Controllers (50%) and absent from treated patients. The majority of TRBV2 public clonotypes (77%) were encoded by more than one nucleotide sequence. The mean number of unique nucleotide sequences encoding a TRBV2 public clonotype was 2.28, versus 1.10 for private TRBV2 clonotypes, again suggestive of convergent V(D)J recombination contributing to the amplification of Gag293-specific public clonotypes. Taken together, TRAV24 and TRBV2 sequence analyses revealed a highly biased Gag293-specific TCR repertoire, characterized by a high degree of TCR sharing in the Controller group. TCR biases were of type III (identical CDR3 sequences), but also of type of type II (conserved CDR3 motifs) (35, 37), and concerned a significant fraction of both TCR chains.

It was noteworthy that the majority of public clonotypes were restricted by multiple HLA DR alleles. Specifically, 83% of TRAV24 public clonotypes and 56% of TRBV2 public clonotypes were identified in samples sorted with at least 2 different HLA DR tetramers (**Table 3, A** and **B**). The most prevalent TRAV24 public clonotype, TRAV24-F, was restricted by the 4 HLA DR alleles tested (DR1, DR11, DR15, DRB5), while the most prevalent TRBV2 public clonotype, TRBV2-13, was restricted by 3 of these alleles (DR1, DR15, and DRB5). Thus, highly prevalent public clonotypes showed a high level of HLA cross-restriction, which could help explain their prevalence in patients of diverse genetic backgrounds.

We next evaluated the degree of overlap of the Gag293-specific clonotypic repertoires restricted by two distinct HLA DR alleles in a same patient. This intrapatient comparison was done for 3 HIV Controllers, for whom two cell lines sorted with different tetramers (DR15/DRB5, or DR11/DRB5) were analyzed in parallel (**Table 8**). These experiments revealed a consistent but moderate degree of clonotypic repertoire overlap, with a mean of 12% TRAV24 clonotypes and 11% of TRBV2 clonotypes shared between the two Tet+ samples (**Table 9**). However, the shared clonotypes were highly expressed, as they represented a mean of 44% TRAV24 sequences and 27% TRBV2 sequences analyzed. Public clonotypes were disproportionately represented among shared clonotypes, with a mean of 83% of shared TRAV24 clonotypes and 47% of shared TRBV2 clonotypes. Thus, while the majority of Gag293-specific clonotypes did not show cross-restriction, the subset of clonotypes restricted by multiple HLA DR alleles appeared dominant and more frequently public.

**Table 8: Intrapatient comparison of Gag293-specific clonotypic repertoires obtained with different MHC II tetramers**

The Gag293-specific repertoire was compared in two cell lines obtained from the same patient but sorted with two distinct MHC II tetramers.
The TRAV24 clonotypes (A) and TRBV2 clonotypes (B) shared between the two tetramer+ samples are highlighted in grey.
Comparisons are shown for 3 HIV Controllers. Public clonotypes are in bold type. Clonotypes tested functionally are marked by an asterisk.

**A- TRAV24 clonotypes (SEQ ID NO: 1060 to SEQ ID NO: 1142)**

| HIC 1 DR15+ | | | |
|---|---|---|---|
| **V-GENE** | **J-GENE** | **JUNCTION (AA)** | **% nt seq** |
| TRAV24 | TRAJ38*01 | CARDDRKLIW | 1,89 |
| TRAV24 | TRAJ34*01 | CASGNTDKLIF | 1,89 |
| TRAV24 | TRAJ39*01 | CAFCNAGNMLTF | 1,89 |
| TRAV24 | TRAJ17*01 | **CAFKAAGNKLTF*** | 5,66 |
| TRAV24 | TRAJ17*01 | CAFTAAGNKLTF | 1,89 |
| TRAV24 | TRAJ17*01 | CALENAGNKLTF | 1,89 |
| TRAV24 | TRAJ39*01 | CALGNAGNMLTF | 64,15 |
| TRAV24 | TRAJ57*01 | CSPQGGSEKLVF | 1,89 |
| TRAV24 | TRAJ6*01 | CAFIPGGSYIPTF | 1,89 |
| TRAV24 | TRAJ42*01 | CASDGGSQGNLIF | 1,89 |
| TRAV24 | TRAJ32*02 | **CASYGGATNKLIF** | 15,09 |
| Total | | 11 | 100,00 |

| HIC1 DRB5+ | | | |
|---|---|---|---|
| **V-GENE** | **J-GENE** | **JUNCTION (AA)** | **% nt seq** |
| TRAV24 | TRAJ34*01 | CAFDNTDKLIF | 6,82 |
| TRAV24 | TRAJ17*01 | **CAFKAAGNKLTF*** | 11,36 |
| TRAV24 | TRAJ39*01 | CALGNAGNMLTF | 4,55 |
| TRAV24 | TRAJ17*01 | CSFKAAGNKLTF | 2,27 |
| TRAV24 | TRAJ17*01 | CSFKGAGNKLIF | 2,27 |
| TRAV24 | TRAJ57*01 | CSPQGGCEKLVF | 2,27 |
| TRAV24 | TRAJ6*01 | CAFIPGGSYIPTF | 2,27 |
| TRAV24 | TRAJ32*02 | CALYGGATNKLIF | 2,27 |
| TRAV24 | TRAJ32*02 | CASYGGAPHDLIF | 2,27 |
| TRAV24 | TRAJ32*02 | CASYGGATKILFV | 2,27 |
| TRAV24 | TRAJ32*02 | **CASYGGATNKLIF** | 61,37 |
| Total | | 11 | 100,00 |

| HIC 3 DR11+ | | | |
|---|---|---|---|
| **V-GENE** | **J-GENE** | **JUNCTION (AA)** | **% nt seq** |
| TRAV24 | TRAJ17*01 | CAPKSRQQANF | 0,64 |
| TRAV24 | TRAJ17*01 | **CAFKAAGNKLTF*** | 46,16 |
| TRAV24 | TRAJ17*01 | **CAFRAAGNKLTF** | 1,28 |
| TRAV24 | TRAJ17*01 | **CALKAAGNKLTF** | 0,64 |
| TRAV24 | TRAJ39*01 | CALKQAGNKLTF | 0,64 |
| TRAV24 | TRAJ39*01 | CALRQAGNMLTF | 3,85 |
| TRAV24 | TRAJ17*01 | CAMKAAGNKLTF | 1,28 |
| TRAV24 | TRAJ17*01 | CANRAAGNKLTF | 0,64 |
| TRAV24 | TRAJ17*01 | **CASKAAGNKLTF*** | 2,56 |
| TRAV24 | TRAJ17*01 | CAYKAAGNKLTF | 0,64 |
| TRAV24 | TRAJ17*01 | CGFKAAGNKLTF | 0,64 |
| TRAV24 | TRAJ17*01 | CGWKAAGNKLTF | 0,64 |
| TRAV24 | TRAJ17*01 | CSLKAAGNKLTF | 1,28 |
| TRAV24 | TRAJ17*01 | CSLRAAGNKLTF | 0,64 |
| TRAV24 | TRAJ17*01 | **CSRRAAGNKLTF*** | 11,54 |
| TRAV24 | TRAJ17*01 | CSWKAAGNKLTF | 2,56 |
| TRAV24 | TRAJ17*01 | CSWRAAGNKLTF | 1,28 |
| TRAV24 | TRAJ17*01 | CTKKAAGNKLTF | 21,15 |
| TRAV24 | TRAJ17*01 | CTLKAAGNKLTF | 0,64 |
| TRAV24 | TRAJ17*01 | CTNKAAGNKLTF | 0,64 |
| TRAV24 | TRAJ17*01 | CTRKAAGNKLTF | 0,64 |
| Total | | 22 | 100,00 |

| HIC 3 DRB5+ | | | |
|---|---|---|---|
| **V-GENE** | **J-GENE** | **JUNCTION (AA)** | **% nt seq** |
| TRAV24 | TRAJ17*01 | **CALKAAGNKLTF** | 1,79 |
| TRAV24 | TRAJ17*01 | CALRAAGNKLTF | 1,79 |
| TRAV24 | TRAJ17*01 | CALRAAGNMLTF | 3,58 |
| TRAV24 | TRAJ39*01 | CALRQAGNMLTF | 62,50 |
| TRAV24 | TRAJ17*01 | CASQAAGNKLTF | 1,79 |
| TRAV24 | TRAJ17*01 | **CSRRAAGNKLTF*** | 28,57 |
| Total | | 6 | 100,00 |

| HIC 7 DRB5+ | | | |
|---|---|---|---|
| **V-GENE** | **J-GENE** | **JUNCTION (AA)** | **% nt seq** |
| TRAV24 | TRAJ20*01 | CAFGDYKLSF | 3,64 |
| TRAV24 | TRAJ17*01 | CAFHAAGNKLTF | 1,82 |
| TRAV24 | TRAJ17*01 | **CAFKAAGNKLTF*** | 18,18 |
| TRAV24 | TRAJ10*01 | CAFKGGGNKLTF | 1,82 |
| TRAV24 | TRAJ17*01 | CAFNAAGNKLTF | 5,45 |
| TRAV24 | TRAJ17*01 | CAFQAAGNKLTF | 16,36 |
| TRAV24 | TRAJ10*01 | CAFRGGGNKLTF | 20,00 |
| TRAV24 | TRAJ39*01 | CAFRRAGNMLTF | 1,82 |
| TRAV24 | TRAJ17*01 | **CAHKAAGNKLTF** | 5,45 |
| TRAV24 | TRAJ17*01 | CAHKGAGNKLTF | 1,82 |
| TRAV24 | TRAJ32*02 | **CAEYGGATNKLIF** | 5,45 |
| TRAV24 | TRAJ32*01 | **CANYGGATNKLIF** | 3,64 |
| TRAV24 | TRAJ57*01 | CAPGGGGSEKLVF | 3,64 |
| TRAV24 | TRAJ32*02 | **CAPYGGATNKLIF** | 3,64 |
| TRAV24 | TRAJ32*01 | **CASYGGATNKLIF** | 7,28 |
| Total | | 15 | 100,00 |

| HIC 7 DR1502+ | | | |
|---|---|---|---|
| **V-GENE** | **J-GENE** | **JUNCTION (AA)** | **% nt seq** |
| TRAV24 | TRAJ31*01 | CAFDNARLMF | 1,03 |
| TRAV24 | TRAJ31*01 | CASYGAALMF | 1,03 |
| TRAV24 | TRAJ36*01 | CAYGANNLFF | 1,03 |
| TRAV24 | TRAJ34*01 | CAFDNADKLIF | 1,03 |
| TRAV24 | TRAJ34*01 | CAFDNTDKLIF | 2,06 |
| TRAV24 | TRAJ39*01 | CAFCNAGNMLTF | 1,03 |
| TRAV24 | TRAJ17*01 | **CAFKAAGNKLTF*** | 21,65 |
| TRAV24 | TRAJ39*01 | CALGNAGNMLTF | 5,15 |
| TRAV24 | TRAJ17*01 | **CALKAAGNKLTF** | 2,06 |
| TRAV24 | TRAJ17*01 | **CASKAAGNKLTF*** | 40,21 |
| TRAV24 | TRAJ57*01 | CSPQGGSEKLVF | 2,06 |
| TRAV24 | TRAJ36*01 | CVFQTGGNNLFF | 1,03 |
| TRAV24 | TRAJ32*02 | CACYGGATNKLIF | 1,03 |
| TRAV24 | TRAJ15*01 | CAFDNQAANNLIF | 1,03 |
| TRAV24 | TRAJ15*01 | CAFDNQAGTALIF | 3,09 |
| TRAV24 | TRAJ32*02 | **CASYGGATNKLIF** | 12,37 |
| TRAV24 | TRAJ42*01 | CAYCGGSPNNLIF | 1,03 |
| TRAV24 | TRAJ42*01 | CASDYGGSQGNLIF | 1,03 |
| TRAV24 | TRAJ42*01 | CASYDGGTQGNLIV | 1,03 |
| Total | | 19 | 100,00 |

**B- TRBV2 clonotypes (SEQ ID NO: 1143 to SEQ ID NO: 1254)**

| HIC 1 DR15+ | | | |
|---|---|---|---|
| **J-GENE** | **D-GENE** | **JUNCTION (AA)** | **% nt seq** |
| TRBJ2-7*01 | TRBD1*01 | CASLGPLRHEQYF | 3,33 |
| TRBJ2-3*01 | TRBD2*01 | CASKPLVSTDTQYF | 1,67 |
| TRBJ2-1*01 | TRBD2*01 | CASLERTSGGEQFF | 1,67 |
| TRBJ2-1*01 | TRBD1*01 | CASTRDRTKNEQFF | 1,67 |
| TRBJ2-1*01 | TRBD2*01 | CASSALASGGDEQFF | 1,67 |
| TRBJ2-3*01 | TRBD2*01 | **CASSALASGTDTQYF** | 1,67 |
| TRBJ2-7*01 | TRBD2*01 | CASSELTSRTYEQYF | 1,67 |
| TRBJ1-5*01 | TRBD2*01 | CASSERVSGNQPQHF | 3,33 |
| TRBJ2-1*01 | TRBD2*01 | CASSPMASGGDEQFF | 1,67 |
| TRBJ2-7*01 | TRBD2*02 | **CASSRRTSGTYEQYF** | 1,67 |
| TRBJ2-1*01 | TRBD2*01 | CASSVMASRGNEQFF | 1,67 |
| TRBJ1-5*01 | TRBD2*01 | CASQRGARGGNQPQHF | 1,67 |
| TRBJ1-4*01 | TRBD1*01 | CASRARTGATNEKLFF | 1,67 |
| TRBJ2-3*01 | TRBD2*01 | CASSAKTSGGSDTQYF | 1,67 |
| TRBJ2-3*01 | TRBD2*01 | CASSALASGGRDTQYF | 1,67 |
| TRBJ2-1*01 | TRBD2*01 | CASSARTSGGLDEQFF | 1,67 |
| TRBJ2-3*01 | TRBD2*01 | **CASSARTSGGSDTQYF** | 1,67 |
| TRBJ2-3*01 | TRBD2*01 | CASSKRASGGTDTQYF | 3,33 |
| TRBJ2-3*01 | TRBD2*01 | **CASSLRTSGGSDTQYF** | 1,67 |
| TRBJ2-1*01 | TRBD2*01 | CASSRLTSGGRNEQFF | 3,33 |
| TRBJ2-3*01 | TRBD2*01 | CASSSKTSGGTDTQYF | 1,67 |
| TRBJ2-1*01 | TRBD2*01 | CASSSRTSGGQDEQFF | 1,67 |
| TRBJ1-5*01 | TRBD2*01 | CATSRGARGSNQPQHF | 56,67 |
| Total | | 23 | 100,00 |

| HIC 1 DRB5+ | | | |
|---|---|---|---|
| **J-GENE** | **D-GENE** | **JUNCTION (AA)** | **% nt seq** |
| TRBJ2-7*01 | TRBD2*01 | CACRIRTSGGEQYF | 1,92 |
| TRBJ2-4*01 | TRBD2*02 | CASARERTKNIQYF | 3,85 |
| TRBJ2-1*01 | TRBD2*01 | CASIPRTSGGLQFF | 1,92 |
| TRBJ2-3*01 | TRBD2*01 | CASKALVSTDTQYF | 1,92 |
| TRBJ2-3*01 | TRBD2*01 | CASKDRTSGDTQYF | 1,92 |
| TRBJ2-7*01 | TRBD2*01 | CASRIRTSGGEQYF | 5,77 |
| TRBJ2-5*01 | TRBD2*01 | CASRLLVSQETQYF | 1,92 |
| TRBJ2-7*01 | TRBD2*01 | CASSALAGVNEQFF | 1,92 |
| TRBJ2-3*01 | TRBD2*01 | CASSELVSGGLHRY | 1,92 |
| TRBJ2-7*01 | TRBD1*01 | CASSPTVNTYEQYF | 3,85 |
| TRBJ2-3*01 | TRBD1*01 | CASQLLVQHTDTQYF | 3,85 |
| TRBJ2-3*01 | TRBD2*01 | **CASSALASGTDTQYF** | 3,85 |
| TRBJ2-1*01 | TRBD2*01 | CASSPMASGGDEQFF | 1,92 |
| TRBJ2-7*01 | TRBD2*01 | **CASSPRTSGTYEQYF** | 5,77 |
| TRBJ2-7*01 | TRBD2*01 | CASSPWARGGDEQFF | 3,85 |
| TRBJ2-1*01 | TRBD2*01 | CASSRWASGGDEQFF | 3,85 |
| TRBJ2-1*01 | TRBD2*01 | CASSVMASRGNEQFF | 5,77 |
| TRBJ2-3*01 | TRBD2*01 | CASSARTSGGGDTQYF | 17,31 |
| TRBJ2-1*01 | TRBD2*01 | CASSARTSGGQDEQFF | 1,92 |
| TRBJ2-3*01 | TRBD2*01 | CASSARTSGGRDTQYF | 1,92 |
| TRBJ2-3*01 | TRBD2*01 | **CASSARTSGGSDTQYF** | 1,92 |
| TRBJ2-5*01 | TRBD2*01 | CASSLLTSGGRETQYF | 15,38 |
| TRBJ2-1*01 | TRBD2*01 | CASSRLTSGGRNEQFF | 1,92 |
| TRBJ2-3*01 | TRBD2*01 | CASSRRTSGGSDTQYF | 1,92 |
| TRBJ2-3*01 | TRBD2*01 | CASSSKTSGGTDTQYF | 1,92 |
| Total | | 25 | 100,00 |

| **HIC 3 DR11+** | | | |
|---|---|---|---|
| **J-GENE** | **D-GENE** | **JUNCTION (AA)** | **% nt seq** |
| TRBJ2-3*01 | TRBD1*01 | CASHRTYTDTQYF | 1,60 |
| TRBJ1-1*01 | TRBD1*01 | **CASSGQTNTEAFF** | 8,00 |
| TRBJ1-2*01 | TRBD1*01 | CASRWTATSYGYTF | 12,00 |
| TRBJ1-2*01 | TRBD1*01 | CASSPTTTGYGYTF | 0,80 |
| TRBJ2-2*01 | TRBD1*01 | CASHEGAGGFGELFF | 0,80 |
| TRBJ2-2*01 | TRBD1*01 | CASHEGAGGYGELFF | 1,60 |
| TRBJ2-1*01 | TRBD1*01 | CASSAGTRGVGEQFF | 0,80 |
| TRBJ2-3*01 | TRBD2*01 | **CASSALASGTDTQYF** | 0,80 |
| TRBJ2-7*01 | TRBD2*01 | CASSDAASGVGEQYF | 4,80 |
| TRBJ2-1*01 | TRBD2*02 | CASSDLASGTNEQFF | 0,80 |
| TRBJ2-1*01 | TRBD2*01 | CASSDRASGVGEQFF | 0,80 |
| TRBJ2-1*01 | TRBD2*01 | CASSDRTSGPHEQFF | 4,80 |
| TRBJ2-1*01 | TRBD2*02 | CASSGLAGGMDEQFF* | 5,60 |
| TRBJ2-1*01 | TRBD1*01 | CASSPGARGIDEQFF | 0,80 |
| TRBJ2-1*01 | TRBD2*01 | **CASSPGTSGVGEQFF*** | 12,80 |
| TRBJ2-1*01 | TRBD1*01 | CASSPGTSGVGKQFF | 0,80 |
| TRBJ2-7*01 | TRBD2*01 | CASSPRTSGGGEQYF | 0,80 |
| TRBJ1-5*01 | TRBD1*01 | CASSPSARGNQPQHF | 1,60 |
| TRBJ2-7*01 | TRBD2*02 | CASSPTTSGRGEQYF | 1,60 |
| TRBJ2-1*01 | TRBD2*01 | CASSSGTSGAGEQFF | 0,80 |
| TRBJ2-1*01 | TRBD2*01 | CASSSGTSGVGEQFF | 27,20 |
| TRBJ2-7*01 | TRBD2*01 | CASSVGTSGVGEQYF | 8,80 |
| TRBJ2-7*01 | TRBD2*01 | CASSYGASGVGEQFF | 0,80 |
| TRBJ2-1*01 | TRBD2*01 | CASSYRTSGPREQFF | 0,80 |
| Total | | 24 | 100,00 |

| **HIC 3 DRB5+** | | | |
|---|---|---|---|
| **J-GENE** | **D-GENE** | **JUNCTION (AA)** | **% nt seq** |
| TRBJ1-1*01 | TRBD1*01 | **CASSGQTNTEAFF** | 1,69 |
| TRBJ2-1*01 | TRBD2*02 | CASARLAGGTDEQFF | 35,59 |
| TRBJ2-3*01 | TRBD2*01 | CASSAMASGSDTQYF | 3,39 |
| TRBJ2-3*01 | TRBD2*01 | **CASSELASGTDTQYF** | 1,69 |
| TRBJ2-1*01 | TRBD2*01 | **CASSPGTSGVGEQFF*** | 45,76 |
| TRBJ1-5*01 | TRBD1*01 | CASSPSARGNQPQHF | 5,08 |
| TRBJ2-7*01 | TRBD2*01 | CASSVGTSGVGEQYF | 6,78 |
| Total | | 7 | 100,00 |

| HIC 7 DRB5+ | | | |
|---|---|---|---|
| **J-GENE** | **D-GENE** | **JUNCTION (AA)** | **% nt seq** |
| TRBJ2-7*01 | TRBD2*01 | CASSPRASGGEQYF | 3,85 |
| TRBJ1-4*01 | TRBD2*01 | CASSVRRNNEKLFF | 3,85 |
| TRBJ2-7*01 | TRBD2*01 | CASNRRTSGTYEQYF | 1,28 |
| TRBJ2-3*01 | TRBD2*01 | CASSALASGGDTQYF | 3,85 |
| TRBJ2-1*01 | TRBD2*01 | CASSARASGGDEQFF | 5,13 |
| TRBJ1-5*01 | TRBD1*01 | CASSARTSGGQPQHF | 5,13 |
| TRBJ1-5*01 | TRBD1*01 | CASSARTSGNQPQHF | 14,10 |
| TRBJ2-1*01 | TRBD2*02 | CASSELASGINEQFF | 6,41 |
| TRBJ2-1*01 | TRBD2*01 | **CASSELTSGGDEQFF** | 1,28 |
| TRBJ2-3*01 | TRBD2*02 | CASSKRTSGGDTQYF | 1,28 |
| TRBJ2-7*01 | TRBD2*01 | CASSLRTSGGDEQYF | 3,85 |
| TRBJ2-3*01 | TRBD2*01 | CASSPLTSATDTQYF | 1,28 |
| TRBJ2-1*01 | TRBD2*01 | CASSPRASGGDEQFF | 6,41 |
| TRBJ2-7*01 | TRBD2*01 | **CASSPRTSGGDEQYF** | 1,28 |
| TRBJ2-3*01 | TRBD2*01 | CASRLRTSGGTDTQYF | 2,56 |
| TRBJ2-3*01 | TRBD2*01 | CASRRLAGFMADTQYF | 1,28 |
| TRBJ2-3*01 | TRBD2*01 | CASSARTSAGTDTQYF | 7,69 |
| TRBJ2-3*01 | TRBD2*01 | CASSARTSGGADTHYF | 1,28 |
| TRBJ2-3*01 | TRBD2*01 | **CASSARTSGGADTQYF** | 2,56 |
| TRBJ2-3*01 | TRBD2*01 | **CASSARTSGGSDTQYF** | 3,84 |
| TRBJ2-3*01 | TRBD2*01 | **CASSARTSGGTDTQYF** | 3,84 |
| TRBJ2-3*01 | TRBD2*01 | CASSERTSGGRDTQYF | 1,28 |
| TRBJ2-3*01 | TRBD2*01 | CASSGRTSGGSDTQYF | 3,85 |
| TRBJ2-3*01 | TRBD2*01 | CASSLRASGGSDTQYF | 1,28 |
| TRBJ2-3*01 | TRBD2*01 | CASSLRTSGGADTQYF | 1,28 |
| TRBJ2-3*01 | TRBD2*01 | CASSQRTSGGADTQYF | 1,28 |
| TRBJ2-3*01 | TRBD2*01 | CASSRLTSGGSDTQYF | 5,13 |
| TRBJ2-3*01 | TRBD2*01 | CASSRRTSGGLDTQYF | 1,28 |
| TRBJ2-1*01 | TRBD2*01 | CASSRRTSGGPNEQFF | 1,28 |
| TRBJ2-3*01 | TRBD2*01 | **CASSRRTSGGTDTQYF*** | 1,28 |
| Total | | 30 | 100,00 |

| HIC 7 DR1502+ | | | |
|---|---|---|---|
| **J-GENE** | **D-GENE** | **JUNCTION (AA)** | **% nt seq** |
| TRBJ2-3*01 | TRBD2*01 | CASSALASGGDTQYF | 20,83 |
| TRBJ2-1*01 | TRBD2*02 | **CASSRLAGGMDEQFF*** | 56,94 |
| TRBJ2-3*01 | TRBD2*01 | **CASSRRTSGGTDTQYF*** | 22,22 |
| Total | | 3 | 100,00 |

**Table 9: Intrapatient comparisons of Gag293-specific clonotypic repertoires obtained with different MHC II tetramers - Summary**

The Gag293-specific repertoire was compared in cell lines obtained from the same patient but sorted with two distinct MHC II tetramers. Comparisons were made for 3 HIV Controllers (HIC1, HIC3, HIC7). Clonotype AA: unique CDR3 sequence, in amino acids.
The % of shared clonotypes AA is obtained from the number of shared clonotypes AA divided by the total number of clonotypes AA obtained for the two tetramer+ samples.
The % of shared sequences is obtained from the number of nt sequences corresponding to shared clonotypes AA divided by the total number of sequences obtained for the two tetramer+ samples.
The % of public clonotypes is computed among the shared clonotypes AA.

| Patient | Tetramers used | % shared TRAV24 clonotypes AA | % shared TRAV24 sequences | % public in shared TRAV24 clonotypes AA |
|---|---|---|---|---|
| HIC1 | DRB1*1501 / DRB5*0101 | 22,22 | 83,51 | 50 |
| HIC3 | DRB1*1101 / DRB5*0101 | 7,69 | 16,98 | 100 |
| HIC7 | DRB5*0101 / DRB1*1502 | 6,25 | 30,92 | 100 |
| **Mean** | | **12,05** | **43,80** | **83,33** |

| Patient | Tetramers used | % shared TRBV2 clonotypes AA | % shared TRBV2 sequences | % public in shared TRBV2 clonotypes AA |
|---|---|---|---|---|
| HIC1 | DRB1*1501 / DRB5*0101 | 11,63 | 12,5 | 40 |
| HIC3 | DRB1*1101 / DRB5*0101 | 14,81 | 40,22 | 50 |
| HIC7 | DRB5*0101 / DRB1*1502 | 6,45 | 28,57 | 50 |
| **Mean** | | **10,96** | **27,10** | **46,67** |

***Ex vivo detection of Gag293-specific public clonotypes in HIV Controllers***

The Gag293-specific TCR repertoire was initially analyzed in primary CD4+ T cell lines, as the preferential depletion of specific CD4+ T cells in HIV-infected patients did not allow a direct *ex vivo* analysis of Tet+ cells. However, the exceptional immunological status of HIV Controllers made the *ex vivo* analysis feasible for a subset of these patients. Four samples sorted from Controller PBMC containing >2,000 Tet+ cells were analyzed by Immunoscope and sequencing. The DRB5 tetramer was used in these experiments, as it yielded the best signal/noise ratio (example in **Figure 4A**). Quantification of TRAV24 expression showed an amplification of this family in 3/4 HIC samples obtained *ex vivo,* as compared to the percentage of TRAV24 detected in PBMC from 7 healthy donors (1.0 ± 0.2%) (**Figure 4B**). Quantification of TRBV2 showed an amplification in 4/4 HIC samples obtained *ex vivo,* as compared to PBMC from healthy donors (3.7 ± 1.0%) (**Figure 4B**).

The frequency of each TRAV24 clonotype found *ex vivo* was computed relative to total TRAV24 sequences found in the same patient (**Table 4, 3rd column**) and in the matched cell line (**4th column**). *Ex vivo* clonotypes represented in median 66% of sequences found in matched cell lines, indicating a large overlap between the *ex vivo* and *in vitro* repertoires (**Figure 4C**). Similarly, the TRBV2 clonotypes detected *ex vivo* (**Table 7**), represented in median 45% of sequences found in matched cell lines (**Figure 4C**). Importantly, the frequency of TRAV24 public motifs (AV24-1 + AV24-2) did not differ significantly between *ex vivo* and cell line derived sequences, and could be as high as 87% (**Figure 4D**). Frequencies of TRBV2 public motifs (BV2-1 + BV2-2) were also comparable *ex vivo* and in matched cell lines (**Figure 4E**). Public motifs were present at median values of 44% and 48% in the *ex vivo* TRAV24 and TRBV2 sequence datasets, respectively, emphasizing the high level of motif sharing in the Gag293-specific repertoire.

Detailed analysis of TRAV24 sequences obtained *ex vivo* revealed the presence of previously identified public clonotypes in each of the patient tested (highlighted in color, **Table 4**). Public clonotypes were present at median frequencies of 44% and 25% in *ex vivo* TRAV24 and TRBV2 sequences, respectively, which did not differ significantly from frequencies observed in matched cell lines (not shown). Overall, the *ex vivo* analysis confirmed the high degree of TCR sharing among HIV Controllers. It was striking that some of the most prevalent public clonotypes, such as AFKAAGNKLT, could represent more than half of TRAV24 sequences in the Gag293-specific repertoire (54.7% in HIC7). This suggested that public clonotypes could shape the properties of the Gag-specific response in controlled HIV infection.

**Table 4** Listing of TRAV24 clonotypes found in Gag293-specific CD4+ T cells sorted ex vivo. The representation of each clonotype in the TRAV24 sequence set obtained ex vivo (3rd column) and in the corresponding cell line (4th column) is reported (SEQ ID NO: 176 to 195).

| **HIC1 DRB5+ ex vivo (n=119 seq.)** | | | |
|---|---|---|---|
| **J-GENE** | **JUNCTION (AA)** | **% ex vivo** | **% in cell line** |
| TRAJ38*01 | CARDDRKLIW | 4,20 | - |
| TRAJ17*01 | CAFTAAGNKLTF | 8,40 | - |
| TRAJ39*01 | CALGNAGNMLTF | 27,73 | 4,65 |
| TRAJ6*01 | CAFIPGGSYIPTF | 3,36 | 2,33 |
| TRAJ32*02 | **CASYGGATNKLIF** | 21,85 | 62,79 |
| TRAJ45*01 | CALDSGGGADGLTF | 34,45 | - |

| **HIC2 DRB5+ ex vivo (n=56 seq.)** | | | |
|---|---|---|---|
| **J-GENE** | **JUNCTION (AA)** | **% ex vivo** | **% in cell line** |
| TRAJ17*01 | **CAFKAAGNKLTF** | 17,86 | 56,06 |
| TRAJ39*01 | CARRNAGNMLTF | 12,50 | - |
| TRAJ17*01 | **CASKAAGNKLTF** | 69,64 | 19,70 |

| **HIC3 DRB5+ ex vivo (n=76 seq.)** | | | |
|---|---|---|---|
| **J-GENE** | **JUNCTION (AA)** | **% ex vivo** | **% in cell line** |
| TRAJ17*01 | **CALKAAGNKLTF** | 6,58 | 1,79 |
| TRAJ39*01 | CALRQAGNMLTF | 93,42 | 62,50 |

| HIC7 DRB5+ ex vivo (n=106 seq) | | | |
|---|---|---|---|
| J-GENE | JUNCTION (AA) | % ex vivo | % in cell line |
| TRAJ13*02 | CAPGGYQKVTF | 4,72 | - |
| TRAJ22*01 | CAWGSARQLTF | 4,72 | - |
| TRAJ17*01 | **CAFKAAGNKLTF** | 54,72 | 18,18 |
| TRAJ17*01 | CAFNAAGNKLTF | 5,66 | 5,45 |
| TRAJ26*01 | CAFVAAGQNFVF | 4,72 | - |
| TRAJ32*02 | **CAEYGGATNKLIF** | 3,77 | 5,45 |
| TRAJ32*01 | **CANYGGATNKLIF** | 6,60 | 3,64 |
| TRAJ32*02 | **CASYGGATNKLIF** | 0,94 | 7,27 |
| TRAJ24*02 | CALMTTDSWGKLQF | 14,15 | - |

***TCR transfer confers Gag293 recognition in the context of multiple HLA-DR alleles***

We set to functionally characterize the most prevalent TRAV24 public clonotype AFKAAGNKLT (clonotype TRAV24-F) by pairing it with different TRBV2 chains and testing the activity of reconstituted TCRs. The chosen TRBV2 clonotypes n° 24 (CASSRLAGGMDEQFF) and 25 (CATTPGASGISEQFF) were the most abundant in cell line of patient HIC2 (10.38% and 33.96% of TRBV2 chains, respectively; **Table 7**), and were co-expressed at high levels with clonotype TRAV24-F, resulting in a high probability of functional TCR chain pairing. The third TRBV2 clonotype, n° 5 (CASSGLAGGMDEQFF), was derived from patient HIC3, who also expressed TRAV24-F at high level. Clonotype 5 differed by a single residue (R to G) from clonotype 24, and thus provided information on the contribution of the CDR3β arginine to TCR function. TRBV2 clonotypes 24 and 5 carried the public motif BV2-1, while TRBV2 clonotype 25 was private (see **Table 10** for a list of all TCR constructs). Full-length TRA and TRB genes were cloned into a T2A lentiviral vector, ensuring equimolar expression of the two chains. The resulting TCRs, F24, F25, and F5, were transduced in J76, a mutant Jurkat cell line defective for endogenous TCR expression, which provides a favorable cellular context for TCR transfer assays. After transduction, J76 cells expressed equivalent levels of the 3 TCRs at the cell surface (≥ 60% TCR+ cells), and recovered a high level of CD3 surface expression (**Figure 5A**).

To test the specificity of the transferred TCRs, transduced J76 were labeled with a panel of Gag293-loaded HLA-DR tetramers. The F24 TCR was found to bind 3 out of the 4 tetramer tested, with an efficiency that was higher for DR11 as compared to DRB5 and DR1 (**Figure 5B**). Quantification of tetramer binding (**Figure 5C**) showed that the three TCRs recognized Gag293 in the context of DR11, with a labeling that was strong for F24 (72% Tet+), intermediate for F25 (44% Tet+), and low for F5 (3% Tet+). F24 bound the DRB5 tetramer at intermediate levels (33% Tet+), while binding was weak for F25 (1.5%), and undetectable for F5. Binding with the DR1 tetramer showed a similar pattern. The DR4 tetramer provided a negative control, as it did not bind any of the TCRs tested. These experiments indicated that the highly prevalent public clonotype TRAV24-F could confer Gag293 recognition in the context of multiple HLA-DR molecules, with an efficiency that depended both on the presenting HLA-DR allele and on the nature of the TRBV2 chain.

Tetramer titration experiments showed the expected hierarchy between the 3 TCRs (F24>F25>F5) but did not prove sensitive enough to determine TCR avidity for F25 and F5 (**Figure 10**). To precisely determine TCR affinity, we expressed the F24, F25, and F5 TCRs as soluble recombinant proteins and measured their binding to Gag293-HLA-DR monomers by suface plasmon resonance (SPR). The soluble TCRs were passed over chips coated with immobilized DR11, DRB5, or DR1 monomers loaded with Gag293 (example of sensorgram in **Figure 5D**). In agreement with tetramer binding assays, the three TCRs recognized Gag293-HLA-DR complexes with different affinities (F24>F25>F5). This hierarchy was conserved across the different HLA-DR alleles tested (**Figure 5E**). Of note, the F24 TCR recognized the Gag293-DR11 complex with a very high affinity of 0.86 μM (**Figure 5F**), which is rarely seen for MHC II restricted TCRs (38). F24 bound the Gag293-DRB5 and Gag293-DR1 complexes with affinities that were three- and eight-fold lower, respectively, but still remained in the high affinity range (<10 μM). The F25 and F5 TCRs also interacted with the Gag293-DR11 complex with relatively high affinities (3 and 11 μM, respectively), while affinities were intermediate or low for the two other allomorphs tested. The percentage of Tet+ cells in TCR-transduced J76 cells, when measurable, correlated well with affinities measured by SPR (R=0.92; **Figure 5G**), consistent with the notion that tetramer binding tightly depends on TCR affinity. These results showed that association of the TRAV24-F public clonotype with highly expressed TRBV2 clonotypes could, for certain combinations, generate HLA cross-restricted TCRs of unusually high affinity.

***Transfer of TCRs containing public TRAV24 and TRBV2 clonotypes confers high antigen sensitivity to J76 cells***

TCR function was tested by monitoring the induction of the early activation marker CD69 in transduced J76 cells. Murine fibroblastic L cells engineered to express a single human HLA-DR allele were used as APC, allowing a precise control of the restricting HLA molecule. L cells expressing DR1, DR3, DR4, DR7, DR11, DR15, or DRB5 were pulsed with decreasing doses of Gag293 peptide and cocultured with J76 cells transduced with the F24, F25, and F5 TCRs (**Figure 6A**). The three TCRs could signal and trigger T cell activation, as indicated by the induction of CD69 in ≥50% of J76 cells in the presence of DR11 APC. The F24 TCR showed the broadest reactivity, as it induced strong T cell activation in the presence of DR11, DR15, DRB5, and DR1 APC, and weak but consistent T cell activation in the presence of DR7 APC (**Table 10**). When restricted by DR11, F24 antigen sensitivity reached $4x10^{-7}$ M as measured by the EC50 for CD69 induction, which represents a high sensitivity for a cell culture system devoid of costimulatory molecules. The F25 TCR did not react with DR7, and showed intermediate reactivity with the other four HLA-DR alleles. The F5 TCR showed the narrowest restriction, with an antigen sensitivity of $2x10^{-6}$ M in the presence of DR11 APC, low responses in the presence of DR15 and DRB5 APC, and undetectable responses in other cases. TCR affinities measured by SPR for the DR11, DRB5, and DR1 alleles correlated well with antigen sensitivities measured in transduced J76 (R=0.85, P=0.016; **Figure 6D**), suggesting that TCR affinity dictated TCR function.

Two other TRAV24 public clonotypes, TRAV24-S (ASKAAGNKLT) and TRAV24-RR (SRRAAGNKLT) conferred cross-restriction by 4 HLA-DR alleles when paired to TRBV2 chains n°24 and 5, respectively (**Table 10**), emphasizing that broad HLA cross-restriction was a frequent property of public clonotypes. We then tested the pairing of TRAV24-F with two TRBV2 public clonotypes: TRBV2-4 (ASSPGTSGVGEQF), shared by 2 Controllers, and TRBV2-13 (ASSRRTSGGTDTQY), the most prevalent TRBV2 clonotype, shared by 4 Controllers. The resulting TCRs, F4 and F13, were cross-restricted by 3 HLA-DR alleles (DR11, DR15 and DRB5) (**Figure 6B**). While F4 had relatively good antigen sensitivity ($10^{-6}$ M) when restricted DR11, it gave only low responses with the two other alleles. In contrast, F13 showed good antigen sensitivity with DR11 ($7.5x10^{-7}$ M) and intermediate sensitivity with the two other alleles (**Table 10**). F13, like F24, carried an R at position 5 of the TRBV2 CDR3 junction, which may contribute to the efficient detection of Gag293 in varied HLA backgrounds. Two TCRs derived from private clonotypes from treated patients were studied for comparison. TCRs HD5 and HY9 comprised the most prevalent TRAV24 and TRBV2 clonotypes from treated patients HAART3 and HAART6, respectively (**Table 10**). These TCRs yielded responses of medium avidity when restricted by DR15 and DR1 ($10^{-5}$ M ≤ EC50 ≤ $10^{-6}$ M), gave only low responses in the presence of DRB5 L cells, and did not react with DR11 L cells (**Figure 6C**). While these TCRs from treated patients could react with several HLA-DR alleles, their degree of cross-restriction appeared narrower than that observed for Controller TCRs. Taken together, the analysis of recombinant TCRs specific for Gag293 highlighted that the most prevalent public clonotypes conferred efficient Gag293 recognition in the context of multiple HLA-DR alleles. Interestingly, antigen sensitivity measured for the 8 TCR tested in the presence of DR11 correlated with the number of HLA-DR alleles restricting each of these TCRs (R=-0.87, P=0.002; Figure 6E), suggesting that both properties depended on the same TCR features.

We next verified that the recombinant TCRs could recognize native HIV capsid antigens naturally processed by APC, in addition to peptide-pulsed APC. To this goal, we used infected monocyte-derived dendritic cells (MDDC) as alternative APC. MDDC from DR11+ healthy donors were infected with an HIV-1 pseudotyped virus and incubated with J76 cells expressing the F24, F25, or F5 TCR. The TCR-transduced cells showed robust CD69 induction in the presence of infected MDDC, while CD69 expression remained moderate in the absence of infection (**Figure 6F**). CD69 induction followed the same hierarchy as that observed when L cells were used as APC (F24>F25>F5), indicating that TCR properties were conserved in different antigen presentation systems.

As the public clonotypes consistently generated TCRs with optimal antigen sensitivity in a DR11 context, it was important to verify that differences in antigen sensitivity between the HIC and the HAART group did not depend on a bias in DR11 expression. Among the 29 patients included in the study, 3 Controllers and 4 treated patients expressed a DR11 allele, including HLA DRB1*1101, DRB1*1122, and DRB1*1165 (**Table 5**). Comparison of the last Gag293 peptide dilution that induced a specific cell line showed that, among DR11 patients, 3 out of 3 Controllers and 1 in 4 treated patient responded at a dilution <10-5 M (**Figure 11A**). Trends for a higher Gag293-specific ELISPOT response and for more prominent TRAV24 biases were also noted in DR11 Controllers as compared to DR11 treated patients (**Figure 11B and C**). Taken together, these findings support the notion that particular clonotypes expressed in controlled HIV infection, rather than mere DR11 expression, are associated with high avidity Gag293-specific responses.

Table **5: MHC typing and clinical characteristics of patients included in the study** Primary CD4+ T cell lines were generated from 14 HIV Controllers (HIC) and 15 treated patients (HAART) genotyped for HLA-DRB1.

For a subgroup of 8 HIC and 8 HAART patients, CD4+ T cell lines were sorted with HLA-matched tetramers loaded with the Gag293 peptide.

The tetramer used for the sort is reported in the rightmost column. VL: viral load; N.A. not available.

| Patient ID | Age yrs | Duration of Infection yrs | CD4 /mm3 | VL copies/ mL | MHC Class II DRB1 alleles | | Tetramer used for sort |
|---|---|---|---|---|---|---|---|
| **HIC 1** | 56,4 | 25,0 | 850 | <50 | DRB1*1501/1507/1510 (DRB5) | | DRB1*1501 |
| **HIC 2** | 50,8 | 24,2 | 1020 | <50 | DRB1*1122 | DRB1*1501/ 1507/ 1510 (DRB5) | DRB5*0101 |
| **HIC 3** | 54,7 | 22,0 | 1050 | <50 | DRB1*0102 | DRB1*1101 | DRB1*1101 |
| **HIC 4** | 61,4 | 26,0 | 852 | <50 | DRB1*1501 (DRB5) | | DRB1*1501 |

| | | | | | | | |
|---|---|---|---|---|---|---|---|
| **HIC 5** | 53,6 | 11,0 | 742 | <50 | DRB1*0101 | DRB1*1607 | DRB1*0101 |
| **HIC 6** | 34,6 | 13,1 | 1048 | <50 | DRB1*1101 | DRB1*1204/ 1201/ 1202/ 1307 | DRB1*1101 |
| **HIC 7** | 41,7 | 11,7 | 744 | <50 | DRB1*0701/ 0703/ 0704/ 0705/ 0706 | DRB1*1501/ 1502 (DRB5) | DRB5*0101 |
| **HIC 8** | 48,5 | 21,0 | 799 | <50 | DRB1*0102 | DRB1*1301 | DRB1*0101 |
| **HIC 9** | 49,7 | 14,6 | 754 | <50 | DRB1*0101 | DRB1*0701 | - |
| **HIC 11** | 45,0 | 26,0 | 1042 | <50 | DRB1*0101 | DRB1*1501 (DRB5) | - |
| **HIC 12** | 50,9 | 25,5 | 648 | <50 | DRB1*0301 | DRB1*1359 | - |
| **HIC 13** | 34,0 | 9,8 | 899 | <50 | DRB1*1360/ 1402 | DRB1*1401 /1602 (DRB5) | - |
| **HIC 14** | 56,6 | 18,3 | 1400 | <50 | DRB1*1301/ 1302 | DRB1*0703 | - |
| **HIC 15** | 43,6 | 14,7 | 1063 | <50 | DRB1*0101 | DRB1*0703 | - |
| **Median HIC** | 50,3 | 19,6 | 875 | <50 | | | |
| **HAART 1** | 41,4 | 7,1 | 516 | <50 | DRB1*0101/ 0107 | DRB1*1502/ 1504/ 1506/ 1514 (DRB5) | DRB5*0101 |
| **HAART 2** | 44,5 | 10,2 | 432 | <50 | DRB1*1502 (DRB5) | DRB1*1608 | DRB5*0101 |
| **HAART 3** | 52,5 | 25,3 | 682 | <50 | DRB1*0101/ 0107 | DRB1*0703 | DRB1*0101 |
| **HAART 4** | 53,0 | 14,5 | 570 | <50 | DRB1*0703 | DRB1*1122 | DRB1*1101 |
| **HAART 5** | 48,6 | 9,0 | 948 | <50 | DRB1*0102 | DRB1*1410 | DRB1*0101 |
| **HAART 6** | 54,5 | 6,8 | 266 | <50 | DRB1*0101/0107 | DRB1*0322 | DRB1*0101 |
| **HAART 7** | 55,9 | 18,1 | 846 | <50 | DRB1*0701 | DRB1*1122 | DRB1*1101 |
| **HAART 8** | 39,5 | 20,1 | 847 | <50 | DRB1*1501 (DRB5) | | DRB5*0101 |
| **HAART 9** | 47,7 | 19,7 | 534 | <50 | N.A. | N.A. | - |
| **HAART 10** | 54,0 | 11,2 | 422 | <50 | DRB1*0301 | DRB1*1501 (DRB5) | - |
| **HAART 11** | 52,1 | 12,9 | 937 | <50 | DRB1*0701 | DRB1*1001 | - |
| **HAART 12** | 43,0 | 12,1 | 558 | <50 | DRB1*1165 | DRB1*1301 | - |
| **HAART 13** | 45,0 | 11,9 | 1534 | <50 | DRB1*0401 | DRB1*1101 | - |
| **HAART 14** | 47,5 | 11,9 | 557 | <50 | DRB1*0901 | DRB1*1301 | - |

| | | | | | | | |
|---|---|---|---|---|---|---|---|
| **HAART 15** | 41,4 | 7,1 | 785 | <50 | DRB1*0101 | DRB1*0301 | - |
| **Median HAART** | 47,7 | 11,9 | 570 | <50 | | | |

***Transfer of the F24 TCR confers polyfunctionality to primary CD4+ and CD8+ T cells***

We next evaluated the properties of the F24, F25, and F5 TCRs when transferred directly into primary T cells, a system physiologically more relevant to potential TCR transfer applications. Healthy donor PBMC were transduced with concentrated TCR lentivector stocks, and analyzed for exogenous TCR expression with a TRBV2-specific mAb. Mock-transduced CD4+ T cells expressed endogenous TRBV2 at levels below 5%, while TCR-transduced CD4+ T cells showed TRBV2 expression rates in the 25%-35% range (**Figure 12A**). The hierarchy of HLA-DR tetramer binding was consistent with that observed in J76 cells, with F24 showing significant binding to 4 different tetramers (DR11, DR15, DRB5, and DR1), F25 reacting mostly to the DR11 and DRB5 tetramers and minimally to the DR1 tetramer, and F5 showing barely detectable tetramer binding (**Figure 12B-C**).

To evaluate TCR function, PMBC from DR11, DR15, DR1, or DRB5 donors were TCR-transduced and cocultured with autologous MDDC pulsed with Gag293. A panel of 5 markers, including the cytokines TNF-α, IL-2, and IFN-γ, the chemokine MIP-1β/CCL4, and the degranulation marker CD107a, was assayed by intracellular cytokine staining (ICS). CD4+ T cells transduced with the F24, F25, and F5 TCRs showed abundant cytokine production and degranulation capacity after high dose Gag293 peptide stimulation (**Figure 7A**). A specific ICS response was detected in ≥50% CD4+ T cells transduced with F24, while in the same experiment TRBV2 expression increased by only 25% post-transduction (**Figure 12A**), pointing to efficient cytokine production even in cells expressing limiting amounts of transduced TCR. ICS responses were then measured in the presence of decreasing Gag293 peptide concentrations (as shown for F24 transduced cells, **Figure 7B**). The hierarchy of marker induction was conserved across peptide doses, with high expression of TNF-α and IL-2, intermediate expression of MIP-1β, and more limited expression of CD107a and IFN-γ. Of note, specific expression of these 5 markers persisted until very low peptide doses ($10^{-9}$ to $10^{-10}$M). Antigen sensitivity was quantified for the 3 TCRs, by measuring the EC50 for induction of the 5 markers tested (**Figure 7C**). This analysis confirmed the functional hierarchy between the TCRs (F24>F25>F5), which followed TCR affinity ranking. F24 conferred a highly sensitive detection of Gag293 in primary cells, with EC50 values comprised between $10^{-9}$ and $10^{-8}$ M when measuring TNF-α, CD107a, or MIP-1β production. IFN-γ responses appeared comparatively less sensitive, with EC50 values comprised between $10^{-8}$ and $10^{-7}$ M, while IL-2 gave intermediate values. Thus, only TCRs with particularly high affinity, such as F24, may lead to full Th1 differentiation with IFN-γ production in situations of low antigen availability.

Polyfunctionality, or the capacity to express multiple cytokines simultaneously, is a hallmark of Controller T cells, and is thought to provide superior effector functions (17, 23, 39). Quantification of the number of functions (or markers) co-expressed by TCR transduced cells revealed a high degree of polyfunctionality (**Figure 7F**). Indeed, the majority (>65%) of F24, F25, and F5 expressing cells could be deemed polyfunctional, as they expressed more than 3 functions after high dose Gag293 stimulation. CD4+ T cells co-expressing the full set of markers (5 functions) were detected until the $10^{-8}$ M peptide dose for F24, and until the $10^{-7}$ M peptide dose for F25 and F5. After F24 transfer, polyfunctional cells expressing at least 3 functions still represented more than half of CD4+ T cells responding to $10^{-9}$ M peptide, emphasizing that polyfunctional responses could be achieved in response to minimal amounts of Gag antigen.

Interestingly, CD8+ T cells transduced with the F24 TCR also showed a Gag293-specific cytokine response, with detectable induction of the 5 markers tested (**Figure 7D** and **7E**). Cytokine response in CD8+ T cells transduced with F25 and F5 remained low or undetectable (not shown). The F24-dependent response in CD8+ T cells could be blocked by treatment with a pan-HLA-DR antibody, indicating that it was restricted by MHC II (**Figure 13A**). Thus, the high-affinity TCR F24 was able to confer a Gag293-specific response in absence of CD4 coreceptor expression, in a situation where availability of the Lck kinase may be limiting. F24 transfer induced 3 functions or more in >25% of specific CD8+ T cells until the $10^{-7}$ M peptide dose (**Figure 13B**), indicating that this high-affinity TCR could confer polyfunctional responses to CD8+ T cells. Thus, Controller clonotypes could be used to generate TCRs with uncommon properties, including affinities in the micromolar range, broad HLA-DR cross-restriction, high antigen sensitivity, and polyfunctionality in both the CD4+ and CD8+ T cell subsets.

**DISCUSSION**

Exploration of the TCR repertoire specific for the most immunoprevalent CD4 epitope in HIV-1 Gag revealed a markedly biased repertoire focused on the TRAV24 and TRBV2 gene families. Highly prevalent public motifs and public clonotypes were preferentially shared by HIV Controllers, suggesting that particular TCR determinants contributed to the efficiency of the antiviral CD4 response in these patients. This notion was reinforced by functional analysis of the most prevalent public clonotypes, as these were able to confer HLA cross-restricted, highly sensitive, polyfunctional responses against Gag antigens, indicative of superior function. These findings, which to our knowledge provide the first assessment of the HIV-specific CD4+ T cell repertoire at the clonotypic level, emphasize that intrinsic TCR determinants, rather than low antigenemia, determine the remarkable properties of the cellular response in controlled HIV infection.

It was noteworthy that both the TRAV24 and TRBV2 repertoires specific for Gag293 showed a high degree of motif and clonotype sharing. So far, most studies of the human TCR repertoire have focused on TRB genes, but recent analyses suggest that the human TRA repertoire is even more diverse (40). Our findings provide evidence for a dual bias in controlled HIV infection, with both TCR chains showing a significant degree of conservation. The most prevalent TRAV24 clonotype (AFKAAGNKLT) was present in 6/8 Controllers (75%) studied. In comparison, CD8 TRB public clonotypes reported in previous studies of the HIV-specific repertoire were shared between 2 to 4 patients (14, 15, 41, 42). When the analysis was extended to less stringent definitions of TCR biases, close to half of Controller TRAV24 and TRBV2 clonotypes fitted defined public motifs, while the corresponding frequencies were of 29% and 10% respectively for clonotypes of treated patients. Such strong bias in the Gag293-specific TCR repertoire of Controllers could not be attributed to amplification of a few clonotypes during culture as the repertoire of Controllers remained highly diverse, with a median of 36 distinct TRBV2 clonotypes per 100 CDR3 sequences. In addition, similar frequencies of public motifs were detected *in vitro* and *ex vivo* when the TCR repertoire of Controllers could be analyzed in both conditions. Rather, these findings suggest that strong selective pressures shaped the Gag293-specific repertoire during the establishment of viral control.

Public clonotype occurrence should statistically be an extremely rare event, given the huge number of TCRs that can be generated by V(D)J recombination (>$10^{15}$) and the extensive genetic diversity observed in human TCR repertoires, with an estimated > $10^7$ distinct clonotypes per individual (43). However, public clonotypes have been described in several chronic viral infections of humans, especially those caused by members of the herpesviridae family (32, 35, 37, 43). CD8 public clonotypes specific for conserved regions of Gag have also been identified in the context of Controlled HIV-1 infection, particularly in patients expressing the protective alleles HLA-B57 and B27 (11, 15, 41), though they can also be detected in patients carrying non-protective HLA-B alleles (42). Recent findings indicate that the frequency of HIV-specific public clonotypes correlates with the magnitude of the CD8 response, suggesting an important contribution to antiviral efficacy (42). Whether CD8 public clonotypes are generally more abundant in controlled rather than progressive HIV infection remains debated (44, 45), but it has become clear that the particular set of CD8 public clonotypes present in Controllers is more efficient at suppressing viral replication and at recognizing viral escape mutants than that of progressor patients (11, 12). A high frequency of Gag-specific CD8 public clonotypes has also been associated with a favorable outcome in the SIVmac model, in primary infection as well as post-vaccination (46). The finding that the Gag-specific CD4 repertoire of Controllers is more biased than the corresponding CD8 repertoire and is enriched in highly efficient public clonotypes supports the notion that public clonotypes contribute to HIV/SIV control. A reason for the advantage conferred by public clonotypes in the antiviral response may lie in their intrinsically high frequency. Analyses of the naive TCR repertoire have shown that public clonotypes, which are by definition shared by several individuals, are also present at significantly higher frequencies than private clonotypes in the naive repertoire of each individual (47).

The presence of a larger pool of naive T cells ready to respond to a given pathogen is thought to trigger a more rapid initiation of the immune response, which can provide a key advantage to limit viral dissemination at an early stage. This advantage may be particularly critical in the cases of viruses such as HIV and SIV, which can induce a massive CD4+ T cell depletion and ensuing immunosuppression during the first week of infection (48). In effect, abundant public clonotypes endowed with efficient functions may act as a rapid antiviral response force, similar to populations of innate cells expressing conserved TCRs, such as iNKT or MAIT cells (38, 49).

The high prevalence of Gag293 public clonotypes may result from a high probability of being generated during V(D)J recombination. The most prevalent TRAV24 public clonotype, AFKAAGNKLTF, could be simply generated from germline sequences by limited nucleotide trimming, without requiring N/P mutations. In addition, 72% the 36 Gag293 specific public clonotypes were encoded by more than one nucleotide CDR3 sequence, compatible with the notion of convergent recombination (36). Interestingly, a study of the preimmune CD4+ T cell repertoire in healthy individuals identified several clones reactive with HIV antigens, including one that responded to Gag293 with high antigen sensitivity (50), supporting the idea that high-avidity Gag293-specific precursors may be comparatively abundant in the naive repertoire. Another reason for the high prevalence of Gag293-specific public clonotypes may lie in their extensive HLA cross-restriction. The Gag293 peptide shows promiscuous binding to at least 14 HLA-DR and DQ allomorphs, enabling multiple possibilities for cross-restriction (30, 51, 52). Functional analyses indicated that the F24 TCR bound at least 5 distinct Gag293-MHC II complexes, with an efficient recognition of DR1, DR11, DR15, DRB5, and a limited but consistent recognition of DR7. The correlation between the degree of cross-restriction and the antigen sensitivity of the 8 TCRs tested suggests that broad HLA cross-restriction is a characteristic of high-affinity TCRs, which may better tolerate punctual MHC polymorphisms. This notion is supported by a study of human CD4 responses to JC virus, where HLA II cross-restricted clones showed higher antigen sensitivity and greater *in vivo* expansion than clones with a more narrow restriction (53). Widespread cross-restriction may help explain why HLA II allele frequencies are not strongly biased in carriers of chronic viral infections. HIV Controllers show a highly significant enrichment for protective HLA I alleles, while biases in HLA II allele distribution have been reported but appear less prominent (23, 52, 54). Considering the evidence for broad HLA II cross-restriction of HIV-specific public TCRs, the limited HLA II biases in the Controller population does not rule out a significant contribution of CD4+ T cells to HIV control.

The co-expression of TRAV24 and TRBV2 public clonotypes generated functional TCRs that, for some combinations, displayed a remarkably high affinity for Gag293-MHC II complexes. Human TCRs generally show affinities in the 1 to 100 μM range by SPR analysis (38). MHC II restricted TCRs show a trend for a lower affinity as compared to MHC I restricted TCRs, with average Kd values of 70 versus 35 μM, respectively. With a Kd of 0.86 μM for the Gag293-DR11 complex, F24 demonstrated one of the highest affinities reported so far for an MHC II restricted TCR, ranking second in a list of 22 human and mouse TCRs for which structural data is available (38). The molecular determinants of a high avidity interaction for the Gag293-MHC complex appear to depend on both the TRAV24 and TRBV2 chains, and on the restricting HLA allele. The most conserved CDR3 residues in public motifs represent likely candidates for contact residues with Gag293. However, conserved CDR1/CDR2 residues may also contact the Gag293 peptide, considering the predominance of the TRAV24 and TRBV2 families. Future structural studies should help determine the respective roles of the CDR3 versus the CDR1/2 regions in shaping high-avidity interactions for Gag293-MHC complexes. The expression of high-affinity TCRs for Gag293 in Controllers resulted in high antigen sensitivity as measured by CD69 induction, with a good correlation between the two parameters (R=0.85). Thus, TCR biophysical properties largely accounted for TCR function, consistent with multiple studies in animal models (55, 56). It should be noted, however, that in some studies TCRs with very high or supraphysiological affinities were found to have reduced functions (57). A proposed explanation is that a pMHC that interacts too tightly with a very high affinity TCR cannot disengage to contact additional TCRs, and thus does not achieve serial TCR triggering and T cell response amplification (58). We did not observe decreased functions for the TCR/pMHC II combinations showing the highest affinities by SPR, suggesting that TCR affinities in the order of 1 μM are still in the physiological range for optimal TCR function in human CD4+ T cells.

The reasons for the preferential amplification of high-affinity Gag293-specific TCRs in Controllers compared to treated patients remain to be elucidated. A particular genetic background or a limited duration of acute infection may have promoted the amplification of such TCRs in Controllers (59). Conversely, patients who initially progressed to disease may have lost the high avidity CD4+ T cell population due to preferential infection and depletion, a notion supported by the restricted CD4 TCR repertoire observed in treated patients, and by rapid loss of certain CD4 specificities after the acute infection stage (6). It will be informative to determine whether other immunoprevalent HIV epitopes elicit a response pattern similar to that observed for Gag293, with the induction of a highly biased, high affinity TCR repertoire in controlled infection. The preferential detection of Gag-specific CD4 responses in HIV Controllers (22) suggests that several regions of Gag may be targeted by high affinity TCRs, though this remains to be investigated. High-affinity CD4 TCRs are endowed with strong proliferative capacity (55), which helps explain how they could reach such a dominance in controlled HIV infection. The high affinity of Controller TCRs also translated into efficient cytokine secretion upon Gag293 peptide stimulation. Primary CD4+ T cells transduced with the F24 TCR showed EC50 ranging between $10^{-8}$ M and $10^{-9}$ M peptide for the different cytokines studied, indicative of a remarkably high antigen sensitivity for the CD4+ T cell subset. Polyfunctionality, a property which has been associated with HIV control (39), showed a clear dependence on TCR affinity. Comparison of the F24, F25, and F5 TCRs showed that a difference of 1.1 log in affinity resulted in a 2 log difference in the peptide concentration at which half the specific cells retained 3 functions. Thus, TCR affinity appears critical in determining the range of cytokine produced, particularly at low antigen dose. Of interest, not all cytokines were equally dependent on the strength of the TCR signal, with TNF-α showing the lowest requirements, and IFN-γ the most stringent. The notion of a hierarchy in cytokine production is consistent with a report showing that the affinity of Gag-specific T cell clones dictated their cytokine expression profile (10). It is relevant that for CD4+ T cells, in contrast to CD8+ T cells, IFN-γ is the most "demanding" cytokine, as it can be produced only by high avidity cells upon limiting antigenic stimulation. IFN-γ plays a key role in CD4+ T cell helper function, through its capacity to upregulate MHC II in APC and prime them for efficient antigen presentation. In addition, IFN-γ production by tissue CD4+ T cells plays an underappreciated role in the recruitment of immune effectors, through the triggering of chemokine cascades (60). In controlled HIV infection, high avidity Gag-specific CD4+ T cells may play a similar role, by keeping the immune system in constant alert, and rapidly recruiting CD8+ T cells and NK cells to sites of HIV replication upon the occurrence of viral replication blips. In addition, IFN-γ secretion may play a direct antiviral role, through the induction of interferon stimulated genes (ISG) that inhibit HIV replication (61). For instance, Th1 cells are thought to be less infectable than Th2 cells due to a higher expression of the HIV restriction factor APOBEC3G (62). Highly differentiated Th1 effectors that re-express CD45RA appear particularly resistant to HIV infection (63). Gag-specific CD4+ T cells in Controllers maintain a Th1 differentiation status with persistent IFN-γ production (24), raising the possibility that such cells possess a degree of resistance to HIV infection. Thus, high TCR affinity, with the associated capacity for IFN-γ expression at low antigen dose, may contribute to HIV control at several levels, through sensitive immune surveillance, rapid triggering of helper functions, and direct antiviral effector mechanisms.

Of interest, the F24 TCR was able to confer polyfunctional cytokine responses when transferred into CD8+ T cells. The responses were MHC II restricted, indicating that the F24 TCR could interact with the Gag293-MHC II complex in the absence of the CD4 coreceptor. The CD4 molecule is not thought to contribute significantly to the affinity of the pMHC/TCR/CD4 complex, but plays an important role in relocating the kinase Lck close to TCR/CD3 signaling complex (64). The comparatively lower cytokine responses in CD8+ than in CD4+ T cells may thus result from a lower number of Lck molecules available for triggering intracellular signals. The transfer of the F24 TCR to CD8+ T cells still conferred the 5 functions tested at the $10^{-5}$ M to $10^{-7}$ M antigen dose, suggesting that transduced CD8+ T cells could become efficient effectors in foci of productive HIV replication. These findings open the possibility of reprogramming CD8+ T cells to target the highly conserved MHR region of capsid, which could be advantageous given the high fitness cost associated with mutations in this region (65). The report that a CMV-based protective SIV vaccine elicited a high frequency of unconventional MHC II-restricted CD8 responses highlights the potential benefits of CD8 T cell reprogramming (66). Studies of TCR transfer for cancer immunotherapy have shown the interest of transferring the same TCR in both CD4+ and CD8+ T cells to enhance tumoricidal activity (67). Similarly, the transfer of a high-avidity Gag-specific TCR in both CD4+ and CD8+ T cell populations may be of interest for adoptive T cell therapies targeting HIV, in order to trigger a full set of antiviral functions.

In conclusion, study of TCRs specific for the immunoprevalent CD4 epitope in capsid revealed that particular clonotypes are associated with HIV control. The TCR repertoire of Controllers was characterized by a high prevalence of public TRAV24 and TRBV2 chains. Reconstituted TCRs showed affinities that reached the micromolar range, at the high end of values obtained for naturally expressed TCRs. Public clonotypes conferred MHC II cross-restriction, high antigen sensitivity and polyfunctionality to CD4+ T cells, suggesting a key role in shaping the properties of an efficient CD4 response. The most prevalent public clonotype also proved functional in CD8+ T cells, suggesting that it could be used to target the highly conserved capsid MHR region in patients of diverse HLA types. Inducing or transferring such clonotypes may contribute to the development of immunotherapeutic approaches that aim at a functional cure of HIV infection.

***Table 6: List of TRAV24 clonotypes specific for Gag293 (SEQ ID NO: 198 to 314 for Junction (AA) sequences and SEQ ID NO: 315 to 454 for Junction (nt) sequences***

TRAV24 clonotypes obtained from 8 Controller cell lines, 8 treated patient cell lines, and 4 ex vivo Controller samples are listed. The V(D)J gene nomenclature is that of the IMGT database (www.imgt.org). Public clonotypes are in bold type. Clonotypes tested functionally are marked by an asterisk.

| **HIC 1 DR15+** | | | | | | | |
|---|---|---|---|---|---|---|---|
| **V-GENE** | **J-GENE** | **JUNCTION** | **JUNCTION (AA)** | **frequency** | **% nt seq** | **% AA seq** | **JUNCTION nt number** |
| TRAV24*01, or TRAV24*02 | TRAJ38*01 | tgtgcccgtgacgaccgtaagctgatttgg | CARDDRKLIW | 1 | 1,89 | 1,89 | 30 |
| TRAV24*01 | TRAJ34*01 | tgtgcctctggtaacaccgacaagctcatcttt | CASGNTDKLIF | 1 | 1,89 | 1,89 | 33 |
| TRAV24*01 | TRAJ39*01 | tgtgccttttgtaatgcaggcaacatgctcaccttt | CAFCNAGNMLTF | 1 | 1,89 | 1,89 | 36 |
| TRAV24*01 | TRAJ17*01 | tgtgcctttaaagctgcaggcaacaagctaactt tt | **CAFKAAGNKLTF*** | 2 | 3,77 | 5,66 | |
| TRAV24*01 | TRAJ17*01 | tgtgcctttaaggctgcaggcaacaagctaactt tt | | 1 | 1,89 | | |
| TRAV24*01 | TRAJ17*01 | tgtgcctttacggctgcaggcaacaagctaactt tt | CAFTAAGNKLTF | 1 | 1,89 | 1,89 | |
| TRAV24*01 | TRAJ17*01 | tgtgccctagaaaatgcaggcaacaagctaac tttt | CALENAGNKLTF | 1 | 1,89 | 1,89 | |
| TRAV24*01 | TRAJ39*01 | tgtgccctcggtaatgcaggcaacatgctcacc ttt | CALGNAGNMLTF | 33 | 62,26 | 64,15 | |
| TRAV24*01 | TRAJ39*01 | tgtgccctcggtaatgcaggcaacatgctcacg ttt | | 1 | 1,89 | | |
| TRAV24*01 | TRAJ57*01 | tgctcgcctcagggcggatctgaaaagctggtc ttt | CSPQGGSEKLVF | 1 | 1,89 | 1,89 | |
| TRAV24*01 | TRAJ6*01 | tgtgcctttatcccaggaggaagctacataccta cattt | CAFIPGGSYIPTF | 1 | 1,89 | 1,89 | 39 |
| TRAV24*01 | TRAJ42*01 | tgtgcctctgatggaggaagccaaggcaatctc atcttt | CASDGGSQGNLIF | 1 | 1,89 | 1,89 | |
| TRAV24*01 | TRAJ32*02 | tgtgcctcttatggtggtgctacaaacaagctcat cttt | **CASYGGATN KLIF** | 8 | 15,09 | 15,09 | |
| | | Total | 11 | 53 | 100,00 | 100,00 | |

(continued)

| | | | | | | | |
|---|---|---|---|---|---|---|---|
| **HIC 2 DRB5+** | | | | | | | |
| TRAV24*01 | TRAJ17*01 | tgtgccttcaaagctgcaggcaacaagctaactttt | **CAFKAAGNKLTF*** | 2 | 3,03 | 56,06 | 36 |
| TRAV24*01 | TRAJ17*01 | tgtgcctttaaagctgcaggcaacaagctaactttt | | 35 | 53,03 | | |
| TRAV24*01 | TRAJ17*01 | tgtgcctttaaggttgcaggcaacaagctaactttt | CAFKVAGNKLTF | 1 | 1,52 | 1,52 | |
| TRAV24*01 | TRAJ39*01 | tgtgcctttaggatggcaggcaacatgctcacctttt | CAFRMAGNMLTF | 1 | 1,52 | 1,52 | |
| TRAV24*01 | TRAJ39*01 | tgtgcctttcgtaatgcaggcaacatgctcacctttt | **CAFRNAGNMLTF** | 1 | 1,52 | 1,52 | |
| TRAV24*01 | TRAJ17*01 | tgtgcccacaaagctgcaggcaacaagctaactttt | **CAHKAAGNKLTF** | 2 | 3,03 | 4,55 | |
| TRAV24*01 | TRAJ17*01 | tgtgcccataaagctgcaggcaacaagctaactttt | | 1 | 1,52 | | |
| TRAV24*01 | TRAJ39*01 | tgtgcccctataaatgcaggcaacatgctcaccttt | CAPINAGNMLTF | 6 | 9,09 | 9,09 | |
| TRAV24*01 | TRAJ17*01 | tgtgcccgcaaagctgcaggcaacaagctaactttt | CARKAAGNKLTF | 1 | 1,52 | 1,52 | |
| TRAV24*01 | TRAJ17*01 | tgtgcctctaaagctgcaggcaacaagctaactttt | **CASKAAGNKLTF*** | 13 | 19,70 | 19,70 | |
| TRAV24*01 | TRAJ17*01 | tgtgcctcacgaactgcaggcaacaagctaactttt | CASRTAGNKLTF | 3 | 4,55 | 4,55 | |
| | | Total | 9 | 66 | 100,00 | 100,00 | |
| **HIC 3 DR11+** | | | | | | | |
| TRAV24*01 | TRAJ17*01 | tgtgctccaaagagcaggcaacaagctaacttt | CAPKSRQQANF | 1 | 0,64 | 0,64 | 33 |

(continued)

| **HIC 3 DR11+** | | | | | | | |
|---|---|---|---|---|---|---|---|
| TRAV24*01 | TRAJ17*01 | tgtgccttcaaagctgcaggcaacaagctaact ttt | **CAFKAAGNKLTF*** | 64 | 41,03 | 46,15 | 36 |
| TRAV24*01, or TRAV24*02 | TRAJ17*01 | tgtgcctttaaagctgcaggcaacaagctaactt tt | | 8 | 5,13 | | |
| TRAV24*01, or TRAV24*02 | TRAJ17*01 | tgtgccttcagggctgcaggcaacaagctaact ttt | **CAFRAAGNKLTF** | 2 | 1,28 | 1,28 | |
| TRAV24*01, or TRAV24*02 | TRAJ17*01 | tgtgccttgaaagctgcaggcaacaagctaact ttt | **CALKAAGNKLTF** | 1 | 0,64 | 0,64 | |
| TRAV24*01 | TRAJ39*01 | tgtgccttgaaacaagcaggcaacaagctcac tttt | CALKQAGNKLTF | 1 | 0,64 | 0,64 | |
| TRAV24*01 | TRAJ39*01 | tgtgccttgaggcaagcaggcaacatgctcac cttt | CALRQAGNMLTF | 6 | 3,85 | 3,85 | |
| TRAV24*01, or TRAV24*02 | TRAJ17*01 | tgtgccatgaaagctgcaggcaacaagctaac tttt | CAMKAAGNKLTF | 1 | 0,64 | 1,28 | |
| TRAV24*01, or TRAV24*02 | TRAJ17*01 | tgtgcgatgaaagctgcaggcaacaagctaa ctttt | | 1 | 0,64 | | |
| TRAV24*01, or TRAV24*02 | TRAJ17*01 | tgtgccaacagagctgcaggcaacaagctaa ctttt | CANRAAGNKLTF | 1 | 0,64 | 0,64 | |
| TRAV24*01, or TRAV24*02 | TRAJ17*01 | tgtgcctccaaagctgcaggcaacaagctaac tttt | **CAS KAAG N KLTF*** | 4 | 2,56 | 2,56 | |
| TRAV24*01, or | TRAJ17*01 | tgtgcctacaaagctgcaggcaacaagctaac tttt | CAYKAAGNKLTF | 1 | 0,64 | 0,64 | |
| TRAV24*02 | | | | | | | |

(continued)

| **HIC 3 DR11+** | | | | | | | |
|---|---|---|---|---|---|---|---|
| TRAV24*01, or TRAV24*02 | TRAJ17*01 | tgtggtttcaaagctgcaggcaacaagctaact ttt | CGFKAAGNKLTF | 1 | 0,64 | 0,64 | |
| TRAV24*01, or TRAV24*02 | TRAJ17*01 | tgtggttggaaagctgcaggcaacaagctaac tttt | CGWKAAGNKLTF | 1 | 0,64 | 0,64 | |
| TRAV24*01, or TRAV24*02 | TRAJ17*01 | tgtagtttgaaagctgcaggcaacaagctaact ttt | CSLKAAGNKLTF | 2 | 1,28 | 1,28 | |
| TRAV24*01, or TRAV24*02 | TRAJ17*01 | tgtagtttgagagctgcaggcaacaagctaact ttt | CSLRAAGNKLTF | 1 | 0,64 | 0,64 | |
| TRAV24*01 | TRAJ17*01 | tgtagtcggagggctgcaggcaacaagctaa ctttt | **CSRRAAGNKLTF*** | 18 | 11,54 | 11,54 | |
| TRAV24*01, or TRAV24*02 | TRAJ17*01 | tgtagttggaaagctgcaggcaacaagctaac tttt | CSWKAAGNKLTF | 4 | 2,56 | 2,56 | |
| TRAV24*01, or TRAV24*02 | TRAJ17*01 | tgtagttggagagctgcaggcaacaagctaac tttt | CSWRAAGNKLTF | 1 | 0,64 | 1,28 | |
| TRAV24*01, or TRAV24*02 | TRAJ17*01 | tgtagttggagggctgcaggcaacaagctaac tttt | | 1 | 0,64 | | |
| TRAV24*01, or TRAV24*02 | TRAJ17*01 | tgtaccaagaaagctgcaggcaacaagctaa ctttt | CTKKAAGNKLTF | 1 | 0,64 | 21,15 | |
| TRAV24*01 | TRAJ17*01 | tgtacgaagaaagctgcaggcaacaagctaa ctttt | | 32 | 20,51 | | |
| TRAV24*01, or TRAV24*02 | TRAJ17*01 | tgtaccttgaaagctgcaggcaacaagctaact ttt | CTLKAAGNKLTF | 1 | 0,64 | 0,64 | |

(continued)

| | | | | | | | |
|---|---|---|---|---|---|---|---|
| **HIC 3 DR11+** | | | | | | | |
| TRAV24*01, or TRAV24*02 | TRAJ17*01 | tgtacgaacaaagctgcaggcaacaagctaa ctttt | CTNKAAGNKLTF | 1 | 0,64 | 0,64 | |
| TRAV24*01, or TRAV24*02 | TRAJ17*01 | tgtactaggaaagctgcaggcaacaagctaa ctttt | CTRKAAGNKLTF | 1 | 0,64 | 0,64 | |
| | | Total | 22 | 156 | 100,00 | 100,00 | |
| **HIC 4 DR15+** | | | | | | | |
| TRAV24*01 | TRAJ39*01 | tgtgccttccaaaatgcaggcaacatgctcacc ttt | CAFQNAGNMLTF | 6 | 9,23 | 9,23 | 36 |
| TRAV24*01 | TRAJ17*01 | tgtgcctttagagctgcaggcaacaagctaactt tt | **CAFRAAGNKLTF** | 1 | 1,54 | 1,54 | |
| TRAV24*01 | TRAJ39*01 | tgtgcgacgcgccgtgcaggcaacatgctcac cttt | **CATRRAGNMLTF** | 6 | 9,23 | 9,23 | |
| TRAV24*01 | TRAJ57*01 | tgtgcctacgagggggcatctgaaaagctggt cttt | **CAYEGASEKLVF** | 3 | 4,62 | 4,62 | |
| TRAV24*01 | TRAJ32*02 | tgtgccgaatatggtggtgctacaaacaagctc atcttt | **CAEYGGATN KLIF** | 2 | 3,08 | 3,08 | 39 |
| TRAV24*01 | TRAJ32*02 | tgtgccccttatggtggtgctacaaacaagctca tcttt | **CAPYGGATN KLIF** | 2 | 3,08 | 3,08 | |
| TRAV24*01 | TRAJ32*02 | tgtgcccgttatggtggtgctgcaaacaagctca tcttt | CARYGGAANKLIF | 1 | 1,54 | 1,54 | |
| TRAV24*01 | TRAJ32*02 | tgtgcccgttatggtggtgctacaaacaagctca tcttt | **CARYGGATN KLIF** | 10 | 15,38 | 16,92 | |
| TRAV24*01 | TRAJ32*02 | tgtgctcgttatggtggtgctacaaacaagctca tcttt | | 1 | 1,54 | | |
| TRAV24*01, or TRAV24*02 | TRAJ32*02 | tgtgcccgctatggtggtggtacaaacaagctc attttt | CARYGGGTNKLIF | 1 | 1,54 | 1,54 | |

(continued)

| | | | | | | | |
|---|---|---|---|---|---|---|---|
| **HIC 4 DR15+** | | | | | | | |
| TRAV24*01 | TRAJ54*01 | tgtgcctccgagtccacgggagcccagaagctggtattt | **CASESTGAQKLVF** | 3 | 4,62 | 4,62 | |
| TRAV24*01 | TRAJ32*02 | tgtgcctcctatggtggtgctacaaacaagctcatcttt | **CASYGGATNKLIF** | 25 | 38,46 | 43,08 | |
| TRAV24*01 | TRAJ32*02 | tgtgcctcgtatggtggtgctacaaacaagctcatcttt | | 2 | 3,08 | | |
| TRAV24*01 | TRAJ32*02 | tgtgcctcttatggtggtgctacaaacaagctcatcttt | | 1 | 1,54 | | |
| TRAV24*01 | TRAJ32*02 | tgtgcctcctatgttggtgctacaaacaagctcatcttt | CASYVGATNKLIF | 1 | 1,54 | 1,54 | |
| | | Total | 12 | 65 | 100,00 | 100,00 | |
| **HIC** 5 **DR1+** | | | | | | | |
| TRAV24*02 | TRAJ38*01 | tgtgccttcgacaaccgtaagctgatttgg | **CAFDNRKLIW** | 31 | 51,67 | 55,00 | 30 |
| TRAV24*01 | TRAJ38*01 | tgtgcctttgacaaccgtaagctgatttgg | | 2 | 3,33 | | |
| TRAV24*01 | TRAJ17*01 | tgtgcctttaaggctgcaggcaacaagctaacttt | **CAFKAAGNKLTF*** | 1 | 1,67 | 1,67 | 36 |
| TRAV24*01 | TRAJ17*01 | tgtgcctttcgagctgcaggcaacaagctaacttt | **CAFRAAGNKLTF** | 2 | 3,33 | 3,33 | |
| TRAV24*01 | TRAJ36*01 | tgtgcctcagaaactggggcaaacaacctcttcttt | CASETGANNLFF | 2 | 3,33 | 3,33 | |
| TRAV24*02 | TRAJ39*01 | tgtgcgacgcgccgtgcaggcaacatgctcaccttt | **CATRRAGNMLTF** | 5 | 8,33 | 8,33 | |
| TRAV24*01 | TRAJ57*01 | tgtgcctacgagggggcatctgaaaagctggtcttt | **CAYEGASEKLVF** | 1 | 1,67 | 1,67 | |
| TRAV24*01 | TRAJ32*02 | tgtgccgaatatggtggtgctacaaacaagctcatcttt | **CAEYGGATNKLIF** | 1 | 1,67 | 1,67 | 39 |
| TRAV24*01 | TRAJ22*01 | tgtgccttcgcttctggttctgcaaggcaactgaccttt | CAFASGSARQLTF | 13 | 21,67 | 21,67 | |

(continued)

| | | | | | | | |
|---|---|---|---|---|---|---|---|
| **HIC** 5 **DR1+** | | | | | | | |
| TRAV24*01 | TRAJ54*01 | tgtgcctccgagtccacgggagcccagaagctggtattt | **CASESTGAQKLVF** | 1 | 1,67 | 1,67 | |
| TRAV24*02 | TRAJ38*01 | tgtgcctttaccccccctggcaacaaccgtaagctgatttgg | CAFYPPGNNRKLIW | 1 | 1,67 | 1,67 | 42 |
| | | Total | 10 | 60 | 100,00 | 100,00 | |
| **HIC 6 DR11+** | | | | | | | |
| TRAV24*01 | TRAJ17*01 | tgtgcatttaaagctgcaggcaacaagctaacttt | **CAFKAAGNKLTF*** | 1 | 1,69 | 23,73 | 36 |
| TRAV24*01 | TRAJ17*01 | tgtgccttcaaagctgcaggcaacaagctaacttt | | 2 | 3,39 | | |
| TRAV24*01 | TRAJ17*01 | tgtgcctttaaagctgcaggcaacaagctaacttt | | 11 | 18,64 | | |
| TRAV24*01 | TRAJ17*01 | tgtgcctttaaagatgcaggcaacaagctaacttt | CAFKDAGNKLTF | 1 | 1,69 | 1,69 | |
| TRAV24*01 | TRAJ39*01 | tgtgcctttcctaatgcaggcaacatgctcacctt | CAFPNAGNMLTF | 5 | 8,47 | 8,47 | |
| TRAV24*01 | TRAJ17*01 | tgtgcctttagggctgcaggcaacaagctaacttt | **CAFRAAGNKLTF** | 1 | 1,69 | 1,69 | |
| TRAV24*01 | TRAJ39*01 | tgtgcctttcggaatgcaggcaacatgctcacctt | **CAFRNAGNMLTF** | 1 | 1,69 | 1,69 | |
| TRAV24*01 | TRAJ17*01 | tgtgctctaaaagctgcaggcaacaagctaacttt | **CALKAAGNKLTF** | 6 | 10,17 | 10,17 | |
| TRAV24*01 | TRAJ17*01 | tgtgctctaaaagatgcaggcaacaagctaactttt | CALKDAGNKLTF | 1 | 1,69 | 1,69 | |
| TRAV24*01 | TRAJ39*01 | tgtgccttaaaaaatgcaggcaacatgctcaccttt | CALKNAGNMLTF | 4 | 6,78 | 6,78 | |
| TRAV24*01 | TRAJ39*01 | tgtgccctcaataatgcaggcaacatgctcaccttt | **CALNNAGNMLTF** | 1 | 1,69 | 1,69 | |

(continued)

| | | | | | | | |
|---|---|---|---|---|---|---|---|
| **HIC 6 DR11+** | | | | | | | |
| TRAV24*01 | TRAJ39*01 | tgtgccctgcggaatgcaggcaacatgctcaccttt | **CALRNAGNMLTF** | 3 | 5,08 | 5,08 | |
| TRAV24*01 | TRAJ17*01 | tgtgcccccaaagctgcaggcaacaagctaactttt | CAPKAAGNKLTF | 12 | 20,34 | 20,34 | |
| TRAV24*01 | TRAJ17*01 | tgtgcccccaaagatgcaggcaacaagctaactttt | CAPKDAGNKLTF | 1 | 1,69 | 1,69 | |
| TRAV24*01 | TRAJ39*01 | tgtgcctccaagaatgcaggcaacatgctcaccttt | CASKNAGNMLTF | 1 | 1,69 | 1,69 | |
| TRAV24*01 | TRAJ39*01 | tgtgcctccatgaatgcaggcaacatgctcaccttt | CASMNAGNMLTF | 7 | 11,86 | 11,86 | |
| TRAV24*01 | TRAJ53*01 | tgtgcctttaagggaggtggaggtagcaactataaactgacattt | CAFKGGGGSNYKLTF | 1 | 1,69 | 1,69 | 45 |
| | | Total | 15 | 59 | 100,00 | 100,00 | |
| **HIC 7 DRB5+** | | | | | | | |
| TRAV24*01 | TRAJ20*01 | tgtgcctttggcgactacaagctcagcttt | CAFGDYKLSF | 2 | 3,64 | 3,64 | 30 |
| TRAV24*01 | TRAJ17*01 | tgtgcctttcacgctgcaggcaacaagctaacttt | CAFHAAGNKLTF | 1 | 1,82 | 1,82 | 36 |
| TRAV24*01 | TRAJ17*01 | tgtgccttcaaagctgcaggcaacaagctaacttt | **CAFKAAGNKLTF*** | 10 | 18,18 | 18,18 | |
| TRAV24*01 | TRAJ10*01 | tgtgcctttaagggaggaggaaacaaactcaccttt | CAFKGGGNKLTF | 1 | 1,82 | 1,82 | |
| TRAV24*01 | TRAJ17*01 | tgtgcctttaacgctgcaggcaacaagctaacttt | CAFNAAGNKLTF | 3 | 5,45 | 5,45 | |
| TRAV24*01 | TRAJ17*01 | tgtgccttccaggctgcaggcaacaagctaacttt | CAFQAAGNKLTF | 9 | 16,36 | 16,36 | |
| TRAV24*01 | TRAJ10*01 | tgtgcctttaggggaggaggaaacaaactcaccttt | CAFRGGGNKLTF | 11 | 20,00 | 20,00 | |
| TRAV24*01 | TRAJ39*01 | tgtgcctttcgaagagcaggcaacatgctcaccttt | CAFRRAGNMLTF | 1 | 1,82 | 1,82 | |

(continued)

| | | | | | | | |
|---|---|---|---|---|---|---|---|
| **HIC 7 DRB5+** | | | | | | | |
| TRAV24*01 | TRAJ17*01 | tgtgcccacaaagctgcaggcaacaagctaa ctttt | **CAHKAAGNKLTF** | 3 | 5,45 | 5,45 | |
| TRAV24*01 | TRAJ17*01 | tgtgcccacaaaggagcaggcaacaagctaa ctttt | CAHKGAGNKLTF | 1 | 1,82 | 1,82 | |
| TRAV24*01 | TRAJ32*02 | tgtgccgaatatggtggtgctacaaacaagctc atcttt | **CAEYGGATN KLIF** | 3 | 5,45 | 5,45 | 39 |
| TRAV24*01 | TRAJ32*01 | tgtgccaactatggcggtgctacaaacaagctc atcttt | **CANYGGATN KLIF** | 2 | 3,64 | 3,64 | |
| TRAV24*01 | TRAJ57*01 | tgtgcccccgggggggcggatctgaaaagct ggtcttt | CAPGGGGSEKLVF | 2 | 3,64 | 3,64 | |
| TRAV24*01 | TRAJ32*02 | tgtgccccttatggtggtgctacaaacaagctca tcttt | **CAPYGGATNKLIF** | 1 | 1,82 | 3,64 | |
| TRAV24*01 | TRAJ32*01 | tgtgctccttatggcggtgctacaaacaagctca tcttt | | 1 | 1,82 | | |
| TRAV24*01 | TRAJ32*01 | tgtgcctcctatggcggtgctacaaacaagctc atcttt | **CASYGGATN KLIF** | 2 | 3,64 | 7,27 | |
| TRAV24*01 | TRAJ32*02 | tgtgcctcctatggtggtgctacaaacaagctca tcttt | | 1 | 1,82 | | |
| TRAV24*01 | TRAJ32*02 | tgtgcctcttatggtggtgctacaaacaagctcat cttt | | 1 | 1,82 | | |
| | | Total | 15 | 55 | 100,00 | 100,00 | |
| **HIC 8 DR1+** | | | | | | | |
| TRAV24*01 | TRAJ31*01 | tgtgggggggataacaatgccagactcatgttt | CGGDNNARLMF | 54 | 77,14 | 77,14 | 33 |
| TRAV24*01 | TRAJ31*01 | tgtgggggggataacaatgccagactcacgttt | CGGDNNARLTF | 1 | 1,43 | 1,43 | |
| TRAV24*01 | TRAJ34*01 | tgtgcctctctctataacaccgacaagctcatctt t | CASLYNTDKLIF | 10 | 14,29 | 14,29 | 36 |
| TRAV24*01 | TRAJ32*01 | tgtgccaactatggcggtgctacaaacaagctc atcttt | **CANYGGATNKLIF** | 5 | 7,14 | 7,14 | 39 |
| | | Total | 4 | 70 | 100,00 | 100,00 | |

***Table 7: List of TRBV2 clonotypes specific for Gag293 (SEQ ID NO: 455 to 749 for Junction (AA) sequences and SEQ ID NO: 750 to 1059 for Junction (nt) sequences***

TRBV2 clonotypes obtained from 8 Controller cell lines, 8 treated patient cell lines, and 4 ex vivo Controller samples are listed. The V(D)J gene nomenclature is that of the IMGT database (www.imgt.org). Public clonotypes are in bold type. Clonotypes tested functionally are marked by an asterisk.

| **HIC 1 DR15+** | | | | | | | | |
|---|---|---|---|---|---|---|---|---|
| **V-GENE** | **J-GENE** | **D-GENE** | **JUNCTION** | **JUNCTION (AA)** | **frequency** | **% nt seq** | **%AA seq** | **JUNCTION nt numb er** |
| TRBV2*01 | TRBJ2-7*01 | TRBD1*01 | tgtgccagcttag-ggcccctacggcac-gagcagtacttc | CASLGPLR-HEQYF | 2 | 3,33 | 3,33 | 39 |
| TRBV2*01 | TRBJ2-3*01 | TRBD2*01 | tgtgccagcaaac-cactagttagcacaga-tacgcagtatttt | CASKPLVST-DTQYF | 1 | 1,67 | 1,67 | 42 |
| TRBV2*01, or TRBV2*02 or TRBV2*03 | TRBJ2-1*01 | TRBD2*01 | tgtgccagcctt-gaaaggactagcg-ggggtgagcagttcttc | CASLERTS-GGEQFF | 1 | 1,67 | 1,67 | |
| TRBV2*01 | TRBJ2-1*01 | TRBD1*01 | tgtgccagcacccg-ggacaggacaaa-gaatgagcagttcttc | CAS-TRDRTKNEQFF | 1 | 1,67 | 1,67 | |

(continued)

TRBV2 clonotypes obtained from 8 Controller cell lines, 8 treated patient cell lines, and 4 ex vivo Controller samples are listed. The V(D)J gene nomenclature is that of the IMGT database (www.imgt.org). Public clonotypes are in bold type. Clonotypes tested functionally are marked by an asterisk.

| **HIC 1 DR15+** | | | | | | | | |
|---|---|---|---|---|---|---|---|---|
| **V-GENE** | **J-GENE** | **D-GENE** | **JUNCTION** | **JUNCTION (AA)** | **frequency** | **% nt seq** | **%AA seq** | **JUNCTION nt numb er** |
| TRBV2*01 | TRBJ2-1*01 | TRBD2*01 | tgtgccagcagtgcgtt-ggctagcgggggga-gatgagcagttcttc | CASSALASGGD-EQFF | 1 | 1,67 | 1,67 | 45 |
| TRBV2*01 | TRBJ2-3*01 | TRBD2*01 | tgtgccagcagtgcgt-tagctagcggtacaga-tacgcagtatttt | **CASSALASGT-DTQYF** | 1 | 1,67 | 1,67 | |
| TRBV2*01 | TRBJ2-7*01 | TRBD2*01 | tgtgccagcagt-gaactgacctcta-gaacctacgagcag-tacttc | CASSELT-SRTYEQYF | 1 | 1,67 | 1,67 | |
| TRBV2*01, or TRBV2*02 or TRBV2*03 | TRBJ1-5*01 | TRBD2*01 | tgtgccagcagt-gaacgggtttcg-ggcaatcagccccag-catttt | CASSERVSGN-QPQHF | 2 | 3,33 | 3,33 | |
| TRBV2*01 | TRBJ2-1*01 | TRBD2*01 | tgtgccag-cagccctat-ggctagcg-ggggggatgagcagtt cttc | CASSPMASGGD-EQFF | 1 | 1,67 | 1,67 | |
| TRBV2*01, or TRBV2*02 or TRBV2*03 | TRBJ2-7*01 | TRBD2*02 | tgtgccagcagccg-taggactagcgggact-tacgagcagtacttc | **CASSRRTSG-TYEQYF** | 1 | 1,67 | 1,67 | |
| TRBV2*01 | TRBJ2-1*01 | TRBD2*01 | tgtgccagcagtgtgat-ggctagccgtgggaat-gagcagttcttc | CASSVMAS-RGNEQFF | 1 | 1,67 | 1,67 | |

(continued)

| TRBV2 clonotypes obtained from 8 Controller cell lines, 8 treated patient cell lines, and 4 ex vivo Controller samples are listed. The V(D)J gene nomenclature is that of the IMGT database (www.imgt.org). Public clonotypes are in bold type. Clonotypes tested functionally are marked by an asterisk. | | | | | | | | |
|---|---|---|---|---|---|---|---|---|
| **HIC 1 DR15+** | | | | | | | | |
| **V-GENE** | **J-GENE** | **D-GENE** | **JUNCTION** | **JUNCTION (AA)** | **frequency** | **% nt seq** | **%AA seq** | **JUNCTION nt numb er** |
| TRBV2*01 | TRBJ1-5*01 | TRBD2*01 | tgtgccagccaaag-gggggctcg-ggggggcaat-cagccccagcatttt | CASQR-GARGGNQPQHF | 1 | 1,67 | 1,67 | |
| TRBV2*01, or TRBV2*02 or TRBV2*03 | TRBJ1-4*01 | TRBD1*01 | tgtgccagcagggctc-gaacag-gggcaactaat-gaaaaactgtttttt | CASRARTGAT-NEKLFF | 1 | 1,67 | 1,67 | |
| TRBV2*01 | TRBJ2-3*01 | TRBD2*01 | tgtgccagcagt-gccaagactagcg-ggggctcagatacg-cagtatttt | CASSAKTS-GGSDTQYF | 1 | 1,67 | 1,67 | |
| TRBV2*01 | TRBJ2-3*01 | TRBD2*01 | tgtgccag-cagcgccct-ggctagcgggggccg-ggatacgcagtatttt | CASSALASGG RDTQYF | 1 | 1,67 | 1,67 | |
| TRBV2*01 | TRBJ2-1*01 | TRBD2*01 | tgtgccagcagt-gcccggactagcg-ggggactcgatgag-cagttcttc | CASSARTSG-GLDEQFF | 1 | 1,67 | 1,67 | |

(continued)

TRBV2 clonotypes obtained from 8 Controller cell lines, 8 treated patient cell lines, and 4 ex vivo Controller samples are listed. The V(D)J gene nomenclature is that of the IMGT database (www.imgt.org). Public clonotypes are in bold type. Clonotypes tested functionally are marked by an asterisk.

| **HIC 1 DR15+** | | | | | | | | |
|---|---|---|---|---|---|---|---|---|
| **V-GENE** | **J-GENE** | **D-GENE** | **JUNCTION** | **JUNCTION (AA)** | **frequency** | **% nt seq** | **%AA seq** | **JUNCTION nt numb er** |
| TRBV2*01 | TRBJ2-3*01 | TRBD2*01 | tgtgccagcagt-gccaggactagcg-ggggctcggatacg-cagtatttt | **CASSARTS-GGSDTQYF** | 1 | 1,67 | 1,67 | 48 |
| TRBV2*01, or TRBV2*02 | TRBJ2-3*01 | TRBD2*01 | tgtgccagcagtaa-gagggctagcg-ggggcacagatacg-cagtatttt | CASSKRASGGT-DTQYF | 2 | 3,33 | 3,33 | |
| TRBV2*01, or TRBV2*02 or TRBV2*03 | TRBJ2-3*01 | TRBD2*01 | tgtgccag-cagcctaag-gactagcgggggttca-gatacgcagtatttt | **CASSLRTS-GGSDTQYF** | 1 | 1,67 | 1,67 | |
| TRBV2*01 | TRBJ2-1*01 | TRBD2*01 | tgtgccag-cagccgcttgac-gagcggggggcg-gaatgagcagttcttc | CASSRLTSGG RNEQFF | 2 | 3,33 | 3,33 | |
| TRBV2*01 | TRBJ2-3*01 | TRBD2*01 | tgtgccagcagttctaa-gactagcg-ggggcacagatacg-cagtatttt | CASSSKTSGGT-DTQYF | 1 | 1,67 | 1,67 | |
| TRBV2*01 | TRBJ2-1*01 | TRBD2*01 | tgtgccagcagt-tcaaggactagcg-ggggccaagatgag-cagttcttc | CASSSRTSG-GQDEQFF | 1 | 1,67 | 1,67 | |
| TRBV2*01 | TRBJ1-5*01 | TRBD2*01 | tgtgccacctccagag-gagcgcggggaag-caatcagccccag-catttt | CATSRGARGSN-QPQHF | 34 | 56,67 | 56,67 | |

(continued)

TRBV2 clonotypes obtained from 8 Controller cell lines, 8 treated patient cell lines, and 4 ex vivo Controller samples are listed. The V(D)J gene nomenclature is that of the IMGT database (www.imgt.org). Public clonotypes are in bold type. Clonotypes tested functionally are marked by an asterisk.

| **HIC 1 DR15+** | | | | | | | | |
|---|---|---|---|---|---|---|---|---|
| **V-GENE** | **J-GENE** | **D-GENE** | **JUNCTION** | **JUNCTION (AA)** | **frequency** | **% nt seq** | **%AA seq** | **JUNCTION nt numb er** |
| | | | Total | 23 | 60 | 100,00 | 100,00 | |
| **HIC 2 DRB5+** | | | | | | | | |
| TRBV2*01, or TRBV2*02 or TRBV2*03 | TRBJ1-2*01 | TRBD1*01 | tgtgccagcgccag-gacagggggcgtt-ggctacaccttc | CASARTGGV-GYTF | 1 | 0,94 | 0,94 | 39 |
| TRBV2*01, or TRBV2*02 or TRBV2*03 | TRBJ2-7*01 | TRBD2*02 | tgtgccagcag-gactagcgggac-ctacgagcagtacttc | CASRTSG-TYEQYF | 1 | 0,94 | 0,94 | |
| TRBV2*01, or TRBV2*02 or TRBV2*03 | TRBJ2-7*01 | TRBD1*01 | tgtgccagcaaag-caaaaacggtaacct-acaagcagtacttc | CASKAKTV-TYKQYF | 1 | 0,94 | 0,94 | 42 |
| TRBV2*01, or TRBV2*02 or TRBV2*03 | TRBJ2-7*01 | TRBD2*01 | tgtgccagcaaac-caaaagcggtaac-ctacgagcagtacttc | CASKPKAV-TYEQYF | 1 | 0,94 | 0,94 | |
| TRBV2*01, or TRBV2*02 or TRBV2*03 | TRBJ1-3*01 | TRBD1*01 | tgtgccagcagag-ggacagcgact-ggaaacaccatatatttt | CASRGTATGNTI-YF | 1 | 0,94 | 0,94 | |
| TRBV2*01, or TRBV2*02 or TRBV2*03 | TRBJ1-4*01 | TRBD1*01 | tgtgccagcagac-cgacagcaactaat-gaaaaactgtttttt | CASRPTAT-NEKLFF | 1 | 0,94 | 0,94 | |
| TRBV2*01, or TRBV2*02 or TRBV2*03 | TRBJ2-1*01 | TRBD2*01 | tgtgccagcagt-gaatatgcgactag-caatgagcagttcttc | CASSEYAT-SNEQFF | 1 | 0,94 | 0,94 | |
| TRBV2*01, or TRBV2*02 or TRBV2*03 | TRBJ1-1*01 | TRBD1*01 | tgtgccagcagtcgt-ggacagcggcaca-gatacgcagtattt | CASS-RGQRHRYAVF | 1 | 0,94 | 0,94 | |

(continued)

| HIC 2 DRB5+ | | | | | | | | |
|---|---|---|---|---|---|---|---|---|
| TRBV2*01, or TRBV2*02 or TRBV2*03 | TRBJ2-7*01 | TRBD2*02 | tgtgccatctcgcgtcta<br>gcgggaggcatggac-<br>gagcagtacttc | CAISRLAGGMD-<br>EQYF | 1 | 0,94 | 0,94 | 45 |
| TRBV2*01, or TRBV2*02 or TRBV2*03 | TRBJ2-3*01 | TRBD2*01 | tgtgccagcg-<br>gccgactagcatcg-<br>ggcacagatacgcag-<br>tatttt | CASGRLASGT-<br>DTQYF | 2 | 1,89 | 1,89 | |
| TRBV2*01, or TRBV2*02 or TRBV2*03 | TRBJ2-2*01 | TRBD2*01 | tgtgccagcaggag-<br>ggggactagcggcac-<br>cggggagctgtttttt | CASRRGTSGT-<br>GELFF | 1 | 0,94 | 0,94 | |
| TRBV2*01, or TRBV2*02 or TRBV2*03 | TRBJ2-7*01 | TRBD2*01 | tgtgccagcagtgat-<br>ggggctagcggggt-<br>gggagagcagtacttc | CASSDGAS-<br>GVGEQYF | 2 | 1,89 | 1,89 | |
| TRBV2*01, or TRBV2*02 or TRBV2*03 | TRBJ1-6*02 | TRBD1*01 | tgtgccagcagt-<br>gaagctgccagggg-<br>taattcacccctccacttt | CASSEAARGNS-<br>PLHF | 2 | 1,89 | 1,89 | |
| TRBV2*01, or TRBV2*02 or TRBV2*03 | TRBJ2-7*01 | TRBD2*01 | tgtgccagcagtgaag-<br>gggctagcgggctcg-<br>gggagcagtacttc | CASSEGAS-<br>GLGEQYF | 1 | 0,94 | 0,94 | |
| TRBV2*01, or TRBV2*02 or TRBV2*03 | TRBJ2-1*01 | TRBD2*02 | tgtgccagcagt-<br>gaactggctagcg-<br>ggataagtgagcagt-<br>tcttc | CASSELASGISE-<br>QFF | 1 | 0,94 | 0,94 | |
| TRBV2*01, or TRBV2*02 or | TRBJ2-1*01 | TRBD2*01 | tgtgccagcagt-<br>gaacttgctagcg-<br>ggctcgcagagcagt-<br>tcttc | CASSELAS-<br>GLAEQFF | 2 | 1,89 | 1,89 | |
| TRBV2*03 | | | | | | | | |

(continued)

| **HIC 2 DRB5+** | | | | | | | | |
|---|---|---|---|---|---|---|---|---|
| TRBV2*01, or TRBV2*02 or TRBV2*03 | TRBJ2-1*01 | TRBD2*02 | tgtgccagcagt-gaactggctagcg-ggacgggtgagcagt-tcttc | CASSELASGT-GEQFF | 1 | 0,94 | 0,94 | |
| TRBV2*01, or TRBV2*02 or TRBV2*03 | TRBJ2-7*01 | TRBD2*02 | tgtgccagcagt-gaacgggctagcg-ggaccgacgagcag-tacttc | CASSERASGTD-EQYF | 2 | 1,89 | 1,89 | |
| TRBV2*01, or TRBV2*02 or TRBV2*03 | TRBJ2-2*01 | TRBD2*01 | tgtgccagcagt-gaaagggctagcg-gggtcggggagct-gtttttt | CASSERAS-GVGELFF | 2 | 1,89 | 1,89 | |
| TRBV2*01, or TRBV2*02 or TRBV2*03 | TRBJ2-3*01 | TRBD2*01 | tgtgccagcagcg-gactggctagcg-gcacagatacgcag-tatttt | CASSGLASGT-DTQYF | 2 | 1,89 | 1,89 | |
| TRBV2*01, or TRBV2*02 or TRBV2*03 | TRBJ2-2*01 | TRBD2*01 | tgtgccagttcg-ggactagcgtcg-ggcaccggggagct-gtttttt | CASSGLASGT-GELFF | 1 | 0,94 | 0,94 | |
| TRBV2*01, or TRBV2*02 or TRBV2*03 | TRBJ2-7*01 | TRBD2*01 | tgtgccagcagcg-ggatgactagccgatc-ctacgagcagtacttc | CASSGMT-SRSYEQYF | 3 | 2,83 | 2,83 | |
| TRBV2*01, or TRBV2*02 or TRBV2*03 | TRBJ2-2*01 | TRBD2*01 | tgtgccagcagcccg-ggactagccggcac-cggggagctgtttttt | CASSPGLAGT-GELFF | 1 | 0,94 | 0,94 | |
| TRBV2*01, or TRBV2*02 or TRBV2*03 | TRBJ2-3*01 | TRBD2*01 | tgtgccagcagtccgtt-gactagcggaacaga-tacgcagtatttt | **CASSPLTSGT-DTQYF** | 1 | 0,94 | 0,94 | |

(continued)

| HIC 2 DRB5+ | | | | | | | | |
|---|---|---|---|---|---|---|---|---|
| TRBV2*01, or TRBV2*02 or TRBV2*03 | TRBJ1-5*01 | TRBD1*01 | tgtgccagcagtc-ctcgggccag-ggggaat-cagccccagcatttt | CASSPRARGN-QPQHF | 2 | 1,89 | 1,89 | |
| TRBV2*01, or TRBV2*02 or TRBV2*03 | TRBJ2-7*01 | TRBD2*01 | tgtgccag-cagccctcg-gactagcggtccctac-gagcagtacttc | CASSPRTSG-PYEQYF | 1 | 0,94 | 0,94 | |
| TRBV2*01, or TRBV2*02 or TRBV2*03 | TRBJ2-7*01 | TRBD2*02 | tgtgccagcagtc-ctcggactagcg-ggagttacgagcag-tacttc | CASSPRTSG-SYEQYF | 1 | 0,94 | 0,94 | |
| TRBV2*01, or TRBV2*02 or TRBV2*03 | TRBJ2-2*01 | TRBD2*01 | tgtgccag-cagccaag-ggctagcgggcaccg-gggagctgtttttt | CASSQGLAGT-GELFF | 1 | 0,94 | 0,94 | |
| TRBV2*01, or TRBV2*02 or TRBV2*03 | TRBJ2-3*01 | TRBD1*01 | tgtgccagcagtcaatt-agctaggggcacaga-tacgcagtatttt | CASSQLARGT-DTQYF | 1 | 0,94 | 0,94 | |
| TRBV2*01, or TRBV2*02 or TRBV2*03 | TRBJ2-7*01 | TRBD1*01 | tgtgccagcagt-caacttgtatcgctgag-gggggagcagtacttc | CAS-SQLVSLRGEQYF | 1 | 0,94 | 0,94 | |
| TRBV2*01, or TRBV2*02 or TRBV2*03 | TRBJ2-7*01 | TRBD2*02 | tgtgccagcagt-caacggactagcg-ggagcgacgagcag-tacttc | CASSQRTSGSD-EQYF | 1 | 0,94 | 0,94 | |
| TRBV2*01, or TRBV2*02 or TRBV2*03 | TRBJ2-3*01 | TRBD2*01 | tgtgccag-cagccaagttgcg-gggggtacagctacg-cagtatttt | CASSQVAGG-TATQYF | 1 | 0,94 | 0,94 | |

(continued)

| HIC 2 DRB5+ | | | | | | | | |
|---|---|---|---|---|---|---|---|---|
| TRBV2*01, or TRBV2*02 or TRBV2*03 | TRBJ1-5*01 | TRBD2*01 | tgtgccagctcccg-gggggctcg-gggcaat-cagccccagcatttt | CASSRGARGN-QPQHF | 1 | 0,94 | 0,94 | |
| TRBV2*01, or TRBV2*02 or TRBV2*03 | TRBJ2-1 *01 | TRBD2*02 | tgtgccag-cagccgactagcg-ggaggtttaggtgag-cagttcttc | CASSRLAG-GLGEQFF | 2 | 1,89 | 1,89 | |
| TRBV2*01, or TRBV2*02 or TRBV2*03 | TRBJ2-1 *01 | TRBD2*02 | tgtgccag-cagccgactagcg-ggagggatggatgag-cagttcttc | **CASSRLAGGMD-EQFF*** | 11 | 10,38 | 10,38 | |
| TRBV2*01, or TRBV2*02 or TRBV2*03 | TRBJ2-1 *01 | TRBD2*02 | tgtgccag-cagccgactagcg-ggagggacggat-gagcagttcttc | CASSRLAGGTD-EQFF | 1 | 0,94 | 0,94 | |
| TRBV2*01, or TRBV2*02 or TRBV2*03 | TRBJ2-3*01 | TRBD2*01 | tgtgccagcagtcgtct-gactagcggcacaga-tacgcagtatttt | CASSRLTSGT-DTQYF | 2 | 1,89 | 1,89 | |
| TRBV2*01, or TRBV2*02 or TRBV2*03 | TRBJ2-1 *01 | TRBD2*02 | tgtgccag-cagccgagtgacg-ggagggatggatgag-cagttcttc | CASSRVTGGMD-EQFF | 1 | 0,94 | 0,94 | |
| TRBV2*01, or TRBV2*02 or TRBV2*03 | TRBJ2-1 *01 | TRBD2*01 | tgtgccagcac-caaactagcgggggg-tacatctgagcagttct-tc | CASTKLAGGT-SEQFF | 1 | 0,94 | 0,94 | |
| TRBV2*01, or TRBV2*02 or TRBV2*03 | TRBJ2-3*01 | TRBD2*01 | tgtgccagcac-caaactagcgt-ggggcacatatacg-cagtatttt | CASTKLAWG-TYTQYF | 1 | 0,94 | 0,94 | |

(continued)

| | | | | | | | | |
|---|---|---|---|---|---|---|---|---|
| **HIC 2 DRB5+** | | | | | | | | |
| TRBV2*01, or TRBV2*02 or TRBV2*03 | TRBJ2-1 *01 | TRBD2*02 | tgtgccaccacccccg-gggctagcggga-taagtgagcagttcttc | CATTPGASGISE-QFF* | 35 | 33,02 | 33,96 | |
| TRBV2*01, or TRBV2*02 or TRBV2*03 | TRBJ2-1 *01 | TRBD1*01 | tgtgccaccacccccg-gggctagtggga-taagtgagcagttcttc | | 1 | 0,94 | | |
| TRBV2*01, or TRBV2*02 or TRBV2*03 | TRBJ2-7*01 | TRBD1*01 | tgtgccagcagt-gaaaggggacag-ggggcgcggtacgag-cagtacttc | CASSERGQ-GARYEQYF | 1 | 0,94 | 0,94 | 48 |
| TRBV2*01, or TRBV2*02 or TRBV2*03 | TRBJ2-3*01 | TRBD2*01 | tgtgccagcagtcgtat-gactggcg-ggggcacagatacg-cagtatttt | CASSRMTGGGT-DTQYF | 1 | 0,94 | 0,94 | |
| TRBV2*01, or TRBV2*02 or | TRBJ2-3*01 | TRBD2*01 | tgtgccagcagtcg-taggactagcg-ggggcacagatacg-cagtatttt | **CASSRRTSGGT-DTQYF*** | 6 | 5,66 | 5,66 | |
| TRBV2*03 | | | | | | | | |
| | | | Total | 44 | 106 | 100,00 | 100,00 | |
| **HIC 3 DR11+** | | | | | | | | |
| TRBV2*01, or TRBV2*02 or TRBV2*03 | TRBJ2-3*01 | TRBD1*01 | tgtgccagtcaccgga-catacacagatacg-cagtatttt | CASHRTYT-DTQYF | 2 | 1,60 | 1,60 | 39 |
| TRBV2*01, or TRBV2*02 or TRBV2*03 | TRBJ1-1*01 | TRBD1*01 | tgtgccagctcag-gacagacgaacact-gaagctttcttt | **CASSGQT-NTEAFF** | 10 | 8,00 | 8,00 | |

(continued)

| **HIC 3 DR11+** | | | | | | | | |
|---|---|---|---|---|---|---|---|---|
| TRBV2*01, or TRBV2*02 or TRBV2*03 | TRBJ1-2*01 | TRBD1*01 | tgtgccagcagat-ggacagcaacctct-tatggctacaccttc | CASRWTATSY-GYTF | 15 | 12,00 | 12,00 | 42 |
| TRBV2*01, or TRBV2*02 or TRBV2*03 | TRBJ1-2*01 | TRBD1*01 | tgtgccagcagtc-ccacaacgacaggg-tatggctacaccttc | CASSPTTTGY-GYTF | 1 | 0,80 | 0,80 | |
| TRBV2*01, or TRBV2*02 or TRBV2*03 | TRBJ2-2*01 | TRBD1*01 | tgtgcctcccacgaag-gggccgggggcttcg-gggagctgtttttt | CASHEGAGGF-GELFF | 1 | 0,80 | 0,80 | 45 |
| TRBV2*01, or TRBV2*02 or TRBV2*03 | TRBJ2-2*01 | TRBD1*01 | tgtgcctcccacgaag-gggccgggggctacg-gggagctgtttttt | CASHEGAGGY-GELFF | 2 | 1,60 | 1,60 | |
| TRBV2*01, or TRBV2*02 or TRBV2*03 | TRBJ2-1 *01 | TRBD1*01 | tgtgccagcagtgccg-gaactagaggggt-gggggagcagttcttc | CAS-SAGTRGVGEQF F | 1 | 0,80 | 0,80 | |
| TRBV2*01, or TRBV2*02 or TRBV2*03 | TRBJ2-3*01 | TRBD2*01 | tgtgccagcagtgcttt-ggctagcggcacaga-tacgcagtatttt | **CASSALASGT-DTQYF** | 1 | 0,80 | 0,80 | |
| TRBV2*01, or TRBV2*02 or TRBV2*03 | TRBJ2-7*01 | TRBD2*01 | tgtgccagcagt-gacgcggctagcggt-gtgggcgagcagtact-tc | CASSDAAS-GVGEQYF | 6 | 4,80 | 4,80 | |
| TRBV2*01, or TRBV2*02 or TRBV2*03 | TRBJ2-1 *01 | TRBD2*02 | tgtgccagcagtgatct-ggctagcgggac-gaatgagcagttcttc | CASSDLASGT-NEQFF | 1 | 0,80 | 0,80 | |
| TRBV2*01, or TRBV2*02 or TRBV2*03 | TRBJ2-1 *01 | TRBD2*01 | tgtgccagcagtgac-cgggctagcg-gggtcggggagcagt-tcttc | CASSDRAS-GVGEQFF | 1 | 0,80 | 0,80 | |

(continued)

| HIC 3 DR11+ | | | | | | | | |
|---|---|---|---|---|---|---|---|---|
| TRBV2*01, or TRBV2*02 or TRBV2*03 | TRBJ2-1 *01 | TRBD2*01 | tgtgccagcagt-gacaggactagcg-gtccccatgagcagt-tcttc | CASSDRTSG-PHEQFF | 6 | 4,80 | 4,80 | |
| TRBV2*01, or TRBV2*02 or TRBV2*03 | TRBJ2-1 *01 | TRBD2*02 | tgtgccagcagcg-gactagcgggag-gaatggatgagcagt-tcttc | CASSGLAGGMD-EQFF* | 7 | 5,60 | 5,60 | |
| TRBV2*01, or TRBV2*02 or TRBV2*03 | TRBJ2-1 *01 | TRBD1*01 | tgtgccagcagccccg-gggcgagaggaatt-gatgagcagttcttc | CASSPGARGID-EQFF | 1 | 0,80 | 0,80 | |
| TRBV2*01, or TRBV2*02 or TRBV2*03 | TRBJ2-1 *01 | TRBD2*01 | tgtgcaag-cagccccg-ggactagcggagtt-ggtgagcagttcttc | **CASSPGTS-GVGEQFF*** | 15 | 12,00 | 12,80 | |
| TRBV2*01, or TRBV2*02 or TRBV2*03 | TRBJ2-1 *01 | TRBD2*01 | tgtgcaag-cagccccg-ggactagcggagtt-ggtgagcagtttttc | | 1 | 0,80 | | |
| TRBV2*01, or TRBV2*02 or TRBV2*03 | TRBJ2-1 *01 | TRBD1*01 | tgtgcaag-cagccccgggac-gagcggagttgg-taagcagtttttc | CASSPGTSGVG-KQFF | 1 | 0,80 | 0,80 | |
| TRBV2*01, or TRBV2*02 or TRBV2*03 | TRBJ2-7*01 | TRBD2*01 | tgtgccag-cagccccag-gactagcgggggag-gcgagcagtacttc | CASSPRTSG-GGEQYF | 1 | 0,80 | 0,80 | |
| TRBV2*01, or TRBV2*02 or TRBV2*03 | TRBJ1-5*01 | TRBD1*01 | tgtgccagcagtc-ccagtgctcgcg-gcaatcagccccag-catttt | CASSPSARGN-QPQHF | 2 | 1,60 | 1,60 | |

(continued)

| | | | | | | | | |
|---|---|---|---|---|---|---|---|---|
| **HIC 3 DR11+** | | | | | | | | |
| TRBV2*01, or TRBV2*02 or TRBV2*03 | TRBJ2-7*01 | TRBD2*02 | tgtgccag-cagcccgac-gactagcgggagag-gcgagcagtacttc | CASSPTTS-GRGEQYF | 2 | 1,60 | 1,60 | |
| TRBV2*01, or TRBV2*02 or TRBV2*03 | TRBJ2-1 *01 | TRBD2*01 | tgtgccagcagttccg-ggactagcggggccg-gggagcagttcttc | CASSSGTS-GAGEQFF | 1 | 0,80 | 0,80 | |
| TRBV2*01, or TRBV2*02 or TRBV2*03 | TRBJ2-1 *01 | TRBD2*01 | tgtgccagcagttccg-ggactagcggagtt-ggtgagcagttcttc | CASSSGTS-GVGEQFF | 1 | 0,80 | 27,20 | |
| TRBV2*01, or TRBV2*02 or TRBV2*03 | TRBJ2-1 *01 | TRBD2*01 | tgtgccagcagttccg-ggactagcggggtcg-gggagcagttcttc | CASSSGTS-GVGEQFF | 33 | 26,40 | | |
| TRBV2*01, or TRBV2*02 or TRBV2*03 | TRBJ2-7*01 | TRBD2*01 | tgtgccagcagtgtcg-ggactagcggggt-gggcgagcagtacttc | CASSVGTS-GVGEQYF | 11 | 8,80 | 8,80 | |
| TRBV2*01, or TRBV2*02 or TRBV2*03 | TRBJ2-7*01 | TRBD2*01 | tgtgccagcagttacg-gggctagcggggt-gggggagcagttcttc | CASSYGAS-GVGEQFF | 1 | 0,80 | 0,80 | |
| TRBV2*01, or TRBV2*02 or TRBV2*03 | TRBJ2-1 *01 | TRBD2*01 | tgtgccagcagtta-caggactagcg-ggccccgggagcagt-tcttc | CASSYRTSG-PREQFF | 1 | 0,80 | 0,80 | |
| | | | Total | 24 | 125 | 100,00 | 100,00 | |
| **HIC 4 DR15+** | | | | | | | | |
| TRBV2*01, or TRBV2*02 or | TRBJ1-2*01 | TRBD2*01 | tgtgccagcaggaaa-gaaggatctaggcta-cacctc | CASRKEGSRLHL | 1 | 0,66 | 0,66 | 36 |
| TRBV2*03 | | | | | | | | |

(continued)

| HIC 4 DR15+ | | | | | | | | |
|---|---|---|---|---|---|---|---|---|
| TRBV2*01, or TRBV2*02 or TRBV2*03 | TRBJ1-2*01 | TRBD1*01 | tgtgccagcagtgaca-gaacgacatgtggcta-caccttc | CASSDRTTC-GYTF | 1 | 0,66 | 0,66 | 39 |
| TRBV2*01, or TRBV2*02 or TRBV2*03 | TRBJ1-2*01 | TRBD1*01 | tgtgccagcagtgaaa-gaaggatctatggcta-caccttc | CASSERRIY-GYTF | 5 | 3,29 | 3,29 | |
| TRBV2*01, or TRBV2*02 or TRBV2*03 | TRBJ2-1 *01 | TRBD2*01 | tgtgccagca-gagccctagcgtcg-gggggtgagcagttct-tc | CASRALAS-GGEQFF | 5 | 3,29 | 3,29 | 42 |
| TRBV2*01, or TRBV2*02 or TRBV2*03 | TRBJ2-3*01 | TRBD2*01 | tgtgccagcagtgctt-tagctagcggaga-taagcagtatttt | CASSALASGD-KQYF | 1 | 0,66 | 0,66 | |
| TRBV2*01, or TRBV2*02 or TRBV2*03 | TRBJ2-3*01 | TRBD2*01 | tgtgccagcagtgcttt-ggctagcggaga-tacgcagtatttt | CASSALAS-GDTQYF | 1 | 0,66 | 0,66 | |
| TRBV2*01, or TRBV2*02 or TRBV2*03 | TRBJ2-1 *01 | TRBD1*01 | tgtgccagcagtgac-gacagggtcggcgat-gagcagttcttc | CASSDDRVGD-EQFF | 1 | 0,66 | 0,66 | |
| TRBV2*01, or TRBV2*02 or TRBV2*03 | TRBJ2-1 *01 | TRBD2*01 | tgtgccagcag-caaactagcgtctgga-gatgagcagttcttc | CASSKLASGD-EQFF | 4 | 2,63 | 2,63 | |
| TRBV2*01, or TRBV2*02 or TRBV2*03 | TRBJ2-1 *01 | TRBD2*02 | tgtgccagcagtgttt-tacctggaggcaat-gatccgctcttc | CASSVLPGGND-PLF | 1 | 0,66 | 0,66 | |
| TRBV2*01, or TRBV2*02 or TRBV2*03 | TRBJ2-1 *01 | TRBD2*01 | tgtgccagcagtgttt-tacctggtcgcaat-gagccgttcttc | CASSVLP-GRNEPFF | 1 | 0,66 | 0,66 | |
| TRBV2*01, or TRBV2*02 or TRBV2*03 | TRBJ2-1 *01 | TRBD1*01 | tgcgccagcagtgttt-tacgtggtggcaat-gagcagtttttc | CASSVL-RGGNEQFF | 1 | 0,66 | 0,66 | |

(continued)

| HIC 4 DR15+ | | | | | | | | |
|---|---|---|---|---|---|---|---|---|
| TRBV2*01, or TRBV2*02 or TRBV2*03 | TRBJ2-1 *01 | TRBD2*01 | tgtgccagcagtgttt-tacgtggtcgcaat-gagccgttcttc | CASSVLR-GRNEPFF | 1 | 0,66 | 0,66 | |
| TRBV2*01, or TRBV2*02 or TRBV2*03 | TRBJ2-1 *01 | | tgtgccagcagtgttt-tacgtggtcgcaat-gagcagttcttc | CASSVLRG RNEQFF | 8 | 5,26 | 5,26 | |
| TRBV2*01, or TRBV2*02 or TRBV2*03 | TRBJ2-1 *01 | TRBD2*02 | tgtgccagcagtgtt-tcacgaggt-ggcaataagcagtttttc | CASSVS-RGGNKQFF | 1 | 0,66 | 0,66 | |
| TRBV2*01, or TRBV2*02 or TRBV2*03 | TRBJ2-1 *01 | TRBD1*01 | tgtgccagtgtattaat-gaggacgaacaat-gagcagttcttc | **CASVLM-RTNNEQFF** | 9 | 5,92 | 5,92 | |
| TRBV2*01, or TRBV2*02 or TRBV2*03 | TRBJ2-1 *01 | TRBD2*01 | tgctccagcagagcta-gagggtgcgcggg-taagcagtatttc | CSSRARGCAG-KQYF | 1 | 0,66 | 0,66 | |
| TRBV2*01, or TRBV2*02 or TRBV2*03 | TRBJ2-1 *01 | TRBD2*01 | tgtgccagcagt-gccctgactagcg-ggggcgatgagcagt-tcttc | CASSALTSGGD-EQFF | 1 | 0,66 | 0,66 | |
| TRBV2*01, or TRBV2*02 or TRBV2*03 | TRBJ2-1 *01 | TRBD2*01 | tgtgccagcagt-gcaaggactagcg-ggggatccgagcagt-tcttc | CASSARTS-GGSEQFF | 2 | 1,32 | 1,32 | |
| TRBV2*01, or TRBV2*02 or TRBV2*03 | TRBJ2-7*01 | TRBD2*02 | tgtgccagcagt-gctaggactagcg-ggagtgacgagcag-tacttc | CASSARTSGSD-EQYF | 2 | 1,32 | 1,32 | |
| TRBV2*01, or TRBV2*02 or TRBV2*03 | TRBJ2-3*01 | TRBD1*01 | tgtgccagcagt-gacagggcctcag-gcggggatacgcag-tatttt | CASSDRASG-GDTQYF | 1 | 0,66 | 0,66 | |

(continued)

| HIC 4 DR15+ | | | | | | | | |
|---|---|---|---|---|---|---|---|---|
| TRBV2*01, or TRBV2*02 or TRBV2*03 | TRBJ2-3*01 | TRBD2*01 | tgtgccagcagt-gatcgggctagcg-ggggggatacgcag-tatttt | CASSDRASG-GDTQYF | 1 | 0,66 | 0,66 | 45 |
| TRBV2*01, or TRBV2*02 or TRBV2*03 | TRBJ2-3*01 | TRBD1*01 | tgtgccagcagt-gatcgggctacag-ggggggatacgcag-tatttt | CASSDRAT-GGDTQYF | 1 | 0,66 | 0,66 | |
| TRBV2*01, or TRBV2*02 or TRBV2*03 | TRBJ2-3*01 | TRBD2*01 | tgtgccagcagt-gaaaaggctagcg-ggggggatacgcag-tatttt | CASSEKASG-GDTQYF | 1 | 0,66 | 0,66 | |
| TRBV2*01, or TRBV2*02 or TRBV2*03 | TRBJ2-1 *01 | TRBD2*01 | tgtgccagcagtgaatt-ggctagcg-ggggggatgagcagt-tcttc | CASSELASGGD-EQFF | 2 | 1,32 | 1,32 | |
| TRBV2*01, or TRBV2*02 or TRBV2*03 | TRBJ2-3*01 | TRBD1*01 | tgtgccagcagt-cacaaggcttcag-ggggggataagcag-tatttt | CASSHKASGGD-KQYF | 1 | 0,66 | 0,66 | |
| TRBV2*01, or TRBV2*02 or TRBV2*03 | TRBJ2-3*01 | TRBD1*01 | tgtgccagctctcacat-ggcctcaggcggcga-tacgcagtatttt | CASSHMASG-GDTQYF | 1 | 0,66 | 0,66 | |
| TRBV2*01, or TRBV2*02 or TRBV2*03 | TRBJ2-3*01 | TRBD1*01 | tgtgccagcagt-cacagggcctcag-gcggggctactccg-tatttt | CASSHRASG-GATPYF | 1 | 0,66 | 0,66 | |
| TRBV2*01, or TRBV2*02 or TRBV2*03 | TRBJ2-3*01 | TRBD1*01 | tgtgccagcagtcac-cgggcctcaggt-ggggatactccgcatttt | CASSHRASG-GDTPHF | 1 | 0,66 | 0,66 | |

(continued)

| | | | | | | | | |
|---|---|---|---|---|---|---|---|---|
| **HIC 4 DR15+** | | | | | | | | |
| TRBV2*01, or TRBV2*02 or TRBV2*03 | TRBJ2-3*01 | TRBD1*01 | tgtgccagcagt-cacagggcctcag-gcggggatacgcag-tatttt | **CASSHRASG-GDTQYF** | 31 | 20,39 | 20,39 | |
| TRBV2*01, or TRBV2*02 or | TRBJ2-7*01 | TRBD2*01 | tgtgccagct-caaaactggctagcg-gggccgacgagcag-tacttc | **CASSKLASGAD-EQYF** | 6 | 3,95 | 4,61 | |
| TRBV2*03 | | | | | | | | |
| TRBV2*01, or TRBV2*02 or TRBV2*03 | TRBJ2-7*01 | TRBD2*01 | tgtgccagct-caaaactggctagcg-gggccgacgagcag-tatttc | | 1 | 0,66 | | |
| TRBV2*01, or TRBV2*02 | TRBJ2-7*01 | TRBD1*01 | tgtgccagct-caaaacttactag-gggcgcagataag-cagtatttc | CASSKLTRGAD-KQYF | 1 | 0,66 | 0,66 | |
| TRBV2*01, or TRBV2*02 or TRBV2*03 | TRBJ2-7*01 | TRBD2*01 | tgtgccagcagtaa-gaggactagcggtac-ctacgagcagtacttc | CASSKRTSG-TYEQYF | 1 | 0,66 | 0,66 | |
| TRBV2*01, or TRBV2*02 or TRBV2*03 | TRBJ2-7*01 | TRBD2*01 | tgtgccagcagcctc-cggactagcggctc-ctacgagcagtacttc | CASSLRTSG-SYEQYF | 2 | 1,32 | 1,32 | |
| TRBV2*01, or TRBV2*02 or TRBV2*03 | TRBJ2-7*01 | TRBD2*01 | tgtgccagcagtc-cccggactagcgg-tacctacgagcag-tacttc | **CASSPRTSG-TYEQYF** | 1 | 0,66 | 0,66 | |
| TRBV2*01, or TRBV2*02 or TRBV2*03 | TRBJ2-2*01 | TRBD2*01 | tgtgccagctcgccgcg agtcttctctgtcg-gggagctgtttttt | CASSPRVFS-VGELFF | 4 | 2,63 | 2,63 | |

(continued)

| **HIC 4 DR15+** | | | | | | | | |
|---|---|---|---|---|---|---|---|---|
| TRBV2*01, or TRBV2*02 or TRBV2*03 | TRBJ2-1 *01 | TRBD2*01 | tgtgccag-cagccaaag-ggctagcg-ggggggacgagcagttcttc | CASSQRASGGD-EQFF | 2 | 1,32 | 1,32 | |
| TRBV2*01, or TRBV2*02 or TRBV2*03 | TRBJ2-7*01 | TRBD2*02 | tgtgccagcagta-gacggactagcg-ggacctacgagcag-tacttc | **CASSRRTSG-TYEQYF** | 1 | 0,66 | 0,66 | |
| TRBV2*01, or TRBV2*02 or TRBV2*03 | TRBJ2-7*01 | TRBD2*01 | tgtgccagcagcgtc-cggactagcgggtc-ctacgagcagtacttc | CASSVRTSG-SYEQYF | 1 | 0,66 | 0,66 | |
| TRBV2*01, or TRBV2*02 or TRBV2*03 | TRBJ2-7*01 | TRBD2*02 | tgcaccagcagtgg-taggactagcgggag-ggataagcagtatttc | CTSSGRTSGRD-KQYF | 1 | 0,66 | 0,66 | |
| TRBV2*01, or TRBV2*02 or TRBV2*03 | TRBJ2-3*01 | TRBD1*01 | tgtaccagcagt-cacagggcctcag-gcggggatacgcag-tatttt | CTSSHRASG-GDTQYF | 1 | 0,66 | 0,66 | |

(continued)

| | | | | | | | | |
|---|---|---|---|---|---|---|---|---|
| **HIC 4 DR15+** | | | | | | | | |
| TRBV2*01, or TRBV2*02 or TRBV2*03 | TRBJ2-7*01 | TRBD2*01 | tgtgccagcagt-gccaggattagcg-gggggctcaacgag-cagtacttc | CASSARISG-GLNEQYF | 1 | 0,66 | 0,66 | 48 |
| TRBV2*01, or TRBV2*02 or TRBV2*03 | TRBJ2-3*01 | TRBD2*01 | tgtgccagcagt-gcaaggactagcg-ggggggccgatacg-cagtatttt | **CASSARTSG-GADTQYF** | 1 | 0,66 | 0,66 | |
| TRBV2*01, or TRBV2*02 or TRBV2*03 | TRBJ2-7*01 | TRBD2*01 | tgtgccagcagt-gccaggactagcg-gggggcttgacgag-cagtacttc | CASSARTSG-GLDEQYF | 19 | 12,50 | 12,50 | |
| TRBV2*01, or | TRBJ2-3*01 | TRBD2*01 | tgtgccagcagt-gctcggactagcg-gggggtcagatacg-cagtatttt | **CASSARTS-GGSDTQYF** | 3 | 1,97 | 1,97 | |
| TRBV2*02 or TRBV2*03 | | | | | | | | |
| TRBV2*01, or TRBV2*02 or TRBV2*03 | TRBJ2-3*01 | TRBD2*01 | tgtgccagcag-taaaaggactagcg-ggggggccgatacg-cagtatttt | CASSKRTSG-GADTQYF | 2 | 1,32 | 1,32 | |
| TRBV2*01, or TRBV2*02 or TRBV2*03 | TRBJ2-3*01 | TRBD2*01 | tgtgccagcagtcttag-gactagcg-ggggcacagatacg-cagtatttt | **CASSLRTSGGT-DTQYF** | 1 | 0,66 | 0,66 | |
| TRBV2*01, or TRBV2*02 or TRBV2*03 | TRBJ2-3*01 | TRBD2*01 | tgtgccag-cagccccag-gactagcg-ggggcacagatacgc agtatttt | CASSPRTSGGT-DTQYF | 5 | 3,29 | 3,29 | |

(continued)

| HIC 4 DR15+ | | | | | | | | |
|---|---|---|---|---|---|---|---|---|
| TRBV2*01, or TRBV2*02 or TRBV2*03 | TRBJ2-3*01 | TRBD1*01 | tgtgccagctcac-gacgggcttccg-ggggcactactccg-cattatttt | CASSRRASGGT-TPHYF | 1 | 0,66 | 0,66 | |
| TRBV2*01, or TRBV2*02 or TRBV2*03 | TRBJ2-3*01 | TRBD2*01 | tgtgccagcagtc-gacggactagcg-ggggcacagatacg-cagtatttt | **CASSRRTSGGT-DTQYF*** | 1 | 0,66 | 1,32 | |
| TRBV2*01, or TRBV2*02 or TRBV2*03 | TRBJ2-3*01 | TRBD2*01 | tgtgccagctcac-gacggactagcg-ggggcacagatacg-cagtatttt | | 1 | 0,66 | | |
| TRBV2*01, or TRBV2*02 or TRBV2*03 | TRBJ2-3*01 | TRBD2*02 | tgtgccag-cagtcgtcg-gactagcgggag-ggcggatacgcagtatttt | CASSRRTS-GRADTQYF | 4 | 2,63 | 2,63 | |
| TRBV2*01, or TRBV2*02 or TRBV2*03 | TRBJ2-3*01 | TRBD2*02 | tgtgccagcagtcg-taggactagcg-ggagtctagatacg-cagtatttt | CASSR-RTSGSLDTQYF | 1 | 0,66 | 0,66 | |
| TRBV2*01, or TRBV2*02 or TRBV2*03 | TRBJ2-3*01 | TRBD1*01 | tgtgccagcagcac-caggattagag-ggggcacagataag-cagtatttt | CASSTRIRGGTD-KQYF | 1 | 0,66 | 0,66 | |
| TRBV2*01, or TRBV2*02 or TRBV2*03 | TRBJ2-3*01 | TRBD2*01 | tgtgccagcagtgttag-gactagcg-ggggcacagatacg-cagtatttt | CASSVRTSGGT-DTQYF | 1 | 0,66 | 0,66 | |
| | | | Total | 52 | 152 | 100,00 | 100,00 | |

(continued)

| HIC 5 DR1+ | | | | | | | | |
|---|---|---|---|---|---|---|---|---|
| TRBV2*01 | TRBJ1-2*01 | TRBD1*01 | tgtgccagcaga-gacagtaactatggct-acaccttc | CASRDSNYGYTF | 1 | 1,61 | 1,61 | 36 |
| TRBV2*02 | TRBJ2-5*01 | TRBD1*01 | tgtgccagcagtc-gacggacggagac-ccagtacttc | CASSRRTETQYF | 3 | 4,84 | 4,84 | |
| TRBV2*02 | TRBJ2-4*01 | TRBD1*01 | tgtgccagcagt-gaaaccagggccaa-cattcagtacttc | CASSETRA-NIQYF | 1 | 1,61 | 1,61 | 39 |
| TRBV2*01 | TRBJ2-1 *01 | TRBD2*01 | tgtgccagcagcg-gactagcggcgaat-gagcagttcttc | CASSG-LAANEQFF | 1 | 1,61 | 1,61 | |
| TRBV2*01, or TRBV2*02 | TRBJ1-1*01 | TRBD1*01 | tgtgcctcaagggcag-ggtcggtggccact-gaagctttcttt | CASRAGS-VATEAFF | 1 | 1,61 | 1,61 | 42 |
| TRBV2*01 | TRBJ2-1 *01 | TRBD2*01 | tgtgccagcagt-gggaggactagcg-gcaatgagcagttcttc | CASSGRTS-GNEQFF | 2 | 3,23 | 3,23 | |
| TRBV2*02 | TRBJ2-1 *01 | TRBD1*01 | tgtgccagcagtgtcgt-gggcagttacaatgag-cagttcttc | CASSVVGSYN-EQFF | 34 | 54,84 | 54,84 | |
| TRBV2*01 | TRBJ2-1 *01 | TRBD1*01 | tgtgccagtgtattaat-gaggacgaacaat-gagcagttcttc | **CASVLM-RTNNEQFF** | 4 | 6,45 | 6,45 | |
| TRBV2*02 | TRBJ2-2*01 | TRBD1*01 | tgtgccagcag-ggccgggacctcg-ggcaccggggagct-gtttttt | CASRAGTSGT-GELFF | 1 | 1,61 | 1,61 | |
| TRBV2*01, or TRBV2*02 | TRBJ2-7*01 | TRBD2*02 | tgtgccagcaggaag-gggactagcgggagt-ggcaagcagtacttc | CASRKGTSGSG-KQYF | 2 | 3,23 | 3,23 | |

(continued)

| | | | | | | | | |
|---|---|---|---|---|---|---|---|---|
| **HIC 5 DR1+** | | | | | | | | |
| TRBV2*02 | TRBJ2-1 *01 | TRBD2*01 | tgtgccagcagt-gaaaaggctagcg-gggtggatgagcagt-tcttc | CASSEKASGVD-EQFF | 1 | 1,61 | 1,61 | 45 |
| TRBV2*01 | TRBJ2-3*01 | TRBD2*01 | tgtgccagcagt-gaaagggctagcg-ggcacgatacgcag-tatttt | CASSERASGH-DTQYF | 1 | 1,61 | 1,61 | |
| TRBV2*01 | TRBJ2-3*01 | TRBD1*01 | tgtgccagcagt-cacagggcctcag-gcggggatacgcag-tatttt | **CASSHRASG-GDTQYF** | 1 | 1,61 | 1,61 | |
| TRBV2*01, or TRBV2*02 | TRBJ2-7*01 | TRBD2*01 | tgtgccagct-caaaactggctagcg-gggccgacgagcag-tacttc | **CASSKLASGAD-EQYF** | 1 | 1,61 | 1,61 | |
| TRBV2*01 | TRBJ2-7*01 | TRBD2*01 | tgtgccagcag-taaacaggctagcg-ggggggacgagcag-tacttc | CASSKQASGGD-EQYF | 8 | 12,90 | 12,90 | |
| | | | Total | 15 | 62 | 100,00 | 100,00 | |
| **HIC 6 DR11+** | | | | | | | | |
| TRBV2*01, or TRBV2*02 or TRBV2*03 | TRBJ2-7*01 | TRBD2*01 | tgtgccagcaga-gaatacgcgactag-caacgagcagtacttc | CASREYAT-SNEQYF | 1 | 1,52 | 1,52 | 42 |
| TRBV2*01, or TRBV2*02 or TRBV2*03 | TRBJ1-2*01 | TRBD2*01 | tgtgccagcagt-gaaatggcgaccg-ggttgcgctacaccttc | CASSEMAT-GLRYTF | 1 | 1,52 | 1,52 | |
| TRBV2*01, or TRBV2*02 or TRBV2*03 | TRBJ1-2*01 | TRBD1*01 | tgtgccagcagt-gaaacggcgacag-ggttgcgctacaccttc | CASSETAT-GLRYTF | 4 | 6,06 | 6,06 | |

(continued)

| HIC 6 DR11+ | | | | | | | | |
|---|---|---|---|---|---|---|---|---|
| TRBV2*01, or TRBV2*02 or TRBV2*03 | TRBJ1-6*02 | TRBD2*01 | tgtgccag-cacgctaacacgggt-taattcacccctccacttt | CASTLTRVNSPL-HF | 2 | 3,03 | 3,03 | |
| TRBV2*01, or TRBV2*02 or TRBV2*03 | TRBJ2-7*01 | TRBD2*01 | tgtgccagcaat-caacggactagcg-ggccttacgagcag-tacttc | CASNQRTSG-PYEQYF | 1 | 1,52 | 1,52 | |
| TRBV2*01, or TRBV2*02 or TRBV2*03 | TRBJ2-1 *01 | TRBD2*01 | tgtgccagcagtgattt-ggctagcggcacag-gggagcagttcttc | CASSDLASGT-GEQFF | 2 | 3,03 | 3,03 | |
| TRBV2*01, or TRBV2*02 or TRBV2*03 | TRBJ2-1 *01 | TRBD1*01 | tgtgccagcagt-gaagctgcag-ggggctacggtgag-cagttcttc | CASSEAAGGY-GEQFF | 1 | 1,52 | 1,52 | 45 |
| TRBV2*01, or TRBV2*02 or TRBV2*03 | TRBJ1-5*01 | TRBD1*01 | tgtgccagcagt-gaatttgccag-gggcaat-cagccccagcatttt | CASSEFARGN-QPQHF | 1 | 1,52 | 1,52 | |
| TRBV2*01, or TRBV2*02 or TRBV2*03 | TRBJ1-5*01 | TRBD1*01 | tgtgccagcagtgaat-tcgtaagggacaat-cagccccagcatttt | CASSEFVRDN-QPQHF | 1 | 1,52 | 1,52 | |
| TRBV2*01, or TRBV2*02 or TRBV2*03 | TRBJ1-5*01 | TRBD1*01 | tgtgccagcagtgaat-tcgtaaggggcaat-cagccccagcatttt | CASSEFVRGN-QPQHF | 4 | 6,06 | 6,06 | |
| TRBV2*01, or TRBV2*02 or TRBV2*03 | TRBJ1-5*01 | TRBD1*01 | tgtgccagcagtgaag-gggcggctggcaat-cagccccagcatttt | **CASSEGAAGN-QPQHF** | 1 | 1,52 | 1,52 | |
| TRBV2*01, or TRBV2*02 or TRBV2*03 | TRBJ2-1 *01 | TRBD1*01 | tgtgccagcagtgaag-gagctaggggcgt-gggggagcagttcttc | CASSEG-ARGVGEQFF | 1 | 1,52 | 1,52 | |

(continued)

| **HIC 6 DR11+** | | | | | | | | |
|---|---|---|---|---|---|---|---|---|
| TRBV2*01, or TRBV2*02 or TRBV2*03 | TRBJ2-5*01 | TRBD2*01 | tgtgccagcagtgaag-gggctagcggtacg-ggggcccagtacttc | CASSEGASGT-GAQYF | 1 | 1,52 | 1,52 | |
| TRBV2*01, or TRBV2*02 or TRBV2*03 | TRBJ2-1 *01 | TRBD1*01 | tgtgccagcagtgaag-gcgctagtggcgtag-gggagcagttcttc | CASSEGAS-GVGEQFF | 1 | 1,52 | 1,52 | |
| TRBV2*01, or TRBV2*02 or TRBV2*03 | TRBJ2-1 *01 | TRBD2*01 | tgtgccagcagtgag-ggtactagcacctt-tcgtgagcagttcttc | CASSEGTST-FREQFF | 2 | 3,03 | 3,03 | |
| TRBV2*01, or TRBV2*02 or TRBV2*03 | TRBJ2-2*01 | TRBD2*01 | tgtgccagcagtgaatt-agcgagcggcaccg-gggagctgtttttt | CASSELASGT-GELFF | 1 | 1,52 | 1,52 | |
| TRBV2*01, or TRBV2*02 or TRBV2*03 | TRBJ1-5*01 | TRBD2*02 | tgtgccagcagt-ggggcagcgag-gggcaat-cagccccagcatttt | CASSGAARGN-QPQHF | 5 | 7,58 | 7,58 | |
| TRBV2*01, or TRBV2*02 or TRBV2*03 | TRBJ2-7*01 | TRBD2*01 | tgtgccagcag-caaactgactagcg-ggggatacgagcag-tacttc | CASSKLTSG-GYEQYF | 2 | 3,03 | 3,03 | |
| TRBV2*01, or TRBV2*02 or TRBV2*03 | TRBJ2-1 *01 | TRBD2*01 | tgtgccagcagtcccg-ggactagcggggtt-ggtgagcagttcttc | **CASSPGTS-GVGEQFF*** | 2 | 3,03 | 3,03 | |
| TRBV2*01, or TRBV2*02 or TRBV2*03 | TRBJ2-1 *01 | TRBD2*01 | tgtgccag-cagtcgtctagcg-gggggtttcgatgag-cagttcttc | CASSRLAGGFD-EQFF | 2 | 3,03 | 3,03 | |
| TRBV2*01, or TRBV2*02 or TRBV2*03 | TRBJ2-3*01 | TRBD2*01 | tgtgccagcagtc-gactagcg-gggggcacagatacg-cagtatttt | CASSRLAGGT-DTQYF | 2 | 3,03 | 3,03 | |

(continued)

| | | | | | | | | |
|---|---|---|---|---|---|---|---|---|
| **HIC 6 DR11+** | | | | | | | | |
| TRBV2*01, or TRBV2*02 or TRBV2*03 | TRBJ2-3*01 | TRBD2*01 | tgtgccagcagta-gactagcgtct-ggcacagatacgcag-tatttt | CASSRLASGT-DTQYF | 5 | 7,58 | 7,58 | |
| TRBV2*01, or TRBV2*02 or TRBV2*03 | TRBJ2-7*01 | TRBD2*01 | tgtgccagcagtgtatt-ggctagcgggctgggt-gagcagtacttc | CASSVLAS-GLGEQYF | 1 | 1,52 | 1,52 | |
| TRBV2*01, or TRBV2*02 or | TRBJ1-5*01 | TRBD1*01 | tgtgccagcagacag-ggggcgaggggag-gcaatcagccccag-cattt | CASRQ-GARGGNQPQHF | 6 | 9,09 | 9,09 | 48 |
| TRBV2*03 | | | | | | | | |
| TRBV2*01, or TRBV2*02 or TRBV2*03 | TRBJ1-5*01 | TRBD1*01 | tgtgccagctcacag-ggggcgcg-ggggggaaat-cagccccagcatttt | CASSQGARGGN-QPQHF | 1 | 1,52 | 1,52 | |
| TRBV2*01, or TRBV2*02 or TRBV2*03 | TRBJ2-7*01 | TRBD2*01 | tgtgccagcagtcgatt-agcgggggggagctc-ctacgagcagtacttc | CASSR-LAGGSSYEQYF | 2 | 3,03 | 3,03 | |
| TRBV2*01, or TRBV2*02 or TRBV2*03 | TRBJ2-3*01 | TRBD2*01 | tgtgccag-cagccgcct-gactagcg-ggggggcagatacgc agtatttt | CASSRLTSG-GADTQYF | 1 | 1,52 | 1,52 | |
| TRBV2*01, or TRBV2*02 or TRBV2*03 | TRBJ2-3*01 | TRBD2*01 | tgtgccag-cagccgccg-gactagcg-ggggcacagatacgc agtatttt | **CASSRRTSGGT-DTQYF*** | 12 | 18,18 | 18,18 | |
| | | | Total | 28 | 66 | 100,00 | 100,00 | |

(continued)

| HIC 7 DRB5+ | | | | | | | | |
|---|---|---|---|---|---|---|---|---|
| TRBV2*01, or TRBV2*02 or TRBV2*03 | TRBJ2-7*01 | TRBD2*01 | tgtgccagcagtc-ctcgggcctcg-gggggagagcag-tacttc | CASSPRAS-GGEQYF | 3 | 3,85 | 3,85 | 42 |
| TRBV2*01, or TRBV2*02 or TRBV2*03 | TRBJ1-4*01 | TRBD2*01 | tgtgccagcagtgt-gcggcggaataat-gaaaaactgtttttt | CASSVRRN-NEKLFF | 3 | 3,85 | 3,85 | |
| TRBV2*01, or TRBV2*02 or TRBV2*03 | TRBJ2-7*01 | TRBD2*01 | tgtgccagcaac-cgaaggactagcg-gaacctacgagcag-tacttc | CASNRRTSG-TYEQYF | 1 | 1,28 | 1,28 | 45 |
| TRBV2*01, or TRBV2*02 or TRBV2*03 | TRBJ2-3*01 | TRBD2*01 | tgtgccagcagcg-cactagcgtccg-ggggagatacgcag-tatttt | CASSALASGG DTQYF | 3 | 3,85 | 3,85 | |
| TRBV2*01, or TRBV2*02 or TRBV2*03 | TRBJ2-1 *01 | TRBD2*01 | tgtgccagcagt-gcacgggctagcg-ggggggatgagcagt-tcttc | CASSARASGGD-EQFF | 3 | 3,85 | 5,13 | |
| TRBV2*01, or TRBV2*02 or TRBV2*03 | TRBJ2-1 *01 | TRBD2*01 | tgtgccagcagt-gcacgggctagcg-ggggggatgag-cagtttttc | | 1 | 1,28 | | |
| TRBV2*01, or TRBV2*02 or TRBV2*03 | TRBJ1-5*01 | TRBD1*01 | tgtgccagcagt-gcccggacgagt-gggggtcagccccag-catttt | CASSARTSG-GQPQHF | 4 | 5,13 | 5,13 | |
| TRBV2*01, or TRBV2*02 or TRBV2*03 | TRBJ1-5*01 | TRBD1*01 | tgtgccag-cagcgcccgga-catcgggcaat-cagccccagcatttt | CASSARTSGN-QPQHF | 11 | 14,10 | 14,10 | |

(continued)

| HIC 7 DRB5+ | | | | | | | | |
|---|---|---|---|---|---|---|---|---|
| TRBV2*01, or TRBV2*02 or TRBV2*03 | TRBJ2-1 *01 | TRBD2*02 | tgtgccagcagt-gaactggctagcg-ggatcaatgagcagt-tcttc | CASSELAS-GINEQFF | 5 | 6,41 | 6,41 | |
| TRBV2*01, or TRBV2*02 or TRBV2*03 | TRBJ2-1*01 | TRBD2*01 | tgtgccagcagtgagtt-gactagcg-ggggggatgagcagt-tcttc | **CASSELTSGGD-EQFF** | 1 | 1,28 | 1,28 | |
| TRBV2*01, or TRBV2*02 or TRBV2*03 | TRBJ2-3*01 | TRBD2*02 | tgtgccagcagtaa-gaggacctctggag-gagatacgcagtatttt | CASSKRTSG-GDTQYF | 1 | 1,28 | 1,28 | |
| TRBV2*01, or TRBV2*02 or TRBV2*03 | TRBJ2-7*01 | TRBD2*01 | tgtgccag-cagcctaag-gactagcg-ggggggatgagcagta cttc | CASSLRTSGGD-EQYF | 3 | 3,85 | 3,85 | |
| TRBV2*01, or TRBV2*02 or TRBV2*03 | TRBJ2-3*01 | TRBD2*01 | tgtgccag-cagccccct-gactagcgccacaga-tacgcagtatttt | CASSPLTSAT-DTQYF | 1 | 1,28 | 1,28 | |
| TRBV2*01, or TRBV2*02 or TRBV2*03 | TRBJ2-1*01 | TRBD2*01 | tgtgccagcagtc-ctcgggctagcg-ggggcgatgagcagt-tcttc | CASSPRASGGD-EQFF | 5 | 6,41 | 6,41 | |
| TRBV2*01, or TRBV2*02 or TRBV2*03 | TRBJ2-7*01 | TRBD2*01 | tgtgccagcagtc-cccggactagcg-ggggcgacgagcag-tacttc | **CASSPRTSGGD-EQYF** | 1 | 1,28 | 1,28 | |
| TRBV2*01, or TRBV2*02 or TRBV2*03 | TRBJ2-3*01 | TRBD2*01 | tgtgccagcaggctc-cggactagcgggggt-acagatacgcagtatttt | CASRLRTSGGT-DTQYF | 2 | 2,56 | 2,56 | |

(continued)

| **HIC 7 DRB5+** | | | | | | | | |
|---|---|---|---|---|---|---|---|---|
| TRBV2*01, or TRBV2*02 or TRBV2*03 | TRBJ2-3*01 | TRBD2*01 | tgtgccagcagac-gactagcggggtttat-ggcagatacgcag-tatttt | CASRRLAGF-MADTQYF | 1 | 1,28 | 1,28 | 48 |
| TRBV2*01, or TRBV2*02 or TRBV2*03 | TRBJ2-3*01 | TRBD2*01 | tgtgccagcagt-gcacggactagcgcg-ggcacagatacgcag-tatttt | CASSARTSAGT-DTQYF | 1 | 1,28 | 7,69 | |
| TRBV2*01, or TRBV2*02 or TRBV2*03 | TRBJ2-3*01 | TRBD2*01 | tgtgccagcagt-gcacggactagcgct-ggcacagatacgcag-tatttt | | 5 | 6,41 | | |
| TRBV2*01, or TRBV2*02 or TRBV2*03 | TRBJ2-3*01 | TRBD2*01 | tgtgccagcagt-gccaggactagcg-gggggcagatacg-cattatttt | CASSARTSG-GADTHYF | 1 | 1,28 | 1,28 | |
| TRBV2*01, or TRBV2*02 or TRBV2*03 | TRBJ2-3*01 | TRBD2*01 | tgtgccagcagt-gccaggactagcg-gggggcagatacg-cagtatttt | **CASSARTSG-GADTQYF** | 1 | 1,28 | 2,56 | |
| TRBV2*01, or TRBV2*02 or TRBV2*03 | TRBJ2-3*01 | TRBD2*01 | tgtgccagcagt-gctaggactagcg-gggggcagatacg-cagtatttt | | 1 | 1,28 | | |
| TRBV2*01, or TRBV2*02 or TRBV2*03 | TRBJ2-3*01 | TRBD2*01 | tgtgccag-cagcgcccg-gactagcggggggt-cagatacgcagtatttt | **CASSARTS-GGSDTQYF** | 1 | 1,28 | 3,85 | |
| TRBV2*01, or TRBV2*02 or | TRBJ2-3*01 | TRBD2*01 | tgtgccagcagt-gccaggactagcg-gggggtcagatacg-cagtatttt | | 2 | 2,56 | | |

(continued)

| | | | | | | | | |
|---|---|---|---|---|---|---|---|---|
| **HIC 7 DRB5+** | | | | | | | | |
| TRBV2*03 | | | | | | | | |
| TRBV2*01, or TRBV2*02 or TRBV2*03 | TRBJ2-3*01 | TRBD2*01 | tgtgccagcagt-gccaggactagcg-gaggcacagatacg-cagtatttt | **CASSARTSGGT-DTQYF** | 2 | 2,56 | 3,85 | |
| TRBV2*01, or TRBV2*02 or TRBV2*03 | TRBJ2-3*01 | TRBD2*01 | tgtgccagcagt-gctaggactagcg-ggggcacagatacg-cagtatttt | | 1 | 1,28 | | |
| TRBV2*01, or TRBV2*02 or TRBV2*03 | TRBJ2-3*01 | TRBD2*01 | tgtgccagcagt-gaacggactagcg-ggggacgcgatacg-cagtatttt | CASSERTSG-GRDTQYF | 1 | 1,28 | 1,28 | |
| TRBV2*01, or TRBV2*02 or TRBV2*03 | TRBJ2-3*01 | TRBD2*01 | tgtgccagcagcgg-taggactagcg-ggggatcggatacg-cagtatttt | CASSG RTS-GGSDTQYF | 3 | 3,85 | 3,85 | |
| TRBV2*01, or TRBV2*02 or TRBV2*03 | TRBJ2-3*01 | TRBD2*01 | tgtgccagcagtcttag-ggctagcggggggt-cagatacgcagtatttt | CASSLRAS-GGSDTQYF | 1 | 1,28 | 1,28 | |
| TRBV2*01, or TRBV2*02 or TRBV2*03 | TRBJ2-3*01 | TRBD2*01 | tgtgccagcagtct-gaggactagcg-ggggggcagatacg-cagtatttt | CASSLRTSG-GADTQYF | 1 | 1,28 | 1,28 | |
| TRBV2*01, or TRBV2*02 or TRBV2*03 | TRBJ2-3*01 | TRBD2*01 | tgtgccag-cagccaacg-gactagcg-ggggggcagatacgc agtatttt | CASSQRTSG-GADTQYF | 1 | 1,28 | 1,28 | |

(continued)

| | | | | | | | | |
|---|---|---|---|---|---|---|---|---|
| **HIC 7 DRB5+** | | | | | | | | |
| TRBV2*01, or TRBV2*02 or TRBV2*03 | TRBJ2-3*01 | TRBD2*01 | tgtgccag-cagccgact-gactagcgggggat-cagatacgcagtatttt | CASSRLTS-GGSDTQYF | 4 | 5,13 | 5,13 | |
| TRBV2*01, or TRBV2*02 or TRBV2*03 | TRBJ2-3*01 | TRBD2*01 | tgtgccag-cagtcgccg-gactagcgggggtcta-gatacgcagtatttt | CASSRRTSG-GLDTQYF | 1 | 1,28 | 1,28 | |
| TRBV2*01, or TRBV2*02 or TRBV2*03 | TRBJ2-1*01 | TRBD2*01 | tgtgccagcagtcg-gcggactagcg-gggggcccaatgag-cagttcttc | CASSRRTSGGP-NEQFF | 1 | 1,28 | 1,28 | |
| TRBV2*01, or TRBV2*02 or TRBV2*03 | TRBJ2-3*01 | TRBD2*01 | tgtgccagcagtcg-gaggactagcg-ggggcacagatacg-cagtatttt | **CASSRRTSGGT-DTQYF*** | 1 | 1,28 | 1,28 | |
| | | | Total | 30 | 78 | 100,00 | 100,00 | |
| **HIC 8 DR1+** | | | | | | | | |
| TRBV2*01, or TRBV2*02 or TRBV2*03 | TRBJ2-7*01 | TRBD1*01 | tgtgccagcagtgaag-ggtgggaaccctac-gagcagtacttc | CASSEG-WEPYEQYF | 1 | 1,49 | 1,49 | 42 |
| TRBV2*01, or TRBV2*02 or TRBV2*03 | TRBJ2-1*01 | TRBD2*01 | tgtgccagcagtgagtt-gactagcg-ggggggatgagcagt-tcttc | **CASSELTSGGD-EQFF** | 63 | 94,03 | 94,03 | 45 |
| TRBV2*01, or TRBV2*02 or TRBV2*03 | TRBJ2-1*01 | TRBD2*01 | tgtgccagcagtgagtt-gactagcg-ggggggatgag-cagttgttc | CASSELTSGGD-EQLF | 1 | 1,49 | 1,49 | |

(continued)

| | | | | | | | | |
|---|---|---|---|---|---|---|---|---|
| **HIC 8 DR1+** | | | | | | | | |
| TRBV2*01, or TRBV2*02 or TRBV2*03 | TRBJ2-3*01 | TRBD2*01 | tgtgccagcagt-gccaggactagcg-gaggcacagatacg-cagtatttt | **CASSARTSGGT-DTQYF** | 1 | 1,49 | 1,49 | 48 |
| TRBV2*01, or TRBV2*02 or TRBV2*03 | TRBJ1-2*01 | TRBD1*01 | tgtgccaccac-cgccgccgggacag-gggtagacggaaac-tatggctacaccttc | CATTAAGT-GVDGNYGYTF | 1 | 1,49 | 1,49 | 54 |
| | | | Total | 5 | 67 | 100 | 100 | |
| **HIC 1 DRB5+ ex vivo** | | | | | | | | |
| TRBV2*01 | TRBJ2-1*01 | TRBD2*01 | tgtgccagccagcg-gggaaattcctacaat-gagcagttcttc | CASQRGNSYN-EQFF | 2 | 4,17 | 4,17 | 42 |
| TRBV2*01 | TRBJ2-7*01 | TRBD2*01 | tgtgccagcagaatc-cggactagcg-ggggggagcagtact-tc | CASRIRTS-GGEQYF | 11 | 22,92 | 22,92 | |
| TRBV2*01 | TRBJ2-1*01 | TRBD1*01 | tgtgccagcacccg-ggacaggacaaa-gaatgagcagttcttc | CAS-TRDRTKNEQFF | 1 | 2,08 | 2,08 | |
| TRBV2*01 | TRBJ2-1*01 | TRBD2*01 | tgtgccagcagtgcgtt-ggctagcgggga-gatgagcagttcttc | CASSALASGGD-EQFF | 1 | 2,08 | 2,08 | |
| TRBV2*01 | TRBJ2-1*01 | TRBD2*01 | tgtgccagcagtctc-cggactagcgggga-gatgagcagttcttc | CASSLRTSGGD-EQFF | 2 | 4,17 | 4,17 | |
| TRBV2*01 | TRBJ2-1*01 | TRBD2*01 | tgtgccag-cagccctat-ggctagcg-ggggggatgagcagtt cttc | CASSPMASGGD-EQFF | 2 | 4,17 | 4,17 | |

(continued)

| HIC 1 DRB5+ ex vivo | | | | | | | | |
|---|---|---|---|---|---|---|---|---|
| TRBV2*01 | TRBJ2-3*01 | TRBD2*01 | tgtgccag-cagcccccg-gactagcggcacaga-tacgcagtatttt | CASSPRTSGT-DTQYF | 2 | 4,17 | 4,17 | 45 |
| TRBV2*01 | TRBJ2-7*01 | TRBD2*02 | tgtgccagcagccg-taggactagcgggact-tacgagcagtacttc | **CASSRRTSG-TYEQYF** | 1 | 2,08 | 2,08 | |
| TRBV2*01 | TRBJ2-1*01 | TRBD2*01 | tgtgccagcagtgtgat-ggctagccgtgggaat-gagcagttcttc | CASSVMAS-RGNEQFF | 1 | 2,08 | 2,08 | |
| TRBV2*01 | TRBJ2-1*01 | TRBD2*01 | tgtgtcagcagtgcgtt-ggctagcgggggga-gatgagcagttcttc | CVSSALASGGD-EQFF | 1 | 2,08 | 2,08 | |
| TRBV2*01 | TRBJ2-3*01 | TRBD2*01 | tgtgccagcaataaatt-gactagcgggggccg-ggatacgcagtatttt | CASNKLTSG-GRDTQYF | 4 | 8,33 | 8,33 | 48 |
| TRBV2*01 | TRBJ2-3*01 | TRBD2*01 | tgtgccagcagt-gccaggactagcg-ggggctcggatacg-cagtatttt | **CASSARTS-GGSDTQYF** | 4 | 8,33 | 8,33 | |
| TRBV2*01 | TRBJ2-1*01 | TRBD2*01 | tgtgccagcag-caaaaggactagcg-gggcgactgatgag-cagttcttc | CASSKRTSGAT-DEQFF | 2 | 4,17 | 4,17 | |
| TRBV2*01 | TRBJ2-5*01 | TRBD2*01 | tgtgccagcagtttgtt-gactagcgggggacg-ggagacccagtacttc | CASSLLTSG-GRETQYF | 6 | 12,50 | 12,50 | |
| TRBV2*01 | TRBJ2-1*01 | TRBD2*01 | tgtgccag-cagccgcttgac-gagcggggggcg-gaatgagcagttcttc | CASSRLTSG-GRNEQFF | 2 | 4,17 | 4,17 | |

(continued)

| | | | | | | | | |
|---|---|---|---|---|---|---|---|---|
| **HIC 1 DRB5+ ex vivo** | | | | | | | | |
| TRBV2*01 | TRBJ2-3*01 | TRBD2*01 | tgtgccagcagctc-caggactagcg-ggggcacagatacg-cagtatttt | CASSSRTSGGT-DTQYF | 3 | 6,25 | 6,25 | |
| TRBV2*01 | TRBJ1-5*01 | TRBD2*01 | tgtgccacctccagag-gagcgcggggaag-caatcagccccag-catttt | CATSRGARGSN-QPQHF | 3 | 6,25 | 6,25 | |
| | | | Total | 17 | 48 | 100,00 | 100,00 | |
| **HIC 2 DRB5+ ex vivo** | | | | | | | | |
| TRBV2*01, orTRBV2*02 orTRBV2*03 | TRBJ2-2*01 | TRBD1*01 | tgtgccagcaga-gacggcctcg-gggagctgtttttt | CASRDGLGELFF | 1 | 2,00 | 2,00 | 36 |
| TRBV2*01, | TRBJ1-1*01 | TRBD1*01 | tgtgccagctcag-gacagacgaacact-gaagctttcttt | **CASSGQT-NTEAFF** | 3 | 6,00 | 6,00 | 39 |
| orTRBV2*02 orTRBV2*03 | | | | | | | | |
| TRBV2*01 | TRBJ2-3*01 | TRBD2*01 | tgtgccagcagt-gcactggctagcg-gaacagatacgcag-tatttt | **CASSALASGT-DTQYF** | 1 | 2,00 | 2,00 | |
| TRBV2*01 | TRBJ2-5*01 | TRBD2*01 | tgtgccagcagtgaatt-ggctagcgggcag-ggatcccagtacttc | CASSELASGQG-SQYF | 5 | 10,00 | 10,00 | |
| TRBV2*01 | TRBJ2-7*01 | TRBD2*02 | tgtgccagcagt-gaactggctagcg-ggagctacgagcag-tacttc | CASSELASG-SYEQYF | 3 | 6,00 | 6,00 | |

(continued)

| | | | | | | | | |
|---|---|---|---|---|---|---|---|---|
| **HIC 2 DRB5+ ex vivo** | | | | | | | | |
| TRBV2*01, orTRBV2*02 orTRBV2*03 | TRBJ2-1*01 | TRBD2*02 | tgtgccagcagt-gaactggctagcg-ggacgggtgagcagt-tcttc | CASSELASGT-GEQFF | 1 | 2,00 | 2,00 | 45 |
| TRBV2*01, orTRBV2*02 orTRBV2*03 | TRBJ2-7*01 | TRBD2*01 | tgtgccag-cagccctcg-gactagcggtccctac-gagcagtacttc | CASSPRTSG-PYEQYF | 3 | 6,00 | 6,00 | |
| TRBV2*01, orTRBV2*02 orTRBV2*03 | TRBJ2-3*01 | TRBD2*01 | tgtgccag-cagccaatt-gactagcggcacaga-tacgcagtatttt | CASSQLTSGT-DTQYF | 2 | 4,00 | 4,00 | |
| TRBV2*01, orTRBV2*02 orTRBV2*03 | TRBJ2-1*01 | TRBD2*02 | tgtgccagcagtc-gactagcgggag-gatttgatgagcagttct-tc | CASSRLAGGFD-EQFF | 1 | 2,00 | 2,00 | |
| TRBV2*01 | TRBJ2-3*01 | TRBD2*01 | tgtgccagcagtcggtt-ggctagcggcacaga-tacgcagtatttt | CASSRLASGT-DTQYF | 4 | 8,00 | 8,00 | |
| TRBV2*01, orTRBV2*02 orTRBV2*03 | TRBJ1-5*01 | TRBD1*01 | tgtgccagcagtcg-gacagtctcgggcaat-cagccccagcatttt | CASSRTVSGN-QPQHF | 1 | 2,00 | 2,00 | |
| TRBV2*01, orTRBV2*02 orTRBV2*03 | TRBJ2-7*01 | TRBD2*01 | tgtgccagcagttcgtt-ggctagcagac-cctacgagcagtacttc | CASSSLAS-RPYEQYF | 1 | 2,00 | 2,00 | |
| TRBV2*01, orTRBV2*02 orTRBV2*03 | TRBJ2-1*01 | TRBD2*01 | tgtgccagcacgaag-ggcgctagcgggtcg-ggtgagcagttcttc | CASTKGASGS-GEQFF | 2 | 4,00 | 4,00 | |
| TRBV2*01 | TRBJ2-1*01 | TRBD2*02 | tgtgccaccacccccg-gggctagcggga-taagtgagcagttcttc | CATTPGASGISE-QFF* | 14 | 28,00 | 28,00 | |

(continued)

| | | | | | | | | |
|---|---|---|---|---|---|---|---|---|
| **HIC 2 DRB5+ ex vivo** | | | | | | | | |
| TRBV2*01 | TRBJ2-7*01 | TRBD1*01 | tgtgccagcagt-gaaaggggacag-ggggcgcggtacgag-cagtacttc | CASSERGQ-GARYEQYF | 5 | 10,00 | 10,00 | 48 |
| TRBV2*01, orTRBV2*02 orTRBV2*03 | TRBJ2-3*01 | TRBD2*01 | tgtgccag-cagccgaag-gactagcggag-gcacagatacgcagtatttt | **CASSRRTSGGT-DTQYF*** | 2 | 4,00 | 6,00 | |
| TRBV2*01, orTRBV2*02 orTRBV2*03 | TRBJ2-3*01 | TRBD2*01 | tgtgccag-cagccgaag-gactagcggaggta-cagatacgcagtatttt | | 1 | 2,00 | | |
| | | | Total | 16 | 50 | 100,00 | 100,00 | |
| **HIC 3 DRB5+ ex vivo** | | | | | | | | |
| TRBV2*01 | TRBJ2-1*01 | TRBD2*02 | tgt-gccagcgcccgactagcgggaggtaccgat-gagcagttcttc | CASARLAGGTD-EQFF | 1 | 4,00 | 4,00 | 45 |
| TRBV2*01 | TRBJ1-5*01 | TRBD2*01 | tgtgccag-cagcgcgaag-gcccgcgggaat-cagccccagcatttt | CASSAKARGN-QPQHF | 2 | 8,00 | 8,00 | |
| TRBV2*01 | TRBJ2-3*01 | TRBD2*01 | tgtgccagcagcg-cactagcg-gggggaacagatacg-cagtatttt | CASSALAGGT-DTQYF | 3 | 12,00 | 12,00 | |
| TRBV2*01 | TRBJ2-3*01 | TRBD2*01 | tgtgccagcagtgcttt-ggctagcggcacaga-tacgcagtatttt | **CASSALASGT-DTQYF** | 5 | 20,00 | 20,00 | |

(continued)

| **HIC 3 DRB5+ ex vivo** | | | | | | | | |
|---|---|---|---|---|---|---|---|---|
| TRBV2*01 | TRBJ2-7*01 | TRBD2*01 | tgtgccagcagt-gacgcggctagcggt-gtgggcgagcagtact-tc | CASSDAAS-GVGEQYF | 4 | 16,00 | 16,00 | |
| TRBV2*01 | TRBJ1-5*01 | TRBD1*01 | tgtgccagcagcg-gacaggcgag-gggcaat-cagccccagcatttt | CASSGQARGN-QPQHF | 4 | 16,00 | 16,00 | |
| TRBV2*01 | TRBJ2-1*01 | TRBD2*01 | tgtgcaag-cagccccg-ggactagcggagtt-ggtgagcagttcttc | **CASSPGTS-GVGEQFF*** | 4 | 16,00 | 16,00 | |
| TRBV2*01 | TRBJ1-5*01 | TRBD1*01 | tgtgccagcagtc-ccagtgctcgcg-gcaatcagccccag-catttt | CASSPSARGN-QPQHF | 1 | 4,00 | 4,00 | |
| TRBV2*01 | TRBJ2-7*01 | TRBD2*01 | tgtgccagcagtgtcg-ggactagcggggt-gggcgagcagtacttc | CASSVGTS-GVGEQYF | 1 | 4,00 | 4,00 | |
| | | | Total | 9 | 25 | 100,00 | 100,00 | |

(continued)

| **HIC 7 DRB5+ ex vivo** | | | | | | | | |
|---|---|---|---|---|---|---|---|---|
| TRBV2*01, orTRBV2*02 orTRBV2*03 | TRBJ2-7*01 | TRBD2*01 | tgtgccagccag-gcaagcggccgatc-ctacgagcagtacttc | CASQAS-GRSYEQYF | 4 | 3,67 | 3,67 | 42 |
| TRBV2*01 | TRBJ2-5*01 | TRBD1*01 | tgtgccagcagt-gaattttgggggcaa-gagacccagtacttc | CASSE-FWGQETQYF | 1 | 0,92 | 0,92 | |
| TRBV2*01 | TRBJ2-1*01 | TRBD2*01 | tgtgccagcagt-gagctggctagcg-gggatgagcagttcttc | CASSELASGD-EQFF | 9 | 8,26 | 8,26 | |
| TRBV2*01 | TRBJ2-7*01 | TRBD1*01 | tgtgccagcagtg-tatcgcag-gggagcgacgag-cagtacttc | CASSVSQGSD-EQYF | 1 | 0,92 | 0,92 | |
| TRBV2*01, orTRBV2*02 orTRBV2*03 | TRBJ2-7*01 | TRBD2*01 | tgtgccagctgtcccat-ggctagccgatcctac-gagcagtacttc | CASCP-MASRSYEQYF | 1 | 0,92 | 0,92 | |
| TRBV2*01 | TRBJ1-2*01 | TRBD1*01 | tgtgccagcattatcg-gttcccaag-gggcctatggctacac-cttc | CASI IGSQGAY-GYTF | 1 | 0,92 | 0,92 | |
| TRBV2*01 | TRBJ2-3*01 | TRBD2*01 | tgtgccagcagcg-cactagcgtccg-ggggagatacgcag-tatttt | CASSALASGG DTQYF | 6 | 5,50 | 5,50 | |
| TRBV2*01, orTRBV2*02 orTRBV2*03 | TRBJ2-1*01 | TRBD2*01 | tgtgccagcagt-gcacgggctagcg-ggggggatgagcagt-tcttc | CASSARASGGD-EQFF | 4 | 3,67 | 3,67 | |

(continued)

| **HIC 7 DRB5+ ex vivo** | | | | | | | | |
|---|---|---|---|---|---|---|---|---|
| TRBV2*01, orTRBV2*02 orTRBV2*03 | TRBJ1-5*01 | TRBD1*01 | tgtgccag-cagcgcccgga-catcgggcaat-cagccccagcatttt | CASSARTSGN-QPQHF | 5 | 4,59 | 4,59 | 45 |
| TRBV2*01, orTRBV2*02 | TRBJ 1-1 *01 | TRBD1*01 | tgtgccagcagt-gaacgcg-ggacagccaacact-gaagctttcttt | CASSERG-TANTEAFF | 1 | 0,92 | 0,92 | |
| TRBV2*01, orTRBV2*02 orTRBV2*03 | TRBJ2-3*01 | TRBD2*01 | tgtgccagcagct-tcaggactagcg-ggggtgatacgcag-tatttt | CASSFRTSG-GDTQYF | 2 | 1,83 | 1,83 | |
| TRBV2*01, orTRBV2*02 | TRBJ2-3*01 | TRBD2*01 | tgtgccagcag-taagcgggctagcg-ggggagatacgcag-tatttt | CASSKRASG-GDTQYF | 2 | 1,83 | 1,83 | |
| TRBV2*01, orTRBV2*02 orTRBV2*03 | TRBJ2-1*01 | TRBD2*01 | tgtgccagcagtc-ctcgggctagcg-ggggcgatgagcagt-tcttc | CASSPRASGGD-EQFF | 3 | 2,75 | 2,75 | |
| TRBV2*01 | TRBJ2-7*01 | TRBD2*01 | tgtgccagcagtc-cccggactagcg-ggggcgacgagcag-tacttc | **CASSPRTSGGD-EQYF** | 7 | 6,42 | 6,42 | |
| TRBV2*01, orTRBV2*02 | TRBJ2-7*01 | TRBD2*02 | tgtgccag-cagccccag-gactagcgggac-ctacgagcagtacttc | **CASSPRTSG-TYEQYF** | 1 | 0,92 | 15,60 | |
| TRBV2*01 | TRBJ2-7*01 | TRBD2*02 | tgtgccagcagtc-ctcggactagcg-ggacctacgagcag-tacttc | | 16 | 14,68 | | |

(continued)

| **HIC 7 DRB5+ ex vivo** | | | | | | | | |
|---|---|---|---|---|---|---|---|---|
| TRBV2*01 | TRBJ2-7*01 | TRBD1*01 | tgtgccag-cagccccgtggccag-ggggccttacgag-cagtacttc | CASSPVARG-PYEQYF | 1 | 0,92 | 0,92 | |
| TRBV2*01 | TRBJ2-7*01 | TRBD2*01 | tgtgccagcagt-caactgactagca-gaacctacgagcag-tacttc | CASSQLT-SRTYEQYF | 1 | 0,92 | 0,92 | |
| TRBV2*01, orTRBV2*02 | TRBJ2-3*01 | TRBD2*01 | tgtgccagcagt-gccaggactagcg-gggggtcagatacg-cagtatttt | **CASSARTS-GGSDTQYF** | 6 | 5,50 | 5,50 | |
| TRBV2*01, orTRBV2*02 orTRBV2*03 | TRBJ2-3*01 | TRBD2*01 | tgtgccag-cagcgcccg-gactagcg-ggggcacagatacgc agtatttt | **CASSARTSGGT-DTQYF** | 1 | 0,92 | 11,01 | |
| TRBV2*01 | TRBJ2-3*01 | TRBD2*01 | tgtgccagcagt-gccaggactagcg-gaggcacagatacg-cagtatttt | | 11 | 10,09 | | |
| TRBV2*01, orTRBV2*02 | TRBJ2-3*01 | TRBD2*01 | tgtgccagcagcgg-taggactagcg-ggggatcggatacg-cagtatttt | CASSGRTS-GGSDTQYF | 4 | 3,67 | 3,67 | |
| TRBV2*01 | TRBJ2-3*01 | TRBD2*01 | tgtgccagcagtct-gaggactagcg-ggggggcagatacg-cagtatttt | CASSLRTSG-GADTQYF | 1 | 0,92 | 0,92 | |
| TRBV2*01, orTRBV2*02 orTRBV2*03 | TRBJ2-3*01 | TRBD2*01 | tgtgccagcagtct-caggactagcg-ggggttcagatacg-cagtatttt | **CASSLRTS-GGSDTQYF** | 2 | 1,83 | 1,83 | |

(continued)

| **HIC 7 DRB5+ ex vivo** | | | | | | | | |
|---|---|---|---|---|---|---|---|---|
| TRBV2*01, orTRBV2*02 orTRBV2*03 | TRBJ2-3*01 | TRBD2*01 | tgtgccagcagtcctct-gactagcg-ggggcacagatacg-cagtatttt | CASSPLTSGGT-DTQYF | 1 | 0,92 | 0,92 | 48 |
| TRBV2*01, orTRBV2*02 | TRBJ2-3*01 | TRBD2*01 | tgtgccagcagtcg-gaggactagcg-gggcctcagatacg-cagtatttt | CASSRRTSGAS-DTQYF | 1 | 0,92 | 0,92 | |
| TRBV2*01, orTRBV2*02 orTRBV2*03 | TRBJ2-1*01 | TRBD2*01 | tgtgccagcagtc-gacgtactagcg-ggggggccgatgag-cagttcttc | CASSRRTSG-GADEQFF | 1 | 0,92 | 0,92 | |
| TRBV2*01, orTRBV2*02 orTRBV2*03 | TRBJ2-3*01 | TRBD2*01 | tgtgccag-cagccgacg-gactagcg-gggggacggatacgc agtatttt | **CASSRRTSGGT-DTQYF*** | 2 | 1,83 | 5,50 | |
| TRBV2*01 | TRBJ2-3*01 | TRBD2*01 | tgtgccagcagccg-gcggactagcg-ggggcacagatacg-cagtatttt | | 2 | 1,83 | | |
| TRBV2*01 | TRBJ2-3*01 | TRBD2*01 | tgtgccagcagta-gaaggactagcggt-ggcacagatacgcag-tatttt | | 1 | 0,92 | | |
| TRBV2*01, orTRBV2*02 | TRBJ2-3*01 | TRBD2*01 | tgtgccagcagta-gacggactagcg-gggggacagatacg-cagtatttt | | 1 | 0,92 | | |
| TRBV2*01, orTRBV2*02 orTRBV2*03 | TRBJ2-1*01 | TRBD2*01 | tgtgccagcagca-gacgaactagcg-ggggatacgatgag-cagttcttc | CASSRRTSG-GYDEQFF | 2 | 1,83 | 1,83 | |

(continued)

| HIC 7 DRB5+ ex vivo | | | | | | | | |
|---|---|---|---|---|---|---|---|---|
| TRBV2*01 | TRBJ2-3*01 | TRBD2*01 | tgtgccagcag-tagccggactagcg-ggggatcagatacg-cagtatttt | CASSSRTS-GGSDTQYF | 5 | 4,59 | 4,59 | |
| TRBV2*01 | TRBJ2-3*01 | TRBD2*01 | tgtgccagcagtgttcg-gactagcggggggt-cagatacgcagtatttt | CASSVRTS-GGSDTQYF | 1 | 0,92 | 0,92 | |
| TRBV2*01 | TRBJ2-7*01 | TRBD1*01 | tgtgccagcag-tataagtttacccg-ggacactttattacgag-cagtacttc | CASSISLPGT-LYYEQYF | 1 | 0,92 | 0,92 | 51 |
| | | | Total | 30 | 109 | 100,00 | 100,00 | |

**Table 10: Antigen sensitivity of the TCRs tested by transduction in J76 cells. SEQ ID NOs: 1255 to 1274**

The EC50 value for CD69 induction in J76 transduced with 10 different TCRs is reported.

Each TCR was tested with a panel of 7 L cell transfectants used as APC. Each L cell line expressed a single human HLA-DR allele.

The EC50 corresponds to the Gag293 peptide concentration (in M) at which half maximal CD69 induction was observed.

Responses too low for EC50 determination are indicated by a dash.

The CDR3 junction sequence is reported for the TRA and TRB chains of each TCR tested. Sequences in bold correspond to public clonotypes.

| TCR name | TRA CDR3 Junction | TRA motif | CDR3 length | TRB CDR3 Junction | TRB motif | CDR3 length | DR11 EC50 | DR15 EC50 | DRB5 EC50 | DR1 EC50 | DR7 EC50 | DR4 EC50 | DR3 EC50 |
|---|---|---|---|---|---|---|---|---|---|---|---|---|---|
| F24 | **CAFKAAGNKLTF** | AV24-1 | 10 | **CASSRLAGGMDEQFF** | BV2-1 | 13 | 4,15E-07 | 2,17E-06 | 4,46E-06 | 2,53E-06 | 8,69E-06 | - | - |
| F25 | **CAFKAAGNKLTF** | AV24-1 | 10 | CATTPGASGISEQF | - | 13 | 7,48E-07 | 8,91E-06 | 2,36E-05 | 3,48E-05 | - | - | - |
| F5 | **CAFKAAGNKLTF** | AV24-1 | 10 | CASSGLAGGMDEQFF | BV2-1 | 13 | 2,12E-06 | 1,60E-05 | - | - | - | - | - |
| S24 | **CASKAAGNKLTF** | AV24-1 | 10 | **CASSRLAGGMDEQFF** | BV2-1 | 13 | 6,19E-07 | 3,57E-06 | 9,23E-06 | 9,98E-06 | - | - | - |
| S25 | **CASKAAGNKLTF** | AV24-1 | 10 | CATTPGASGISEQF | - | 13 | 3,79E-06 | - | - | - | - | - | - |
| RR5 | **CSRRAAGNKLTF** | AV24-1 | 10 | CASSGLAGGMDEQF | BV2-1 | 13 | 1,25E-06 | 8,46E-06 | 1,10E-05 | 1,11E-05 | - | - | - |
| F4 | **CAFKAAGNKLTF** | AV24-1 | 10 | **CASSPGTSGVGEQFF** | BV2-1 | 13 | 1,39E-06 | 6,78E-05 | 8,42E-06 | - | - | - | - |
| F13 | **CAFKAAGNKLTF** | AV24-1 | 10 | **CASSRRTSGGTDTQYF** | BV2-2 | 14 | 7,54E-07 | 2,61E-06 | 4,10E-06 | - | - | - | - |
| HD5 | CASDYGGSQGNLIF | - | 12 | CASSEFRTRGYTF | - | 11 | - | 8,26E-06 | 5,86E-06 | 1,08E-05 | - | - | - |
| HY9 | CAYGANNLFF | - | 8 | CASRPLHSVYEQYF | - | 12 | - | 1,99E-06 | 3,84E-06 | 3,04E-06 | - | - | - |

## MATERIALS AND METHODS

### Study design

HIV Controllers (HIC group; n=14) were recruited through the CO21 CODEX cohort implemented by Agence Nationale de Recherche sur le SIDA et les hepatites virales (ANRS). HIV Controllers were defined as HIV-1 infected patients who had been seropositive for >5 years, had received no antiretroviral treatment, and for whom >90% of plasma viral load measurements were undetectable by standard assays. All HIV Controllers included in the study had current viral loads <50 copies/mL. The group of efficiently treated patients (HAART group; n=14) had received antiretroviral therapy for a minimum of 5 years and showed long term HIV-1 suppression with viral loads <50 copies/mL. Treated patients were recruited at the Raymond Poincaré and Bicêtre hospitals (France). Patients were included in the TCR study if their genotype matched at least one of the following alleles: DRB1*0101 (DR1), DRB1*1101 (DR11), DRB1*1501 (DR15), orDRB5*0101 (DRB5). Healthy donors were anonymous volunteers who donated blood at Etablissement Français du Sang.

### MHC II tetramer labeling of primary CD4+ T cell lines and patient PBMC

The 20-mer Gag293 peptide (FRDYVDRFYKTLRAEQASQE), spanning amino acids 293-312 in HIV-1 HXB2 Gag, was used to stimulate Gag-specific CD4+ T cell lines, as previously described (27). Cell lines were tested for Gag293 specificity by IFN-$\gamma$ ELISPOT assay and, if positive, labeled with an HLA-DR-matched Gag293-loaded tetramer and sorted. For ex vivo tetramer labeling, $10^7$ Controller PBMC were tetramer-labeled and sorted, with a minimum of 2,000 Tet+ events acquired for TCR diversity analysis.

### Analysis of TCR genetic diversity by Immunoscope and sequencing

TCR diversity was evaluated in sorted Tet+ cell RNA by the Immunoscope technique, as previously described (32). PCR products corresponding to the two amplified familes,TRAV24 and TRBV2, were cloned, sequenced, and analyzed with tools of IMGT database (34). Public motifs enriched in CDR3 sequences were detected with the MEME motif discovery software (http://meme.nbcr.net).

### Analysis of TCR affinity by surface plasmon resonance

Soluble TCRs were produced in bacterial expression vectors as previously described (68). The affinity of the soluble TCRs for immobilized Gag293-loaded HLA-DR monomers (DR11, DRB5, and DR1) was measured on a BIAcore 3000 instrument.

### TCR transfer experiments

Lentivectors expressing full-length TRAV24 and TRBV2 chains in equimolar amounts were produced by transfecting 293-T cells, concentrated by ultracentrifugation, and used to transduce the Jurkat-derived TCR-negative J76 cell line. TCR function was evaluated by measuring CD69 induction in transduced J76 cells incubated with Gag293-loaded HLA-DR-expressing L cells. Alternatively, monocyte-derived dendritic cells infected with pseudotyped pNL4-3-deltaEnv-EGFP were used as APC. For TCR transfer in primary cells, PBMC were PHA-stimulated for 48h before transduction with highly concentrated lentivector stocks. TCR function was evaluated by intracellular cytokine staining for IFN-$\gamma$, IL-2, MIP-1$\beta$, TNF-$\alpha$ and CD107a. All possible combinations of these 5 markers were analyzed by boolean gating, before analysis of polyfunctionality with the SPICE software.

### Antibodies

The following antibodies were used for membrane or intracellular staining: CD8-AlexaFluor488 (AF488, clone RPA-T8), CD3-eFluor® 780-Allophycocyanin (eF780-APC, clone UCHT1), TCR-Allophycocyanin (APC, clone IP26) and IL-2-APC (clone MQ1-17H12) (all from eBioscience); CD4 BD Horizon™ R-phycoerythrin-CF594 (PE-CF594, clone RPA-T4), CD4-R-phycoerythrin-cyanine 7 (PE-Cy7, clone SK3), CD69-R-phycoerythrin (PE, clone FN50), CD107a-AlexaFluor700 (AF700, clone H4A3), Perforin-AF488 (clone $\delta$G9), IFN$\gamma$-PE-Cy7 (clone B27), MIP1$\beta$-PE (clone D21-1351) (all from BD Biosciences); CD14-Viogreen (clone TUK4), and CD19-VioGreen (clone LT19) (from Miltenyi Biotec); TRBV2-PE (clone IMMU 546, Beckman Coulter); CD14-Brilliant Violet 510™ (BV510, clone M5E2), and CD19-BV510 (clone HIB19), CD8-Brilliant Violet 785™ (BV785, clone RPA-T8), HLA-DR-PE-Cy7 (clone LN3), CD45RA-Brilliant Violet 421™ (BV421, clone HI100), CCR7-PE-Cy7 (clone G043H7), and TNF$\alpha$-BV421 (clone MAb11) (all from Biolegend).

The fixable viability dye eFluor® 506 (eF506, eBioscience) was added to restrict the analysis to live cells.

**Cell culture**

Transformed cell lines: The mutant Jurkat cell line J76, which lacks endogenous TCR expression, was provided by Dr Mirjam Heemskerk (69). J76 cells were maintained in RPMI 1640 medium supplemented with100 ug/ml penicillin/streptomycin, 1% Hepes buffer, and 2 mM L-glutamine (complete RPMI) in the presence of 10% fetal bovine serum (FBS). Murine fibroblasts (L cells) stably transfected to express a single human HLA-DR allele (DR1, DR3, DR4, DR7, DR11, DR15, or DRB5) were used as antigen presenting cells (70). L cells were maintained in complete RMPI supplemented with 10% FBS and 1% Non-Essential Amino Acids (Life Technologies).

Primary cells: Peripheral blood mononuclear cells (PBMC) were isolated from heparinized blood via density gradient centrifugation on Ficoll-Paque PLUS (GE Healthcare Life Sciences) and were either cryopreserved or used freshly for the preparation of monocyte derived dendritic cells (MDDC). The latter were obtained by positive selection of CD14+ monocytes using magnetic Microbeads (Miltenyi Biotec). Monocytes were plated at $2x10^6$ cells per mL in synthetic AIM-V medium (Life Technologies) supplemented with 10 ng/mL GM-CSF and 20 ng/mL IL-4 (both from Miltenyi Biotec) and incubated for 5-7 days at 37°C in a 5% CO2 incubator. Differentiated immature MDDC were collected and cryopreserved until further use.

Primary CD4+ T cell lines: The 20-mer Gag293 peptide (FRD YVD RFY KTL RAE QAS QE), spanning amino acids 293 to 312 in HIV-1 HXB2 Gag, was used in highly purified form (>99% pure; Proteogenix SAS) to stimulate Gag-specific CD4+ T cell lines. PBMCs from HIV-1 infected patients were plated at $2x10^6$ cells per well in 24-well plates in the presence of decreasing amount of Gag293 peptide ($10^{-5}$M to $10^{-11}$M) in complete RPMI supplemented with 10% human AB serum, 0.5 μM AZT, 5 nM Saquinavir and 5 ng/ml recombinant IL-7 (Cytheris). Recombinant IL-2 (Chiron, Novartis) was added to a final concentration of 100 U/ml starting from day 2, and every 2 days afterwards. When CD4+ T cell lines reached doubling time (observed number of cells = 2 x number of input cells), they were tested for Gag293 specificity by IFN-γ ELISPOT assay, as described previously (71).

**MHC class II tetramer labeling**

Patients were genotyped for the HLA-DRB1 gene at a 4 digit resolution using the INNO-LiPA HLA-DRB1 Plus kit (Fujirebio). Patients were included in the study if their genotype matched at least one of the following alleles: DR1, DR11, DR15, or DRB5. APC-labeled MHC II tetramers for the DR1, DRB15, DRB5, DRB1*0301 (DR3), and DRB1*0701 (DR7) alleles were obtained through the NIH Tetramer Core Facility at Emory University, USA. HLA-DRB1*0401 (DR4) biotinylated monomers were provided by Dr Fabrice Lemaitre (Institut Pasteur, Paris). DR11 biotinylated monomers were obtained through the tetramer Core laboratory of the Benaroya Research Institute (Seattle, USA). Monomers were loaded with 0.2 mg/ml peptide by incubation at 37°C for 72 h in the presence of 2.5 mg/ml n-octyl-β-D-glucopyranoside and protease inhibitors. Peptide-loaded monomers were tetramerized using APC-conjugated streptavidin (eBioscience). For each tetramer loaded with the Gag293 peptide, a corresponding control tetramer was loaded with an irrelevant peptide (either the CLIP peptide PVSKMRMATPLLMQA for the DR1, DR15, DR11 tetramers; or an Annexin II peptide DVPKWISIMTERSVPH for the DRB5 tetramer).

The MHC II tetramer labeling protocol was adapted from (72). Primary CD4+ T cells lines were incubated with 1μg MHC II tetramer/$10^6$ cells at a concentration ≥1 μg/mL in complete RPMI supplemented with 15% human AB serum for 90 min at 4°C. Antibodies for surface markers were added for the last 30 min of labeling, using the following combination: CD3-eF780-APC, CD4-PE-CF594, CD8-BV785, CD45RA-BV421, CCR7-PE-Cy7, CD14-VioGreen, and CD19-VioGreen. Gag293-specific tetramer-labeled (Tet+) cells were visualized in the CD3+, CD4+, CD14-, CD19-, CD8- lymphocyte gate, and were sorted using a FACSAria II cell sorter (BD Biosciences) installed in a microbiological safety cabinet. Each Gag293-tetramer labeled sample was matched with a control-tetramer labeled sample processed in identical conditions. Sorted Tet+ cells were resuspended in RLT buffer (Qiagen) and kept frozen at -80°C until RNA extraction. Sorted samples that yielded a minimum of 20,000 Tet+ events were selected for clonotypic analysis. Patient PBMC were labeled with MHC II tetramer and sorted as described above, with a minimum of $10^7$ cells labeled per sample. Sorted PBMC samples that yielded a minimum of 2,000 Tet+ events were used for clonotypic analysis.

J76 cells expressing recombinant TCRs were labeled as above, except that incubation with the MHC II tetramer was performed in complete RPMI supplemented with 15% FBS for 1h at 37°C, followed by incubation for 30 min at 4°C with the following antibody combination: CD3-eF780-APC, CD4-PE-Cy7, αβTCR-APC and eF506-viability dye. The percentage of Tet+ cells was measured in the live CD3+, CD4+ gate after acquisition on an LSR Fortessa cytometer (BD Biosciences).

CDR3 length polymorphism analysis and CDR3 sequencing

The expression and diversity of 35 TCRα variable gene (TRAV) families and of 24 TCRβ variable gene (TRBV) families were evaluated in Tet+ cells by the Immunoscope technique, as described previously (73, 74). TRAV and TRBV gene expression was measured by quantitative RT-PCR, followed by an analysis of the length distribution of the amplified CDR3 products on a capillary sequencer. Briefly, total RNA was extracted from Tet+ cells using the RNeasy mini or micro kits (Qiagen), depending on available cell number. Next, cDNA was obtained by reverse transcription with 500μg/mL oligo (dT)17 and 200 U of Superscript II reverse transcriptase (Life technologies). A cDNA aliquot was amplified with each of 35 TRAV and 24 TRBV family-specific primers, in combination with a constant region TRAC or a TRBC primer, respectively (73, 74). Amplification was performed in the presence of a family-specific TaqMan probe on an ABI 7300 real time PCR device (Applied Biosystems). An aliquot of each PCR reaction was used as template in a run-off reaction with a nested fluorescent TRAC- or TRBC-specific primer, to generate TRAV- or TRBV-specific single stranded DNA products. These fluorescent DNA samples were separated on an ABI-PRISM 3730 DNA analyzer (Applied Biosystems) and quantified for size and intensity with the Immunoscope software. Fluorescence intensity (in arbitrary units) was plotted in function of the CDR3 length in amino acids.

PCR products corresponding to the TRAV24 and TRBV2 families were cloned and sequenced. The primers used to amplify the full-length chains were: TRAV24 forward primer: 5'-CCG AGG CCT TGT TTG TAA TG-3'; TRAC reverse primer: 5'GTG AAT AGG CAG ACA GAC TTG T-3'; TRBV2 forward primer: 5'-GGT CCG GAA TGG ATA CCT GGC TCG TAT GCT GGG C-3'; TRBC reverse primer: 5'-CCG GTC GAC CTA GCC TCT GGA ATC CTT TCT CTT GAC C-3'. PCR products were cloned into the pCR-Blunt-II-TOPO vector (Life technologies), transformed in E. coli, and analyzed by DNA sequencing (Eurofins Genomics).

**Analysis of the TCR clonotypic repertoire**

TRA and TRB sequences were analyzed with the software suite from the International ImMunoGeneTics (IMGT) Information System (75). The V(D)J gene nomenclature used is that of the IMGT database (www.imgt.org). Clonotypic diversity of the TRAV24 and TRBV2 repertoires was evaluated (i) by counting the number of unique amino acid clonotypes (clonotypes AA) per 100 CDR3 nucleotide sequences (ii) by computing Simpson's diversity index, using the EstimateS software version 9.1.0 (76). Simpson's diversity index takes into account both the number of clonotypes and the frequency of each clonotype in the dataset, and is maximal when all clonotypes have an equal representation. The number of N and P mutations introduced during the V(D)J recombination process was determined by comparing the observed CDR3 sequences to their germline counterparts, using the Junction analysis module of the IMGT/HighV-QUEST software (75). The distribution of TRAV, TRAJ, TRBV, TRBJ, and TRBD genes in the sequence set was computed with the statistics module of IMGT/HighV-QUEST. The CDR3 lengths corresponded to the number of a.a. comprised between, but not including, the two conserved residues C104 and F/W118, as defined by the IMGT-ONTOLOGY unique numbering system. In contrast, the "CDR3 junctions" included the conserved C104 and F/W118 residues. Kurtosis, which measures the "peakedness" of a distribution, was used to evaluate biases in CDR3 lengths. Kurtosis was measured in the Prism v6.0 software (GraphPad). A Gaussian distribution has a kurtosis of zero, while a flatter distribution has negative kurtosis, and a more "peaked" distribution has positive kurtosis.

Motifs enriched in the TRAV24 and TRBV2 CDR3 sequence sets were first identified with the MEME motif discovery software version 4.10.0 (77) available at http://meme-suite.org. The MEME software chooses the width and number of occurrence of each motif automatically in order to minimize the "E-value" of the motif, i.e. the probability of finding an equally well conserved pattern in random sequences. Motifs were searched in discriminative mode, to identify public motifs enriched in the HIC compared to the HAART dataset. Motifs were represented as sequence logos, where the relative sizes of the letters indicate their frequencies in the sequence set, and the total height of the letters represents the information content of the position, in bits. Based on the initial motif analysis, simpler public motifs included within the MEME motifs were identified and counted using the "Protein Pattern Find" module of the Sequence Manipulation Suite (78).

**Analysis of soluble recombinant TCRs by surface plasmon resonance**

Soluble TCRs were engineered by inserting disulfide linkage between the TRAC and TRBC constant domains and truncating the transmembrane and cytoplasmic regions, as previously described (79). The soluble TCRα and TCRβ chains were expressed separately as inclusion bodies, refolded together, and purified before surface plasmon resonance (SPR) analysis. All SPR experiments were conducted at 25°C on a BIAcore 3000 instrument in the presence of TBS buffer (10mM Tris-HCl, pH 8, 150mM NaCl and 0.005% surfactant P20). The TBS buffer was supplemented with 1% BSA to prevent non-specific binding. The pHLA-II complexes were immobilized onto a Streptadivin-coated sensor chip with 1,000-1,200 Response Unit (RU) per flow cell. A flow cell containing a pHLA-I complex was used as negative control.

Experiments were conducted as previously described (79), with a concentration range of 0.78-100 μM of pHLA-II complexes. The BIAevaluationVersion 3.1 software was used for data analysis with the 1:1 Langmuir binding model.

**TCR lentivector construction**

Full-length TCRα and TCRβ chains amplified from Gag293-specific Tet+ cells were cloned into lentiviral expression vectors. Full-length TRAV24+ chains were amplified with a forward primer containing the TRAV24 leader sequence with an NheI restriction site and a Kosak sequence added in 5' (5'-CGG CTA GCC GCC ACC ATG GAG AAG AAT CCT TTG GCA GCC-3') and a reverse primer containing the 3' of TRAC and a NotI site (5'-TTA GCG GCC GCG CTG GAC CAC AGC CGC AGC G-3'). Full-length TRBV2+ chains were amplified with a forward primer containing the TRBV2 leader sequence and a BspEI site in 5' (5'- GGT CCG GAA TGG ATA CCT GGC TCG TAT GCT GGG C-3') and a reverse primer containing the 3' of TRBC and a SalI site (5'- CCG GTC GAC CTA GCC TCT GGA ATC CTT TCT CTT GAC C-3'). The TCRα and TCRβ chains were first cloned separately in the pCR-Blunt-II-TOPO vector, and then combined into the pCDH-EF1-MCS-T2A vector (SBI System Bioscience), with a self-cleaving T2A sequence inserted in between, ensuring an equimolar expression of the two chains from the same transcript. All constructs were verified by DNA sequencing.

The sequence of the plasmid termed "pCDH-F24-TCR" herein, encoding the alpha and beta chains of the "F24" TCR reported in Table S8 is provided under SEQ ID NO: 57 (8241 nucleotides)

**TCR lentivector and HIV-1 pseudotype preparation**

TCR lentivectors were transfected in 293T cells with Lipofectamine 3000 (Life technologies) in the presence of the pPACKH1 Packaging Plasmid Mix (SBI System Bioscience). 48h after transfection, supernatants were collected, filtered using a 0.45 μm filter, and concentrated by ultracentrifugation at 23,000 g for 90 min at 4°C on a 20% sucrose cushion. Viral particles were resuspended in PBS and frozen in aliquots at -80°C until use. Gag p24 concentration was measured with the Alliance HIV-1 p24 Antigen ELISA kit (Perkin Elmer).

Single cycle pseudotyped HIV-1 particles (ΨHIV-1) were produced by calcium phosphate transfection of 25 μg of pNL4-3-deltaEnv-EGFP (from the NIH AIDS Reagent Program) and 10 μg of pVSV-G plasmids in 293T cells. Virus-like particles (VLP) expressing Vpx were obtained similarly by co-transfection of the pSIV3+ vector (80) with pVSV-G. Viruses were harvested at 48h later and concentrated as described above.

**TCR transduction**

For TCR transfer, 0.5 x $10^6$ J76 cells were resuspended in 0.5 mL complete RPMI medium supplemented with 10% FBS in a 24-well plate. TCR lentiviral particles (200 ng of p24) were added to each well and thoroughly resuspended by pipetting. After 3h, 0.5 mL fresh medium was added to each well. Transduced J76 cells were analyzed at day 3 by staining with CD4-PE-Cy7, TCR-APC, CD3-eF780-APC antibodies and the eF510-viability dye. Samples were acquired on an LSR Fortessa flow cytometer (BD Biosciences) to determine TCR expression and relocalization of the CD3 complex to the cell surface as a measure of transduction efficiency.

To transfer TCRs in primary T cells, healthy donor PBMCs were pre-activated with 5 μg/mL PHA and 50 UI/mL IL-2 for 48h. PHA blasts were collected and plated at $10^5$ cells/well in a 24-well plate. TCR lentiviral particles (400 ng of p24) were mixed with 5 μL Lentiblast solution A + 5 μL Lentiblast solution B (OZ Biosciences), and added to each well. Complete RPMI medium supplemented with 10% FBS and 50 UI/ mL IL-2 was added up to a volume of 0.5 mL and plates were centrifuged at 1,000 g for 1h at 32°C, before incubation o/n at 37°C. The following day, fresh medium and 50 UI/mL IL-2 were added up to a volume of 1 mL. 48 to 72h later, transduced PBMC were labeled with TRBV2-PE, CD4-PE-CF594, CD8-BV785, CD3-eF780-APC antibodies, and the eF506-viability dye. Samples analyzed by flow cytometry as above, and quantified for an increase in TRBV2 expression level in the live CD3+ CD4+ CD8- lymphocyte gate to measure transduction efficiency.

**Analyses of TCR functions**

J76 cell activation assay: L cells expressing a single HLA-DR allele were pulsed with serial dilutions (from 2 x $10^{-5}$ to $10^{-11}$ M) of Gag293 peptide. 5 x $10^4$ TCR-transduced J76 cells and 5 x $10^4$ peptide-pulsed L cells were co-cultured in a 96-well plate o/n at 37°C. On the next day, cells were labeled with CD69-PE, CD4-PE-Cy7, TCR-APC, CD3-eF780-APC antibodies and eF506-viability dye. Samples were acquired in a 96-well plate using a FACSCanto II flow cytometer and analyzed to estimate the induction of CD69 expression as a measure of J76 cell activation upon Gag293-HLA-DR recognition.

To measure J76 cell activation upon stimulation with HIV-1-derived, endogenously processed Gag proteins, MDDC were incubated for 3h with 50 ng p24 of ΨHIV-1 per 0.15 x $10^6$ cells in presence of Vpx VLP. Cells were then extensively washed to remove unbound viral particles and co-cultured o/n at 37°C with TCR-transduced J76 cells at a 1:1 ratio. After co-culture, cells were harvested, washed, treated with FcR Blocker (Miltenyi Biotec), stained with CD69-PE, HLA-DR-PE-Cy7, TCR-APC, CD3-eF780-APC antibodies and eF506-viability dye, and analyzed by flow cytometry as above.

Intracellular cytokine staining (ICS) in primary T cells: PBMC from healthy donors expressing at least one of 4 HLA-DR alleles (DR11, DR1, DR15, or DRB5) were transduced with TCR lentivectors and tested 7 to 9 days after transduction. Autologous MDDC (2.5 x $10^4$) were pulsed with serial dilutions of from $10^{-5}$ to $10^{-11}$ M of Gag293 peptide and co-cultured at a 1:1 ratio with transduced PBMC. Cells were co-cultured for 1h at 37°C before addition of 1μg/ml Brefeldin A (eBioscience), and further incubated o/n. Negative controls consisted in TCR-transduced PBMC incubated with unpulsed MDDC and in mock-transduced PBMC incubated with peptide-pulsed MDDC. Positive controls were obtained by pulsing MMDC with 1μg/ml Staphylococcal Enterotoxin A (Toxin Technology, Sarasota, FL), or by using TCR-transduced PBMC stimulated with 50 ng/mL phorbol 12-myristate 13-acetate (PMA) and 0.25 μg/ml ionomycin in the absence of MDDC.

For ICS, cells were washed, treated with FcR Blocker, and stained for surface antigens with CD4-PE-CF594, CD8-BV785, CD14-BV510, CD3-eF780-APC antibodies, and eF506-viability dye. Cells were fixed and permeabilized using the CytoFix/Cytoperm kit (BD Biosciences) before staining for intacellular cytokines with IL-2-APC, MIP1β-PE, IFNγ-PE-Cy7, TNFα-BV421, and CD107a antibodies. Fluorescence was acquired on an LSR Fortessa flow cytometer. Intracellular cytokine production was evaluated in the live CD14-CD3+CD4+CD8- or CD14-CD3+CD4-CD8+ lymphocyte gates. The percentage of cytokine-producing T cells was determined after subtracting the percentage of cytokine-positive events in unstimulated controls. All flow cytometry experiments were analyzed with the Flowjo v8.8 software (Tree Star). Polyfunctionality was assessed for a panel of 5 functions that included the production of IFN-γ, IL-2, MIP-1β, TNF-α and CD107a. All possible combinations of the 5 markers were analyzed by boolean gating in Flowjo. The resulting data table was converted to the matrix symmetric positive definite (SPD) format, and then analyzed using the SPICE software version 5.3 (81), with a cytokine positivity threshold of 0.1%.

HLA-blocking experiments in primary cells: MDDC were brought to a concentration of 5 x $10^6$/mL in complete RPMI medium supplemented with 10% FBS. MDDC were pretreated with 10 μg/mL of an HLA-DR blocking antibody (Biolegend, clone: L243) or a pan-MHC I blocking antibody (Biolegend, clone: W6/32) for 1 h at 37°C prior to Gag293 peptide stimulation. Incubation with 10 μg/mL of an isotypic IgG2a control antibody (Biolegend, clone: MOPC-173) was used as a negative control for HLA blocking. MDDC were then pulsed with $10^{-5}$ M Gag293 peptide and cocultured at a 1:1 ratio with TCR-transduced PBMC, as described above. Cytokine production was evaluated by ICS in the CD8+ T cell gate.

**Statistical analyses**

P values <0.05 were considered statistically significant. Statistics were computed with the Prism v6.0 software (GraphPad) and the R version 3.2.3 software (https://www.r-project.org/). Differences between groups were analyzed with the non parametric Mann-Whitney U test, with the exception of total clonotypic repertoires, for which means where compared with unpaired t tests. Differences in cell line response frequencies and HLA-DR allele frequencies were analyzed in contingency tables with Fisher's exact test. Differences in proportions of CDR3 lengths were computed with a 2-sample test for equality of proportions with continuity correction R. Correlations were analyzed with the non-parametric Spearman's coefficient. Half maximal effective concentrations (EC50) were obtained after non-linear curve fit using a sigmoidal dose response model in Prism. All significant differences between groups (P<0.05) were reported on data plots.

**BIBLIOGRAPHY**

1. Deeks SG, et al. Systemic effects of inflammation on health during chronic HIV infection. Immunity. 2013;39(4):633-45.
2. Connors M, et al. HIV infection induces changes in CD4+ T-cell phenotype and depletions within the CD4+ T-cell repertoire that are not immediately restored by antiviral or immune-based therapies. Nat Med. 1997;3(5):533-40.
3. Gorochov G, et al. Perturbation of CD4+ and CD8+ T-cell repertoires during progression to AIDS and regulation of the CD4+ repertoire during antiviral therapy. Nat Med. 1998;4(2):215-21.
4. Rosenberg ES, et al. Vigorous HIV-1-specific CD4+ T cell responses associated with control of viremia. Science. 1997;278(5342):1447-50.
5. Douek DC, et al. HIV preferentially infects HIV-specific CD4+ T cells. Nature. 2002;417(6884):95-8.
6. Lubong Sabado R, et al. In vitro priming recapitulates in vivo HIV-1 specific T cell responses, revealing rapid loss of virus reactive CD4 T cells in acute HIV-1 infection. PLoS One. 2009;4(1):e4256.

7. Lambotte O, et al. HIV controllers: a homogeneous group of HIV-1-infected patients with spontaneous control of viral replication. Clin Infect Dis. 2005;41 (7):1053-6.
8. Chakrabarti LA, and Simon V. Immune mechanisms of HIV control. Curr Opin Immunol. 2010;22(4):488-96.
9. Saez-Cirion A, et al. HIV controllers exhibit potent CD8 T cell capacity to suppress HIV infection ex vivo and peculiar CTL activation phenotype. Proc Natl Acad Sci U S A. 2007;104(16):6776-81.
10. Almeida JR, et al. Antigen sensitivity is a major determinant of CD8+ T-cell polyfunctionality and HIV-suppressive activity. Blood. 2009;113(25):6351-60.
11. Ladell K, et al. A molecular basis for the control of preimmune escape variants by HIV-specific CD8+ T cells. Immunity. 2013;38(3):425-36.
12. Chen H, et al. TCR clonotypes modulate the protective effect of HLA class I molecules in HIV-1 infection. Nat Immunol. 2012;13(7):691-700.
13. Migueles SA, et al. Lytic granule loading of CD8+ T cells is required for HIV-infected cell elimination associated with immune control. Immunity. 2008;29(6):1009-21.
14. Almeida JR, et al. Superior control of HIV-1 replication by CD8+ T cells is reflected by their avidity, polyfunctionality, and clonal turnover. J Exp Med. 2007;204(10):2473-85.
15. Iglesias MC, et al. Escape from highly effective public CD8+ T-cell clonotypes by HIV. Blood. 2011;118(8):2138-49.
16. Younes SA, et al. HIV-1 viremia prevents the establishment of interleukin 2-producing HIV-specific memory CD4+ T cells endowed with proliferative capacity. J Exp Med. 2003;198(12):1909-22.
17. Harari A, et al. Functional heterogeneity of memory CD4 T cell responses in different conditions of antigen exposure and persistence. J Immunol. 2005;174(2):1037-45.
18. van Grevenynghe J, et al. Transcription factor FOXO3a controls the persistence of memory CD4(+) T cells during HIV infection. Nat Med. 2008;14(3):266-74.
19. Potter SJ, et al. Preserved central memory and activated effector memory CD4+ T-cell subsets in human immunodeficiency virus controllers: an ANRS EP36 study. J Virol. 2007;81(24):13904-15.
20. Kaufmann DE, et al. Limited durability of viral control following treated acute HIV infection. PLoS Med. 2004;1(2):e36.
21. Saez-Cirion A, et al. Restriction of HIV-1 replication in macrophages and CD4+ T cells from HIV controllers. Blood. 2011;118(4):955-64.
22. Ranasinghe S, et al. HIV-Specific CD4 T Cell Responses to Different Viral Proteins Have Discordant Associations with Viral Load and Clinical Outcome. J Virol. 2012;86(1):277-83.
23. Ferre AL, et al. HIV controllers with HLA-DRB1*13 and HLA-DQB1*06 alleles have strong, polyfunctional mucosal CD4+ T-cell responses. J Virol. 2010;84(21):11020-9.
24. Vingert B, et al. HIV controllers maintain a population of highly efficient Th1 effector cells in contrast to patients treated in the long term. J Virol. 2012;86(19):10661-74.
25. Kaufmann DE, et al. Upregulation of CTLA-4 by HIV-specific CD4+ T cells correlates with disease progression and defines a reversible immune dysfunction. Nat Immunol. 2007;8(11):1246-54.
26. Porichis F, et al. Responsiveness of HIV-specific CD4 T cells to PD-1 blockade. Blood. 2011.
27. Vingert B, et al. HIV controller CD4+ T cells respond to minimal amounts of Gag antigen due to high TCR avidity. PLoS Pathog. 2010;6(2):e1000780.
28. Alexander-Miller MA. High-avidity CD8+ T cells: optimal soldiers in the war against viruses and tumors. Immunol Res. 2005;31(1):13-24.
29. Berger CT, et al. High functional avidity CTL responses to HLA-B-restricted Gag-derived epitopes associate with relative HIV control. J Virol. 2011.
30. Kaufmann DE, et al. Comprehensive analysis of human immunodeficiency virus type 1-specific CD4 responses reveals marked immunodominance of gag and nef and the presence of broadly recognized peptides. J Virol. 2004;78(9):4463-77.
31. Wooldridge L, et al. Tricks with tetramers: how to get the most from multimeric peptide-MHC. Immunology. 2009;126(2):147-64.
32. Lim A, et al. Frequent contribution of T cell clonotypes with public TCR features to the chronic response against a dominant EBV-derived epitope: application to direct detection of their molecular imprint on the human peripheral T cell repertoire. J Immunol. 2000;165(4):2001-11.
33. Hacein-Bey-Abina S, et al. Efficacy of gene therapy for X-linked severe combined immunodeficiency. N Engl J Med. 2010;363(4):355-64.
34. Alamyar E, et al. IMGT((R)) tools for the nucleotide analysis of immunoglobulin (IG) and T cell receptor (TR) V-(D)-J repertoires, polymorphisms, and IG mutations: IMGT/V-QUEST and IMGT/HighV-QUEST for NGS. Methods Mol Biol. 2012;882:569-604.
35. Turner SJ, et al. Structural determinants of T-cell receptor bias in immunity. Nat Rev Immunol. 2006;6(12):883-94.

36. Venturi V, et al. A mechanism for TCR sharing between T cell subsets and individuals revealed by pyrosequencing. J Immunol. 2011;186(7):4285-94.
37. Gras S, et al. T-cell receptor bias and immunity. Curr Opin Immunol. 2008;20(1):119-25.
38. Rossjohn J, et al. T cell antigen receptor recognition of antigen-presenting molecules. Annu Rev Immunol. 2015;33:169-200.
39. Betts MR, et al. HIV nonprogressors preferentially maintain highly functional HIV-specific CD8+ T cells. Blood. 2006;107(12):4781-9.
40. Liu P, et al. Characterization of human alphabetaTCR repertoire and discovery of D-D fusion in TCRbeta chains. Protein Cell. 2014;5(8):603-15.
41. Gillespie GM, et al. Strong TCR conservation and altered T cell cross-reactivity characterize a B*57-restricted immune response in HIV-1 infection. J Immunol. 2006;177(6):3893-902.
42. Kloverpris HN, et al. CD8+ TCR Bias and Immunodominance in HIV-1 Infection. J Immunol. 2015.
43. Miles JJ, et al. Bias in the alphabeta T-cell repertoire: implications for disease pathogenesis and vaccination. Immunol Cell Biol. 2011;89(3):375-87.
44. Mendoza D, et al. HLA B*5701-positive long-term nonprogressors/elite controllers are not distinguished from progressors by the clonal composition of HIV-specific CD8+ T cells. J Virol. 2012;86(7):4014-8.
45. Simons BC, et al. Despite biased TRBV gene usage against a dominant HLA B57-restricted epitope, TCR diversity can provide recognition of circulating epitope variants. J Immunol. 2008;181(7):5137-46.
46. Price DA, et al. Public clonotype usage identifies protective Gag-specific CD8+ T cell responses in SIV infection. J Exp Med. 2009;206(4):923-36.
47. Warren RL, et al. Exhaustive T-cell repertoire sequencing of human peripheral blood samples reveals signatures of antigen selection and a directly measured repertoire size of at least 1 million clonotypes. Genome Res. 2011;21(5):790-7.
48. Mattapallil JJ, et al. Massive infection and loss of memory CD4+ T cells in multiple tissues during acute SIV infection. Nature. 2005;434(7037):1093-7.
49. Godfrey DI, et al. The burgeoning family of unconventional T cells. Nat Immunol. 2015;16(11):1114-23.
50. Campion SL, et al. Proteome-wide analysis of HIV-specific naive and memory CD4(+) T cells in unexposed blood donors. J Exp Med. 2014;211(7):1273-80.
51. Jones RB, et al. Human immunodeficiency virus type 1 escapes from interleukin-2-producing CD4+ T-cell responses without high-frequency fixation of mutations. J Virol. 2009;83(17):8722-32.
52. Ranasinghe S, et al. Association of HLA-DRB1-restricted CD4 T cell responses with HIV immune control. Nature Medicine. 2013;19(7):930-3.
53. Yousef S, et al. TCR bias and HLA cross-restriction are strategies of human brain-infiltrating JC virus-specific CD4+ T cells during viral infection. J Immunol. 2012;189(7):3618-30.
54. Malhotra U, et al. Role for HLA class II molecules in HIV-1 suppression and cellular immunity following antiretroviral treatment. J Clin Invest. 2001; 107(4):505-17.
55. Williams MA, et al. Rapid culling of the CD4+ T cell repertoire in the transition from effector to memory. Immunity. 2008;28(4):533-45.
56. Belyakov IM, et al. Impact of vaccine-induced mucosal high-avidity CD8+ CTLs in delay of AIDS viral dissemination from mucosa. Blood. 2006;107(8):3258-64.
57. Corse E, et al. Attenuated T cell responses to a high-potency ligand in vivo. PLoS Biol. 2010;8(9).
58. Valitutti S. The Serial Engagement Model 17 Years After: From TCR Triggering to Immunotherapy. Front Immunol. 2012;3:272.
59. Thorbom G, et al. Clonotypic composition of the CD4+ T cell response to a vectored retroviral antigen is determined by its speed. J Immunol. 2014;193(4):1567-77.
60. Swain SL, et al. Expanding roles for CD4(+) T cells in immunity to viruses. Nat Rev Immunol. 2012;12(2):136-48.
61. Blanco-Melo D, et al. Intrinsic cellular defenses against human immunodeficiency viruses. Immunity. 2012;37(3):399-411.
62. Vetter ML, et al. Differences in APOBEC3G expression in CD4+ T helper lymphocyte subtypes modulate HIV-1 infectivity. PLoS Pathog. 2009;5(2):e1000292.
63. Oswald-Richter K, et al. Identification of a CCR5-expressing T cell subset that is resistant to R5-tropic HIV infection. PLoS Pathog. 2007;3(4):e58.
64. Reinherz EL, and Wang JH. Codification of bidentate pMHC interaction with TCR and its co-receptor. Trends Immunol. 2015.
65. Rihn SJ, et al. Extreme genetic fragility of the HIV-1 capsid. PLoS Pathog. 2013;9(6):e1003461.
66. Hansen SG, et al. Cytomegalovirus vectors violate CD8+ T cell epitope recognition paradigms. Science. 2013;340(6135):1237874.
67. Ghorashian S, et al. CD8 T cell tolerance to a tumor-associated self-antigen is reversed by CD4 T cells engineered to express the same T cell receptor. J Immunol. 2015;194(3):1080-9.

68. Gras S, et al. The shaping of T cell receptor recognition by self-tolerance. Immunity. 2009;30(2):193-203.

69. Heemskerk MH, et al. Redirection of antileukemic reactivity of peripheral T lymphocytes using gene transfer of minor histocompatibility antigen HA-2-specific T-cell receptor complexes expressing a conserved alpha joining region. Blood. 2003;102(10):3530-40.

70. Klohe EP, et al. Analysis of the molecular specificities of anti-class II monoclonal antibodies by using L cell transfectants expressing HLA class II molecules. J Immunol. 1988;141(6):2158-64.

71. Vingert B, et al. HIV controller CD4+ T cells respond to minimal amounts of Gag antigen due to high TCR avidity. PLoS Pathog. 2010;6(2):e1000780.

72. Seth N, et al. Expansion and contraction of HIV-specific CD4 T cells with short bursts of viremia, but physical loss of the majority of these cells with sustained viral replication. J Immunol. 2005;175(10):6948-58.

73. Lim A, et al. Frequent contribution of T cell clonotypes with public TCR features to the chronic response against a dominant EBV-derived epitope: application to direct detection of their molecular imprint on the human peripheral T cell repertoire. J Immunol. 2000;165(4):2001-11.

74. Hacein-Bey-Abina S, et al. Efficacy of gene therapy for X-linked severe combined immunodeficiency. N Engl J Med. 2010;363(4):355-64.

75. Alamyar E, et al. IMGT((R)) tools for the nucleotide analysis of immunoglobulin (IG) and T cell receptor (TR) V-(D)-J repertoires, polymorphisms, and IG mutations: IMGT/V-QUEST and IMGT/HighV-QUEST for NGS. Methods Mol Biol. 2012;882(569-604.

76. Colwell RK. EstimateS: Statistical estimation of species richness and shared species from samples. Version 9. http://purloclcorg/estimates. 2013.

77. Bailey TL, et al. The MEME Suite. Nucleic Acids Res. 2015.

78. Stothard P. The sequence manipulation suite: JavaScript programs for analyzing and formatting protein and DNA sequences. Biotechniques. 2000;28(6):1102, 4.

79. Gras S, et al. The shaping of T cell receptor recognition by self-tolerance. Immunity. 2009;30(2):193-203.

80. Negre D, et al. Characterization of novel safe lentiviral vectors derived from simian immunodeficiency virus (SIVmac251) that efficiently transduce mature human dendritic cells. Gene Ther. 2000;7(19):1613-23.

81. Roederer M, et al. SPICE: exploration and analysis of post-cytometric complex multivariate datasets. Cytometry A. 2011;79(2):167-74.

**Claims**

1. A T-cell receptor (TCR) which is specific for the epitope located between positions 293-312 in the GAG protein of HIV-1 having the amino-acid sequence FRDYVDRF[Y/F]KTLRAEQA[S/T]QE (SEQ ID NO: 1), comprising an alpha chain and a beta chain whose variable domains each comprise three complementarity determining regions CDR1, CDR2 and CDR3, wherein

   a. the amino-acid sequence of the CDR1 and CDR2 on the alpha variable chain is encoded by the human TRAV24 gene, and
   b. the amino-acid sequence of the CDR1 and CDR2 on the beta variable chain is encoded by the human TRBV2 gene, and
   c. the TCR has a sensitivity for said epitope of the GAG protein of HIV-1 that is measured as the capability of CD4+ T cells which express the TCR, to proliferate and to differentiate into TNF-alpha and/or IFN-gamma secreting effectors upon stimulation with said epitope with half-maximal responses for TNF-alpha or IFN-gamma production (EC50) being achieved in particular with less than $10^{-7}$ M of Gag293 epitope concentration.

2. A TCR which is specific for the epitope located between positions 293-312 in the GAG protein of HIV-1 having the amino-acid sequence FRDYVDRF[Y/F]KTLRAEQA[S/T]QE (SEQ ID NO: 1), comprising an alpha chain and a beta chain whose variable domains each comprise three complementarity determining regions CDR1, CDR2 and CDR3, wherein

   a. the amino-acid sequence of the CDR1 and CDR2 on the alpha variable chain is encoded by the human TRAV24 gene, and
   b. the amino-acid sequence of the CDR1 and CDR2 on the beta variable chain is encoded by the human TRBV2 gene, and/or
   c. the amino-acid sequence of the CDR3 on the alpha chain comprises a motif selected amongst: [A/S]X[K/R]AAGNKLT (SEQ ID NO: 2), AXYGGATNKLI (SEQ ID NO: 3), AX[R/N][R/N]AGNMLTF (SEQ ID NO: 4), AXD[N/D]RKLI (SEQ ID NO: 5) or AXE[S/G]X[G/A][A/S][Q/E]KLV (SEQ ID NO: 6), X being any amino-acid, and/or
   d. the amino-acid sequence of the CDR3 on the beta chain comprises a motif selected amongst: ASSX[R/G/L][T/A][S/G]GXX[E/D/T][Q/T][F/Y]) (SEQ ID NO: 7), ASSX[R/G/L][T/A][S/G/A]GXX[E/D/T/P][Q/T][F/Y/H] (SEQ ID NO: 8), ASSGXXNTEAF (SEQ ID NO: 9) or ASVLMRT[N/R]NEQF (SEQ ID NO: 10), X being any amino-acid.

3. The TCR of claim 1 or 2, which specifically recognizes a peptide from the GAG protein of HIV-1 having the amino-acid sequence FRDYVDRF[Y/F]KTLRAEQA[S/T]QE (SEQ ID NO: 1) when presented by a major histocompatibility complex (MHC) molecule, in particular when said peptide is presented by a peptide-MHC II complex (by antigen-presenting cells).

**4.** The TCR of any one of claims 1 to 3, which has at least one of the following properties:

a. an affinity for the epitope located between positions 293-312 of the GAG protein of HIV-1 measured by a Kd value m that is equal or less than 20 μM by SPR analysis and in particular that is equal or less than 10 μM, more particularly equal or less than 1 μM, more particularly between the range of 0.5 to 7 μM, especially the range of 0.5 to 1 μM, by SPR analysis and/or
b. it is polyfunctional as measured by assaying induction of at least 3 cytokines, in particular 5 cytokines in the group of TNF-alpha, IL-2, INF-gamma, MIP-1beta and degranulation marker CD107a, and preferably cytokines including IFN-gamma and TNF-alpha by primary CD4+T cells expressing said TCR upon limiting antigenic stimulation using the Gag293 peptide.

**5.** The TCR of any one of claims 1 to 4, wherein:

a. the amino-acid sequence of the CDR3 on the alpha chain is or comprises a sequence as disclosed in any one of SEQ ID NO: 11 to 27, and/or
b. the amino-acid sequence of the CDR3 on the beta chain is or comprises a sequence as disclosed in any one of SEQ ID NO: 28 to 46, or
c. the amino-acid sequence of the CDR3 on the alpha and/or beta chain comprises a variant having at least 80 % amino-acids sequence identity with the sequences disclosed in a. and b. respectively,

the length of the amino-acid sequence of the CDR3 on the alpha chain being from 9 and 16 amino-acid residues, in particular 12 amino-acid residues, the length of the amino-acid sequence of the CDR3 on the beta chain being from 11 and 18 amino-acid residues, in particular 15 amino-acid residues.

**6.** The TCR of any one of claims 1 to 5, wherein:

a. the amino-acid sequence of the CDR3 on the alpha chain is or comprises a sequence selected from : CAFKAAGNKLTF (SEQ ID NO: 47), CASKAAGNKLTF (SEQ ID NO: 48), and CSRRAAGNKLTF (SEQ ID NO: 49), and/or
b. the amino-acid sequence of the CDR3 on the beta chain is or comprises a sequence selected from : CASSRLAGGMDEQF (SEQ ID NO: 50), CATTPGASGISEQF (SEQ ID NO: 51), CASSPGTSGVEQFF (SEQ ID NO: 52), and CASSRRTSGGTDTQYF (SEQ ID NO: 53), or
c. the amino-acid sequence of the CDR3 on the alpha and/or beta chain comprises a variant having at least 80 % amino-acids sequence identity with the sequences disclosed in a. and b. respectively,

the length of the amino-acid sequence of the CDR3 on the alpha chain being from 9 and 16 amino-acid residues, in particular 12 amino-acid residues, the length of the amino-acid sequence of the CDR3 on the beta chain being from 11 and 18 amino-acid residues, in particular 15 amino-acid residues.

**7.** The TCR of any one of claims 1 to 6, wherein:

- the amino-acid sequence of the CDR1 and CDR2 on the alpha variable chain encoded by the human TRAV24 gene, in particular has an amino-acid sequence for the CDR1alpha corresponding to the positions 49 to 54 in SEQ ID NO: 58 and has an amino-acid sequence for the CDR2alpha corresponding to the positions 72 to 77 in SEQ ID NO: 58 and
- the amino-acid sequence of the CDR1 and CDR2 on the beta variable chain encoded by the human TRBV2 gene, in particular has an amino-acid sequence for the CDR1beta corresponding to the positions 46 to 50 in SEQ ID NO: 59 and has an amino-acid sequence for the CDR2beta corresponding to the positions 68 to 73 in SEQ ID NO: 59 and
- the amino-acid sequence of the CDR3 on the alpha chain that is: CAFKAAGNKLTF (SEQ ID NO: 11), and the amino-acid sequence of the CDR3 on the beta chain that is: CASSRLAGGMDEQFF (SEQ ID NO: 512), or
- the amino-acid sequence of the CDR3 on the alpha and/or beta chain comprises a variant having at least 80 % amino-acids sequence identity with the sequences disclosed above for the CDR3 found on the alpha and beta chains, respectively, the length of the amino-acid sequence of the CDR3 on the alpha chain being from 9 and 16 amino-acid residues, in particular 12 amino-acid residues, the length of the amino-acid sequence of the CDR3 on the beta chain being from 11 and 18 amino-acid residues, in particular 15 amino-acid residues.

**8.** The TCR of any one of claims 1 to 7, wherein:

- the amino-acid sequence of its alpha chain is as disclosed in SEQ ID NO: 58 or SEQ ID NO: 60, and
- the amino-acid sequence of its beta chain is as disclosed in SEQ ID NO: 59 or SEQ ID NO: 61, or
- the amino-acid sequence of its alpha and/or beta chain is a variant having at least 80 % amino-acids sequence identity with the sequences disclosed above.

**9.** The TCR of any one of claims 1 to 8, which is a recombinant TCR and/or a chimeric TCR such as a single chain TCR, a soluble TCR, a single chain TCR fragment.

**10.** A nucleic acid molecule encoding at least one chain of TCR as defined in any one of the preceding claims, or a fragment thereof selected in the group of nucleic acid molecules encoding the alpha chain, the variable domain of the alpha chain, the CDR3 domain of the alpha chain, the beta chain, the variable domain of the beta chain, the CDR3 domain of the beta chain, the soluble form of the TCR.

**11.** A nucleic acid molecule encoding at least a part of the alpha chain of a human TCR and/or at least a part of the beta chain of a TCR, in which :

a. the nucleic acid molecule encoding at least a part of the alpha chain, especially the variable domain of the alpha chain, of a TCR comprises at least the nucleotide sequence of the human TRAV24 gene coding the CDR1 and the CDR2 of a TCR and further comprises the **junctional rearranged nucleotide sequence** disclosed in any one of SEQ ID NO: 79 to SEQ ID NO: 120 or a variant **thereof** having at least 80 % sequence identity with these sequences, and/or
b. the nucleic acid molecule encoding at least a part of the beta chain, especially the variable domain of the beta chain, of a TCR comprises at least the nucleotide sequence of the human TRBV2 gene coding the CDR1 and the CDR2 of a TCR and further comprises the **junctional rearranged nucleotide sequence** disclosed in any one of SEQ ID NO: 121 to SEQ ID NO: 161 or a variant **thereof** having at least 80 % sequence identity with these sequences.

**12.** The nucleic acid molecule according to claim 11, which comprises or consists of the sequence disclosed in SEQ ID NO: 62, and/or comprises or consists of the sequence disclosed in SEQ ID NO: 63, or variant thereof having at least 80 % nucleotide sequence identity with the sequences.

**13.** The nucleic acid molecule according to any one of claim 11 or 12, which comprises a nucleic acid sequence coding for the alpha chain and/or the beta chain of a TCR as defined in any one of claims 1 to 8.

**14.** A recombinant nucleic acid molecule which comprises the nucleic acid molecule coding for the alpha chain and the nucleic acid molecule of the beta chain of a TCR wherein both nucleic acid molecules are linked to form a single molecule, optionally are linked by a polynucleotide encoding a 2A peptide, and each of said nucleic acid molecule is as defined in any one of claims 10 to 13.

**15.** A vector, in particular a plasmid, especially a lentiviral transfer vector, comprising at least one nucleic acid molecule according to any one of claims 10 to 14.

**16.** A lentiviral transfer vector according to claim 15, which comprises lentiviral cis-active elements including long terminal repeats (LTRs) or modified LTRs including partially deleted 3'LTR, psi (Ψ) packaging signal, optionally Rev responsive element (RRE), wherein the nucleic acid molecule according to any one of claims 10 to 14 are contained in a transcription unit and wherein said vector optionally further comprises a lentiviral DNA flap encompassing the fragment of the lentiviral genome framed by the regions of central polypurine tract (cPPT) and central termination sequence (CTS) and/or wherein the transcription unit optionally comprises a self-cleaving 2A sequence inserted between sequences encoding alpha and beta TCR chains.

**17.** The lentiviral transfer vector according to any one of claims 15 to 16, wherein the lentiviral sequences are from the genomic sequence of a viral species selected from the group consisting of: human immunodeficiency virus (HIV), simian immunodeficiency virus (SIV), visna/maedi virus (VMV), caprine arthritis-encephalitis virus (CAEV), equine infectious anemia virus (EIAV), feline immunodeficiency virus (FIV), and bovine immunodeficiency virus (BIV)

**18.** A method to produce recombinant lentiviral vector particles, comprising or consisting of:

a) transfecting the recombinant lentiviral transfer vector according to any one of claims 15 to 17, into a host cell, for example a HEK-293T cell line;
b) co-transfecting the cell of step a) with (i) a plasmid vector encoding the envelope glycoprotein G of a VSV, in particular the VSV-G of Indiana or of New Jersey VSV strains and (ii) with a plasmid vector encoding the lentiviral GAG and POL protein or mutated non integrative POL protein of a lentivirus, in particular of a HIV-1 lentivirus, as packaging construct;
c) recovering the recombinant lentiviral particles expressing recombinant TCR.

**19.** Recombinant lentiviral vector particles the genome of which comprises the recombinant lentiviral transfer vector according to any one of claims 15 to 17, in particular recombinant lentiviral vector particles which are pseudotyped with a vesicular stomatitis virus glycoprotein G (VSV-G) protein, in particular the VSV-G protein of the VSV Indiana strain or the VSV protein of the New Jersey strain.

**20.** A method for obtaining a collection of recombinant cells expressing a TCR or a recombinant TCR as defined in any one of claims 1 to 9, comprising the steps of :

a. Transducing cells capable of expressing a TCR at their surface with recombinant lentiviral vector particles according to claim19, and
b. Culturing the transduced cells in conditions that permit the TCR to be expressed at their surface, and
c. Obtaining and/or recovering a recombinant cell collection expressing said recombinant TCR and optionally further isolating said expressed recombinant TCR.

**21.** A collection of recombinant human cells presenting a TCR as claimed in any one of claims 1 to 9 or a collection of cells transduced by a lentiviral vector according to claim 19, in particular a collection of T-cells, in particular primary T cells or CD4+ T cells which is formulated for administration to a human host.

**22.** The recombinant lentiviral vector particles according to claim 19 or obtained by the method of claim 18, or collection of cells according to claim 21 or obtained by the method of claim 20, for use as a medicament in the immunotherapeutic treatment of a human patient seropositive for HIV, in particular HIV-1, in particular for active control of HIV infection.

**23.** The recombinant lentiviral vector particles or collection of cells for use according to claim 22, wherein the human patient is under antiretroviral therapy following a HIV infection, in particular a HIV-1 infection, especially a human patient undergoing HAART therapy (highly active antiretroviral therapy), more particularly a human patient who has been under antiretroviral therapy, in particular a patient showing pharmacological control of HIV infection.

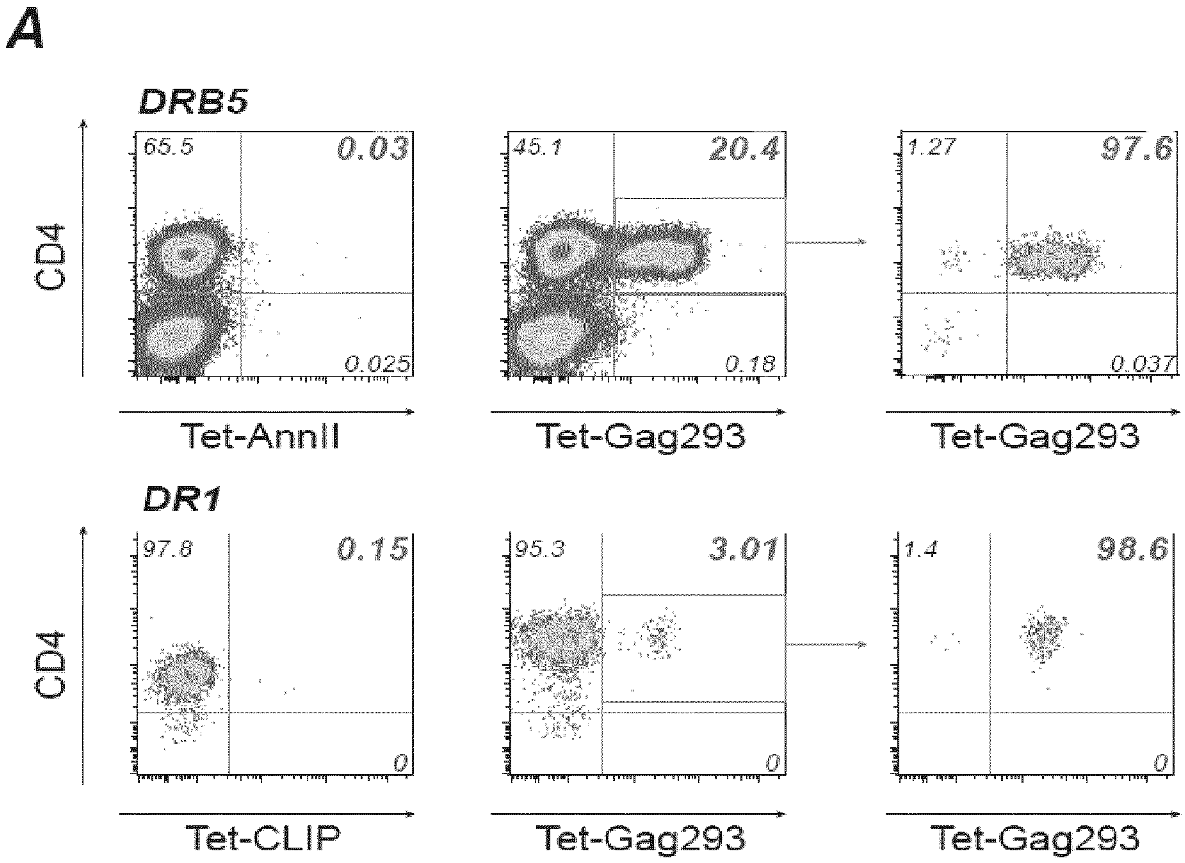


Fig. 1A

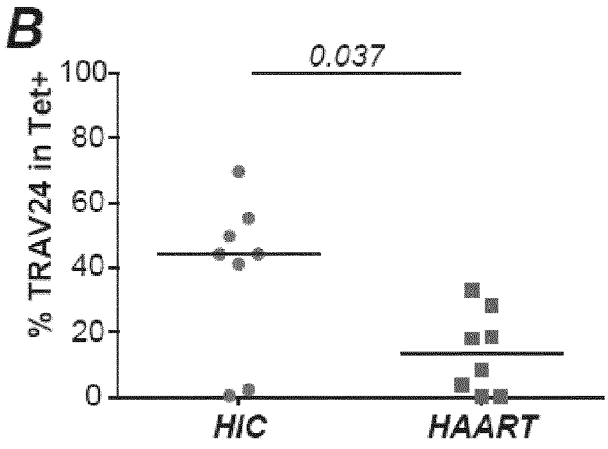


Fig. 1B

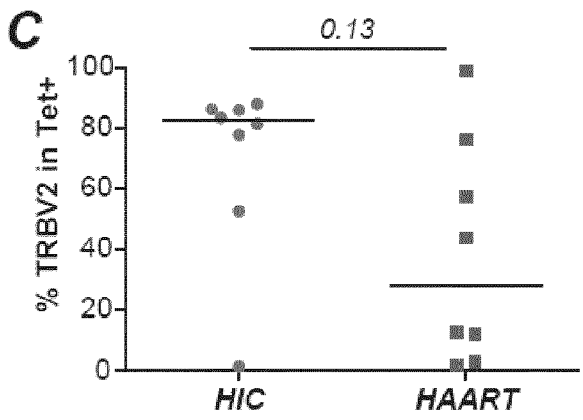


Fig. 1C

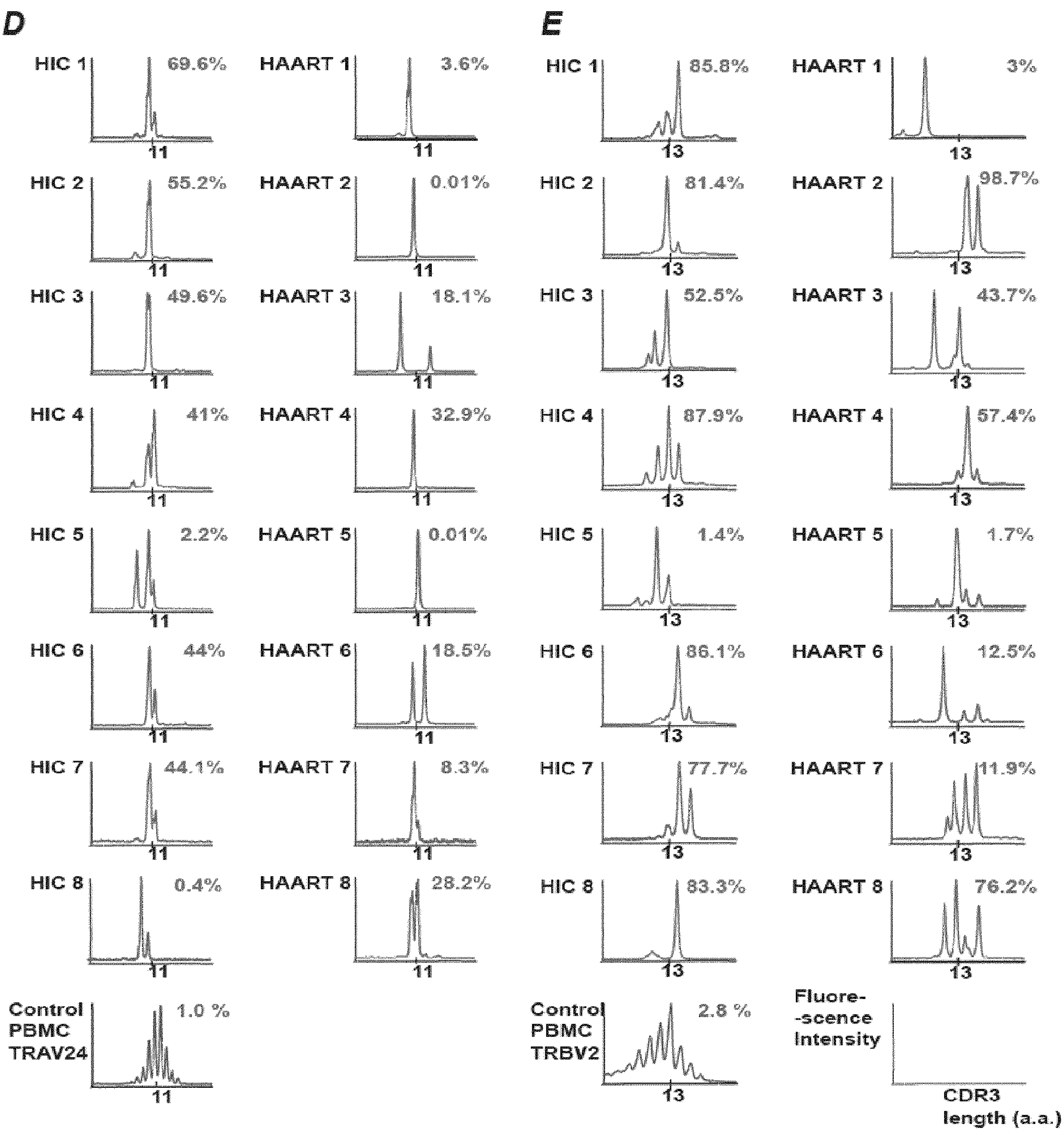
D
HIC 1
69.6%
11
HAART 1
3.6%
11
HIC 2
55.2%
11
HAART 2
0.01%
11
HIC 3
49.6%
11
HAART 3
18.1%
11
HIC 4
41%
11
HAART 4
32.9%
11
HIC 5
2.2%
11
HAART 5
0.01%
11
HIC 6
44%
11
HAART 6
18.5%
11
HIC 7
44.1%
11
HAART 7
8.3%
11
HIC 8
0.4%
11
HAART 8
28.2%
11
Control PBMC TRAV24
1.0 %
11
E
HIC 1
85.8%
13
HAART 1
3%
13
HIC 2
81.4%
13
HAART 2
98.7%
13
HIC 3
52.5%
13
HAART 3
43.7%
13
HIC 4
87.9%
13
HAART 4
57.4%
13
HIC 5
1.4%
13
HAART 5
1.7%
13
HIC 6
86.1%
13
HAART 6
12.5%
13
HIC 7
77.7%
13
HAART 7
11.9%
13
HIC 8
83.3%
13
HAART 8
76.2%
13
Control PBMC TRBV2
2.8 %
13
Fluore-
-scence
Intensity
CDR3
length (a.a.)

Fig. 1D Fig. 1E

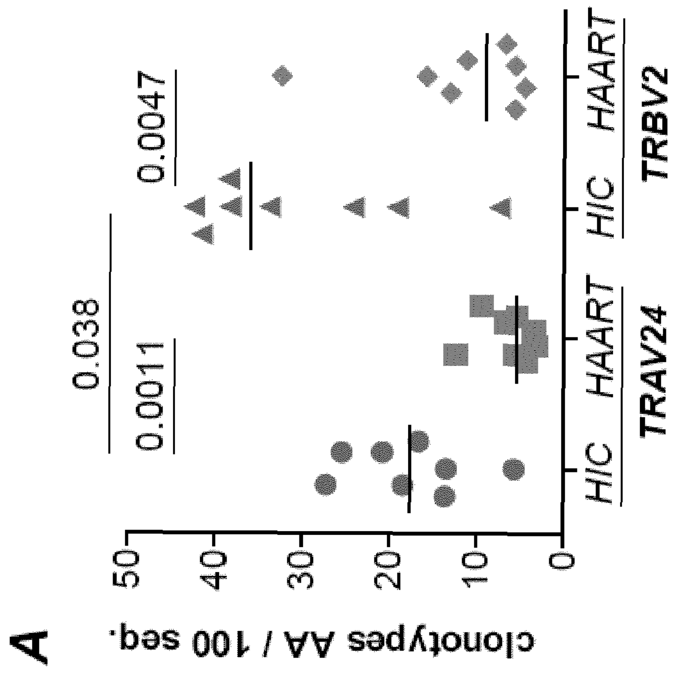
A
clonotypes AA / 100 seq.
0
10
20
30
40
50
HIC
HAART
TRAV24
HIC
HAART
TRBV2
0.038
0.0011
0.0047

Fig. 2A

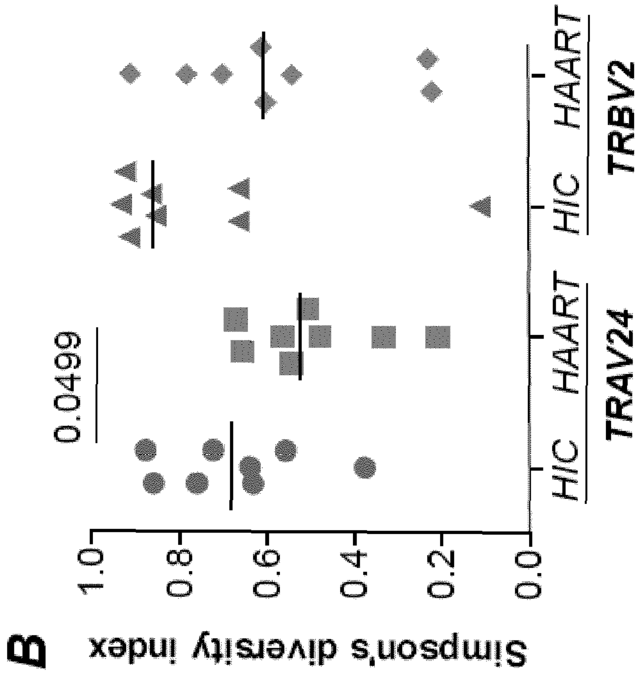
B
Simpson's diversity index
0.0
0.2
0.4
0.6
0.8
1.0
HIC
HAART
TRAV24
HIC
HAART
TRBV2
0.0499

Fig. 2B

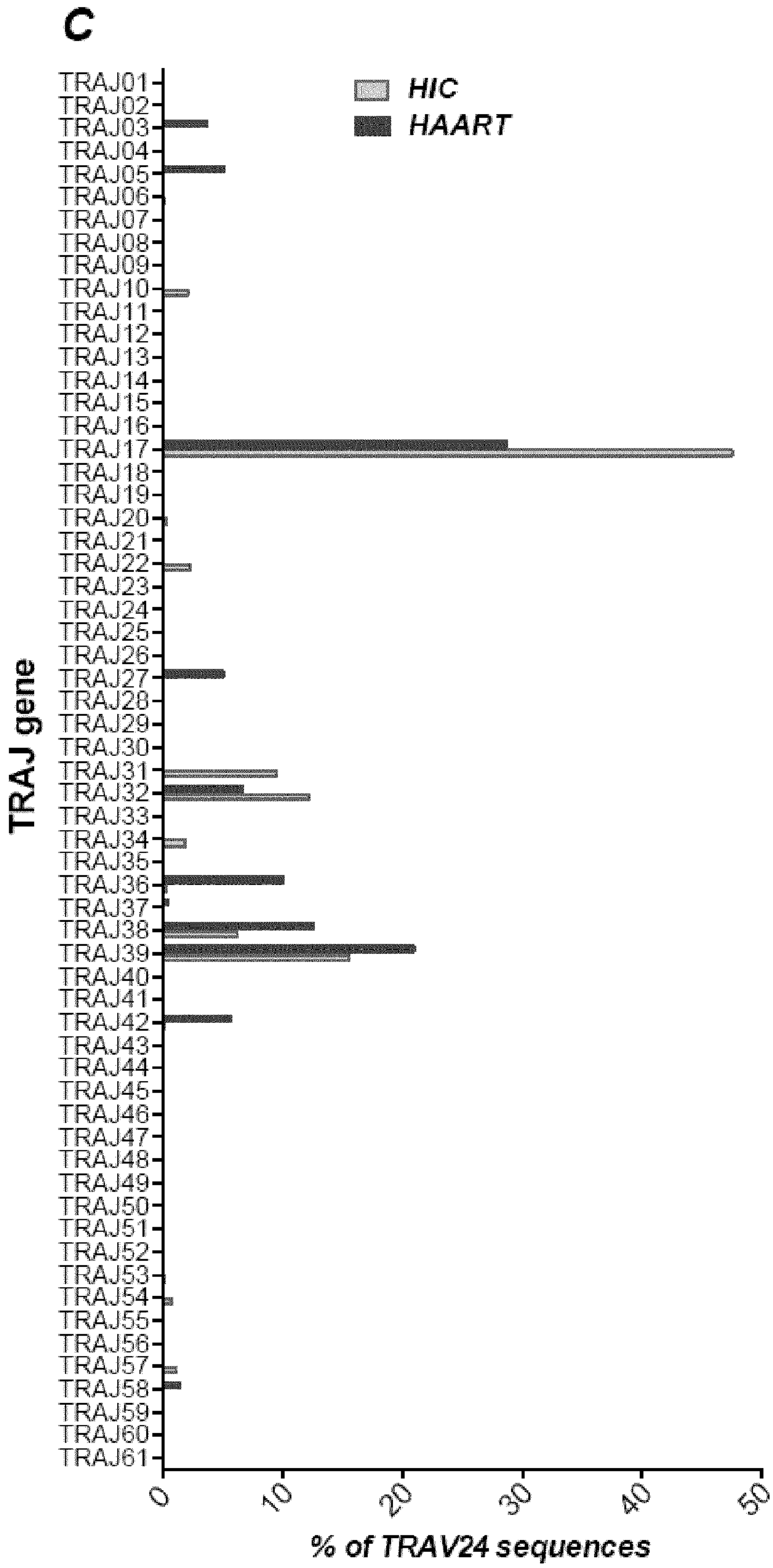
C
HIC
HAART
TRAJ gene
TRAJ01
TRAJ02
TRAJ03
TRAJ04
TRAJ05
TRAJ06
TRAJ07
TRAJ08
TRAJ09
TRAJ10
TRAJ11
TRAJ12
TRAJ13
TRAJ14
TRAJ15
TRAJ16
TRAJ17
TRAJ18
TRAJ19
TRAJ20
TRAJ21
TRAJ22
TRAJ23
TRAJ24
TRAJ25
TRAJ26
TRAJ27
TRAJ28
TRAJ29
TRAJ30
TRAJ31
TRAJ32
TRAJ33
TRAJ34
TRAJ35
TRAJ36
TRAJ37
TRAJ38
TRAJ39
TRAJ40
TRAJ41
TRAJ42
TRAJ43
TRAJ44
TRAJ45
TRAJ46
TRAJ47
TRAJ48
TRAJ49
TRAJ50
TRAJ51
TRAJ52
TRAJ53
TRAJ54
TRAJ55
TRAJ56
TRAJ57
TRAJ58
TRAJ59
TRAJ60
TRAJ61
0
10
20
30
40
50
% of TRAV24 sequences

Fig. 2C

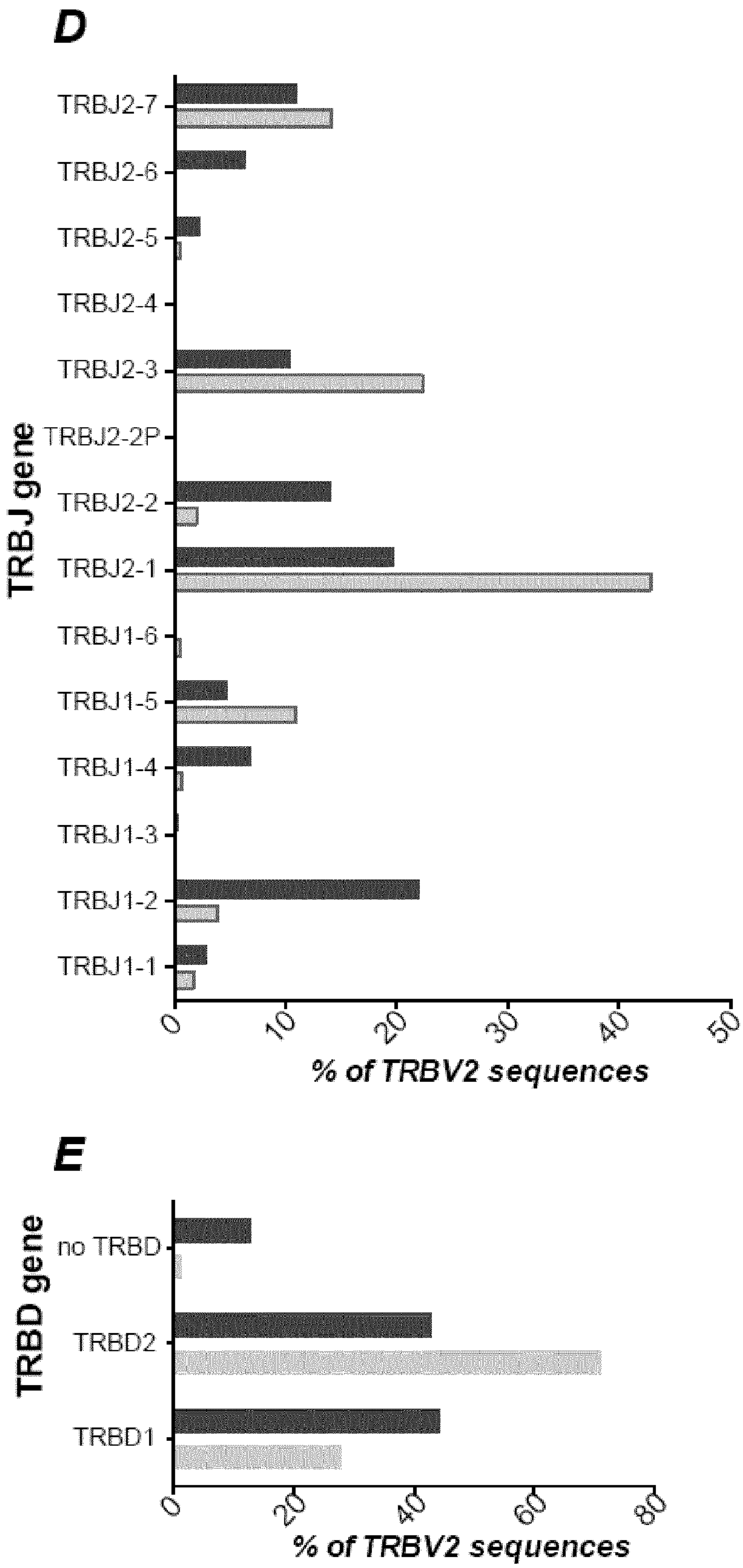
D
TRBJ gene
TRBJ2-7
TRBJ2-6
TRBJ2-5
TRBJ2-4
TRBJ2-3
TRBJ2-2P
TRBJ2-2
TRBJ2-1
TRBJ1-6
TRBJ1-5
TRBJ1-4
TRBJ1-3
TRBJ1-2
TRBJ1-1
0
10
20
30
40
50
% of TRBV2 sequences
E
TRBD gene
no TRBD
TRBD2
TRBD1
0
20
40
60
80
% of TRBV2 sequences

Fig. 2D, 2E

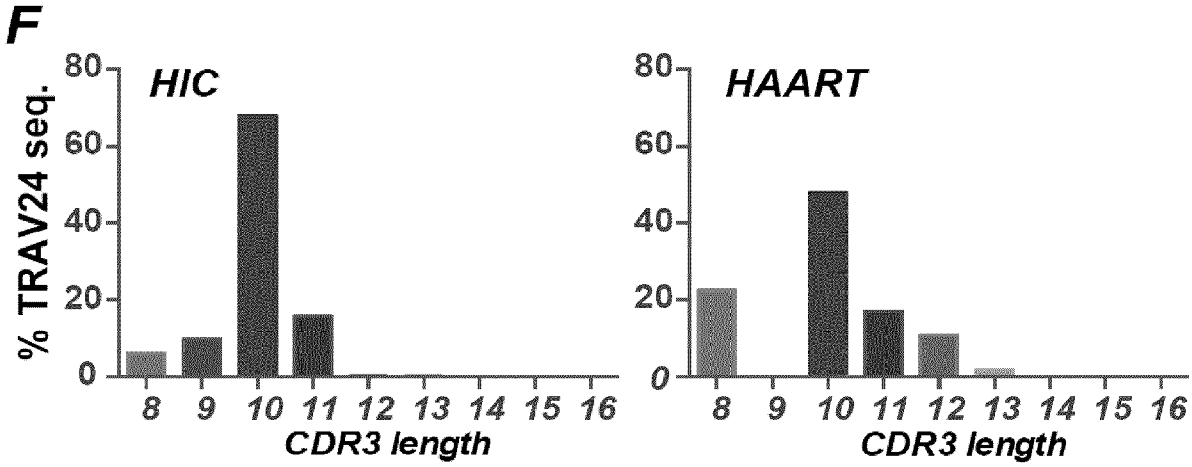
F
HIC
HAART
% TRAV24 seq.
80
60
40
20
0
8 9 10 11 12 13 14 15 16
CDR3 length

Fig. 2F

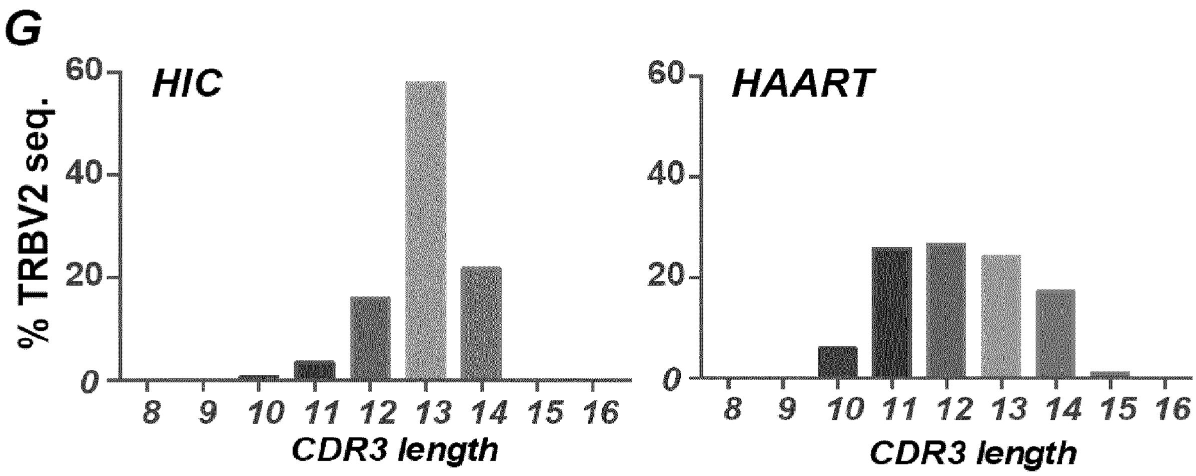
G
HIC
HAART
% TRBV2 seq.
60
40
20
0
8 9 10 11 12 13 14 15 16
CDR3 length

Fig. 2G

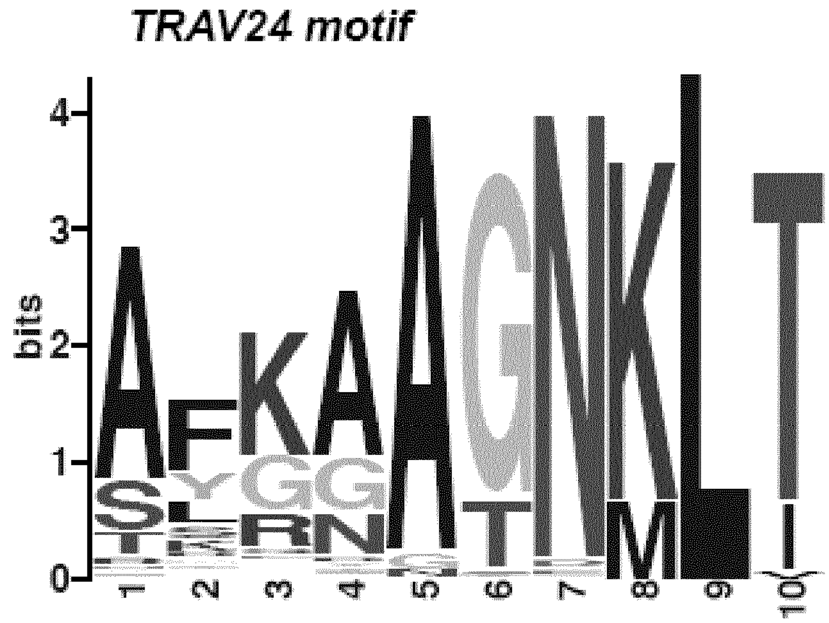
TRAV24 motif
bits
0
1
2
3
4
1
2
3
4
5
6
7
8
9
10

Fig. 3A

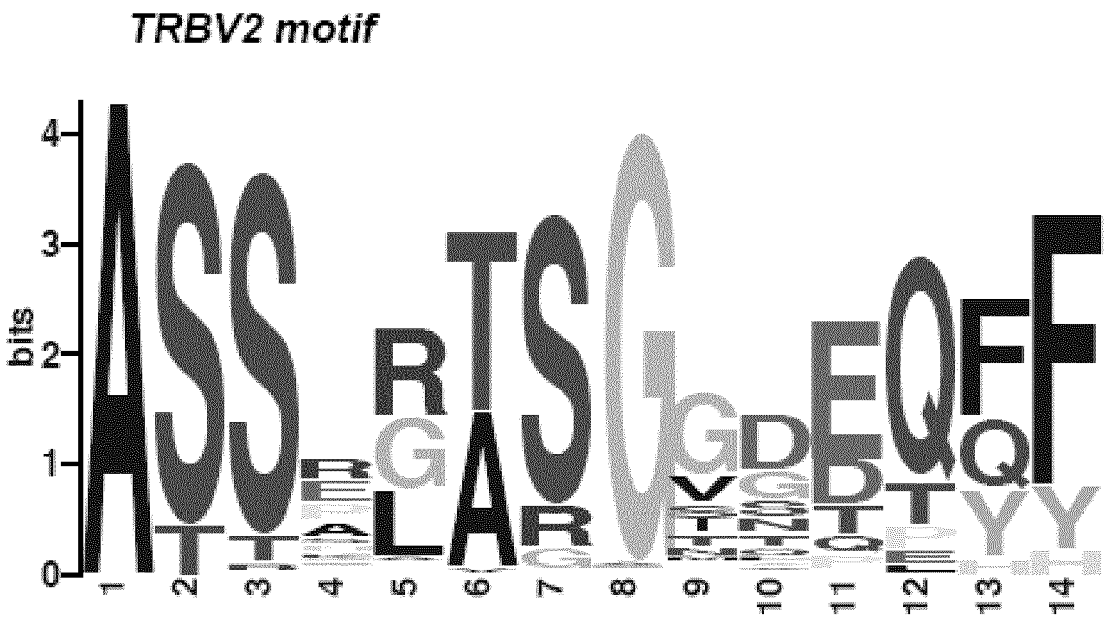
TRBV2 motif
bits
0
1
2
3
4
1
2
3
4
5
6
7
8
9
10
11
12
13
14

Fig. 3B

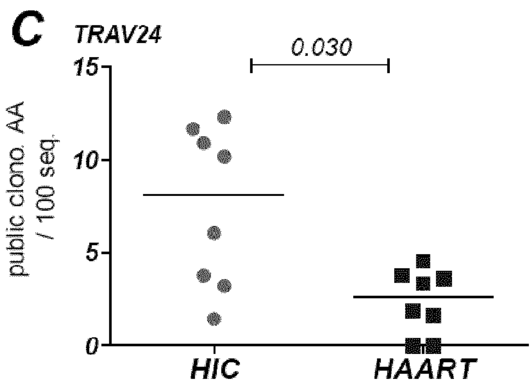


Fig. 3C

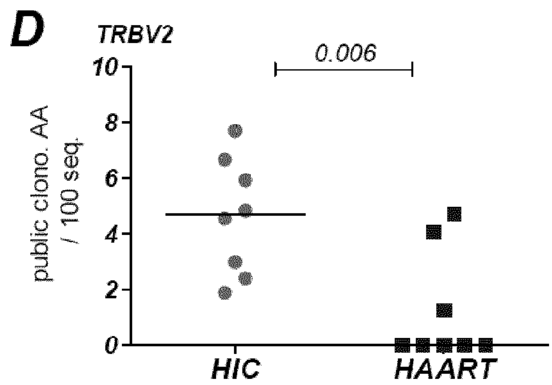


Fig. 3D

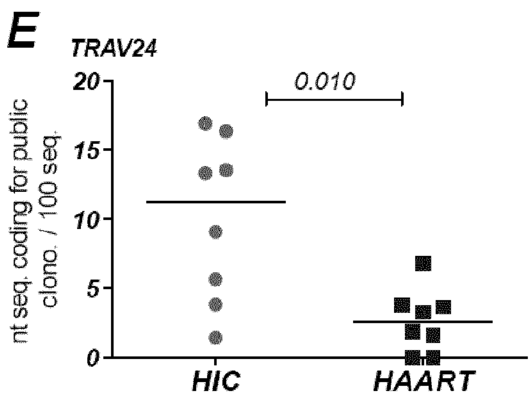


Fig. 3E

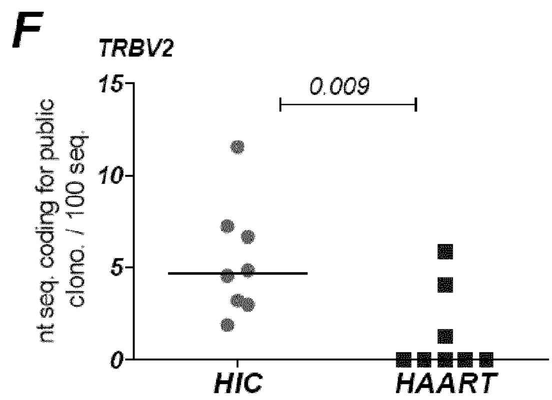


Fig. 3F

**G**

| Seq. | V name | 3'V-REGION | P | N | 5'J-REGION | J name | N | trim | %AFKAAGNKLT |
|---|---|---|---|---|---|---|---|---|---|
| 1 | TRAV24*01 | tgtgcctttta | | | ......aagctgcaggcaacaagctaacttt | TRAJ17*01 | 0 | 6 | 64.9 % |
| 2 | TRAV24*01 | tgtgcctttta | | ag | ........gctgcaggcaacaagctaacttt | TRAJ17*01 | 2 | 8 | 0.9 % |
| 3 | TRAV24*01 | tgtgcctt.. | | | ....caaagctgcaggcaacaagctaacttt | TRAJ17*01 | 0 | 6 | 33.8 % |
| 4 | TRAV24*01 | tgtgc..... | | attt | .....aaagctgcaggcaacaagctaacttt | TRAJ17*01 | 4 | 10 | 0.4 % |

Fig. 3G

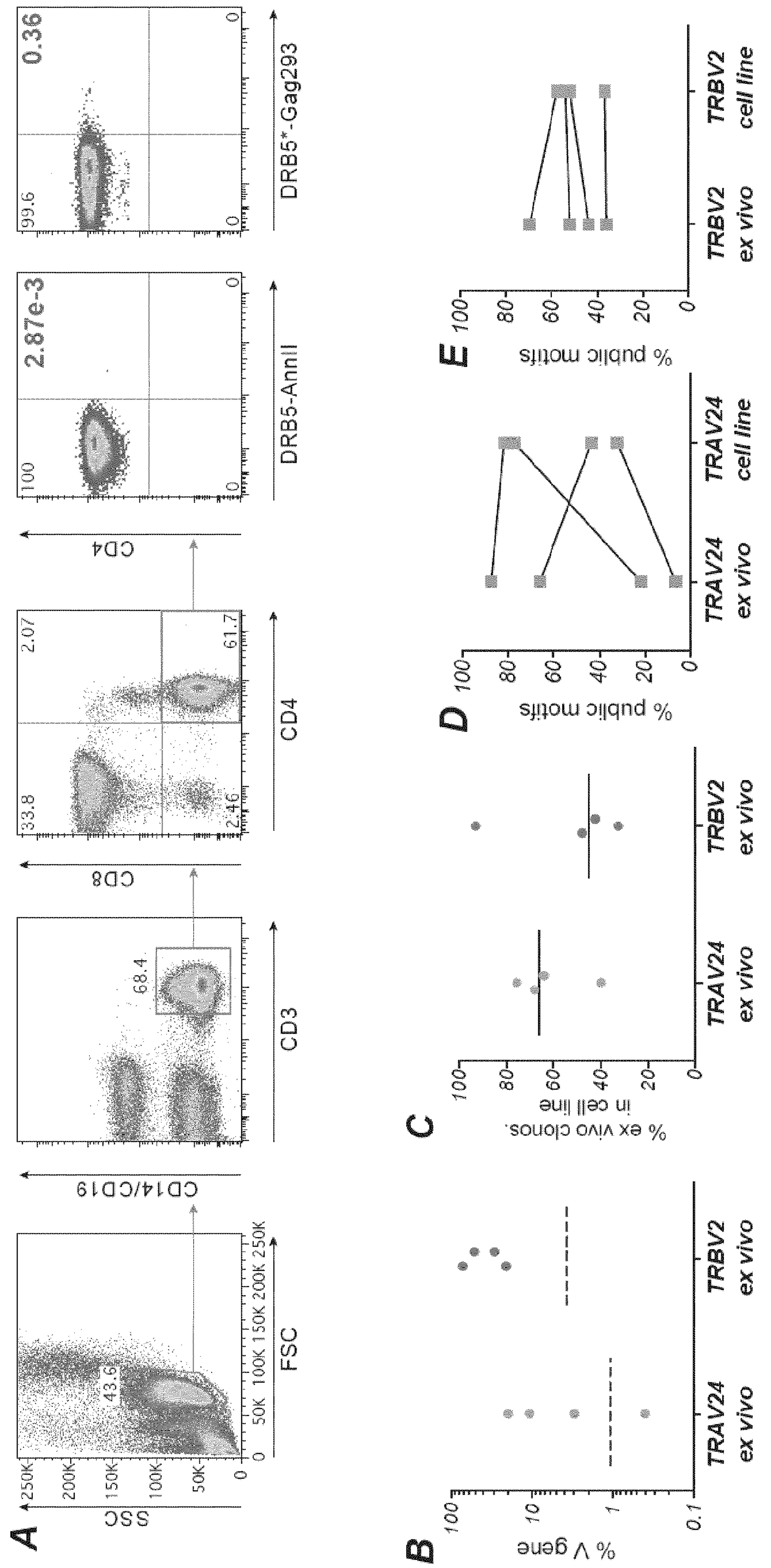


Fig. 4A, 4B, 4C, 4D, 4E

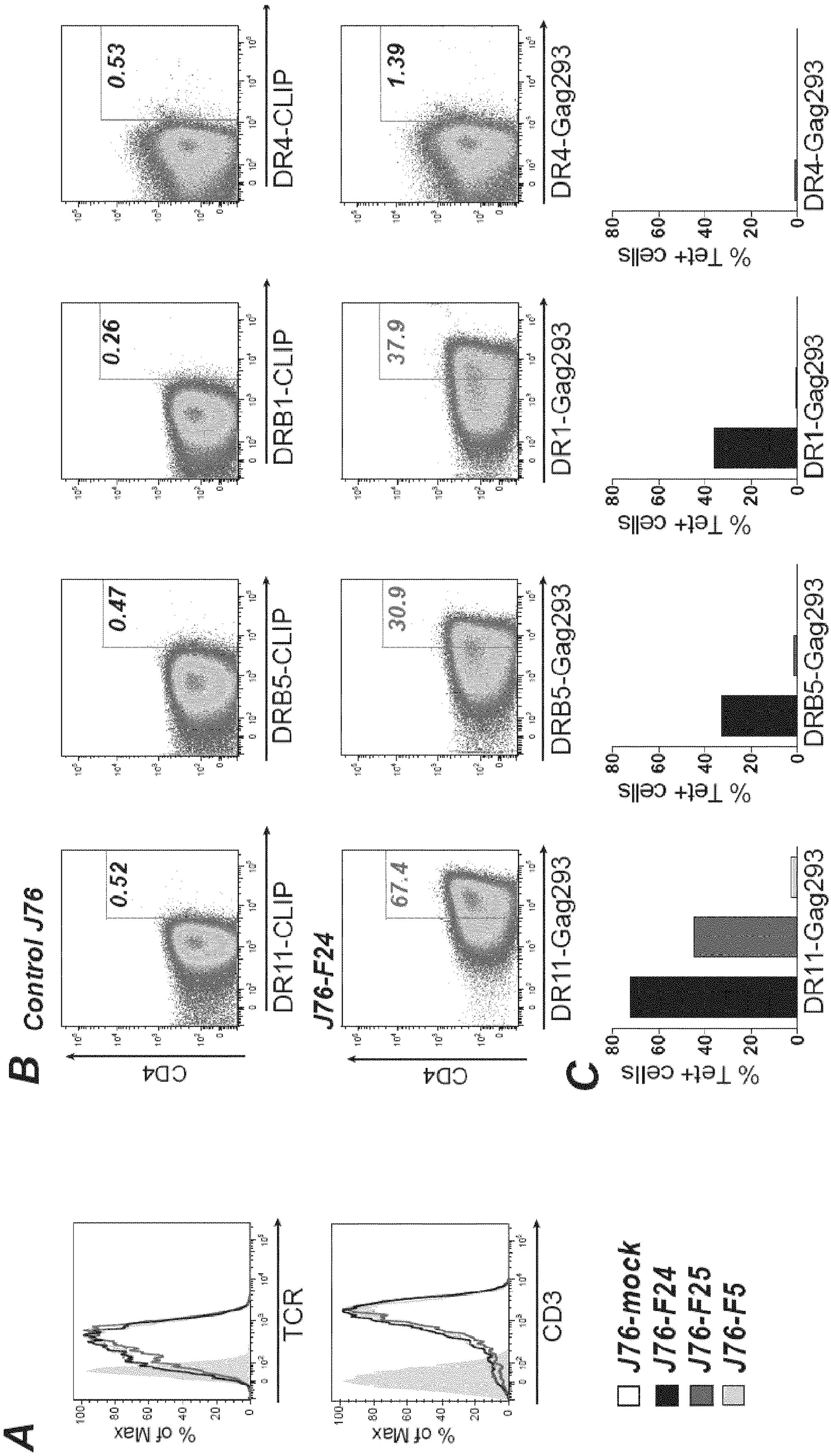
A
% of Max
TCR
CD3
J76-mock
J76-F24
J76-F25
J76-F5
B
Control J76
J76-F24
CD4
0.52
0.47
0.26
0.53
67.4
30.9
37.9
1.39
DR11-CLIP
DRB5-CLIP
DRB1-CLIP
DR4-CLIP
DR11-Gag293
DRB5-Gag293
DR1-Gag293
DR4-Gag293
C
% Tet+ cells

Fig. 5A, 5B, 5C

**D**

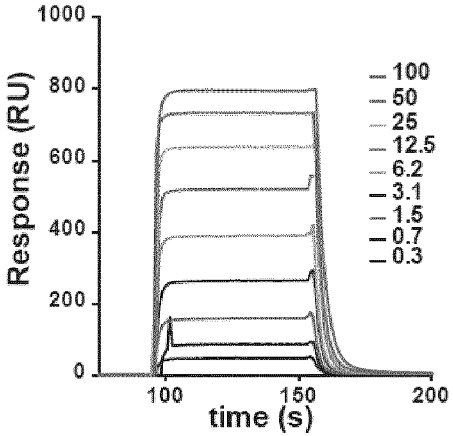


**F**

| TCRs | DR11 Kd eq (µM) | DRB5 Kd eq (µM) | DR1 Kd eq (µM) |
|---|---|---|---|
| F24 | 0.86 ± 0.15 | 2.58 ± 0.19 | 6.97 ± 0.22 |
| F25 | 3.58 ± 0.25 | 16.00 ± 0.60 | 51.50± 3.00 |
| F5 | 11.06 ± 1.73 | 74.45 ± 6.25 | > 100 |

**G**

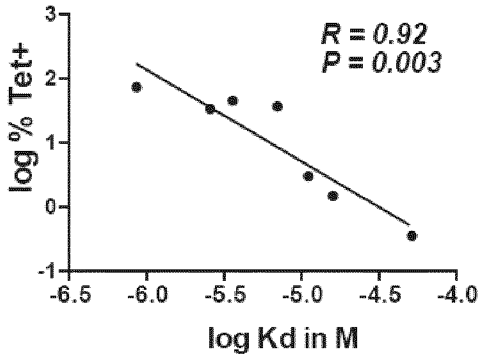


**E**

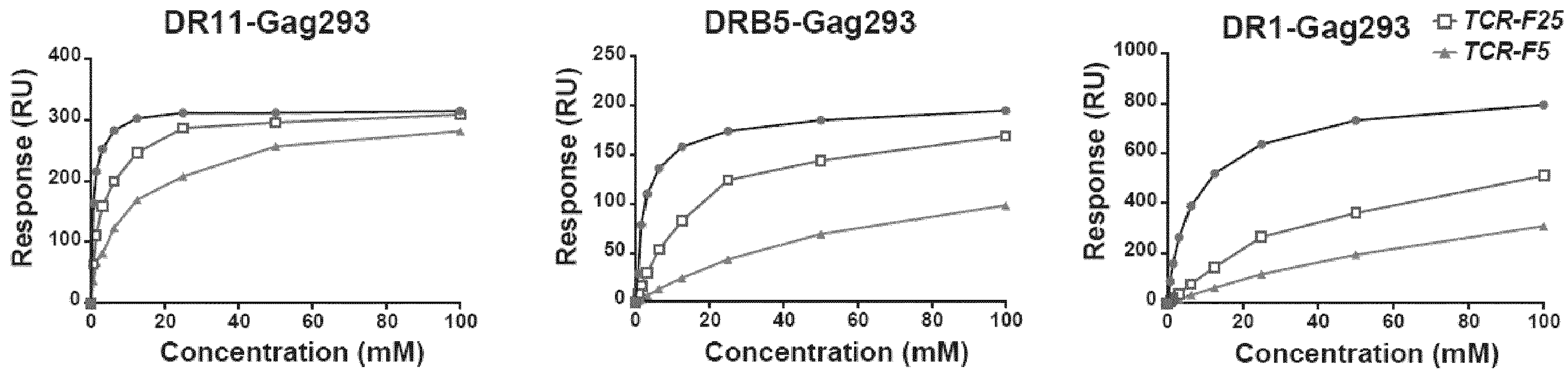


Fig. 5D, 5E, 5F, 5G

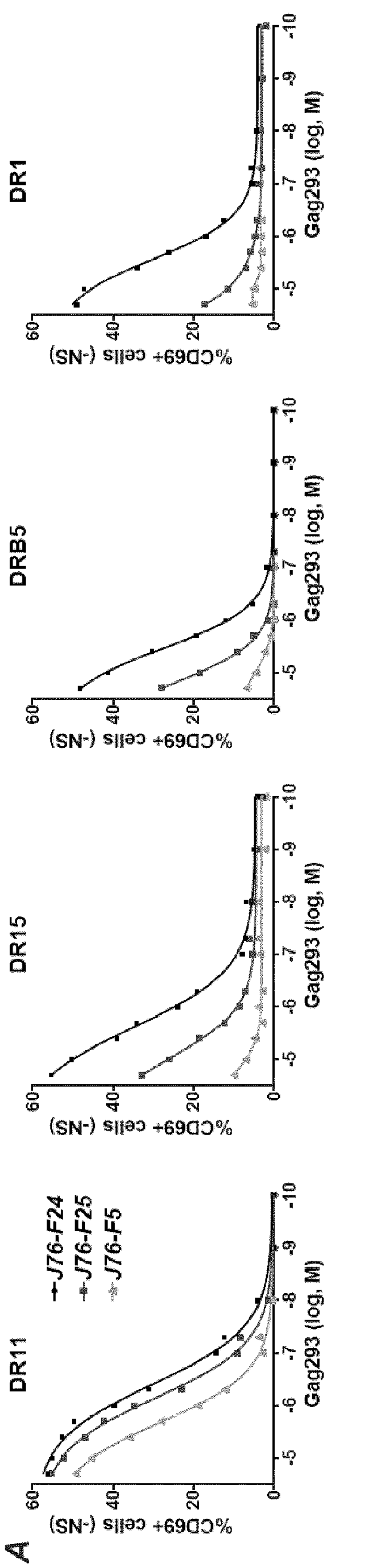
A
DR11
DR15
DRB5
DR1
J76-F24
J76-F25
J76-F5
%CD69+ cells (-NS)
Gag293 (log, M)

Fig. 6A

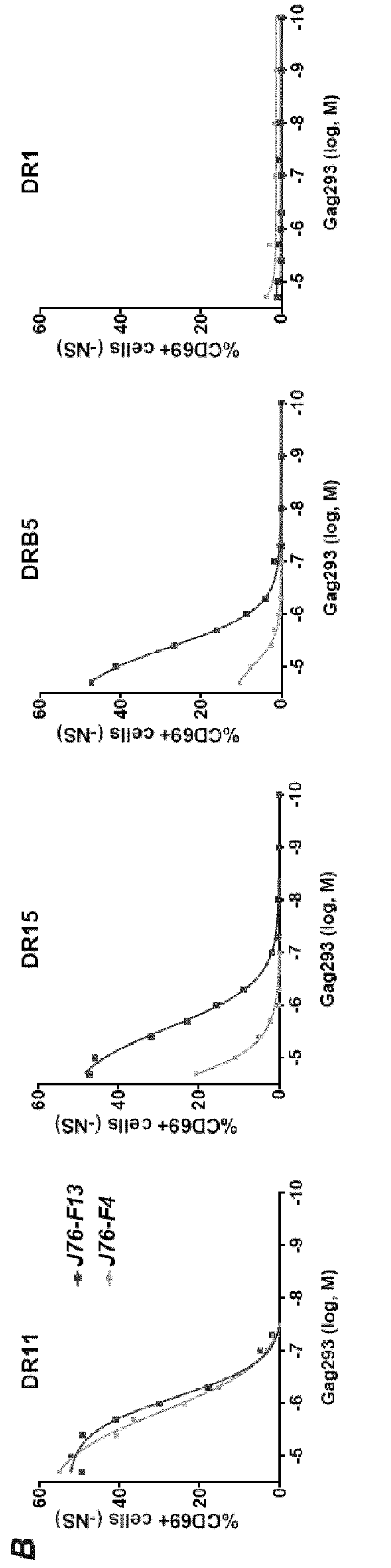
B
DR11
DR15
DRB5
DR1
J76-F13
J76-F4
%CD69+ cells (-NS)
Gag293 (log, M)

Fig. 6B

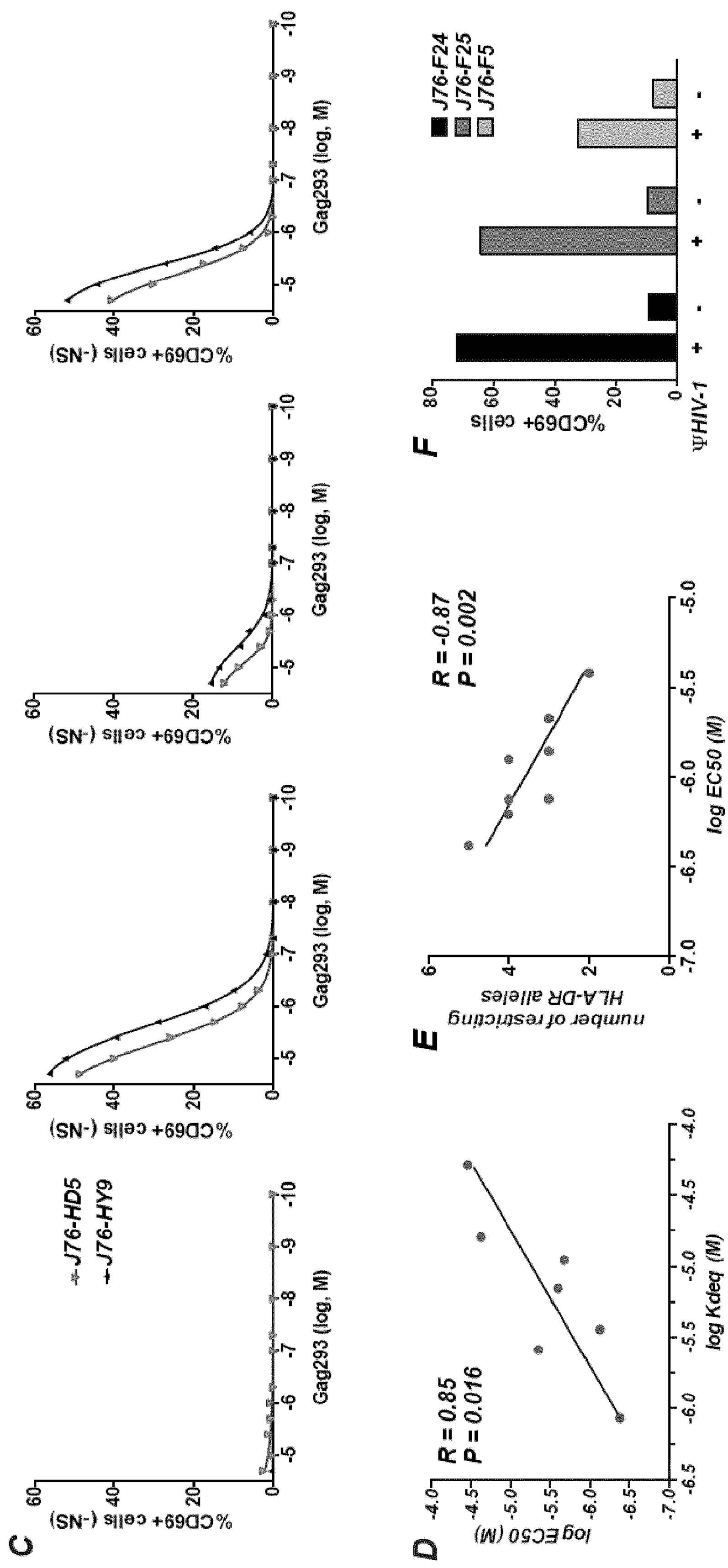


Fig. 6C, 6D, 6E, 6F

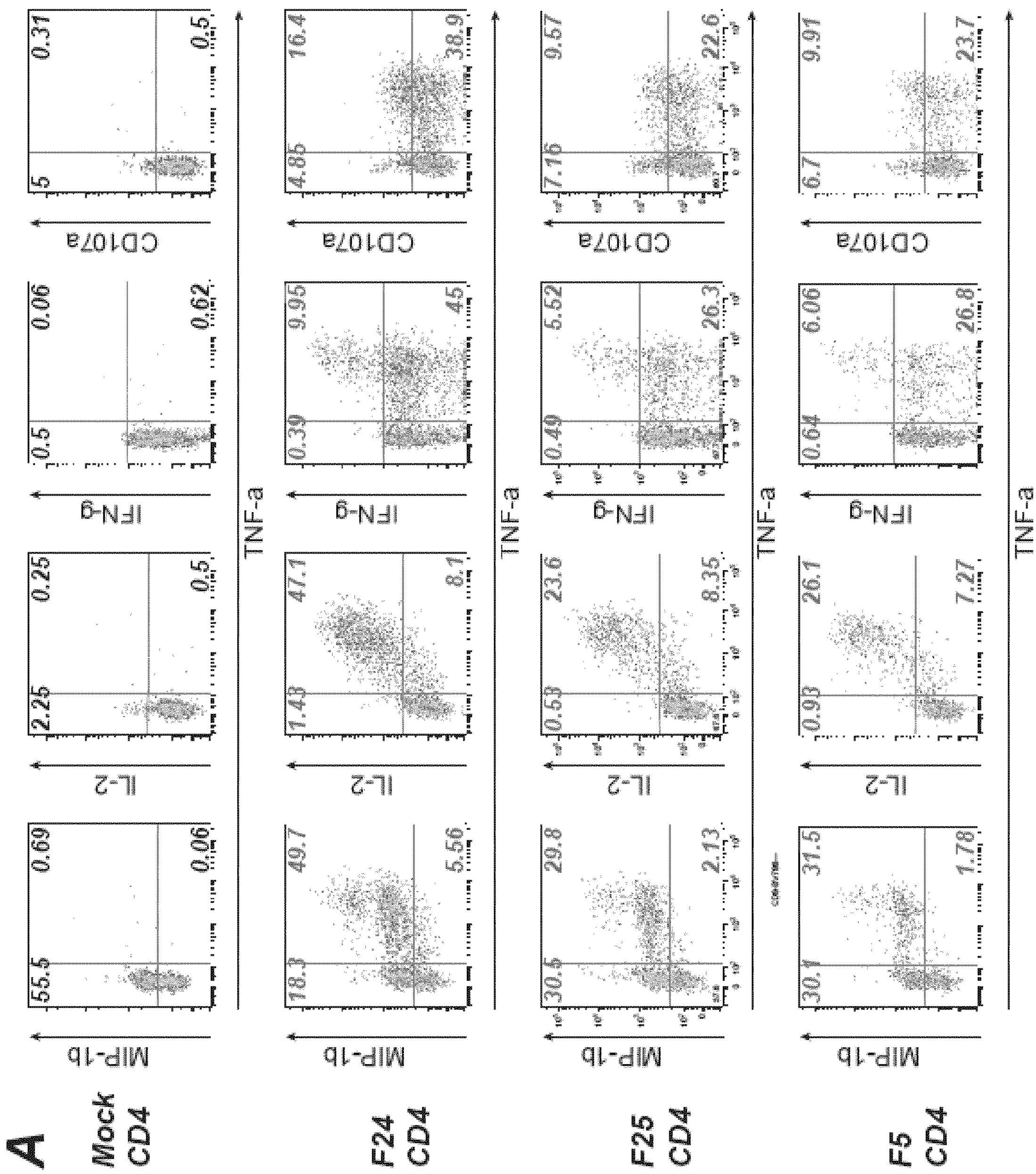


Fig. 7A

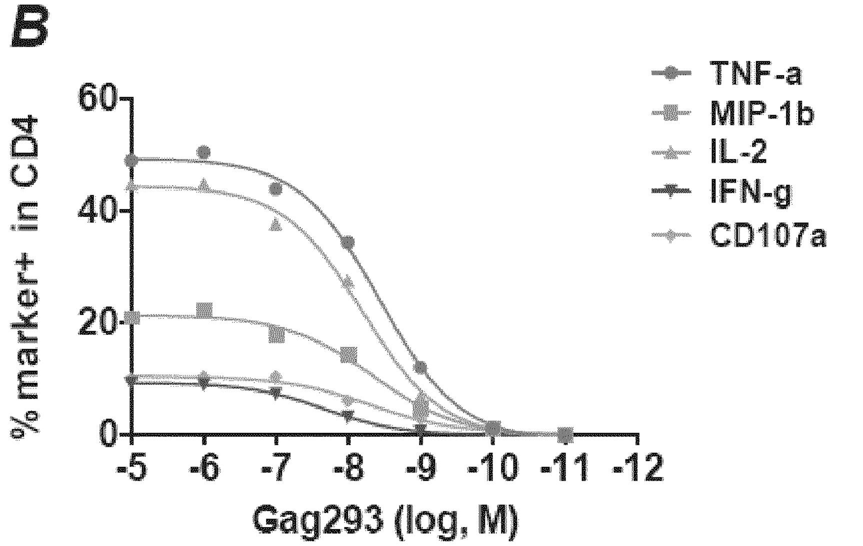
B
TNF-a
MIP-1b
IL-2
IFN-g
CD107a
60
40
20
0
% marker+ in CD4
-5
-6
-7
-8
-9
-10
-11
-12
Gag293 (log, M)

Fig. 7B

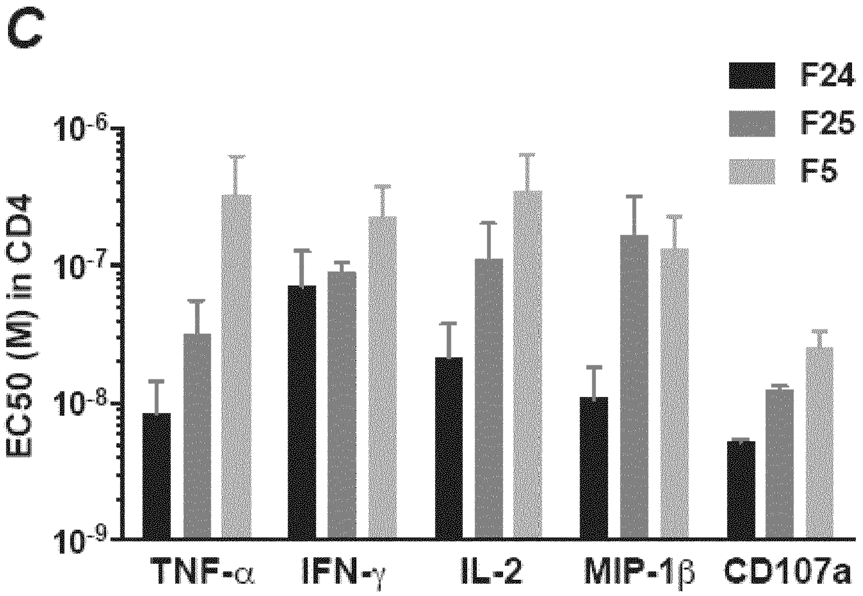
C
F24
F25
F5
$10^{-6}$
$10^{-7}$
$10^{-8}$
$10^{-9}$
EC50 (M) in CD4
TNF-α
IFN-γ
IL-2
MIP-1β
CD107a

Fig. 7C

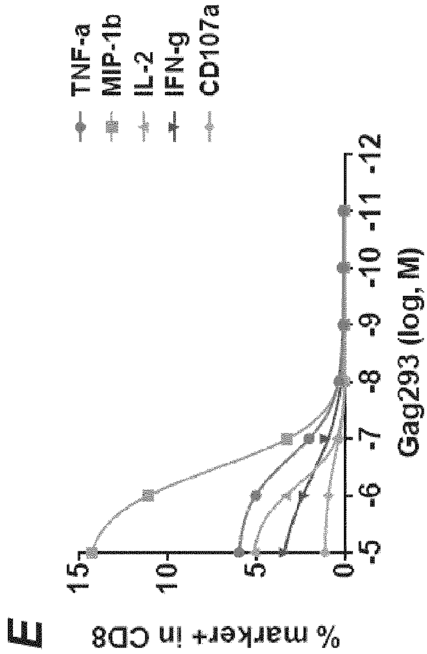
E
% marker+ in CD8
15
10
5
0
-5 -6 -7 -8 -9 -10 -11 -12
Gag293 (log, M)
TNF-a
MIP-1b
IL-2
IFN-g
CD107a

Fig. 7E

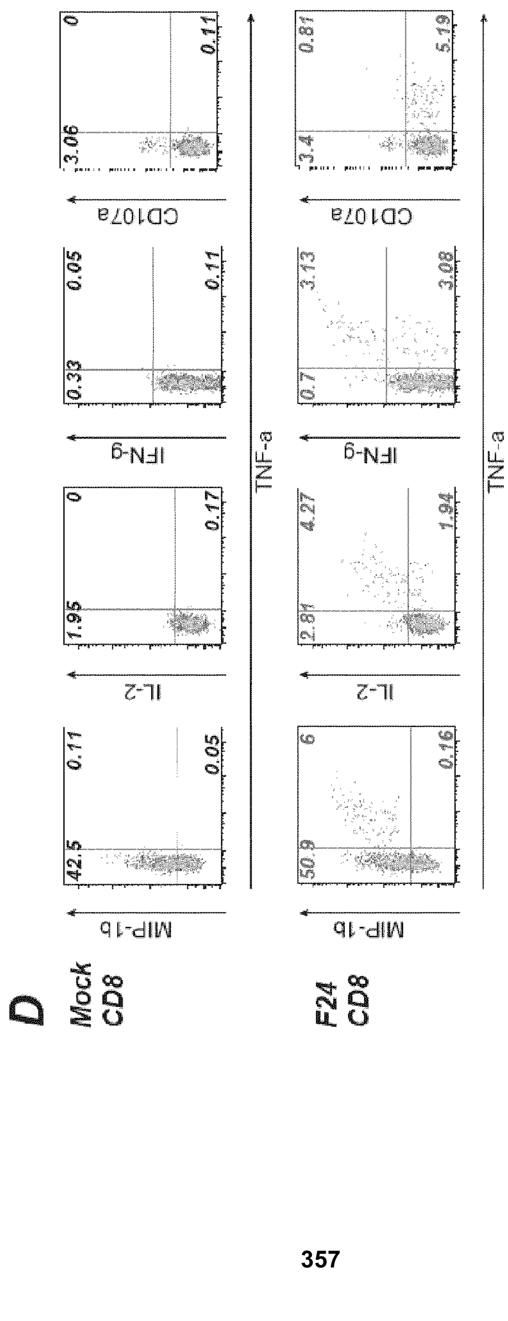
D
Mock CD8
F24 CD8
MIP-1b
IL-2
IFN-g
CD107a
TNF-a
42.5
0.11
0.05
1.95
0
0.17
0.33
0.05
0.11
3.06
0
0.11
50.9
6
0.16
2.81
4.27
1.94
0.7
3.13
3.08
3.4
0.81
5.19

Fig. 7D

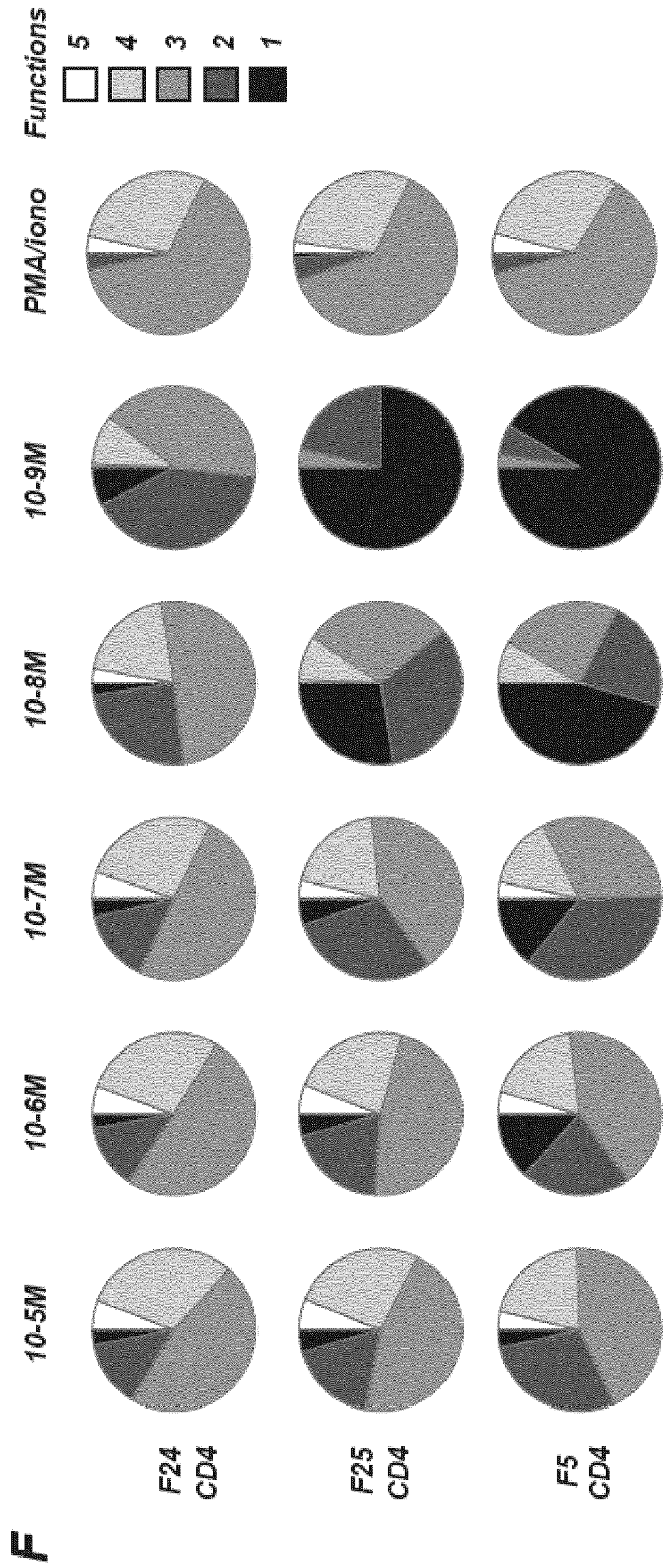
F
10-5M
10-6M
10-7M
10-8M
10-9M
PMA/iono
Functions
5
4
3
2
1
F24 CD4
F25 CD4
F5 CD4

Fig. 7F

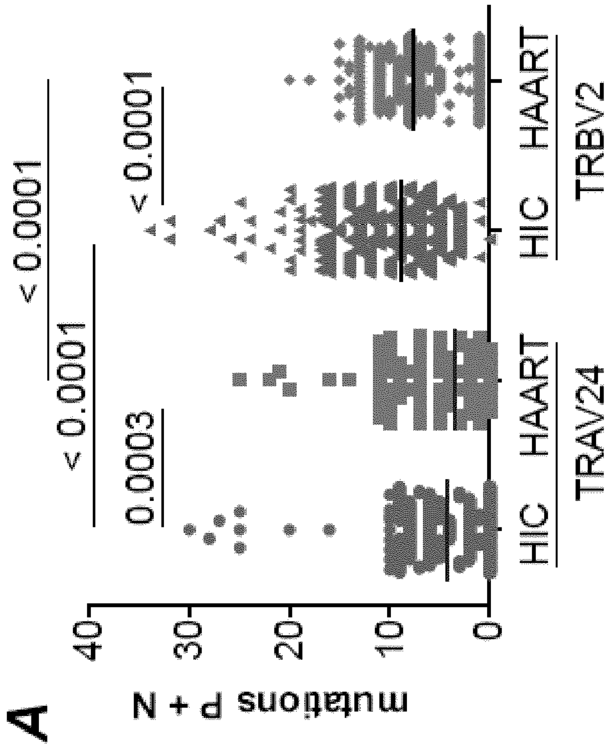
A
mutations P + N
40
30
20
10
0
0.0003
< 0.0001
< 0.0001
< 0.0001
HIC
HAART
TRAV24
HIC
HAART
TRBV2

Fig. 8A

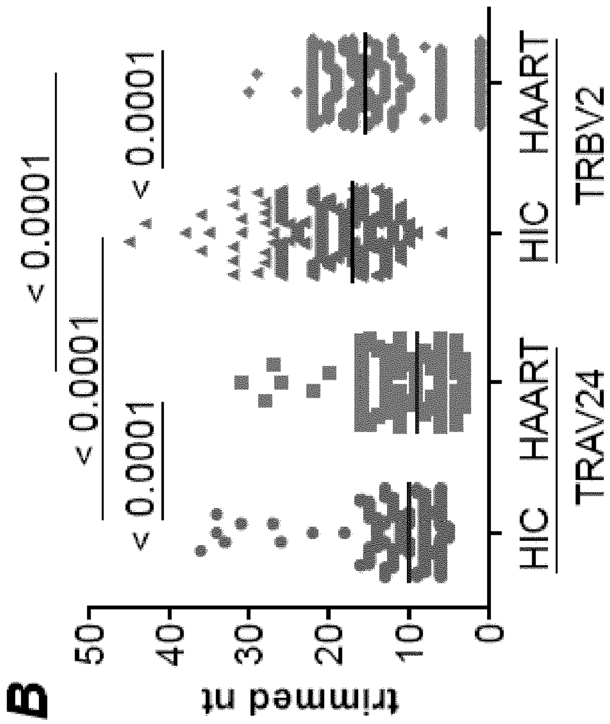
B
trimmed nt
50
40
30
20
10
0
< 0.0001
< 0.0001
< 0.0001
< 0.0001
HIC
HAART
TRAV24
HIC
HAART
TRBV2

Fig. 8B

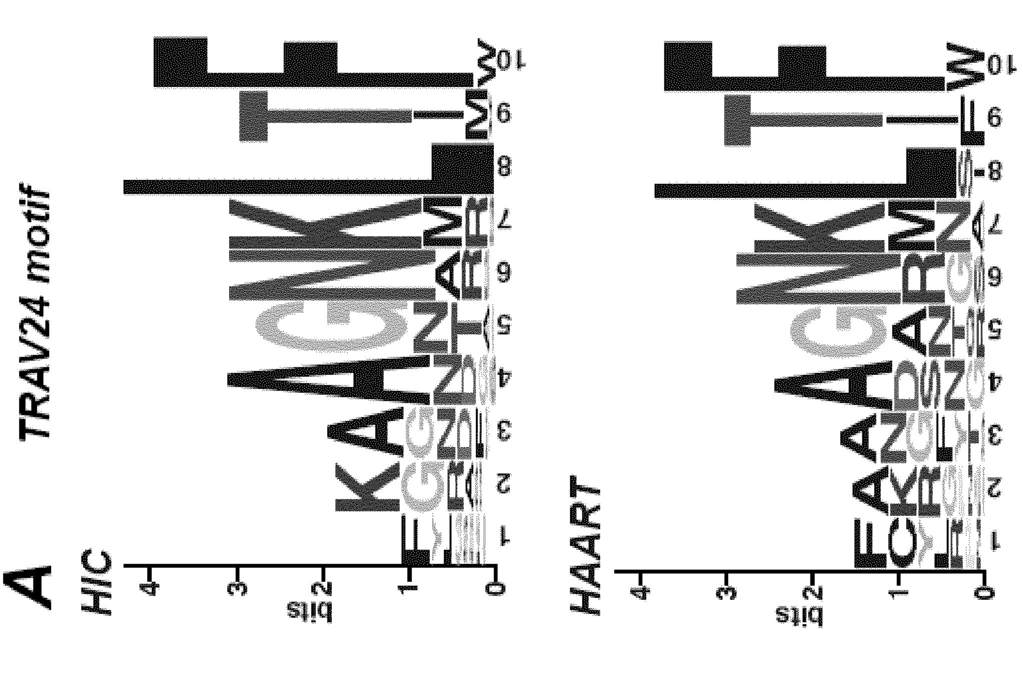


Fig. 9A

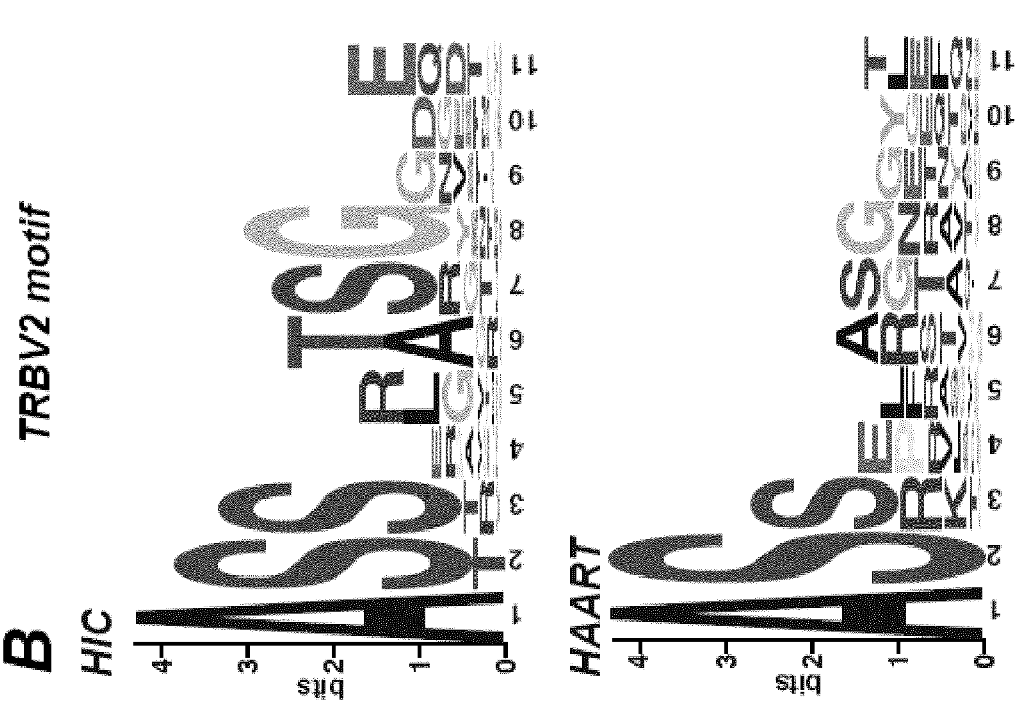


Fig. 9B

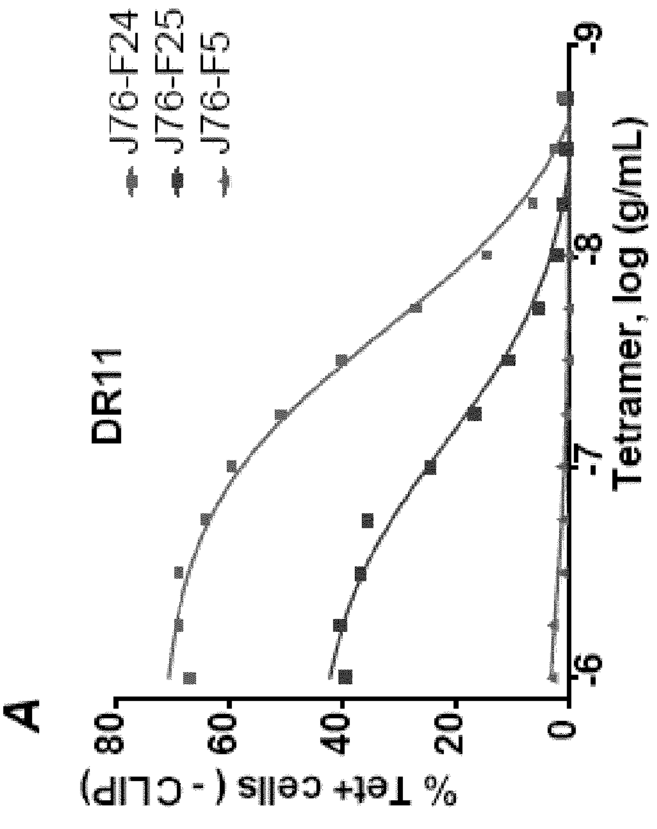


Fig. 10A

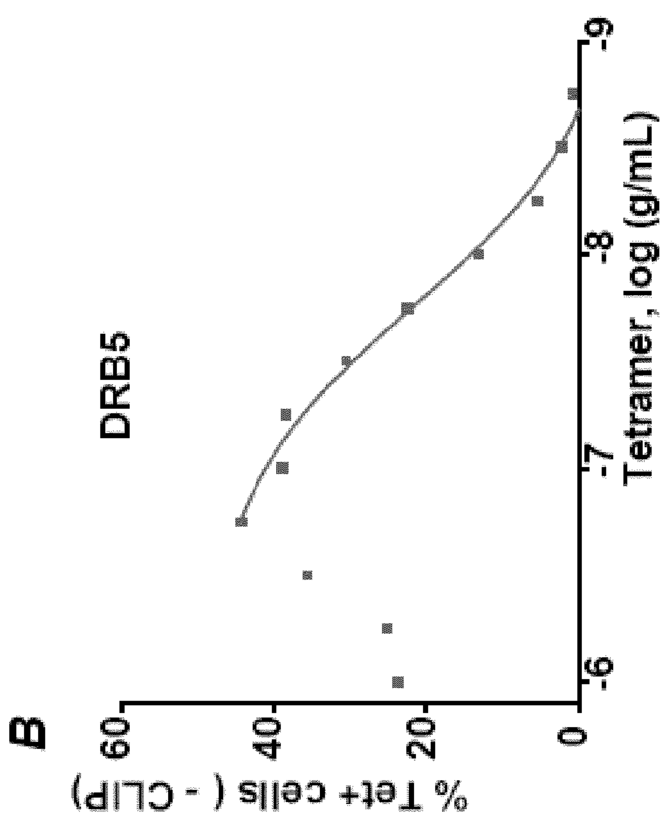


Fig. 10B

C

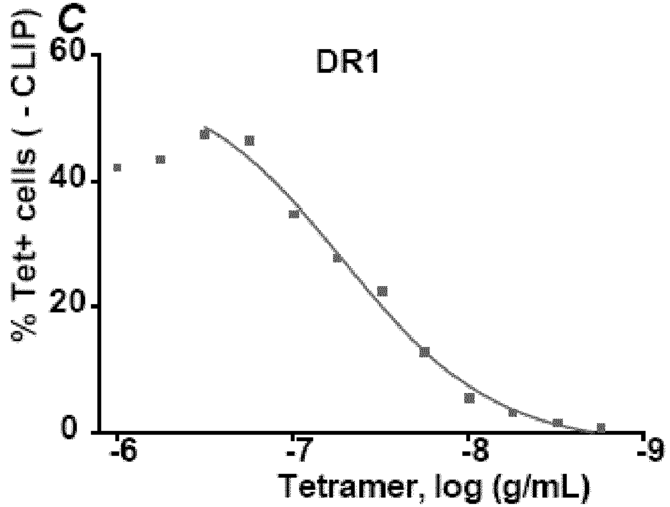


D

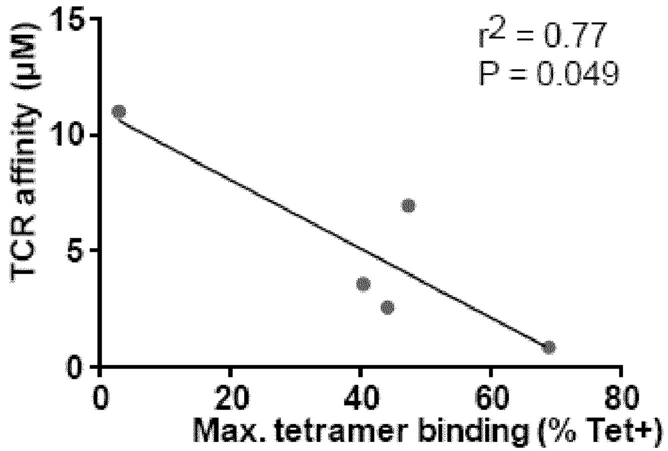


E

| TCRs | DR11 EC50 (g/mL) | DRB5 EC50 (g/mL) | DR1 EC50 (g/mL) |
|---|---|---|---|
| F24 | 2.24E-08 | 1.73E-08 | 5.17E-08 |
| F25 | 7.58E-08 | ND | ND |
| F5 | ND | ND | ND |

Fig. 10C, 10D, 10E

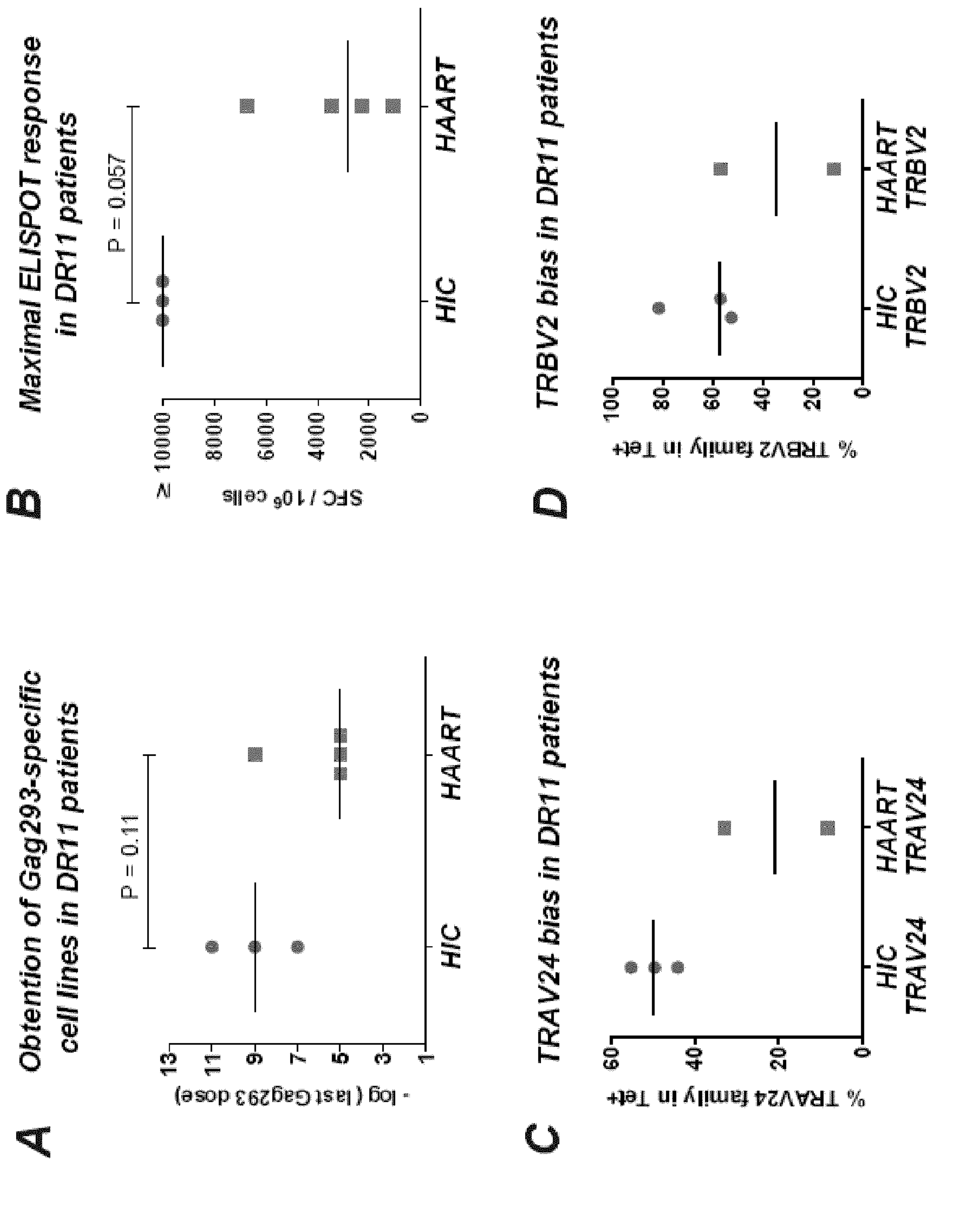


Fig.11A, 11B, 11C, 11D

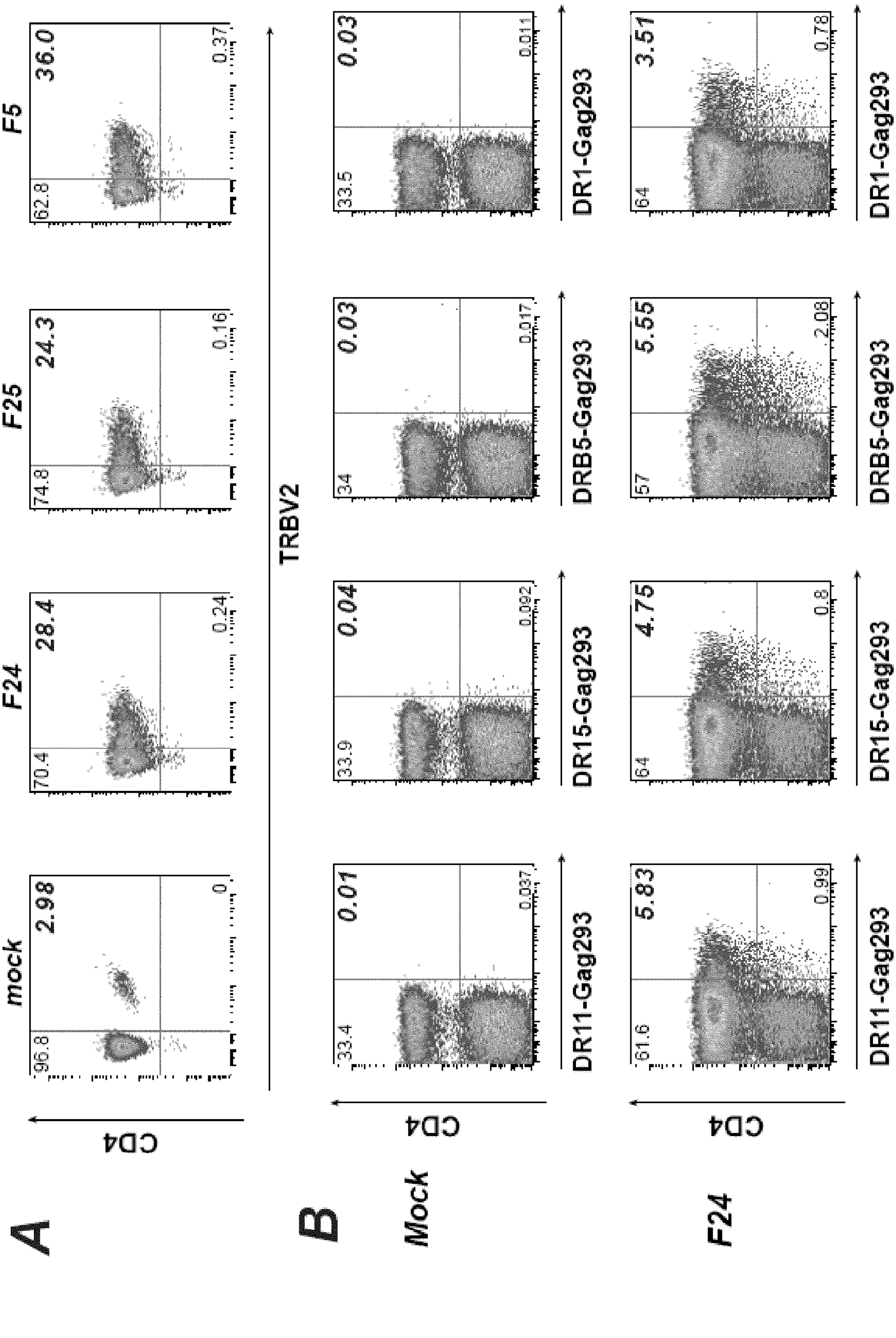
A
B
mock
F24
F25
F5
2.98
28.4
24.3
36.0
96.8
70.4
74.8
62.8
0
0.24
0.16
0.37
CD4
TRBV2
Mock
F24
0.01
0.04
0.03
0.03
33.4
33.9
34
33.5
0.037
0.092
0.017
0.011
5.83
4.75
5.55
3.51
61.6
64
57
64
0.99
0.8
2.08
0.78
DR11-Gag293
DR15-Gag293
DRB5-Gag293
DR1-Gag293

Fig. 12A, 12B

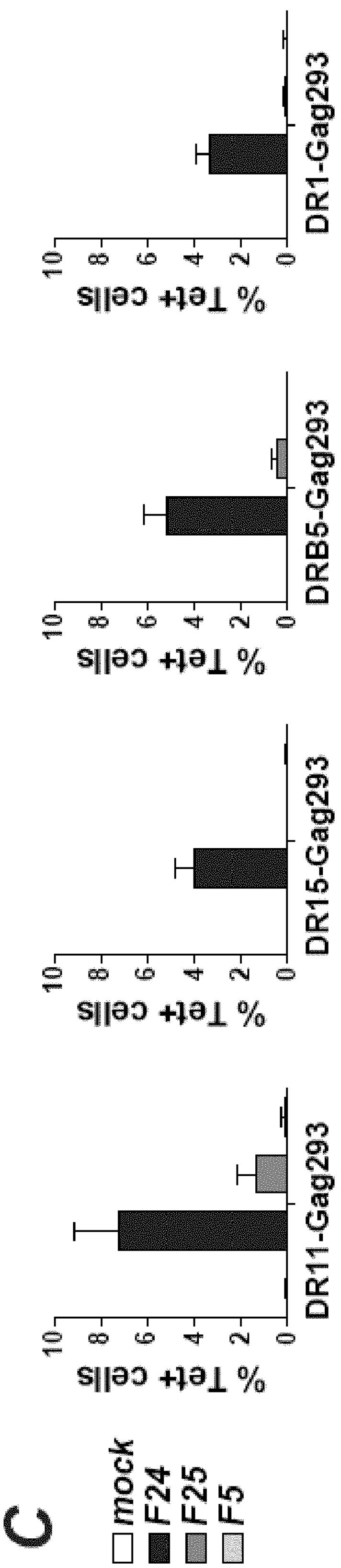
C
mock
F24
F25
F5
% Tet+ cells
10
8
6
4
2
0
DR11-Gag293
DR15-Gag293
DRB5-Gag293
DR1-Gag293

Fig. 12C

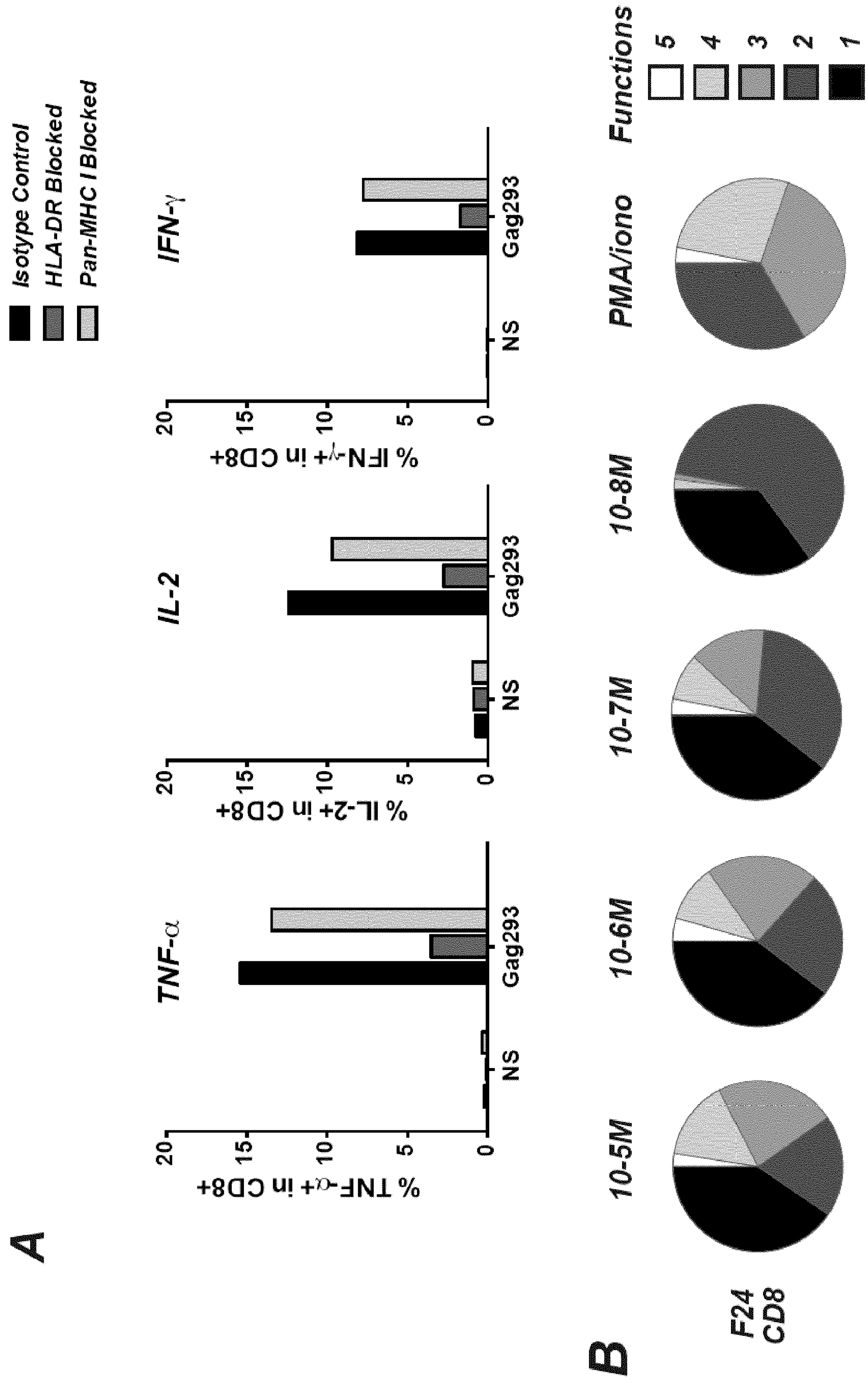
A
Isotype Control
HLA-DR Blocked
Pan-MHC I Blocked
TNF-α
IL-2
IFN-γ
% TNF-α+ in CD8+
% IL-2+ in CD8+
% IFN-γ+ in CD8+
20
15
10
5
0
NS
Gag293
B
F24
CD8
10-5M
10-6M
10-7M
10-8M
PMA/iono
Functions
5
4
3
2
1

Fig. 13A, 13B

Europäisches Patentamt
European Patent Office
Office européen des brevets

# EUROPEAN SEARCH REPORT

Application Number
EP 16 30 5218

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | BENOÎT VINGERT ET AL: "HIV Controller CD4+ T Cells Respond to Minimal Amounts of Gag Antigen Due to High TCR Avidity", PLOS PATHOGENS, vol. 6, no. 2, 26 February 2010 (2010-02-26), page e1000780, XP055268224, DOI: 10.1371/journal.ppat.1000780 * the whole document * -& BENOÎT VINGERT ET AL: "Supplementary Data: HIV Controller CD4+ T Cells Respond to Minimal Amounts of Gag Antigen Due to High TCR Avidity", PLOS PATHOGENS, vol. 6, no. 2, 26 February 2010 (2010-02-26), page e1000780, XP055268969, DOI: 10.1371/journal.ppat.1000780 * the whole document * -& BENOÎT VINGERT ET AL: "Supplementary Data: HIV Controller CD4+ T Cells Respond to Minimal Amounts of Gag Antigen Due to High TCR Avidity", PLOS PATHOGENS, vol. 6, no. 2, 26 February 2010 (2010-02-26), page e1000780, XP055268969, DOI: 10.1371/journal.ppat.1000780 * the whole document * ----- | 1-23 | INV. C07K14/725 A61K48/00 A61K35/17 C07K16/10 C07K14/16 C12N5/0783 C12N15/867 A61P31/18 |
| | -/-- | | TECHNICAL FIELDS SEARCHED (IPC) C07K A61K C12N |

The present search report has been drawn up for all claims

1

| Place of search | Date of completion of the search | Examiner |
|---|---|---|
| Munich | 27 April 2016 | Madruga, Jaime |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 4

Europäisches Patentamt
European Patent Office
Office européen des brevets

# EUROPEAN SEARCH REPORT

Application Number
EP 16 30 5218

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | B. VINGERT ET AL: "HIV Controllers Maintain a Population of Highly Efficient Th1 Effector Cells in Contrast to Patients Treated in the Long Term", JOURNAL OF VIROLOGY., vol. 86, no. 19, 25 July 2012 (2012-07-25), pages 10661-10674, XP055268230, US ISSN: 0022-538X, DOI: 10.1128/JVI.00056-12 * the whole document * | 1-23 | |
| X,D | HUABIAO CHEN ET AL: "TCR clonotypes modulate the protective effect of HLA class I molecules in HIV-1 infection", NATURE IMMUNOLOGY, vol. 13, no. 7, 10 June 2012 (2012-06-10), pages 691-700, XP055268624, GB ISSN: 1529-2908, DOI: 10.1038/ni.2342 | 10,11, 14-19, 22,23 | |
| Y | * the whole document * -& HUABIAO CHEN ET AL: "Supplementary data: TCR clonotypes modulate the protective effect of HLA class I molecules in HIV-1 infection", NATURE IMMUNOLOGY, vol. 13, no. 7, 10 June 2012 (2012-06-10), pages 691-700, XP055268800, GB ISSN: 1529-2908, DOI: 10.1038/ni.2342 * the whole document * | 1-15 | TECHNICAL FIELDS SEARCHED (IPC) |

-/--

1

The present search report has been drawn up for all claims

| Place of search | Date of completion of the search | Examiner |
|---|---|---|
| Munich | 27 April 2016 | Madruga, Jaime |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 30 5218

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | D. MENDOZA ET AL: "HLA B*5701-Positive Long-Term Nonprogressors/Elite Controllers Are Not Distinguished from Progressors by the Clonal Composition of HIV-Specific CD8+ T Cells", JOURNAL OF VIROLOGY., vol. 86, no. 7, 25 January 2012 (2012-01-25), pages 4014-4018, XP055268643, US ISSN: 0022-538X, DOI: 10.1128/JVI.06982-11 | 11, 16-19, 22,23 | |
| Y | * the whole document * | 1-15 | |
| X | WO 2007/117588 A2 (GEN HOSPITAL CORP [US]; LICHTERFELD MATHIAS [US]; YU XU G [US]; ALTFEL) 18 October 2007 (2007-10-18) | 10-12, 14-19, 22,23 | |
| Y | * the whole document * * claims; tables 1,2; sequences 47, 52 * | 1-15 | |
| Y | WO 2008/038002 A2 (MEDIGENE LTD [GB]; BENNETT ALAN DAVID [GB]; JAKOBSEN BENT KARSTEN [GB]) 3 April 2008 (2008-04-03) * the whole document * * claims; examples; sequences 12,13 * | 1-15 | TECHNICAL FIELDS SEARCHED (IPC) |
| Y | WO 2006/103429 A2 (AVIDEX LTD [GB]; JAKOBSEN BENT KARSTEN [GB]; LI YI [GB]; DUNN STEVEN M) 5 October 2006 (2006-10-05) * the whole document * * claims; sequence 7 * | 16-23 | |
| Y | US 9 228 007 B1 (KITCHEN SCOTT G [US] ET AL) 5 January 2016 (2016-01-05) * the whole document * * claims; sequence 28 * | 16-23 | |

-/--

The present search report has been drawn up for all claims

1

| Place of search | Date of completion of the search | Examiner |
|---|---|---|
| Munich | 27 April 2016 | Madruga, Jaime |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 30 5218

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MICHAEL S BENNETT ET AL: "Fine-tuning of T-cell receptor avidity to increase HIV epitope variant recognition by cytotoxic T lymphocytes", AIDS, vol. 24, no. 17, 13 November 2010 (2010-11-13), pages 2619-2628, XP055268250, GB ISSN: 0269-9370, DOI: 10.1097/QAD.0b013e32833f7b22 * the whole document * ----- | 16-23 | |

TECHNICAL FIELDS SEARCHED (IPC)

The present search report has been drawn up for all claims

1

| Place of search | Date of completion of the search | Examiner |
|---|---|---|
| Munich | 27 April 2016 | Madruga, Jaime |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 16 30 5218

This annex lists the patent family members relating to the patent documents cited in the above-mentioned European search report. The members are as contained in the European Patent Office EDP file on
The European Patent Office is in no way liable for these particulars which are merely given for the purpose of information.

27-04-2016

| Patent document cited in search report | Publication date | Patent family member(s) | Publication date |
|---|---|---|---|
| WO 2007117588 A2 | 18-10-2007 | AU 2007235320 A1 | 18-10-2007 |
| | | CA 2648403 A1 | 18-10-2007 |
| | | CN 101490078 A | 22-07-2009 |
| | | EP 2007911 A2 | 31-12-2008 |
| | | JP 2009536922 A | 22-10-2009 |
| | | KR 20090015034 A | 11-02-2009 |
| | | US 2008015139 A1 | 17-01-2008 |
| | | WO 2007117588 A2 | 18-10-2007 |
| WO 2008038002 A2 | 03-04-2008 | EP 2087000 A2 | 12-08-2009 |
| | | US 2010166722 A1 | 01-07-2010 |
| | | WO 2008038002 A2 | 03-04-2008 |
| WO 2006103429 A2 | 05-10-2006 | AU 2006228308 A1 | 05-10-2006 |
| | | CA 2602463 A1 | 05-10-2006 |
| | | CN 103059128 A | 24-04-2013 |
| | | CN 105461802 A | 06-04-2016 |
| | | EP 1863842 A2 | 12-12-2007 |
| | | EP 2275441 A1 | 19-01-2011 |
| | | EP 2301964 A1 | 30-03-2011 |
| | | EP 2985291 A1 | 17-02-2016 |
| | | JP 5612623 B2 | 22-10-2014 |
| | | JP 5885220 B2 | 15-03-2016 |
| | | JP 2008535826 A | 04-09-2008 |
| | | JP 2012144543 A | 02-08-2012 |
| | | JP 2014205671 A | 30-10-2014 |
| | | NZ 561338 A | 30-04-2010 |
| | | NZ 584523 A | 26-11-2010 |
| | | US 2009214551 A1 | 27-08-2009 |
| | | US 2013295063 A1 | 07-11-2013 |
| | | WO 2006103429 A2 | 05-10-2006 |
| US 9228007 B1 | 05-01-2016 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0127300 A
- WO 0127304 A
- WO 9955892 A
- WO 2009019612 A


### Non-patent literature cited in the description


- **GIUDICELLI V ; LEFRANC MP.** IMGT-ONTOLOGY 2012. *Front Genet.,* 23 May 2012, vol. 3, 79
- **LEFRANC MP.** IMGT unique numbering for the variable (V), constant (C), and groove (G) domains of IG, TR, MH, IgSF, and MhSF. Cold Spring Harb Protoc, 01 June 2011, 633-42
- **ZENNOU et al.** *Cell,* 2000, vol. 101, 173-185
- **DEEKS SG et al.** Systemic effects of inflammation on health during chronic HIV infection. *Immunity,* 2013, vol. 39 (4), 633-45
- **CONNORS M et al.** HIV infection induces changes in CD4+ T-cell phenotype and depletions within the CD4+ T-cell repertoire that are not immediately restored by antiviral or immune-based therapies. *Nat Med.,* 1997, vol. 3 (5), 533-40
- **GOROCHOV G et al.** Perturbation of CD4+ and CD8+ T-cell repertoires during progression to AIDS and regulation of the CD4+ repertoire during antiviral therapy. *Nat Med.,* 1998, vol. 4 (2), 215-21
- **ROSENBERG ES et al.** Vigorous HIV-1-specific CD4+ T cell responses associated with control of viremia. *Science,* 1997, vol. 278 (5342), 1447-50
- **DOUEK DC et al.** HIV preferentially infects HIV-specific CD4+ T cells. *Nature,* 2002, vol. 417 (6884), 95-8
- **LUBONG SABADO R et al.** In vitro priming recapitulates in vivo HIV-1 specific T cell responses, revealing rapid loss of virus reactive CD4 T cells in acute HIV-1 infection. *PLoS One.,* 2009, vol. 4 (1), e4256
- **LAMBOTTE O et al.** HIV controllers: a homogeneous group of HIV-1-infected patients with spontaneous control of viral replication. *Clin Infect Dis.,* 2005, vol. 41 (7), 1053-6
- **CHAKRABARTI LA ; SIMON V.** Immune mechanisms of HIV control. *Curr Opin Immunol.,* 2010, vol. 22 (4), 488-96
- **SAEZ-CIRION A et al.** HIV controllers exhibit potent CD8 T cell capacity to suppress HIV infection ex vivo and peculiar CTL activation phenotype. *Proc Natl Acad Sci U S A.,* 2007, vol. 104 (16), 6776-81
- **ALMEIDA JR et al.** Antigen sensitivity is a major determinant of CD8+ T-cell polyfunctionality and HIV-suppressive activity. *Blood,* 2009, vol. 113 (25), 6351-60
- **LADELL K et al.** A molecular basis for the control of preimmune escape variants by HIV-specific CD8+ T cells. *Immunity,* 2013, vol. 38 (3), 425-36
- **CHEN H et al.** TCR clonotypes modulate the protective effect of HLA class I molecules in HIV-1 infection. *Nat Immunol.,* 2012, vol. 13 (7), 691-700
- **MIGUELES SA et al.** Lytic granule loading of CD8+ T cells is required for HIV-infected cell elimination associated with immune control. *Immunity,* 2008, vol. 29 (6), 1009-21
- **ALMEIDA JR et al.** Superior control of HIV-1 replication by CD8+ T cells is reflected by their avidity, polyfunctionality, and clonal turnover. *J Exp Med.,* 2007, vol. 204 (10), 2473-85
- **IGLESIAS MC et al.** Escape from highly effective public CD8+ T-cell clonotypes by HIV. *Blood,* 2011, vol. 118 (8), 2138-49
- **YOUNES SA et al.** HIV-1 viremia prevents the establishment of interleukin 2-producing HIV-specific memory CD4+ T cells endowed with proliferative capacity. *J Exp Med.,* 2003, vol. 198 (12), 1909-22
- **HARARI A et al.** Functional heterogeneity of memory CD4 T cell responses in different conditions of antigen exposure and persistence. *J Immunol.,* 2005, vol. 174 (2), 1037-45
- **VAN GREVENYNGHE J et al.** Transcription factor FOXO3a controls the persistence of memory CD4(+) T cells during HIV infection. *Nat Med.,* 2008, vol. 14 (3), 266-74
- **POTTER SJ et al.** Preserved central memory and activated effector memory CD4+ T-cell subsets in human immunodeficiency virus controllers: an ANRS EP36 study. *J Virol.,* 2007, vol. 81 (24), 13904-15
- **KAUFMANN DE et al.** Limited durability of viral control following treated acute HIV infection. *PLoS Med.,* 2004, vol. 1 (2), e36

- **SAEZ-CIRION A et al.** Restriction of HIV-1 replication in macrophages and CD4+ T cells from HIV controllers. *Blood,* 2011, vol. 118 (4), 955-64
- **RANASINGHE S et al.** HIV-Specific CD4 T Cell Responses to Different Viral Proteins Have Discordant Associations with Viral Load and Clinical Outcome. *J Virol.,* 2012, vol. 86 (1), 277-83
- **FERRE AL et al.** HIV controllers with HLA-DRB1*13 and HLA-DQB1*06 alleles have strong, polyfunctional mucosal CD4+ T-cell responses. *J Virol.,* 2010, vol. 84 (21), 11020-9
- **VINGERT B et al.** HIV controllers maintain a population of highly efficient Th1 effector cells in contrast to patients treated in the long term. *J Virol.,* 2012, vol. 86 (19), 10661-74
- **KAUFMANN DE et al.** Upregulation of CTLA-4 by HIV-specific CD4+ T cells correlates with disease progression and defines a reversible immune dysfunction. *Nat Immunol.,* 2007, vol. 8 (11), 1246-54
- **PORICHIS F et al.** Responsiveness of HIV-specific CD4 T cells to PD-1 blockade. *Blood,* 2011
- **VINGERT B et al.** HIV controller CD4+ T cells respond to minimal amounts of Gag antigen due to high TCR avidity. *PLoS Pathog.,* 2010, vol. 6 (2), e1000780
- **ALEXANDER-MILLER MA.** High-avidity CD8+ T cells: optimal soldiers in the war against viruses and tumors. *Immunol Res.,* 2005, vol. 31 (1), 13-24
- **BERGER CT et al.** High functional avidity CTL responses to HLA-B-restricted Gag-derived epitopes associate with relative HIV control. *J Virol.,* 2011
- **KAUFMANN DE et al.** Comprehensive analysis of human immunodeficiency virus type 1-specific CD4 responses reveals marked immunodominance of gag and nef and the presence of broadly recognized peptides. *J Virol.,* 2004, vol. 78 (9), 4463-77
- **WOOLDRIDGE L et al.** Tricks with tetramers: how to get the most from multimeric peptide-MHC. *Immunology,* 2009, vol. 126 (2), 147-64
- **LIM A et al.** Frequent contribution of T cell clonotypes with public TCR features to the chronic response against a dominant EBV-derived epitope: application to direct detection of their molecular imprint on the human peripheral T cell repertoire. *J Immunol.,* 2000, vol. 165 (4), 2001-11
- **HACEIN-BEY-ABINA S et al.** Efficacy of gene therapy for X-linked severe combined immunodeficiency. *N Engl J Med.,* 2010, vol. 363 (4), 355-64
- **ALAMYAR E et al.** IMGT((R)) tools for the nucleotide analysis of immunoglobulin (IG) and T cell receptor (TR) V-(D)-J repertoires, polymorphisms, and IG mutations: IMGT/V-QUEST and IMGT/HighV-QUEST for NGS. *Methods Mol Biol.,* 2012, vol. 882, 569-604
- **TURNER SJ et al.** Structural determinants of T-cell receptor bias in immunity. *Nat Rev Immunol.,* 2006, vol. 6 (12), 883-94
- **VENTURI V et al.** A mechanism for TCR sharing between T cell subsets and individuals revealed by pyrosequencing. *J Immunol.,* 2011, vol. 186 (7), 4285-94
- **GRAS S et al.** T-cell receptor bias and immunity. *Curr Opin Immunol.,* 2008, vol. 20 (1), 119-25
- **ROSSJOHN J et al.** T cell antigen receptor recognition of antigen-presenting molecules. *Annu Rev Immunol.,* 2015, vol. 33, 169-200
- **BETTS MR et al.** HIV nonprogressors preferentially maintain highly functional HIV-specific CD8+ T cells. *Blood,* 2006, vol. 107 (12), 4781-9
- **LIU P et al.** Characterization of human alphabetaTCR repertoire and discovery of D-D fusion in TCRbeta chains. *Protein Cell,* 2014, vol. 5 (8), 603-15
- **GILLESPIE GM et al.** Strong TCR conservation and altered T cell cross-reactivity characterize a B*57-restricted immune response in HIV-1 infection. *J Immunol.,* 2006, vol. 177 (6), 3893-902
- **KLOVERPRIS HN et al.** CD8+ TCR Bias and Immunodominance in HIV-1 Infection. *J Immunol.,* 2015
- **MILES JJ et al.** Bias in the alphabeta T-cell repertoire: implications for disease pathogenesis and vaccination. *Immunol Cell Biol.,* 2011, vol. 89 (3), 375-87
- **MENDOZA D et al.** HLA B*5701-positive long-term nonprogressors/elite controllers are not distinguished from progressors by the clonal composition of HIV-specific CD8+ T cells. *J Virol.,* 2012, vol. 86 (7), 4014-8
- **SIMONS BC et al.** Despite biased TRBV gene usage against a dominant HLA B57-restricted epitope, TCR diversity can provide recognition of circulating epitope variants. *J Immunol.,* 2008, vol. 181 (7), 5137-46
- **PRICE DA et al.** Public clonotype usage identifies protective Gag-specific CD8+ T cell responses in SIV infection. *J Exp Med.,* 2009, vol. 206 (4), 923-36
- **WARREN RL et al.** Exhaustive T-cell repertoire sequencing of human peripheral blood samples reveals signatures of antigen selection and a directly measured repertoire size of at least 1 million clonotypes. *Genome Res.,* 2011, vol. 21 (5), 790-7
- **MATTAPALLIL JJ et al.** Massive infection and loss of memory CD4+ T cells in multiple tissues during acute SIV infection. *Nature,* 2005, vol. 434 (7037), 1093-7
- **GODFREY DI et al.** The burgeoning family of unconventional T cells. *Nat Immunol.,* 2015, vol. 16 (11), 1114-23

- **CAMPION SL et al.** Proteome-wide analysis of HIV-specific naive and memory CD4(+) T cells in unexposed blood donors. *J Exp Med.,* 2014, vol. 211 (7), 1273-80
- **JONES RB et al.** Human immunodeficiency virus type 1 escapes from interleukin-2-producing CD4+ T-cell responses without high-frequency fixation of mutations. *J Virol.,* 2009, vol. 83 (17), 8722-32
- **RANASINGHE S et al.** Association of HLA-DRB1-restricted CD4 T cell responses with HIV immune control. *Nature Medicine,* 2013, vol. 19 (7), 930-3
- **YOUSEF S et al.** TCR bias and HLA cross-restriction are strategies of human brain-infiltrating JC virus-specific CD4+ T cells during viral infection. *J Immunol.,* 2012, vol. 189 (7), 3618-30
- **MALHOTRA U et al.** Role for HLA class II molecules in HIV-1 suppression and cellular immunity following antiretroviral treatment. *J Clin Invest.,* 2001, vol. 107 (4), 505-17
- **WILLIAMS MA et al.** Rapid culling of the CD4+ T cell repertoire in the transition from effector to memory. *Immunity,* 2008, vol. 28 (4), 533-45
- **BELYAKOV IM et al.** Impact of vaccine-induced mucosal high-avidity CD8+ CTLs in delay of AIDS viral dissemination from mucosa. *Blood,* 2006, vol. 107 (8), 3258-64
- **CORSE E et al.** Attenuated T cell responses to a high-potency ligand in vivo. *PLoS Biol.,* 2010, vol. 8 (9
- **VALITUTTI S.** The Serial Engagement Model 17 Years After: From TCR Triggering to Immunotherapy. *Front Immunol.,* 2012, vol. 3, 272
- **THORBOM G et al.** Clonotypic composition of the CD4+ T cell response to a vectored retroviral antigen is determined by its speed. *J Immunol.,* 2014, vol. 193 (4), 1567-77
- **SWAIN SL et al.** Expanding roles for CD4(+) T cells in immunity to viruses. *Nat Rev Immunol.,* 2012, vol. 12 (2), 136-48
- **BLANCO-MELO D et al.** Intrinsic cellular defenses against human immunodeficiency viruses. *Immunity,* 2012, vol. 37 (3), 399-411
- **VETTER ML et al.** Differences in APOBEC3G expression in CD4+ T helper lymphocyte subtypes modulate HIV-1 infectivity. *PLoS Pathog.,* 2009, vol. 5 (2), e1000292
- **OSWALD-RICHTER K et al.** Identification of a CCR5-expressing T cell subset that is resistant to R5-tropic HIV infection. *PLoS Pathog.,* 2007, vol. 3 (4), e58
- **REINHERZ EL ; WANG JH.** Codification of bidentate pMHC interaction with TCR and its co-receptor. *Trends Immunol.,* 2015
- **RIHN SJ et al.** Extreme genetic fragility of the HIV-1 capsid. *PLoS Pathog.,* 2013, vol. 9 (6), e1003461
- **HANSEN SG et al.** Cytomegalovirus vectors violate CD8+ T cell epitope recognition paradigms. *Science,* 2013, vol. 340 (6135), 1237874
- **GHORASHIAN S et al.** CD8 T cell tolerance to a tumor-associated self-antigen is reversed by CD4 T cells engineered to express the same T cell receptor. *J Immunol.,* 2015, vol. 194 (3), 1080-9
- **GRAS S et al.** The shaping of T cell receptor recognition by self-tolerance. *Immunity,* 2009, vol. 30 (2), 193-203
- **HEEMSKERK MH et al.** Redirection of antileukemic reactivity of peripheral T lymphocytes using gene transfer of minor histocompatibility antigen HA-2-specific T-cell receptor complexes expressing a conserved alpha joining region. *Blood,* 2003, vol. 102 (10), 3530-40
- **KLOHE EP et al.** Analysis of the molecular specificities of anti-class II monoclonal antibodies by using L cell transfectants expressing HLA class II molecules. *J Immunol.,* 1988, vol. 141 (6), 2158-64
- **SETH N et al.** Expansion and contraction of HIV-specific CD4 T cells with short bursts of viremia, but physical loss of the majority of these cells with sustained viral replication. *J Immunol.,* 2005, vol. 175 (10), 6948-58
- **COLWELL RK.** *EstimateS: Statistical estimation of species richness and shared species from samples,* 2013, http://purloclcorg/estimates
- **BAILEY TL et al.** The MEME Suite. *Nucleic Acids Res.,* 2015
- **STOTHARD P.** The sequence manipulation suite: JavaScript programs for analyzing and formatting protein and DNA sequences. *Biotechniques,* 2000, vol. 28 (6), 1102, , 4
- **NEGRE D et al.** Characterization of novel safe lentiviral vectors derived from simian immunodeficiency virus (SIVmac251) that efficiently transduce mature human dendritic cells. *Gene Ther.,* 2000, vol. 7 (19), 1613-23
- **ROEDERER M et al.** SPICE: exploration and analysis of post-cytometric complex multivariate datasets. *Cytometry A.,* 2011, vol. 79 (2), 167-74

SEQUENCE LISTING

<110> INSTITUT PASTEUR

<120> T CELL RECEPTORS FROM THE HIV-SPECIFIC REPERTOIRE, MEANS FOR THEIR PRODUCTION AND THERAPEUTIC USES THEREOF

<130> B11676 AD/AFL/KN

<140> EPXXXXXXXX.X
<141> 2016-02-24

<160> 1274

<170> PatentIn version 3.5

<210> 1
<211> 20
<212> PRT
<213> Artificial sequence

<220>
<223> Consensus sequence epitope Gag 293

<220>
<221> MISC_FEATURE
<222> (1)..(20)
<223> Gag 293 Consensus

<220>
<221> MISC_FEATURE
<222> (9)..(9)
<223> Xaa can be Tyr or Phe

<220>
<221> MISC_FEATURE
<222> (18)..(18)
<223> Xaa can be Ser or Thr

<400> 1

Phe Arg Asp Tyr Val Asp Arg Phe Xaa Lys Thr Leu Arg Ala Glu Gln
1               5                   10                  15

Ala Xaa Gln Glu
            20

<210> 2
<211> 10
<212> PRT
<213> Artificial sequence

<220>
<223> Motif Trav24 AV24-1

<220>
<221> MISC_FEATURE
<222> (1)..(10)
<223> Motif AV24-1

<220>
<221> MISC_FEATURE
<222> (1)..(1)
<223> Xaa can be Ala or Ser

<220>
<221> MISC_FEATURE
<222> (2)..(2)
<223> Xaa can be any amino-acid

<220>
<221> MISC_FEATURE
<222> (3)..(3)
<223> Xaa can be Lys or Arg

<400> 2

Xaa Xaa Xaa Ala Ala Gly Asn Lys Leu Thr
1 5 10

<210> 3
<211> 11
<212> PRT
<213> Artificial sequence

<220>
<223> Motif TRAV24-2

<220>
<221> MISC_FEATURE
<222> (1)..(11)
<223> Motif TRAV24-2

<220>
<221> MISC_FEATURE
<222> (2)..(2)
<223> Xaa can be any amino-acid

<400> 3

Ala Xaa Tyr Gly Gly Ala Thr Asn Lys Leu Ile
1 5 10

<210> 4
<211> 11
<212> PRT
<213> Artificial sequence

<220>
<223> Motif TRAV24-3

<220>
<221> MISC_FEATURE
<222> (1)..(11)
<223> Motif TRAV24-3

<220>
<221> MISC_FEATURE

<222> (2)..(2)
<223> Xaa can be any amino-acid

<220>
<221> MISC_FEATURE
<222> (3)..(3)
<223> Xaa can be Arg or Asn

<220>
<221> MISC_FEATURE
<222> (4)..(4)
<223> Xaa can be Arg or Asn

<400> 4

Ala Xaa Xaa Xaa Ala Gly Asn Met Leu Thr Phe
1 5 10

<210> 5
<211> 8
<212> PRT
<213> Artificial sequence

<220>
<223> Motif TRAV24-4

<220>
<221> MISC_FEATURE
<222> (1)..(8)
<223> Motif TRAV24-4

<220>
<221> MISC_FEATURE
<222> (2)..(2)
<223> Xaa can be any amino-acid

<220>
<221> MISC_FEATURE
<222> (4)..(4)
<223> Xaa can be Asn or Asp

<400> 5

Ala Xaa Asp Xaa Arg Lys Leu Ile
1 5

<210> 6
<211> 11
<212> PRT
<213> Artificial sequence

<220>
<223> Motif TRAV24-5

<220>
<221> MISC_FEATURE
<222> (1)..(11)
<223> Motif TRAV24-5

<220>
<221> MISC_FEATURE
<222> (2)..(2)
<223> Xaa can be any amino-acid

<220>
<221> MISC_FEATURE
<222> (4)..(4)
<223> Xaa can be Ser or Gly

<220>
<221> MISC_FEATURE
<222> (5)..(5)
<223> Xaa can be any amino-acid

<220>
<221> MISC_FEATURE
<222> (6)..(6)
<223> Xaa can be Gly or Ala

<220>
<221> MISC_FEATURE
<222> (7)..(7)
<223> Xaa can be ala or Ser

<220>
<221> MISC_FEATURE
<222> (8)..(8)
<223> Xaa can be Gln or Glu

<400> 6

Ala Xaa Glu Xaa Xaa Xaa Xaa Xaa Lys Leu Val
1 5 10

<210> 7
<211> 14
<212> PRT
<213> Artificial sequence

<220>
<223> Motif TRBV2-1

<220>
<221> MISC_FEATURE
<222> (1)..(14)
<223> Motif TRBV2-1

<220>
<221> MISC_FEATURE
<222> (4)..(4)
<223> Xaa can be any amino-acid

<220>
<221> MISC_FEATURE
<222> (5)..(5)
<223> Xaa can be Arg or Gly or Leu

<220>
<221> MISC_FEATURE
<222> (6)..(6)

<223> Xaa can be Thr or Ala

<220>
<221> MISC_FEATURE
<222> (10)..(10)
<223> Xaa can be any amino-acid

<220>
<221> MISC_FEATURE
<222> (11)..(11)
<223> Xaa can be any amino-acid

<220>
<221> MISC_FEATURE
<222> (12)..(12)
<223> Xaa can be Glu or Asp or Thr

<220>
<221> MISC_FEATURE
<222> (13)..(13)
<223> Xaa can be Gln or Thr

<220>
<221> MISC_FEATURE
<222> (14)..(14)
<223> Xaa can be Phe or Tyr

<400> 7

Ala Ser Ser Xaa Xaa Xaa Ser Gly Gly Xaa Xaa Xaa Xaa Xaa
1               5                   10

<210> 8
<211> 13
<212> PRT
<213> Artificial sequence

<220>
<223> Motif TRBV2-2-b

<220>
<221> MISC_FEATURE
<222> (1)..(13)
<223> Motif TRBV2-2-b

<220>
<221> MISC_FEATURE
<222> (4)..(4)
<223> Xaa can be any amino-acid

<220>
<221> MISC_FEATURE
<222> (5)..(5)
<223> Xaa can be Arg or Gly or Leu

<220>
<221> MISC_FEATURE
<222> (6)..(6)
<223> Xaa can be Thr or Ala

<220>

<221> MISC_FEATURE
<222> (7)..(7)
<223> Xaa can be Ser or Gly or Ala

<220>
<221> MISC_FEATURE
<222> (9)..(9)
<223> Xaa can be any amino-acid

<220>
<221> MISC_FEATURE
<222> (10)..(10)
<223> Xaa can be any amino-acid

<220>
<221> MISC_FEATURE
<222> (11)..(11)
<223> Xaa can be Glu or Asp or Thr or Pro

<220>
<221> MISC_FEATURE
<222> (12)..(12)
<223> Xaa can be Gln or Thr

<220>
<221> MISC_FEATURE
<222> (13)..(13)
<223> Xaa can be Phe or Tyr or His

<400> 8

```
Ala Ser Ser Xaa Xaa Xaa Xaa Gly Xaa Xaa Xaa Xaa Xaa
1               5                   10
```

<210> 9
<211> 11
<212> PRT
<213> Artificial sequence

<220>
<223> Motif TRBV2-3

<220>
<221> MISC_FEATURE
<222> (1)..(11)
<223> Motif TRBV2-3

<220>
<221> MISC_FEATURE
<222> (5)..(5)
<223> Xaa can be any amino-acid

<220>
<221> MISC_FEATURE
<222> (6)..(6)
<223> Xaa can be any amino-acid

<400> 9

```
Ala Ser Ser Gly Xaa Xaa Asn Thr Glu Ala Phe
1               5                   10
```

<210> 10
<211> 12
<212> PRT
<213> Artificial sequence

<220>
<223> Motif TRBV2-4

<220>
<221> MISC_FEATURE
<222> (1)..(12)
<223> Motif TRBV2-4

<220>
<221> MISC_FEATURE
<222> (8)..(8)
<223> Xaa can be Asn or Arg

<400> 10

Ala Ser Val Leu Met Arg Thr Xaa Asn Glu Gln Phe
1 5 10

<210> 11
<211> 12
<212> PRT
<213> Homo sapiens

<400> 11

Cys Ala Phe Lys Ala Ala Gly Asn Lys Leu Thr Phe
1 5 10

<210> 12
<211> 12
<212> PRT
<213> Homo sapiens

<400> 12

Cys Ala Phe Arg Ala Ala Gly Asn Lys Leu Thr Phe
1 5 10

<210> 13
<211> 12
<212> PRT
<213> Homo sapiens

<400> 13

Cys Ala His Lys Ala Ala Gly Asn Lys Leu Thr Phe
1 5 10

<210> 14
<211> 12
<212> PRT

<213> Homo sapiens

<400> 14

Cys Ala Leu Lys Ala Ala Gly Asn Lys Leu Thr Phe
1 5 10

<210> 15
<211> 12
<212> PRT
<213> Homo sapiens

<400> 15

Cys Ala Ser Lys Ala Ala Gly Asn Lys Leu Thr Phe
1 5 10

<210> 16
<211> 12
<212> PRT
<213> Homo sapiens

<400> 16

Cys Ser Arg Arg Ala Ala Gly Asn Lys Leu Thr Phe
1 5 10

<210> 17
<211> 13
<212> PRT
<213> Homo sapiens

<400> 17

Cys Ala Glu Tyr Gly Gly Ala Thr Asn Lys Leu Ile Phe
1 5 10

<210> 18
<211> 13
<212> PRT
<213> Homo sapiens

<400> 18

Cys Ala Asn Tyr Gly Gly Ala Thr Asn Lys Leu Ile Phe
1 5 10

<210> 19
<211> 13
<212> PRT
<213> Homo sapiens

<400> 19

Cys Ala Pro Tyr Gly Gly Ala Thr Asn Lys Leu Ile Phe
1 5 10

<210> 20
<211> 13
<212> PRT
<213> Homo sapiens

<400> 20

Cys Ala Arg Tyr Gly Gly Ala Thr Asn Lys Leu Ile Phe
1 5 10

<210> 21
<211> 13
<212> PRT
<213> Homo sapiens

<400> 21

Cys Ala Ser Tyr Gly Gly Ala Thr Asn Lys Leu Ile Phe
1 5 10

<210> 22
<211> 10
<212> PRT
<213> Homo sapiens

<400> 22

Cys Ala Phe Asp Asn Arg Lys Leu Ile Trp
1 5 10

<210> 23
<211> 12
<212> PRT
<213> Homo sapiens

<400> 23

Cys Ala Phe Arg Asn Ala Gly Asn Met Leu Thr Phe
1 5 10

<210> 24
<211> 12
<212> PRT
<213> Homo sapiens

<400> 24

Cys Ala Leu Asn Asn Ala Gly Asn Met Leu Thr Phe
1 5 10

<210> 25
<211> 12
<212> PRT
<213> Homo sapiens

<400> 25

Cys Ala Leu Arg Asn Ala Gly Asn Met Leu Thr Phe

1 5 10

<210> 26
<211> 12
<212> PRT
<213> Homo sapiens

<400> 26

Cys Ala Thr Arg Arg Ala Gly Asn Met Leu Thr Phe
1 5 10

<210> 27
<211> 13
<212> PRT
<213> Homo sapiens

<400> 27

Cys Ala Ser Glu Ser Thr Gly Ala Gln Lys Leu Val Phe
1 5 10

<210> 28
<211> 12
<212> PRT
<213> Homo sapiens

<400> 28

Cys Ala Tyr Glu Gly Ala Ser Glu Lys Leu Val Phe
1 5 10

<210> 29
<211> 13
<212> PRT
<213> Homo sapiens

<400> 29

Cys Ala Ser Ser Gly Gln Thr Asn Thr Glu Ala Phe Phe
1 5 10

<210> 30
<211> 15
<212> PRT
<213> Homo sapiens

<400> 30

Cys Ala Ser Ser Glu Gly Ala Ala Gly Asn Gln Pro Gln His Phe
1 5 10 15

<210> 31
<211> 15
<212> PRT
<213> Homo sapiens

<400> 31

Cys Ala Ser Ser Glu Leu Thr Ser Gly Gly Asp Glu Gln Phe Phe
1 5 10 15

<210> 32
<211> 15
<212> PRT
<213> Homo sapiens

<400> 32

Cys Ala Ser Ser Pro Gly Thr Ser Gly Val Gly Glu Gln Phe Phe
1 5 10 15

<210> 33
<211> 14
<212> PRT
<213> Homo sapiens

<400> 33

Cys Ala Ser Val Leu Met Arg Thr Asn Asn Glu Gln Phe Phe
1 5 10

<210> 34
<211> 15
<212> PRT
<213> Homo sapiens

<400> 34

Cys Ala Ser Ser Ala Leu Ala Ser Gly Thr Asp Thr Gln Tyr Phe
1 5 10 15

<210> 35
<211> 16
<212> PRT
<213> Homo sapiens

<400> 35

Cys Ala Ser Ser Ala Arg Thr Ser Gly Gly Ala Asp Thr Gln Tyr Phe
1 5 10 15

<210> 36
<211> 16
<212> PRT
<213> Homo sapiens

<400> 36

Cys Ala Ser Ser Ala Arg Thr Ser Gly Gly Ser Asp Thr Gln Tyr Phe
1 5 10 15

<210> 37
<211> 16

<212> PRT
<213> Homo sapiens

<400> 37

Cys Ala Ser Ser Ala Arg Thr Ser Gly Gly Thr Asp Thr Gln Tyr Phe
1 5 10 15

<210> 38
<211> 15
<212> PRT
<213> Homo sapiens

<400> 38

Cys Ala Ser Ser His Arg Ala Ser Gly Gly Asp Thr Gln Tyr Phe
1 5 10 15

<210> 39
<211> 16
<212> PRT
<213> Homo sapiens

<400> 39

Cys Ala Ser Ser Leu Arg Thr Ser Gly Gly Ser Asp Thr Gln Tyr Phe
1 5 10 15

<210> 40
<211> 16
<212> PRT
<213> Homo sapiens

<400> 40

Cys Ala Ser Ser Leu Arg Thr Ser Gly Gly Thr Asp Thr Gln Tyr Phe
1 5 10 15

<210> 41
<211> 15
<212> PRT
<213> Homo sapiens

<400> 41

Cys Ala Ser Ser Pro Leu Thr Ser Gly Thr Asp Thr Gln Tyr Phe
1 5 10 15

<210> 42
<211> 16
<212> PRT
<213> Homo sapiens

<400> 42

Cys Ala Ser Ser Arg Arg Thr Ser Gly Gly Thr Asp Thr Gln Tyr Phe
1 5 10 15

<210> 43
<211> 15
<212> PRT
<213> Homo sapiens

<400> 43

Cys Ala Ser Ser Lys Leu Ala Ser Gly Ala Asp Glu Gln Tyr Phe
1 5 10 15

<210> 44
<211> 15
<212> PRT
<213> Homo sapiens

<400> 44

Cys Ala Ser Ser Pro Arg Thr Ser Gly Gly Asp Glu Gln Tyr Phe
1 5 10 15

<210> 45
<211> 15
<212> PRT
<213> Homo sapiens

<400> 45

Cys Ala Ser Ser Pro Arg Thr Ser Gly Thr Tyr Glu Gln Tyr Phe
1 5 10 15

<210> 46
<211> 15
<212> PRT
<213> Homo sapiens

<400> 46

Cys Ala Ser Ser Arg Arg Thr Ser Gly Thr Tyr Glu Gln Tyr Phe
1 5 10 15

<210> 47
<211> 12
<212> PRT
<213> Homo sapiens

<400> 47

Cys Ala Phe Lys Ala Ala Gly Asn Lys Leu Thr Phe
1 5 10

<210> 48
<211> 12
<212> PRT
<213> Homo sapiens

<400> 48

Cys Ala Ser Lys Ala Ala Gly Asn Lys Leu Thr Phe
1               5                   10

<210>  49
<211>  12
<212>  PRT
<213>  Homo sapiens

<400>  49

Cys Ser Arg Arg Ala Ala Gly Asn Lys Leu Thr Phe
1               5                   10

<210>  50
<211>  14
<212>  PRT
<213>  Homo sapiens

<400>  50

Cys Ala Ser Ser Arg Leu Ala Gly Gly Met Asp Glu Gln Phe
1               5                   10

<210>  51
<211>  14
<212>  PRT
<213>  Homo sapiens

<400>  51

Cys Ala Thr Thr Pro Gly Ala Ser Gly Ile Ser Glu Gln Phe
1               5                   10

<210>  52
<211>  14
<212>  PRT
<213>  Homo sapiens

<400>  52

Cys Ala Ser Ser Pro Gly Thr Ser Gly Val Glu Gln Phe Phe
1               5                   10

<210>  53
<211>  16
<212>  PRT
<213>  Homo sapiens

<400>  53

Cys Ala Ser Ser Arg Arg Thr Ser Gly Gly Thr Asp Thr Gln Tyr Phe
1               5                   10                  15

<210>  54
<211>  60
<212>  DNA
<213>  Human immunodeficiency virus

<400> 54
tttagagact atgtagaccg gttctataaa actctaagag ccgagcaagc ttcacaggag 60

<210> 55
<211> 343
<212> DNA
<213> Homo sapiens

<400> 55
atggagaaga atcctttggc agccccatta ctaatcctct ggtttcatct tgactgcgtg 60

agcagcatac tgaacgtgga acaaagtcct cagtcactgc atgttcagga gggagacagc 120

accaatttca cctgcagctt cccttccagc aatttttatg ccttacactg gtacagatgg 180

gaaactgcaa aaagccccga ggccttgttt gtaatgactt taaatgggga tgaaaagaag 240

aaaggacgaa taagtgccac tcttaatacc aaggagggtt acagctattt gtacatcaaa 300

ggatcccagc ctgaagactc agccacatac ctctgtgcct tta 343

<210> 56
<211> 290
<212> DNA
<213> Homo sapiens

<400> 56
gaacctgaag tcacccagac tcccagccat caggtcacac agatgggaca ggaagtgatc 60

ttgcgctgtg tccccatctc taatcactta tacttctatt ggtacagaca aatcttgggg 120

cagaaagtcg agtttctggt ttccttttat aataatgaaa tctcagagaa gtctgaaata 180

ttcgatgatc aattctcagt tgaaaggcct gatggatcaa atttcactct gaagatccgg 240

tccacaaagc tggaggactc agccatgtac ttctgtgcca gcagtgaagc 290

<210> 57
<211> 8241
<212> DNA
<213> Artificial sequence

<220>
<223> Plasmid pCDH-F24-TCR

<220>
<221> source
<222> (1)..(8241)
<223> Plasmid pCDH-F24-TCR

<400> 57
acgcgtgtag tcttatgcaa tactcttgta gtcttgcaac atggtaacga tgagttagca 60

acatgcctta caaggagaga aaaagcaccg tgcatgccga ttggtggaag taaggtggta 120

cgatcgtgcc ttattaggaa ggcaacagac gggtctgaca tggattggac gaaccactga 180

attgccgcat tgcagagata ttgtatttaa gtgcctagct cgatacaata aacgggtctc 240

tctggttaga ccagatctga gcctgggagc tctctggcta actagggaac ccactgctta 300
agcctcaata aagcttgcct tgagtgcttc aagtagtgtg tgcccgtctg ttgtgtgact 360
ctggtaacta gagatccctc agaccctttt agtcagtgtg gaaaatctct agcagtggcg 420
cccgaacagg gacctgaaag cgaaagggaa accagagctc tctcgacgca ggactcggct 480
tgctgaagcg cgcacggcaa gaggcgaggg gcggcgactg gtgagtacgc caaaaatttt 540
gactagcgga ggctagaagg agagagatgg gtgcgagagc gtcagtatta agcgggggag 600
aattagatcg cgatgggaaa aaattcggtt aaggccaggg ggaaagaaaa aatataaatt 660
aaaacatata gtatgggcaa gcagggagct agaacgattc gcagttaatc ctggcctgtt 720
agaaacatca gaaggctgta gacaaatact gggacagcta caaccatccc ttcagacagg 780
atcagaagaa cttagatcat tatataatac agtagcaacc ctctattgtg tgcatcaaag 840
gatagagata aaagacacca aggaagcttt agacaagata gaggaagagc aaaacaaaag 900
taagaccacc gcacagcaag cggccactga tcttcagacc tggaggagga gatatgaggg 960
acaattggag aagtgaatta tataaatata aagtagtaaa aattgaacca ttaggagtag 1020
cacccaccaa ggcaaagaga agagtggtgc agagagaaaa aagagcagtg ggaataggag 1080
ctttgttcct tgggttcttg ggagcagcag gaagcactat gggcgcagcg tcaatgacgc 1140
tgacggtaca ggccagacaa ttattgtctg gtatagtgca gcagcagaac aatttgctga 1200
gggctattga ggcgcaacag catctgttgc aactcacagt ctggggcatc aagcagctcc 1260
aggcaagaat cctggctgtg gaaagatacc taaaggatca acagctcctg gggatttggg 1320
gttgctctgg aaaactcatt tgcaccactg ctgtgccttg gaatgctagt tggagtaata 1380
aatctctgga acagatttgg aatcacacga cctggatgga gtgggacaga gaaattaaca 1440
attacacaag cttaatacac tccttaattg aagaatcgca aaaccagcaa gaaaagaatg 1500
aacaagaatt attggaatta gataaatggg caagtttgtg gaattggttt aacataacaa 1560
attggctgtg gtatataaaa ttattcataa tgatagtagg aggcttggta ggtttaagaa 1620
tagtttttgc tgtactttct atagtgaata gagttaggca gggatattca ccattatcgt 1680
ttcagaccca cctcccaacc ccgaggggac ccgacaggcc cgaaggaata gaagaagaag 1740
gtggagagag agacagagac agatccattc gattagtgaa cggatctcga cggtatcggt 1800
taacttttaa aagaaaaggg gggattgggg ggtacagtgc aggggaaaga atagtagaca 1860
taatagcaac agacatacaa actaaagaat tacaaaaaca aattacaaaa ttcaaaattt 1920
tatcgatact agtggatctg cgatcgctcc ggtgcccgtc agtgggcaga gcgcacatcg 1980
cccacagtcc ccgagaagtt ggggggaggg gtcggcaatt gaacgggtgc ctagagaagg 2040
tggcgcgggg taaactggga aagtgatgtc gtgtactggc tccgcctttt tcccgagggt 2100

gggggagaac cgtatataag tgcagtagtc gccgtgaacg ttctttttcg caacgggttt 2160
gccgccagaa cacagctgaa gcttcgaggg gctcgcatct ctccttcacg cgcccgccgc 2220
cctacctgag gccgccatcc acgccggttg agtcgcgttc tgccgcctcc cgcctgtggt 2280
gcctcctgaa ctgcgtccgc cgtctaggta agtttaaagc tcaggtcgag accgggcctt 2340
tgtccggcgc tcccttggag cctacctaga ctcagccggc tctccacgct ttgcctgacc 2400
ctgcttgctc aactctacgt ctttgtttcg ttttctgttc tgcgccgtta cagatccaag 2460
ctgtgaccgg cgcctactct agagctagcc gccaccatgg agaagaatcc tttggtagcc 2520
ccattactaa tcctctggtt tcatcttgac tgcgtgagca gcatactgaa cgtggaacaa 2580
agtcctcagt cactgcatgt tcaggaggga gacagcacca atttcacctg cagcttccct 2640
tccagcaatt tttatgcctt acactggtac agatgggaaa ctgcaaaaag ccccgaggcc 2700
ttgtttgtaa tgactttaaa tggggatgaa aagaagaaag gacgaataag tgccactctt 2760
aataccaagg agggttacag ctatttgtac atcaaaggat cccagcctga agactcagcc 2820
acatacctct gtgcctttaa agctgcaggc aacaagctaa cttttggagg aggaaccagg 2880
gtgctagtta aaccaaatat ccagaagcct gaccctgccg tgtaccagct gagagactct 2940
aaatccagtg acaagtctgt ctgcctattc accgattttg attctcaaac aaatgtgtca 3000
caaagtaagg attctgatgt gtatatcaca gacaaaactg tgctagacat gaggtctatg 3060
gacttcaaga gcaacagtgc tgtggcctgg agcaacaaat ctgactttgc atgtgcaaac 3120
gccttcaaca acagcattat tccagcagac accttcttcc ccagcccaga aagttcctgt 3180
gatgtcaagc tggtcgagaa aagctttgaa acagatacga acctaaactt tcaaaacctg 3240
tcagtgattg ggttccgaat cctcctcctg aaagtggccg ggtttaatct gctcatgacg 3300
ctgcggctgt ggtccagcgc ggccgctgag ggcagaggaa gtcttctaac atgcggtgac 3360
gtggaggaga atcccggccc ttccggaatg gatacctggc tcgtatgctg ggcaattttt 3420
agtctcttga aagcaggact cacagaacct gaagtcaccc agactcccag ccatcaggtc 3480
acacagatgg gacaggaagt gatcttgcgc tgtgtcccca tctctaatca cttatacttc 3540
tattggtaca gacaaatctt ggggcagaaa gtcgagtttc tggtttcctt ttataataat 3600
gaaatctcag agaagtctga aatattcgat gatcaattct cagttgaaag gcctgatgga 3660
tcaaatttca ctctgaagat ccggtccaca aagctggagg actcagccat gtacttctgt 3720
gccagcagcc gactagcggg agggatggat gagcagttct tcgggccagg gacacggctc 3780
accgtgctag aggacctgaa aaacgtgttc ccacccgagg tcgctgtgtt tgagccatca 3840
gaagcagaga tctcccacac ccaaaaggcc acactggtgt gcctggccac aggcttctac 3900
cccgaccacg tggagctgag ctggtgggtg aatgggaagg aggtgcacag tggggtcagc 3960
acagacccgc agcccctcaa ggagcagccc gccctcaatg actccagata ctgcctgagc 4020

agccgcctga gggtctcggc caccttctgg cagaaccccc gcaaccactt ccgctgtcaa 4080
gtccagttct acgggctctc ggagaatgac gagtggaccc aggatagggc caaacctgtc 4140
acccagatcg tcagcgccga ggcctggggt agagcagact gtggcttcac ctccgagtct 4200
taccagcaag ggtcctgtc tgccaccatc ctctatgaga tcttgctagg gaaggccacc 4260
ttgtatgccg tgctggtcag tgccctcgtg ctgatggcca tggtcaagag aaaggattcc 4320
agaggctagg tcgacaatca acctctggat tacaaaattt gtgaaagatt gactggtatt 4380
cttaactatg ttgctccttt tacgctatgt ggatacgctg ctttaatgcc tttgtatcat 4440
gctattgctt cccgtatggc tttcattttc tcctccttgt ataaatcctg gttgctgtct 4500
ctttatgagg agttgtggcc cgttgtcagg caacgtggcg tggtgtgcac tgtgtttgct 4560
gacgcaaccc ccactggttg gggcattgcc accacctgtc agctcctttc cgggactttc 4620
gctttccccc tccctattgc cacggcggaa ctcatcgccg cctgccttgc ccgctgctgg 4680
acaggggctc ggctgttggg cactgacaat tccgtggtgt tgtcggggaa atcatcgtcc 4740
tttccttggc tgctcgcctg tgttgccacc tggattctgc gcgggacgtc cttctgctac 4800
gtcccttcgg ccctcaatcc agcggacctt ccttcccgcg gcctgctgcc ggctctgcgg 4860
cctcttccgc gtcttcgcct tcgccctcag acgagtcgga tctccctttg ggccgcctcc 4920
ccgcctggta cctttaagac caatgactta caaggcagct gtagatctta gccacttttt 4980
aaaagaaaag gggggactgg aagggctaat tcactcccaa cgaaaataag atctgctttt 5040
tgcttgtact gggtctctct ggttagacca gatctgagcc tgggagctct ctggctaact 5100
agggaaccca ctgcttaagc ctcaataaag cttgccttga gtgcttcaag tagtgtgtgc 5160
ccgtctgttg tgtgactctg gtaactagag atccctcaga cccttttagt cagtgtggaa 5220
aatctctagc agtagtagtt catgtcatct tattattcag tatttataac ttgcaaagaa 5280
atgaatatca gagagtgaga ggaacttgtt tattgcagct tataatggtt acaaataaag 5340
caatagcatc acaaatttca caaataaagc atttttttca ctgcattcta gttgtggttt 5400
gtccaaactc atcaatgtat cttatcatgt ctggctctag ctatcccgcc cctaactccg 5460
cccagttccg cccattctcc gccccatggc tgactaattt tttttattta tgcagaggcc 5520
gaggccgcct cggcctctga gctattccag aagtagtgag gaggcttttt tggaggccta 5580
gacttttgca gagacggccc aaattcgtaa tcatggtcat agctgtttcc tgtgtgaaat 5640
tgttatccgc tcacaattcc acacaacata cgagccggaa gcataaagtg taaagcctgg 5700
ggtgcctaat gagtgagcta actcacatta attgcgttgc gctcactgcc cgctttccag 5760
tcgggaaacc tgtcgtgcca gctgcattaa tgaatcggcc aacgcgcggg gagaggcggt 5820
ttgcgtattg ggcgctcttc cgcttcctcg ctcactgact cgctgcgctc ggtcgttcgg 5880

ctgcggcgag cggtatcagc tcactcaaag gcggtaatac ggttatccac agaatcaggg 5940
gataacgcag gaaagaacat gtgagcaaaa ggccagcaaa aggccaggaa ccgtaaaaag 6000
gccgcgttgc tggcgttttt ccataggctc cgcccccctg acgagcatca caaaaatcga 6060
cgctcaagtc agaggtggcg aaacccgaca ggactataaa gataccaggc gtttccccct 6120
ggaagctccc tcgtgcgctc tcctgttccg accctgccgc ttaccggata cctgtccgcc 6180
tttctccctt cgggaagcgt ggcgctttct catagctcac gctgtaggta tctcagttcg 6240
gtgtaggtcg ttcgctccaa gctgggctgt gtgcacgaac cccccgttca gcccgaccgc 6300
tgcgccttat ccggtaacta tcgtcttgag tccaacccgg taagacacga cttatcgcca 6360
ctggcagcag ccactggtaa caggattagc agagcgaggt atgtaggcgg tgctacagag 6420
ttcttgaagt ggtggcctaa ctacggctac actagaagga cagtatttgg tatctgcgct 6480
ctgctgaagc cagttacctt cggaaaaaga gttggtagct cttgatccgg caaacaaacc 6540
accgctggta gcggtggttt ttttgtttgc aagcagcaga ttacgcgcag aaaaaaagga 6600
tctcaagaag atcctttgat cttttctacg gggtctgacg ctcagtggaa cgaaaactca 6660
cgttaaggga ttttggtcat gagattatca aaaaggatct tcacctagat ccttttaaat 6720
taaaaatgaa gttttaaatc aatctaaagt atatatgagt aaacttggtc tgacagttac 6780
caatgcttaa tcagtgaggc acctatctca gcgatctgtc tatttcgttc atccatagtt 6840
gcctgactcc ccgtcgtgta gataactacg atacgggagg gcttaccatc tggccccagt 6900
gctgcaatga taccgcgaga cccacgctca ccggctccag atttatcagc aataaaccag 6960
ccagccggaa gggccgagcg cagaagtggt cctgcaactt tatccgcctc catccagtct 7020
attaattgtt gccgggaagc tagagtaagt agttcgccag ttaatagttt gcgcaacgtt 7080
gttgccattg ctacaggcat cgtggtgtca cgctcgtcgt ttggtatggc ttcattcagc 7140
tccggttccc aacgatcaag gcgagttaca tgatccccca tgttgtgcaa aaaagcggtt 7200
agctccttcg gtcctccgat cgttgtcaga agtaagttgg ccgcagtgtt atcactcatg 7260
gttatggcag cactgcataa ttctcttact gtcatgccat ccgtaagatg cttttctgtg 7320
actggtgagt actcaaccaa gtcattctga gaatagtgta tgcggcgacc gagttgctct 7380
tgcccggcgt caatacggga taataccgcg ccacatagca gaactttaaa agtgctcatc 7440
attggaaaac gttcttcggg gcgaaaactc tcaaggatct taccgctgtt gagatccagt 7500
tcgatgtaac ccactcgtgc acccaactga tcttcagcat cttttacttt caccagcgtt 7560
tctgggtgag caaaaacagg aaggcaaaat gccgcaaaaa agggaataag ggcgacacgg 7620
aaatgttgaa tactcatact cttccttttt caatattatt gaagcattta tcagggttat 7680
tgtctcatga gcggatacat atttgaatgt atttagaaaa ataaacaaat aggggttccg 7740
cgcacatttc cccgaaaagt gccacctgac gtctaagaaa ccattattat catgacatta 7800

```
acctataaaa ataggcgtat cacgaggccc tttcgtctcg cgcgtttcgg tgatgacggt      7860

gaaaacctct gacacatgca gctcccggag acggtcacag cttgtctgta agcggatgcc      7920

gggagcagac aagcccgtca gggcgcgtca gcgggtgttg gcgggtgtcg gggctggctt      7980

aactatgcgg catcagagca gattgtactg agagtgcacc atatgcggtg tgaaataccg      8040

cacagatgcg taaggagaaa ataccgcatc aggcgccatt cgccattcag gctgcgcaac      8100

tgttgggaag ggcgatcggt gcgggcctct tcgctattac gccagctggc gaaaggggga      8160

tgtgctgcaa ggcgattaag ttgggtaacg ccagggtttt cccagtcacg acgttgtaaa      8220

acgacggcca gtgccaagct g                                                8241


<210>  58
<211>  274
<212>  PRT
<213>  Homo sapiens

<400>  58

Met Glu Lys Asn Pro Leu Val Ala Pro Leu Leu Ile Leu Trp Phe His
1               5                   10                  15

Leu Asp Cys Val Ser Ser Ile Leu Asn Val Glu Gln Ser Pro Gln Ser
            20                  25                  30

Leu His Val Gln Glu Gly Asp Ser Thr Asn Phe Thr Cys Ser Phe Pro
        35                  40                  45

Ser Ser Asn Phe Tyr Ala Leu His Trp Tyr Arg Trp Glu Thr Ala Lys
    50                  55                  60

Ser Pro Glu Ala Leu Phe Val Met Thr Leu Asn Gly Asp Glu Lys Lys
65                  70                  75                  80

Lys Gly Arg Ile Ser Ala Thr Leu Asn Thr Lys Glu Gly Tyr Ser Tyr
                85                  90                  95

Leu Tyr Ile Lys Gly Ser Gln Pro Glu Asp Ser Ala Thr Tyr Leu Cys
            100                 105                 110

Ala Phe Lys Ala Ala Gly Asn Lys Leu Thr Phe Gly Gly Gly Thr Arg
        115                 120                 125

Val Leu Val Lys Pro Asn Ile Gln Lys Pro Asp Pro Ala Val Tyr Gln
    130                 135                 140

Leu Arg Asp Ser Lys Ser Ser Asp Lys Ser Val Cys Leu Phe Thr Asp
145                 150                 155                 160
```

Phe Asp Ser Gln Thr Asn Val Ser Gln Ser Lys Asp Ser Asp Val Tyr
                165                 170                 175

Ile Thr Asp Lys Thr Val Leu Asp Met Arg Ser Met Asp Phe Lys Ser
            180                 185                 190

Asn Ser Ala Val Ala Trp Ser Asn Lys Ser Asp Phe Ala Cys Ala Asn
        195                 200                 205

Ala Phe Asn Asn Ser Ile Ile Pro Ala Asp Thr Phe Phe Pro Ser Pro
    210                 215                 220

Glu Ser Ser Cys Asp Val Lys Leu Val Glu Lys Ser Phe Glu Thr Asp
225                 230                 235                 240

Thr Asn Leu Asn Phe Gln Asn Leu Ser Val Ile Gly Phe Arg Ile Leu
                245                 250                 255

Leu Leu Lys Val Ala Gly Phe Asn Leu Leu Met Thr Leu Arg Leu Trp
            260                 265                 270

Ser Ser

<210> 59
<211> 313
<212> PRT
<213> Homo sapiens

<400> 59

Met Asp Thr Trp Leu Val Cys Trp Ala Ile Phe Ser Leu Leu Lys Ala
1               5                   10                  15

Gly Leu Thr Glu Pro Glu Val Thr Gln Thr Pro Ser His Gln Val Thr
            20                  25                  30

Gln Met Gly Gln Glu Val Ile Leu Arg Cys Val Pro Ile Ser Asn His
        35                  40                  45

Leu Tyr Phe Tyr Trp Tyr Arg Gln Ile Leu Gly Gln Lys Val Glu Phe
    50                  55                  60

Leu Val Ser Phe Tyr Asn Asn Glu Ile Ser Glu Lys Ser Glu Ile Phe
65                  70                  75                  80

Asp Asp Gln Phe Ser Val Glu Arg Pro Asp Gly Ser Asn Phe Thr Leu
                85                  90                  95

```
Lys Ile Arg Ser Thr Lys Leu Glu Asp Ser Ala Met Tyr Phe Cys Ala
            100                 105                 110

Ser Ser Arg Leu Ala Gly Gly Met Asp Glu Gln Phe Phe Gly Pro Gly
        115                 120                 125

Thr Arg Leu Thr Val Leu Glu Asp Leu Lys Asn Val Phe Pro Pro Glu
    130                 135                 140

Val Ala Val Phe Glu Pro Ser Glu Ala Glu Ile Ser His Thr Gln Lys
145                 150                 155                 160

Ala Thr Leu Val Cys Leu Ala Thr Gly Phe Tyr Pro Asp His Val Glu
                165                 170                 175

Leu Ser Trp Trp Val Asn Gly Lys Glu Val His Ser Gly Val Ser Thr
            180                 185                 190

Asp Pro Gln Pro Leu Lys Glu Gln Pro Ala Leu Asn Asp Ser Arg Tyr
        195                 200                 205

Cys Leu Ser Ser Arg Leu Arg Val Ser Ala Thr Phe Trp Gln Asn Pro
    210                 215                 220

Arg Asn His Phe Arg Cys Gln Val Gln Phe Tyr Gly Leu Ser Glu Asn
225                 230                 235                 240

Asp Glu Trp Thr Gln Asp Arg Ala Lys Pro Val Thr Gln Ile Val Ser
                245                 250                 255

Ala Glu Ala Trp Gly Arg Ala Asp Cys Gly Phe Thr Ser Glu Ser Tyr
            260                 265                 270

Gln Gln Gly Val Leu Ser Ala Thr Ile Leu Tyr Glu Ile Leu Leu Gly
        275                 280                 285

Lys Ala Thr Leu Tyr Ala Val Leu Val Ser Ala Leu Val Leu Met Ala
    290                 295                 300

Met Val Lys Arg Lys Asp Ser Arg Gly
305                 310
```

<210> 60
<211> 263
<212> PRT
<213> Homo sapiens

<400> 60

Ile Leu Trp Phe His Leu Asp Cys Val Ser Ser Ile Leu Asn Val Glu
1               5                   10                  15

Gln Ser Pro Gln Ser Leu His Val Gln Glu Gly Asp Ser Thr Asn Phe
            20                  25                  30

Thr Cys Ser Phe Pro Ser Ser Asn Phe Tyr Ala Leu His Trp Tyr Arg
        35                  40                  45

Trp Glu Thr Ala Lys Ser Pro Glu Ala Leu Phe Val Met Thr Leu Asn
    50                  55                  60

Gly Asp Glu Lys Lys Lys Gly Arg Ile Ser Ala Thr Leu Asn Thr Lys
65                  70                  75                  80

Glu Gly Tyr Ser Tyr Leu Tyr Ile Lys Gly Ser Gln Pro Glu Asp Ser
                85                  90                  95

Ala Thr Tyr Leu Cys Ala Phe Lys Ala Ala Gly Asn Lys Leu Thr Phe
            100                 105                 110

Gly Gly Gly Thr Arg Val Leu Val Lys Pro Asn Ile Gln Lys Pro Asp
        115                 120                 125

Pro Ala Val Tyr Gln Leu Arg Asp Ser Lys Ser Ser Asp Lys Ser Val
    130                 135                 140

Cys Leu Phe Thr Asp Phe Asp Ser Gln Thr Asn Val Ser Gln Ser Lys
145                 150                 155                 160

Asp Ser Asp Val Tyr Ile Thr Asp Lys Thr Val Leu Asp Met Arg Ser
                165                 170                 175

Met Asp Phe Lys Ser Asn Ser Ala Val Ala Trp Ser Asn Lys Ser Asp
            180                 185                 190

Phe Ala Cys Ala Asn Ala Phe Asn Asn Ser Ile Ile Pro Ala Asp Thr
        195                 200                 205

Phe Phe Pro Ser Pro Glu Ser Ser Cys Asp Val Lys Leu Val Glu Lys
    210                 215                 220

Ser Phe Glu Thr Asp Thr Asn Leu Asn Phe Gln Asn Leu Ser Val Ile
225                 230                 235                 240

Gly Phe Arg Ile Leu Leu Leu Lys Val Ala Gly Phe Asn Leu Leu Met

245 250 255

Thr Leu Arg Leu Trp Ser Ser
260

<210> 61
<211> 294
<212> PRT
<213> Homo sapiens

<400> 61

Glu Pro Glu Val Thr Gln Thr Pro Ser His Gln Val Thr Gln Met Gly
1 5 10 15

Gln Glu Val Ile Leu Arg Cys Val Pro Ile Ser Asn His Leu Tyr Phe
20 25 30

Tyr Trp Tyr Arg Gln Ile Leu Gly Gln Lys Val Glu Phe Leu Val Ser
35 40 45

Phe Tyr Asn Asn Glu Ile Ser Glu Lys Ser Glu Ile Phe Asp Asp Gln
50 55 60

Phe Ser Val Glu Arg Pro Asp Gly Ser Asn Phe Thr Leu Lys Ile Arg
65 70 75 80

Ser Thr Lys Leu Glu Asp Ser Ala Met Tyr Phe Cys Ala Ser Ser Arg
85 90 95

Leu Ala Gly Gly Met Asp Glu Gln Phe Phe Gly Pro Gly Thr Arg Leu
100 105 110

Thr Val Leu Glu Asp Leu Lys Asn Val Phe Pro Pro Glu Val Ala Val
115 120 125

Phe Glu Pro Ser Glu Ala Glu Ile Ser His Thr Gln Lys Ala Thr Leu
130 135 140

Val Cys Leu Ala Thr Gly Phe Tyr Pro Asp His Val Glu Leu Ser Trp
145 150 155 160

Trp Val Asn Gly Lys Glu Val His Ser Gly Val Ser Thr Asp Pro Gln
165 170 175

Pro Leu Lys Glu Gln Pro Ala Leu Asn Asp Ser Arg Tyr Cys Leu Ser
180 185 190

Ser Arg Leu Arg Val Ser Ala Thr Phe Trp Gln Asn Pro Arg Asn His

```
        195                 200                 205

Phe Arg Cys Gln Val Gln Phe Tyr Gly Leu Ser Glu Asn Asp Glu Trp
    210                 215                 220

Thr Gln Asp Arg Ala Lys Pro Val Thr Gln Ile Val Ser Ala Glu Ala
225                 230                 235                 240

Trp Gly Arg Ala Asp Cys Gly Phe Thr Ser Glu Ser Tyr Gln Gln Gly
                245                 250                 255

Val Leu Ser Ala Thr Ile Leu Tyr Glu Ile Leu Leu Gly Lys Ala Thr
            260                 265                 270

Leu Tyr Ala Val Leu Val Ser Ala Leu Val Leu Met Ala Met Val Lys
        275                 280                 285

Arg Lys Asp Ser Arg Gly
    290


<210>  62
<211>  822
<212>  DNA
<213>  Homo sapiens

<400>  62
atggagaaga atcctttggt agccccatta ctaatcctct ggtttcatct tgactgcgtg      60

agcagcatac tgaacgtgga acaaagtcct cagtcactgc atgttcagga gggagacagc     120

accaatttca cctgcagctt cccttccagc aatttttatg ccttacactg gtacagatgg     180

gaaactgcaa aaagccccga ggccttgttt gtaatgactt taaatgggga tgaaaagaag     240

aaaggacgaa taagtgccac tcttaatacc aaggagggtt acagctattt gtacatcaaa     300

ggatcccagc ctgaagactc agccacatac ctctgtgcct ttaaagctgc aggcaacaag     360

ctaacttttg gaggaggaac cagggtgcta gttaaaccaa atatccagaa gcctgacccct     420

gccgtgtacc agctgagaga ctctaaatcc agtgacaagt ctgtctgcct attcaccgat     480

tttgattctc aaacaaatgt gtcacaaagt aaggattctg atgtgtatat cacagacaaa     540

actgtgctag acatgaggtc tatggacttc aagagcaaca gtgctgtggc ctggagcaac     600

aaatctgact ttgcatgtgc aaacgccttc aacaacagca ttattccagc agacaccttc     660

ttccccagcc cagaaagttc ctgtgatgtc aagctggtcg agaaaagctt tgaaacagat     720

acgaacctaa actttcaaaa cctgtcagtg attgggttcc gaatcctcct cctgaaagtg     780

gccgggttta atctgctcat gacgctgcgg ctgtggtcca gc                        822


<210>  63
```

<211> 939
<212> DNA
<213> Homo sapiens

<400> 63
atggatacct ggctcgtatg ctgggcaatt tttagtctct tgaaagcagg actcacagaa 60

cctgaagtca cccagactcc cagccatcag gtcacacaga tgggacagga agtgatcttg 120

cgctgtgtcc ccatctctaa tcacttatac ttctattggt acagacaaat cttggggcag 180

aaagtcgagt ttctggtttc cttttataat aatgaaatct cagagaagtc tgaaatattc 240

gatgatcaat tctcagttga aaggcctgat ggatcaaatt tcactctgaa gatccggtcc 300

acaaagctgg aggactcagc catgtacttc tgtgccagca gccgactagc gggagggatg 360

gatgagcagt tcttcgggcc agggacacgg ctcaccgtgc tagaggacct gaaaaacgtg 420

ttcccacccg aggtcgctgt gtttgagcca tcagaagcag agatctccca cacccaaaag 480

gccacactgg tgtgcctggc cacaggcttc taccccgacc acgtggagct gagctggtgg 540

gtgaatggga aggaggtgca cagtggggtc agcacagacc cgcagcccct caaggagcag 600

cccgccctca atgactccag atactgcctg agcagccgcc tgagggtctc ggccaccttc 660

tggcagaacc cccgcaacca cttccgctgt caagtccagt tctacgggct ctcggagaat 720

gacgagtgga cccaggatag ggccaaacct gtcacccaga tcgtcagcgc cgaggcctgg 780

ggtagagcag actgtggctt cacctccgag tcttaccagc aaggggtcct gtctgccacc 840

atcctctatg agatcttgct agggaaggcc accttgtatg ccgtgctggt cagtgccctc 900

gtgctgatgg ccatggtcaa gagaaaggat tccagaggc 939

<210> 64
<211> 423
<212> DNA
<213> Homo sapiens

<220>
<221> misc_feature
<222> (1)..(1)
<223> n is a, c, g, or t

<400> 64
natatccaga accctgaccc tgccgtgtac cagctgagag actctaaatc cagtgacaag 60

tctgtctgcc tattcaccga ttttgattct caaacaaatg tgtcacaaag taaggattct 120

gatgtgtata tcacagacaa aactgtgcta gacatgaggt ctatggactt caagagcaac 180

agtgctgtgg cctggagcaa caaatctgac tttgcatgtg caaacgcctt caacaacagc 240

attattccag aagacacctt cttccccagc ccagaaagtt cctgtgatgt caagctggtc 300

gagaaaagct ttgaaacaga tacgaaccta aactttcaaa acctgtcagt gattgggttc 360

cgaatcctcc tcctgaaagt ggccgggttt aatctgctca tgacgctgcg gctgtggtcc 420

agc 423

<210> 65
<211> 537
<212> DNA
<213> Homo sapiens

<400> 65
gaggacctga aaaacgtgtt cccacccgag gtcgctgtgt ttgagccatc agaagcagag 60

atctcccaca cccaaaaggc cacactggta tgcctggcca caggcttcta ccccgaccac 120

gtggagctga gctggtgggt gaatgggaag gaggtgcaca gtggggtcag cacagacccg 180

cagcccctca aggagcagcc cgccctcaat gactccagat actgcctgag cagccgcctg 240

agggtctcgg ccaccttctg gcagaacccc cgcaaccact tccgctgtca agtccagttc 300

tacgggctct cggagaatga cgagtggacc caggataggg ccaaacccgt cacccagatc 360

gtcagcgccg aggcctgggg tagagcagac tgtggcttca cctccgagtc ttaccagcaa 420

ggggtcctgt ctgccaccat cctctatgag atcttgctag ggaaggccac cttgtatgcc 480

gtgctggtca gtgccctcgt gctgatggcc atggtcaaga gaaaggattc cagaggc 537

<210> 66
<211> 63
<212> DNA
<213> Homo sapiens

<400> 66
tgatcaaagc tgcaggcaac aagctaactt ttggaggagg aaccagggtg ctagttaaac 60

caa 63

<210> 67
<211> 66
<212> DNA
<213> Homo sapiens

<400> 67
tgaattatgg cggtgctaca aacaagctca tctttggaac tggcactctg cttgctgtcc 60

agccaa 66

<210> 68
<211> 63
<212> DNA
<213> Homo sapiens

<400> 68
tgaataataa tgcaggcaac atgctcacct ttggaggggg aacaaggtta atggtcaaac 60

ccc 63

<210> 69

<211> 60
<212> DNA
<213> Homo sapiens

<400> 69
taattcaggg agcccagaag ctggtatttg gccaaggaac caggctgact atcaacccaa 60

<210> 70
<211> 63
<212> DNA
<213> Homo sapiens

<400> 70
taactcaggg cggatctgaa aagctggtct ttggaaaggg aacgaaactg acagtaaacc 60

cat 63

<210> 71
<211> 48
<212> DNA
<213> Homo sapiens

<400> 71
tgaacactga agctttcttt ggacaaggca ccagactcac agttgtag 48

<210> 72
<211> 48
<212> DNA
<213> Homo sapiens

<400> 72
ctaactatgg ctacaccttc ggttcgggga ccaggttaac cgttgtag 48

<210> 73
<211> 50
<212> DNA
<213> Homo sapiens

<400> 73
tagcaatcag ccccagcatt ttggtgatgg gactcgactc tccatcctag 50

<210> 74
<211> 50
<212> DNA
<213> Homo sapiens

<400> 74
ctcctacaat gagcagttct tcgggccagg gacacggctc accgtgctag 50

<210> 75
<211> 49
<212> DNA
<213> Homo sapiens

<400> 75
agcacagata cgcagtattt tggcccaggc acccggctga cagtgctcg 49

<210> 76
<211> 47
<212> DNA
<213> Homo sapiens

<400> 76
ctcctacgag cagtacttcg ggccgggcac caggctcacg gtcacag 47

<210> 77
<211> 12
<212> DNA
<213> Homo sapiens

<400> 77
gggacagggg gc 12

<210> 78
<211> 16
<212> DNA
<213> Homo sapiens

<400> 78
gggactagcg gggggg 16

<210> 79
<211> 36
<212> DNA
<213> Homo sapiens

<400> 79
tgtgccttta aagctgcagg caacaagcta actttt 36

<210> 80
<211> 36
<212> DNA
<213> Homo sapiens

<400> 80
tgtgccttta aggctgcagg caacaagcta actttt 36

<210> 81
<211> 36
<212> DNA
<213> Homo sapiens

<400> 81
tgtgccttca aagctgcagg caacaagcta actttt 36

<210> 82
<211> 36
<212> DNA
<213> Homo sapiens

<400> 82
tgtgcattta aagctgcagg caacaagcta actttt 36

<210> 83
<211> 36
<212> DNA
<213> Homo sapiens

<400> 83
tgtgccttca gggctgcagg caacaagcta actttt 36

<210> 84
<211> 36
<212> DNA
<213> Homo sapiens

<400> 84
tgtgccttta gagctgcagg caacaagcta actttt 36

<210> 85
<211> 36
<212> DNA
<213> Homo sapiens

<400> 85
tgtgcctttc gagctgcagg caacaagcta actttt 36

<210> 86
<211> 36
<212> DNA
<213> Homo sapiens

<400> 86
tgtgccttta gggctgcagg caacaagcta actttt 36

<210> 87
<211> 36
<212> DNA
<213> Homo sapiens

<400> 87
tgtgcccaca aagctgcagg caacaagcta actttt 36

<210> 88
<211> 36
<212> DNA
<213> Homo sapiens

<400> 88
tgtgcccata aagctgcagg caacaagcta actttt 36

<210> 89
<211> 36
<212> DNA
<213> Homo sapiens

<400> 89
tgtgccttga aagctgcagg caacaagcta actttt 36

<210> 90
<211> 36
<212> DNA
<213> Homo sapiens

<400> 90
tgtgccttaa aagctgcagg caacaagcta actttt 36

<210> 91
<211> 36
<212> DNA
<213> Homo sapiens

<400> 91
tgtgctctaa aagctgcagg caacaagcta actttt 36

<210> 92
<211> 36
<212> DNA
<213> Homo sapiens

<400> 92
tgtgcctcta aagctgcagg caacaagcta actttt 36

<210> 93
<211> 36
<212> DNA
<213> Homo sapiens

<400> 93
tgtgcctcca aagctgcagg caacaagcta actttt 36

<210> 94
<211> 36
<212> DNA
<213> Homo sapiens

<400> 94
tgtagtcgga gggctgcagg caacaagcta actttt 36

<210> 95
<211> 39
<212> DNA
<213> Homo sapiens

<400> 95
tgtgccgaat atggtggtgc tacaaacaag ctcatcttt 39

<210> 96
<211> 39
<212> DNA
<213> Homo sapiens

<400> 96
tgtgccaact atggcggtgc tacaaacaag ctcatcttt 39

<210> 97
<211> 39
<212> DNA
<213> Homo sapiens

<400> 97
tgtgcccctt atggtggtgc tacaaacaag ctcatcttt 39

<210> 98
<211> 39
<212> DNA
<213> Homo sapiens

<400> 98
tgtgctcctt atggcggtgc tacaaacaag ctcatcttt 39

<210> 99
<211> 39
<212> DNA
<213> Homo sapiens

<400> 99
tgtgccccgt atggtggtgc tacaaacaag ctcatcttt 39

<210> 100
<211> 39
<212> DNA
<213> Homo sapiens

<400> 100
tgtgcccgtt atggtggtgc tacaaacaag ctcatcttt 39

<210> 101
<211> 39
<212> DNA
<213> Homo sapiens

<400> 101
tgtgctcgtt atggtggtgc tacaaacaag ctcatcttt 39

<210> 102
<211> 39
<212> DNA
<213> Homo sapiens

<400> 102
tgtgctcggt atggtggtgc tacaaacaag ctcatcttt 39

<210> 103
<211> 39
<212> DNA
<213> Homo sapiens

<400> 103
tgtgcctctt atggtggtgc tacaaacaag ctcatcttt 39

<210> 104
<211> 39
<212> DNA
<213> Homo sapiens

<400> 104
tgtgcctcct atggtggtgc tacaaacaag ctcatcttt 39

<210> 105
<211> 39
<212> DNA
<213> Homo sapiens

<400> 105
tgtgcctcgt atggtggtgc tacaaacaag ctcatcttt 39

<210> 106
<211> 39
<212> DNA
<213> Homo sapiens

<400> 106
tgtgcctcct atggcggtgc tacaaacaag ctcatcttt 39

<210> 107
<211> 30
<212> DNA
<213> Homo sapiens

<400> 107
tgtgccttcg acaaccgtaa gctgatttgg 30

<210> 108
<211> 30
<212> DNA
<213> Homo sapiens

<400> 108
tgtgcctttg acaaccgtaa gctgatttgg 30

<210> 109
<211> 36
<212> DNA
<213> Homo sapiens

<400> 109
tgtgcctttc gtaatgcagg caacatgctc accttt 36

<210> 110
<211> 36
<212> DNA
<213> Homo sapiens

<400> 110
tgtgcctttc ggaatgcagg caacatgctc accttt 36

<210> 111
<211> 36
<212> DNA
<213> Homo sapiens

<400> 111
tgtgccttta ggaatgcagg caacatgctc accttt 36

<210> 112
<211> 36
<212> DNA
<213> Homo sapiens

<400> 112
tgtgccttta gaaatgcagg caacatgctc accttt 36

<210> 113
<211> 36
<212> DNA
<213> Homo sapiens

<400> 113
tgtgccctca ataatgcagg caacatgctc accttt 36

<210> 114
<211> 36
<212> DNA
<213> Homo sapiens

<400> 114
tgtgccttaa ataatgcagg caacatgctc accttt 36

<210> 115
<211> 36
<212> DNA
<213> Homo sapiens

<400> 115
tgtgccctgc ggaatgcagg caacatgctc accttt 36

<210> 116
<211> 36
<212> DNA
<213> Homo sapiens

<400> 116
tgtgccttaa gaaatgcagg caacatgctc accttt 36

<210> 117
<211> 36
<212> DNA
<213> Homo sapiens

<400> 117
tgtgccttac gaaatgcagg caacatgctc accttt 36

<210> 118
<211> 36
<212> DNA
<213> Homo sapiens

<400> 118
tgtgcgacgc gccgtgcagg caacatgctc accttt 36

<210> 119
<211> 39
<212> DNA
<213> Homo sapiens

<400> 119
tgtgcctccg agtccacggg agcccagaag ctggtattt 39

<210> 120
<211> 36
<212> DNA
<213> Homo sapiens

<400> 120
tgtgcctacg aggggcatc tgaaaagctg gtcttt 36

<210> 121
<211> 39
<212> DNA
<213> Homo sapiens

<400> 121
tgtgccagct caggacagac gaacactgaa gctttcttt 39

<210> 122
<211> 45
<212> DNA
<213> Homo sapiens

<400> 122
tgtgccagca gtgaaggggc ggctggcaat cagccccagc atttt 45

<210> 123
<211> 45
<212> DNA
<213> Homo sapiens

<400> 123
tgtgccagca gcgaggggc ggcgggcaat cagccccagc atttt 45

<210> 124
<211> 45
<212> DNA
<213> Homo sapiens

<400> 124
tgtgccagca gtgagttgac tagcgggggg gatgagcagt tcttc 45

<210> 125
<211> 45
<212> DNA
<213> Homo sapiens

<400> 125
tgtgcaagca gccccgggac tagcggagtt ggtgagcagt tcttc 45

<210> 126
<211> 45
<212> DNA
<213> Homo sapiens

<400> 126
tgtgcaagca gccccgggac tagcggagtt ggtgagcagt ttttc 45

<210> 127
<211> 45
<212> DNA
<213> Homo sapiens

<400> 127
tgtgccagca gtcccgggac tagcggggtt ggtgagcagt tcttc 45

<210> 128
<211> 42
<212> DNA
<213> Homo sapiens

<400> 128
tgtgccagtg tattaatgag gacgaacaat gagcagttct tc 42

<210> 129
<211> 45
<212> DNA
<213> Homo sapiens

<400> 129
tgtgccagca gtgcgttagc tagcggtaca gatacgcagt atttt 45

<210> 130
<211> 45
<212> DNA
<213> Homo sapiens

<400> 130
tgtgccagca gtgctttggc tagcggcaca gatacgcagt atttt 45

<210> 131
<211> 45
<212> DNA
<213> Homo sapiens

<400> 131
tgtgccagca gtgccctggc tagtggcaca gatacgcagt atttt 45

<210> 132
<211> 48
<212> DNA
<213> Homo sapiens

<400> 132
tgtgccagca gtgcaaggac tagcgggggg gccgatacgc agtatttt 48

<210> 133
<211> 48
<212> DNA
<213> Homo sapiens

<400> 133
tgtgccagca gtgccaggac tagcgggggg gcagatacgc agtatttt 48

<210> 134
<211> 48
<212> DNA
<213> Homo sapiens

<400> 134
tgtgccagca gtgctaggac tagcgggggg gcagatacgc agtatttt 48

<210> 135
<211> 48
<212> DNA
<213> Homo sapiens

<400> 135
tgtgccagca gtgccaggac tagcgggggc tcggatacgc agtatttt 48

<210> 136
<211> 48
<212> DNA
<213> Homo sapiens

<400> 136
tgtgccagca gtgctcggac tagcgggggg tcagatacgc agtatttt 48

<210> 137
<211> 48
<212> DNA
<213> Homo sapiens

<400> 137
tgtgccagca gcgcccggac tagcgggggg tcagatacgc agtatttt 48

<210> 138
<211> 48
<212> DNA
<213> Homo sapiens

<400> 138
tgtgccagca gtgccaggac tagcgggggg tcagatacgc agtatttt 48

<210> 139
<211> 48
<212> DNA
<213> Homo sapiens

<400> 139
tgtgccagca gtgccaggac tagcggaggc acagatacgc agtatttt 48

<210> 140
<211> 48
<212> DNA
<213> Homo sapiens

<400> 140
tgtgccagca gtgctaggac tagcgggggc acagatacgc agtatttt 48

<210> 141
<211> 45
<212> DNA
<213> Homo sapiens

<400> 141
tgtgccagca gtcacagggc ctcaggcggg gatacgcagt atttt 45

<210> 142
<211> 48
<212> DNA
<213> Homo sapiens

<400> 142
tgtgccagca gcctaaggac tagcgggggt tcagatacgc agtatttt 48

<210> 143
<211> 48
<212> DNA
<213> Homo sapiens

<400> 143
tgtgccagca gccttaggac tagcgggggt tcagatacgc agtatttt 48

<210> 144
<211> 48
<212> DNA
<213> Homo sapiens

<400> 144
tgtgccagca gtcttaggac tagcgggggc acagatacgc agtatttt 48

<210> 145
<211> 48
<212> DNA
<213> Homo sapiens

<400> 145
tgtgccagca gtctccggac tagcgggggc acagatacgc agtatttt 48

<210>  146
<211>  48
<212>  DNA
<213>  Homo sapiens

<400>  146
tgtgccagca gtcttcggac tagcgggga acagatacgc agtatttt    48

<210>  147
<211>  45
<212>  DNA
<213>  Homo sapiens

<400>  147
tgtgccagca gtccgttgac tagcggaaca gatacgcagt atttt    45

<210>  148
<211>  45
<212>  DNA
<213>  Homo sapiens

<400>  148
tgtgccagca gtccattgac tagcggcaca gatacgcagt atttt    45

<210>  149
<211>  48
<212>  DNA
<213>  Homo sapiens

<400>  149
tgtgccagca gtcgtaggac tagcgggggc acagatacgc agtatttt    48

<210>  150
<211>  48
<212>  DNA
<213>  Homo sapiens

<400>  150
tgtgccagca gtcgacggac tagcgggggc acagatacgc agtatttt    48

<210>  151
<211>  48
<212>  DNA
<213>  Homo sapiens

<400>  151
tgtgccagct cacgacggac tagcgggggc acagatacgc agtatttt    48

<210>  152
<211>  48
<212>  DNA
<213>  Homo sapiens

<400>  152
tgtgccagca gccgccggac tagcgggggc acagatacgc agtatttt    48

<210> 153
<211> 48
<212> DNA
<213> Homo sapiens

<400> 153
tgtgccagca gtcggaggac tagcgggggc acagatacgc agtatttt 48

<210> 154
<211> 45
<212> DNA
<213> Homo sapiens

<400> 154
tgtgccagct caaaactggc tagcggggcc gacgagcagt acttc 45

<210> 155
<211> 45
<212> DNA
<213> Homo sapiens

<400> 155
tgtgccagct caaaactggc tagcggggcc gacgagcagt atttc 45

<210> 156
<211> 45
<212> DNA
<213> Homo sapiens

<400> 156
tgtgccagca gtccccggac tagcgggggc gacgagcagt acttc 45

<210> 157
<211> 45
<212> DNA
<213> Homo sapiens

<400> 157
tgtgccagca gcccccggac tagcgggggg gacgagcagt acttc 45

<210> 158
<211> 45
<212> DNA
<213> Homo sapiens

<400> 158
tgtgccagca gtccccggac tagcggtacc tacgagcagt acttc 45

<210> 159
<211> 45
<212> DNA
<213> Homo sapiens

<400> 159
tgtgccagca gtcctaggac tagcggaacc tacgagcagt acttc 45

<210> 160
<211> 45
<212> DNA
<213> Homo sapiens

<400> 160
tgtgccagca gccgtaggac tagcgggact tacgagcagt acttc 45

<210> 161
<211> 45
<212> DNA
<213> Homo sapiens

<400> 161
tgtgccagca gtagacggac tagcgggacc tacgagcagt acttc 45

<210> 162
<211> 39
<212> DNA
<213> Artificial sequence

<220>
<223> forward primer trav24

<220>
<221> source
<222> (1)..(39)
<223> forward primer trav24

<400> 162
cggctagccg ccaccatgga gaagaatcct ttggcagcc 39

<210> 163
<211> 31
<212> DNA
<213> Artificial sequence

<220>
<223> reverse primer trac

<220>
<221> source
<222> (1)..(31)
<223> reverse primer trac

<400> 163
ttagcggccg cgctggacca cagccgcagc g 31

<210> 164
<211> 34
<212> DNA
<213> Artificial sequence

<220>
<223> forward primer trbv2

<220>
<221> source
<222> (1)..(34)
<223> forward primer trbv2

<400> 164
ggtccggaat ggatacctgg ctcgtatgct gggc 34

<210> 165
<211> 37
<212> DNA
<213> Artificial sequence

<220>
<223> reverse primer trbc

<220>
<221> source
<222> (1)..(37)
<223> reverse primer trbc

<400> 165
ccggtcgacc tagcctctgg aatcctttct cttgacc 37

<210> 166
<211> 20
<212> DNA
<213> Artificial sequence

<220>
<223> forward primer trav24

<220>
<221> source
<222> (1)..(20)
<223> forward primer trav24

<400> 166
ccgaggcctt gtttgtaatg 20

<210> 167
<211> 22
<212> DNA
<213> Artificial sequence

<220>
<223> reverse primer trac

<220>
<221> source
<222> (1)..(22)
<223> reverse primer trac

<400> 167
gtgaataggc agacagactt gt 22

<210> 168
<211> 18
<212> PRT
<213> Artificial sequence

<220>
<223> T2A peptide

<220>
<221> MISC_FEATURE
<222> (1)..(18)
<223> T2A peptide

<400> 168

Glu Gly Arg Gly Ser Leu Leu Thr Cys Gly Asp Val Glu Glu Asn Pro
1               5                   10                  15

Gly Pro

<210> 169
<211> 19
<212> PRT
<213> Artificial sequence

<220>
<223> P2A peptide

<220>
<221> MISC_FEATURE
<222> (1)..(19)
<223> P2A peptide

<400> 169

Ala Thr Asn Phe Ser Leu Leu Lys Gln Ala Gly Asp Val Glu Glu Asn
1               5                   10                  15

Pro Gly Pro

<210> 170
<211> 20
<212> PRT
<213> Artificial sequence

<220>
<223> E2A peptide

<220>
<221> MISC_FEATURE
<222> (1)..(20)
<223> E2A peptide

<400> 170

Gln Cys Thr Asn Tyr Ala Leu Leu Lys Leu Ala Gly Asp Val Glu Ser
1 5 10 15

Asn Pro Gly Pro
20

<210> 171
<211> 22
<212> PRT
<213> Artificial sequence

<220>
<223> F2A peptide

<220>
<221> MISC_FEATURE
<222> (1)..(22)
<223> F2A peptide

<400> 171

Val Lys Gln Thr Leu Asn Phe Asp Leu Leu Lys Leu Ala Gly Asp Val
1 5 10 15

Glu Ser Asn Pro Gly Pro
20

<210> 172
<211> 36
<212> DNA
<213> Homo sapiens

<400> 172
tgtgccttta aagctgcagg caacaagcta actttt 36

<210> 173
<211> 36
<212> DNA
<213> Homo sapiens

<400> 173
tgtgccttta aggctgcagg caacaagcta actttt 36

<210> 174
<211> 36
<212> DNA
<213> Homo sapiens

<400> 174
tgtgccttca aagctgcagg caacaagcta actttt 36

<210> 175
<211> 36

<212> DNA
<213> Homo sapiens

<400> 175
tgtgcattta aagctgcagg caacaagcta actttt 36

<210> 176
<211> 10
<212> PRT
<213> Homo sapiens

<400> 176

Cys Ala Arg Asp Asp Arg Lys Leu Ile Trp
1 5 10

<210> 177
<211> 12
<212> PRT
<213> Homo sapiens

<400> 177

Cys Ala Phe Thr Ala Ala Gly Asn Lys Leu Thr Phe
1 5 10

<210> 178
<211> 12
<212> PRT
<213> Homo sapiens

<400> 178

Cys Ala Leu Gly Asn Ala Gly Asn Met Leu Thr Phe
1 5 10

<210> 179
<211> 13
<212> PRT
<213> Homo sapiens

<400> 179

Cys Ala Phe Ile Pro Gly Gly Ser Tyr Ile Pro Thr Phe
1 5 10

<210> 180
<211> 13
<212> PRT
<213> Homo sapiens

<400> 180

Cys Ala Ser Tyr Gly Gly Ala Thr Asn Lys Leu Ile Phe
1 5 10

<210> 181

<211> 14
<212> PRT
<213> Homo sapiens

<400> 181

Cys Ala Leu Asp Ser Gly Gly Gly Ala Asp Gly Leu Thr Phe
1 5 10

<210> 182
<211> 12
<212> PRT
<213> Homo sapiens

<400> 182

Cys Ala Phe Lys Ala Ala Gly Asn Lys Leu Thr Phe
1 5 10

<210> 183
<211> 12
<212> PRT
<213> Homo sapiens

<400> 183

Cys Ala Arg Arg Asn Ala Gly Asn Met Leu Thr Phe
1 5 10

<210> 184
<211> 12
<212> PRT
<213> Homo sapiens

<400> 184

Cys Ala Ser Lys Ala Ala Gly Asn Lys Leu Thr Phe
1 5 10

<210> 185
<211> 12
<212> PRT
<213> Homo sapiens

<400> 185

Cys Ala Leu Lys Ala Ala Gly Asn Lys Leu Thr Phe
1 5 10

<210> 186
<211> 12
<212> PRT
<213> Homo sapiens

<400> 186

Cys Ala Leu Arg Gln Ala Gly Asn Met Leu Thr Phe
1 5 10

<210> 187
<211> 11
<212> PRT
<213> Homo sapiens

<400> 187

Cys Ala Pro Gly Gly Tyr Gln Lys Val Thr Phe
1 5 10

<210> 188
<211> 11
<212> PRT
<213> Homo sapiens

<400> 188

Cys Ala Trp Gly Ser Ala Arg Gln Leu Thr Phe
1 5 10

<210> 189
<211> 12
<212> PRT
<213> Homo sapiens

<400> 189

Cys Ala Phe Lys Ala Ala Gly Asn Lys Leu Thr Phe
1 5 10

<210> 190
<211> 12
<212> PRT
<213> Homo sapiens

<400> 190

Cys Ala Phe Asn Ala Ala Gly Asn Lys Leu Thr Phe
1 5 10

<210> 191
<211> 12
<212> PRT
<213> Homo sapiens

<400> 191

Cys Ala Phe Val Ala Ala Gly Gln Asn Phe Val Phe
1 5 10

<210> 192
<211> 13
<212> PRT
<213> Homo sapiens

<400> 192

Cys Ala Glu Tyr Gly Gly Ala Thr Asn Lys Leu Ile Phe
1 5 10

<210> 193
<211> 13
<212> PRT
<213> Homo sapiens

<400> 193

Cys Ala Asn Tyr Gly Gly Ala Thr Asn Lys Leu Ile Phe
1 5 10

<210> 194
<211> 13
<212> PRT
<213> Homo sapiens

<400> 194

Cys Ala Ser Tyr Gly Gly Ala Thr Asn Lys Leu Ile Phe
1 5 10

<210> 195
<211> 14
<212> PRT
<213> Homo sapiens

<400> 195

Cys Ala Leu Met Thr Thr Asp Ser Trp Gly Lys Leu Gln Phe
1 5 10

<210> 196
<211> 13
<212> PRT
<213> Artificial sequence

<220>
<223> Motif disclosed in Table 2

<220>
<221> MISC_FEATURE
<222> (1)..(13)
<223> Motif disclosed in Table 2

<220>
<221> MISC_FEATURE
<222> (4)..(4)
<223> Xaa is any amino-acid

<220>
<221> MISC_FEATURE
<222> (5)..(5)
<223> Xaa is Arg or Gly or Leu

<220>

<221> MISC_FEATURE
<222> (6)..(6)
<223> Xaa is Thr or Ala

<220>
<221> MISC_FEATURE
<222> (7)..(7)
<223> Xaa is Ser or Gly

<220>
<221> MISC_FEATURE
<222> (9)..(9)
<223> Xaa is any amino-acid

<220>
<221> MISC_FEATURE
<222> (10)..(10)
<223> Xaa is any amino-acid

<220>
<221> MISC_FEATURE
<222> (11)..(11)
<223> Xaa is Glu or Thr

<220>
<221> MISC_FEATURE
<222> (13)..(13)
<223> Xaa is Phe or Tyr

<400> 196

```
Ala Ser Ser Xaa Xaa Xaa Xaa Gly Xaa Xaa Xaa Gln Xaa
1               5                   10
```

<210> 197
<211> 14
<212> PRT
<213> Artificial sequence

<220>
<223> Motif disclosed in Table 2

<220>
<221> MISC_FEATURE
<222> (1)..(14)
<223> Motif disclosed in Table 2

<220>
<221> MISC_FEATURE
<222> (4)..(4)
<223> Xaa is any amino-acid

<220>
<221> MISC_FEATURE
<222> (5)..(5)
<223> Xaa is Arg or Gly or Leu

<220>
<221> MISC_FEATURE
<222> (6)..(6)
<223> Xaa is Thr or Ala

<220>
<221> MISC_FEATURE
<222> (10)..(10)
<223> Xaa is any amino-acid

<220>
<221> MISC_FEATURE
<222> (11)..(11)
<223> Xaa is any amino-acid

<220>
<221> MISC_FEATURE
<222> (12)..(12)
<223> Xaa is Glu or Thr

<220>
<221> MISC_FEATURE
<222> (14)..(14)
<223> Xaa is Phe or Tyr

<400> 197

Ala Ser Ser Xaa Xaa Xaa Ser Gly Gly Xaa Xaa Xaa Gln Xaa
1 5 10

<210> 198
<211> 10
<212> PRT
<213> Homo sapiens

<400> 198

Cys Ala Arg Asp Asp Arg Lys Leu Ile Trp
1 5 10

<210> 199
<211> 11
<212> PRT
<213> Homo sapiens

<400> 199

Cys Ala Ser Gly Asn Thr Asp Lys Leu Ile Phe
1 5 10

<210> 200
<211> 12
<212> PRT
<213> Homo sapiens

<400> 200

Cys Ala Phe Cys Asn Ala Gly Asn Met Leu Thr Phe
1 5 10

<210> 201
<211> 12
<212> PRT

<213> Homo sapiens

<400> 201

Cys Ala Phe Lys Ala Ala Gly Asn Lys Leu Thr Phe
1 5 10

<210> 202
<211> 12
<212> PRT
<213> Homo sapiens

<400> 202

Cys Ala Phe Thr Ala Ala Gly Asn Lys Leu Thr Phe
1 5 10

<210> 203
<211> 12
<212> PRT
<213> Homo sapiens

<400> 203

Cys Ala Leu Glu Asn Ala Gly Asn Lys Leu Thr Phe
1 5 10

<210> 204
<211> 12
<212> PRT
<213> Homo sapiens

<400> 204

Cys Ala Leu Gly Asn Ala Gly Asn Met Leu Thr Phe
1 5 10

<210> 205
<211> 12
<212> PRT
<213> Homo sapiens

<400> 205

Cys Ser Pro Gln Gly Gly Ser Glu Lys Leu Val Phe
1 5 10

<210> 206
<211> 13
<212> PRT
<213> Homo sapiens

<400> 206

Cys Ala Phe Ile Pro Gly Gly Ser Tyr Ile Pro Thr Phe
1 5 10

<210> 207
<211> 13
<212> PRT
<213> Homo sapiens

<400> 207

Cys Ala Ser Asp Gly Gly Ser Gln Gly Asn Leu Ile Phe
1 5 10

<210> 208
<211> 13
<212> PRT
<213> Homo sapiens

<400> 208

Cys Ala Ser Tyr Gly Gly Ala Thr Asn Lys Leu Ile Phe
1 5 10

<210> 209
<211> 12
<212> PRT
<213> Homo sapiens

<400> 209

Cys Ala Phe Lys Ala Ala Gly Asn Lys Leu Thr Phe
1 5 10

<210> 210
<211> 12
<212> PRT
<213> Homo sapiens

<400> 210

Cys Ala Phe Lys Val Ala Gly Asn Lys Leu Thr Phe
1 5 10

<210> 211
<211> 12
<212> PRT
<213> Homo sapiens

<400> 211

Cys Ala Phe Arg Met Ala Gly Asn Met Leu Thr Phe
1 5 10

<210> 212
<211> 12
<212> PRT
<213> Homo sapiens

<400> 212

Cys Ala Phe Arg Asn Ala Gly Asn Met Leu Thr Phe

1 5 10

<210> 213
<211> 12
<212> PRT
<213> Homo sapiens

<400> 213

Cys Ala His Lys Ala Ala Gly Asn Lys Leu Thr Phe
1 5 10

<210> 214
<211> 12
<212> PRT
<213> Homo sapiens

<400> 214

Cys Ala Pro Ile Asn Ala Gly Asn Met Leu Thr Phe
1 5 10

<210> 215
<211> 12
<212> PRT
<213> Homo sapiens

<400> 215

Cys Ala Arg Lys Ala Ala Gly Asn Lys Leu Thr Phe
1 5 10

<210> 216
<211> 12
<212> PRT
<213> Homo sapiens

<400> 216

Cys Ala Ser Lys Ala Ala Gly Asn Lys Leu Thr Phe
1 5 10

<210> 217
<211> 12
<212> PRT
<213> Homo sapiens

<400> 217

Cys Ala Ser Arg Thr Ala Gly Asn Lys Leu Thr Phe
1 5 10

<210> 218
<211> 11
<212> PRT
<213> Homo sapiens

<400> 218

Cys Ala Pro Lys Ser Arg Gln Gln Ala Asn Phe
1 5 10

<210> 219
<211> 12
<212> PRT
<213> Homo sapiens

<400> 219

Cys Ala Phe Lys Ala Ala Gly Asn Lys Leu Thr Phe
1 5 10

<210> 220
<211> 12
<212> PRT
<213> Homo sapiens

<400> 220

Cys Ala Phe Arg Ala Ala Gly Asn Lys Leu Thr Phe
1 5 10

<210> 221
<211> 12
<212> PRT
<213> Homo sapiens

<400> 221

Cys Ala Leu Lys Ala Ala Gly Asn Lys Leu Thr Phe
1 5 10

<210> 222
<211> 12
<212> PRT
<213> Homo sapiens

<400> 222

Cys Ala Leu Lys Gln Ala Gly Asn Lys Leu Thr Phe
1 5 10

<210> 223
<211> 12
<212> PRT
<213> Homo sapiens

<400> 223

Cys Ala Leu Arg Gln Ala Gly Asn Met Leu Thr Phe
1 5 10

<210> 224
<211> 12

<212> PRT
<213> Homo sapiens

<400> 224

Cys Ala Met Lys Ala Ala Gly Asn Lys Leu Thr Phe
1 5 10

<210> 225
<211> 12
<212> PRT
<213> Homo sapiens

<400> 225

Cys Ala Asn Arg Ala Ala Gly Asn Lys Leu Thr Phe
1 5 10

<210> 226
<211> 12
<212> PRT
<213> Homo sapiens

<400> 226

Cys Ala Ser Lys Ala Ala Gly Asn Lys Leu Thr Phe
1 5 10

<210> 227
<211> 12
<212> PRT
<213> Homo sapiens

<400> 227

Cys Ala Tyr Lys Ala Ala Gly Asn Lys Leu Thr Phe
1 5 10

<210> 228
<211> 12
<212> PRT
<213> Homo sapiens

<400> 228

Cys Gly Phe Lys Ala Ala Gly Asn Lys Leu Thr Phe
1 5 10

<210> 229
<211> 12
<212> PRT
<213> Homo sapiens

<400> 229

Cys Gly Trp Lys Ala Ala Gly Asn Lys Leu Thr Phe
1 5 10

<210> 230
<211> 12
<212> PRT
<213> Homo sapiens

<400> 230

Cys Ser Leu Lys Ala Ala Gly Asn Lys Leu Thr Phe
1 5 10

<210> 231
<211> 12
<212> PRT
<213> Homo sapiens

<400> 231

Cys Ser Leu Arg Ala Ala Gly Asn Lys Leu Thr Phe
1 5 10

<210> 232
<211> 12
<212> PRT
<213> Homo sapiens

<400> 232

Cys Ser Arg Arg Ala Ala Gly Asn Lys Leu Thr Phe
1 5 10

<210> 233
<211> 12
<212> PRT
<213> Homo sapiens

<400> 233

Cys Ser Trp Lys Ala Ala Gly Asn Lys Leu Thr Phe
1 5 10

<210> 234
<211> 12
<212> PRT
<213> Homo sapiens

<400> 234

Cys Ser Trp Arg Ala Ala Gly Asn Lys Leu Thr Phe
1 5 10

<210> 235
<211> 12
<212> PRT
<213> Homo sapiens

<400> 235

Cys Thr Lys Lys Ala Ala Gly Asn Lys Leu Thr Phe
1 5 10

<210> 236
<211> 12
<212> PRT
<213> Homo sapiens

<400> 236

Cys Thr Leu Lys Ala Ala Gly Asn Lys Leu Thr Phe
1 5 10

<210> 237
<211> 12
<212> PRT
<213> Homo sapiens

<400> 237

Cys Thr Asn Lys Ala Ala Gly Asn Lys Leu Thr Phe
1 5 10

<210> 238
<211> 12
<212> PRT
<213> Homo sapiens

<400> 238

Cys Thr Arg Lys Ala Ala Gly Asn Lys Leu Thr Phe
1 5 10

<210> 239
<211> 12
<212> PRT
<213> Homo sapiens

<400> 239

Cys Ala Phe Gln Asn Ala Gly Asn Met Leu Thr Phe
1 5 10

<210> 240
<211> 12
<212> PRT
<213> Homo sapiens

<400> 240

Cys Ala Phe Arg Ala Ala Gly Asn Lys Leu Thr Phe
1 5 10

<210> 241
<211> 12
<212> PRT
<213> Homo sapiens

<400> 241

Cys Ala Thr Arg Arg Ala Gly Asn Met Leu Thr Phe
1 5 10

<210> 242
<211> 12
<212> PRT
<213> Homo sapiens

<400> 242

Cys Ala Tyr Glu Gly Ala Ser Glu Lys Leu Val Phe
1 5 10

<210> 243
<211> 13
<212> PRT
<213> Homo sapiens

<400> 243

Cys Ala Glu Tyr Gly Gly Ala Thr Asn Lys Leu Ile Phe
1 5 10

<210> 244
<211> 13
<212> PRT
<213> Homo sapiens

<400> 244

Cys Ala Pro Tyr Gly Gly Ala Thr Asn Lys Leu Ile Phe
1 5 10

<210> 245
<211> 13
<212> PRT
<213> Homo sapiens

<400> 245

Cys Ala Arg Tyr Gly Gly Ala Ala Asn Lys Leu Ile Phe
1 5 10

<210> 246
<211> 13
<212> PRT
<213> Homo sapiens

<400> 246

Cys Ala Arg Tyr Gly Gly Ala Thr Asn Lys Leu Ile Phe
1 5 10

<210> 247

<211> 13
<212> PRT
<213> Homo sapiens

<400> 247

Cys Ala Arg Tyr Gly Gly Gly Thr Asn Lys Leu Ile Phe
1 5 10

<210> 248
<211> 13
<212> PRT
<213> Homo sapiens

<400> 248

Cys Ala Ser Glu Ser Thr Gly Ala Gln Lys Leu Val Phe
1 5 10

<210> 249
<211> 13
<212> PRT
<213> Homo sapiens

<400> 249

Cys Ala Ser Tyr Gly Gly Ala Thr Asn Lys Leu Ile Phe
1 5 10

<210> 250
<211> 13
<212> PRT
<213> Homo sapiens

<400> 250

Cys Ala Ser Tyr Val Gly Ala Thr Asn Lys Leu Ile Phe
1 5 10

<210> 251
<211> 10
<212> PRT
<213> Homo sapiens

<400> 251

Cys Ala Phe Asp Asn Arg Lys Leu Ile Trp
1 5 10

<210> 252
<211> 12
<212> PRT
<213> Homo sapiens

<400> 252

Cys Ala Phe Lys Ala Ala Gly Asn Lys Leu Thr Phe
1 5 10

<210> 253
<211> 12
<212> PRT
<213> Homo sapiens

<400> 253

Cys Ala Phe Arg Ala Ala Gly Asn Lys Leu Thr Phe
1 5 10

<210> 254
<211> 12
<212> PRT
<213> Homo sapiens

<400> 254

Cys Ala Ser Glu Thr Gly Ala Asn Asn Leu Phe Phe
1 5 10

<210> 255
<211> 12
<212> PRT
<213> Homo sapiens

<400> 255

Cys Ala Thr Arg Arg Ala Gly Asn Met Leu Thr Phe
1 5 10

<210> 256
<211> 12
<212> PRT
<213> Homo sapiens

<400> 256

Cys Ala Tyr Glu Gly Ala Ser Glu Lys Leu Val Phe
1 5 10

<210> 257
<211> 13
<212> PRT
<213> Homo sapiens

<400> 257

Cys Ala Glu Tyr Gly Gly Ala Thr Asn Lys Leu Ile Phe
1 5 10

<210> 258
<211> 13
<212> PRT
<213> Homo sapiens

<400> 258

Cys Ala Phe Ala Ser Gly Ser Ala Arg Gln Leu Thr Phe
1 5 10

<210> 259
<211> 13
<212> PRT
<213> Homo sapiens

<400> 259

Cys Ala Ser Glu Ser Thr Gly Ala Gln Lys Leu Val Phe
1 5 10

<210> 260
<211> 14
<212> PRT
<213> Homo sapiens

<400> 260

Cys Ala Phe Tyr Pro Pro Gly Asn Asn Arg Lys Leu Ile Trp
1 5 10

<210> 261
<211> 12
<212> PRT
<213> Homo sapiens

<400> 261

Cys Ala Phe Lys Ala Ala Gly Asn Lys Leu Thr Phe
1 5 10

<210> 262
<211> 12
<212> PRT
<213> Homo sapiens

<400> 262

Cys Ala Phe Lys Asp Ala Gly Asn Lys Leu Thr Phe
1 5 10

<210> 263
<211> 12
<212> PRT
<213> Homo sapiens

<400> 263

Cys Ala Phe Pro Asn Ala Gly Asn Met Leu Thr Phe
1 5 10

<210> 264
<211> 12
<212> PRT

<213> Homo sapiens

<400> 264

Cys Ala Phe Arg Ala Ala Gly Asn Lys Leu Thr Phe
1 5 10

<210> 265
<211> 12
<212> PRT
<213> Homo sapiens

<400> 265

Cys Ala Phe Arg Asn Ala Gly Asn Met Leu Thr Phe
1 5 10

<210> 266
<211> 12
<212> PRT
<213> Homo sapiens

<400> 266

Cys Ala Leu Lys Ala Ala Gly Asn Lys Leu Thr Phe
1 5 10

<210> 267
<211> 12
<212> PRT
<213> Homo sapiens

<400> 267

Cys Ala Leu Lys Asp Ala Gly Asn Lys Leu Thr Phe
1 5 10

<210> 268
<211> 12
<212> PRT
<213> Homo sapiens

<400> 268

Cys Ala Leu Lys Asn Ala Gly Asn Met Leu Thr Phe
1 5 10

<210> 269
<211> 12
<212> PRT
<213> Homo sapiens

<400> 269

Cys Ala Leu Asn Asn Ala Gly Asn Met Leu Thr Phe
1 5 10

<210> 270
<211> 12
<212> PRT
<213> Homo sapiens

<400> 270

Cys Ala Leu Arg Asn Ala Gly Asn Met Leu Thr Phe
1 5 10

<210> 271
<211> 12
<212> PRT
<213> Homo sapiens

<400> 271

Cys Ala Pro Lys Ala Ala Gly Asn Lys Leu Thr Phe
1 5 10

<210> 272
<211> 12
<212> PRT
<213> Homo sapiens

<400> 272

Cys Ala Pro Lys Asp Ala Gly Asn Lys Leu Thr Phe
1 5 10

<210> 273
<211> 12
<212> PRT
<213> Homo sapiens

<400> 273

Cys Ala Ser Lys Asn Ala Gly Asn Met Leu Thr Phe
1 5 10

<210> 274
<211> 12
<212> PRT
<213> Homo sapiens

<400> 274

Cys Ala Ser Met Asn Ala Gly Asn Met Leu Thr Phe
1 5 10

<210> 275
<211> 15
<212> PRT
<213> Homo sapiens

<400> 275

Cys Ala Phe Lys Gly Gly Gly Gly Ser Asn Tyr Lys Leu Thr Phe

1 5 10 15

<210> 276
<211> 10
<212> PRT
<213> Homo sapiens

<400> 276

Cys Ala Phe Gly Asp Tyr Lys Leu Ser Phe
1 5 10

<210> 277
<211> 12
<212> PRT
<213> Homo sapiens

<400> 277

Cys Ala Phe His Ala Ala Gly Asn Lys Leu Thr Phe
1 5 10

<210> 278
<211> 12
<212> PRT
<213> Homo sapiens

<400> 278

Cys Ala Phe Lys Ala Ala Gly Asn Lys Leu Thr Phe
1 5 10

<210> 279
<211> 12
<212> PRT
<213> Homo sapiens

<400> 279

Cys Ala Phe Lys Gly Gly Gly Asn Lys Leu Thr Phe
1 5 10

<210> 280
<211> 12
<212> PRT
<213> Homo sapiens

<400> 280

Cys Ala Phe Asn Ala Ala Gly Asn Lys Leu Thr Phe
1 5 10

<210> 281
<211> 12
<212> PRT
<213> Homo sapiens

<400> 281

Cys Ala Phe Gln Ala Ala Gly Asn Lys Leu Thr Phe
1 5 10

<210> 282
<211> 12
<212> PRT
<213> Homo sapiens

<400> 282

Cys Ala Phe Arg Gly Gly Gly Asn Lys Leu Thr Phe
1 5 10

<210> 283
<211> 12
<212> PRT
<213> Homo sapiens

<400> 283

Cys Ala Phe Arg Arg Ala Gly Asn Met Leu Thr Phe
1 5 10

<210> 284
<211> 12
<212> PRT
<213> Homo sapiens

<400> 284

Cys Ala His Lys Ala Ala Gly Asn Lys Leu Thr Phe
1 5 10

<210> 285
<211> 12
<212> PRT
<213> Homo sapiens

<400> 285

Cys Ala His Lys Gly Ala Gly Asn Lys Leu Thr Phe
1 5 10

<210> 286
<211> 13
<212> PRT
<213> Homo sapiens

<400> 286

Cys Ala Glu Tyr Gly Gly Ala Thr Asn Lys Leu Ile Phe
1 5 10

<210> 287
<211> 13

<212> PRT
<213> Homo sapiens

<400> 287

Cys Ala Asn Tyr Gly Gly Ala Thr Asn Lys Leu Ile Phe
1 5 10

<210> 288
<211> 13
<212> PRT
<213> Homo sapiens

<400> 288

Cys Ala Pro Gly Gly Gly Gly Ser Glu Lys Leu Val Phe
1 5 10

<210> 289
<211> 13
<212> PRT
<213> Homo sapiens

<400> 289

Cys Ala Pro Tyr Gly Gly Ala Thr Asn Lys Leu Ile Phe
1 5 10

<210> 290
<211> 13
<212> PRT
<213> Homo sapiens

<400> 290

Cys Ala Ser Tyr Gly Gly Ala Thr Asn Lys Leu Ile Phe
1 5 10

<210> 291
<211> 11
<212> PRT
<213> Homo sapiens

<400> 291

Cys Gly Gly Asp Asn Asn Ala Arg Leu Met Phe
1 5 10

<210> 292
<211> 11
<212> PRT
<213> Homo sapiens

<400> 292

Cys Gly Gly Asp Asn Asn Ala Arg Leu Thr Phe
1 5 10

<210> 293
<211> 12
<212> PRT
<213> Homo sapiens

<400> 293

Cys Ala Ser Leu Tyr Asn Thr Asp Lys Leu Ile Phe
1 5 10

<210> 294
<211> 13
<212> PRT
<213> Homo sapiens

<400> 294

Cys Ala Asn Tyr Gly Gly Ala Thr Asn Lys Leu Ile Phe
1 5 10

<210> 295
<211> 10
<212> PRT
<213> Homo sapiens

<400> 295

Cys Ala Arg Asp Asp Arg Lys Leu Ile Trp
1 5 10

<210> 296
<211> 12
<212> PRT
<213> Homo sapiens

<400> 296

Cys Ala Phe Thr Ala Ala Gly Asn Lys Leu Thr Phe
1 5 10

<210> 297
<211> 12
<212> PRT
<213> Homo sapiens

<400> 297

Cys Ala Leu Gly Asn Ala Gly Asn Met Leu Thr Phe
1 5 10

<210> 298
<211> 13
<212> PRT
<213> Homo sapiens

<400> 298

Cys Ala Phe Ile Pro Gly Gly Ser Tyr Ile Pro Thr Phe
1 5 10

<210> 299
<211> 13
<212> PRT
<213> Homo sapiens

<400> 299

Cys Ala Ser Tyr Gly Gly Ala Thr Asn Lys Leu Ile Phe
1 5 10

<210> 300
<211> 14
<212> PRT
<213> Homo sapiens

<400> 300

Cys Ala Leu Asp Ser Gly Gly Gly Ala Asp Gly Leu Thr Phe
1 5 10

<210> 301
<211> 12
<212> PRT
<213> Homo sapiens

<400> 301

Cys Ala Phe Lys Ala Ala Gly Asn Lys Leu Thr Phe
1 5 10

<210> 302
<211> 12
<212> PRT
<213> Homo sapiens

<400> 302

Cys Ala Arg Arg Asn Ala Gly Asn Met Leu Thr Phe
1 5 10

<210> 303
<211> 12
<212> PRT
<213> Homo sapiens

<400> 303

Cys Ala Ser Lys Ala Ala Gly Asn Lys Leu Thr Phe
1 5 10

<210> 304
<211> 12
<212> PRT
<213> Homo sapiens

<400> 304

Cys Ala Leu Lys Ala Ala Gly Asn Lys Leu Thr Phe
1 5 10

<210> 305
<211> 12
<212> PRT
<213> Homo sapiens

<400> 305

Cys Ala Leu Arg Gln Ala Gly Asn Met Leu Thr Phe
1 5 10

<210> 306
<211> 11
<212> PRT
<213> Homo sapiens

<400> 306

Cys Ala Pro Gly Gly Tyr Gln Lys Val Thr Phe
1 5 10

<210> 307
<211> 11
<212> PRT
<213> Homo sapiens

<400> 307

Cys Ala Trp Gly Ser Ala Arg Gln Leu Thr Phe
1 5 10

<210> 308
<211> 12
<212> PRT
<213> Homo sapiens

<400> 308

Cys Ala Phe Lys Ala Ala Gly Asn Lys Leu Thr Phe
1 5 10

<210> 309
<211> 12
<212> PRT
<213> Homo sapiens

<400> 309

Cys Ala Phe Asn Ala Ala Gly Asn Lys Leu Thr Phe
1 5 10

<210> 310

<211> 12
<212> PRT
<213> Homo sapiens

<400> 310

Cys Ala Phe Val Ala Ala Gly Gln Asn Phe Val Phe
1 5 10

<210> 311
<211> 13
<212> PRT
<213> Homo sapiens

<400> 311

Cys Ala Glu Tyr Gly Gly Ala Thr Asn Lys Leu Ile Phe
1 5 10

<210> 312
<211> 13
<212> PRT
<213> Homo sapiens

<400> 312

Cys Ala Asn Tyr Gly Gly Ala Thr Asn Lys Leu Ile Phe
1 5 10

<210> 313
<211> 13
<212> PRT
<213> Homo sapiens

<400> 313

Cys Ala Ser Tyr Gly Gly Ala Thr Asn Lys Leu Ile Phe
1 5 10

<210> 314
<211> 14
<212> PRT
<213> Homo sapiens

<400> 314

Cys Ala Leu Met Thr Thr Asp Ser Trp Gly Lys Leu Gln Phe
1 5 10

<210> 315
<211> 30
<212> DNA
<213> Homo sapiens

<400> 315
tgtgcccgtg acgaccgtaa gctgatttgg 30

<210> 316
<211> 33
<212> DNA
<213> Homo sapiens

<400> 316
tgtgcctctg gtaacaccga caagctcatc ttt 33

<210> 317
<211> 36
<212> DNA
<213> Homo sapiens

<400> 317
tgtgcctttt gtaatgcagg caacatgctc accttt 36

<210> 318
<211> 36
<212> DNA
<213> Homo sapiens

<400> 318
tgtgccttta aagctgcagg caacaagcta actttt 36

<210> 319
<211> 36
<212> DNA
<213> Homo sapiens

<400> 319
tgtgccttta aggctgcagg caacaagcta actttt 36

<210> 320
<211> 36
<212> DNA
<213> Homo sapiens

<400> 320
tgtgccttta cggctgcagg caacaagcta actttt 36

<210> 321
<211> 36
<212> DNA
<213> Homo sapiens

<400> 321
tgtgccctag aaaatgcagg caacaagcta actttt 36

<210> 322
<211> 36
<212> DNA
<213> Homo sapiens

<400> 322
tgtgccctcg gtaatgcagg caacatgctc accttt 36

<210> 323
<211> 36
<212> DNA
<213> Homo sapiens

<400> 323
tgtgccctcg gtaatgcagg caacatgctc acgttt 36

<210> 324
<211> 36
<212> DNA
<213> Homo sapiens

<400> 324
tgctcgcctc agggcggatc tgaaaagctg gtcttt 36

<210> 325
<211> 39
<212> DNA
<213> Homo sapiens

<400> 325
tgtgccttta tcccaggagg aagctacata cctacattt 39

<210> 326
<211> 39
<212> DNA
<213> Homo sapiens

<400> 326
tgtgcctctg atggaggaag ccaaggcaat ctcatcttt 39

<210> 327
<211> 39
<212> DNA
<213> Homo sapiens

<400> 327
tgtgcctctt atggtggtgc tacaaacaag ctcatcttt 39

<210> 328
<211> 36
<212> DNA
<213> Homo sapiens

<400> 328
tgtgccttca aagctgcagg caacaagcta actttt 36

<210> 329
<211> 36
<212> DNA
<213> Homo sapiens

<400> 329
tgtgccttta aagctgcagg caacaagcta actttt 36

<210> 330
<211> 36
<212> DNA
<213> Homo sapiens

<400> 330
tgtgccttta aggttgcagg caacaagcta actttt 36

<210> 331
<211> 36
<212> DNA
<213> Homo sapiens

<400> 331
tgtgccttta ggatggcagg caacatgctc accttt 36

<210> 332
<211> 36
<212> DNA
<213> Homo sapiens

<400> 332
tgtgcctttc gtaatgcagg caacatgctc accttt 36

<210> 333
<211> 36
<212> DNA
<213> Homo sapiens

<400> 333
tgtgcccaca aagctgcagg caacaagcta actttt 36

<210> 334
<211> 36
<212> DNA
<213> Homo sapiens

<400> 334
tgtgcccata aagctgcagg caacaagcta actttt 36

<210> 335
<211> 36
<212> DNA
<213> Homo sapiens

<400> 335
tgtgccccta taaatgcagg caacatgctc accttt 36

<210> 336
<211> 36
<212> DNA
<213> Homo sapiens

<400> 336
tgtgcccgca aagctgcagg caacaagcta actttt 36

<210> 337
<211> 36
<212> DNA
<213> Homo sapiens

<400> 337
tgtgcctcta aagctgcagg caacaagcta actttt 36

<210> 338
<211> 36
<212> DNA
<213> Homo sapiens

<400> 338
tgtgcctcac gaactgcagg caacaagcta actttt 36

<210> 339
<211> 33
<212> DNA
<213> Homo sapiens

<400> 339
tgtgctccaa agagcaggca acaagctaac ttt 33

<210> 340
<211> 36
<212> DNA
<213> Homo sapiens

<400> 340
tgtgccttca aagctgcagg caacaagcta actttt 36

<210> 341
<211> 36
<212> DNA
<213> Homo sapiens

<400> 341
tgtgccttta aagctgcagg caacaagcta actttt 36

<210> 342
<211> 36
<212> DNA
<213> Homo sapiens

<400> 342
tgtgccttca gggctgcagg caacaagcta actttt 36

<210> 343
<211> 36
<212> DNA
<213> Homo sapiens

<400> 343
tgtgccttga aagctgcagg caacaagcta actttt 36

<210> 344
<211> 36
<212> DNA
<213> Homo sapiens

<400> 344
tgtgccttga aacaagcagg caacaagctc actttt 36

<210> 345
<211> 36
<212> DNA
<213> Homo sapiens

<400> 345
tgtgccttga ggcaagcagg caacatgctc accttt 36

<210> 346
<211> 36
<212> DNA
<213> Homo sapiens

<400> 346
tgtgccatga aagctgcagg caacaagcta actttt 36

<210> 347
<211> 36
<212> DNA
<213> Homo sapiens

<400> 347
tgtgcgatga aagctgcagg caacaagcta actttt 36

<210> 348
<211> 36
<212> DNA
<213> Homo sapiens

<400> 348
tgtgccaaca gagctgcagg caacaagcta actttt 36

<210> 349
<211> 36
<212> DNA
<213> Homo sapiens

<400> 349
tgtgcctcca aagctgcagg caacaagcta actttt 36

<210> 350
<211> 36
<212> DNA
<213> Homo sapiens

<400> 350
tgtgcctaca aagctgcagg caacaagcta actttt 36

<210> 351
<211> 36
<212> DNA
<213> Homo sapiens

<400> 351
tgtggtttca aagctgcagg caacaagcta actttt 36

<210> 352
<211> 36
<212> DNA
<213> Homo sapiens

<400> 352
tgtggttgga aagctgcagg caacaagcta actttt 36

<210> 353
<211> 36
<212> DNA
<213> Homo sapiens

<400> 353
tgtagtttga aagctgcagg caacaagcta actttt 36

<210> 354
<211> 36
<212> DNA
<213> Homo sapiens

<400> 354
tgtagtttga gagctgcagg caacaagcta actttt 36

<210> 355
<211> 36
<212> DNA
<213> Homo sapiens

<400> 355
tgtagtcgga gggctgcagg caacaagcta actttt 36

<210> 356
<211> 36
<212> DNA
<213> Homo sapiens

<400> 356
tgtagttgga aagctgcagg caacaagcta actttt 36

<210> 357
<211> 36
<212> DNA
<213> Homo sapiens

<400> 357
tgtagttgga gagctgcagg caacaagcta actttt 36

<210> 358
<211> 36
<212> DNA
<213> Homo sapiens

<400> 358
tgtagttgga gggctgcagg caacaagcta actttt 36

<210> 359
<211> 36
<212> DNA
<213> Homo sapiens

<400> 359
tgtaccaaga aagctgcagg caacaagcta actttt 36

<210> 360
<211> 36
<212> DNA
<213> Homo sapiens

<400> 360
tgtacgaaga aagctgcagg caacaagcta actttt 36

<210> 361
<211> 36
<212> DNA
<213> Homo sapiens

<400> 361
tgtaccttga aagctgcagg caacaagcta actttt 36

<210> 362
<211> 36
<212> DNA
<213> Homo sapiens

<400> 362
tgtacgaaca aagctgcagg caacaagcta actttt 36

<210> 363
<211> 36
<212> DNA
<213> Homo sapiens

<400> 363
tgtactagga aagctgcagg caacaagcta actttt 36

<210> 364
<211> 36
<212> DNA
<213> Homo sapiens

<400> 364
tgtgccttcc aaaatgcagg caacatgctc accttt 36

<210> 365
<211> 36
<212> DNA
<213> Homo sapiens

<400> 365
tgtgccttta gagctgcagg caacaagcta actttt 36

<210> 366
<211> 36
<212> DNA
<213> Homo sapiens

<400> 366
tgtgcgacgc gccgtgcagg caacatgctc accttt 36

<210> 367
<211> 36
<212> DNA
<213> Homo sapiens

<400> 367
tgtgcctacg agggggcatc tgaaaagctg gtcttt 36

<210> 368
<211> 39
<212> DNA
<213> Homo sapiens

<400> 368
tgtgccgaat atggtggtgc tacaaacaag ctcatcttt 39

<210> 369
<211> 39
<212> DNA
<213> Homo sapiens

<400> 369
tgtgcccctt atggtggtgc tacaaacaag ctcatcttt 39

<210> 370
<211> 39
<212> DNA
<213> Homo sapiens

<400> 370
tgtgcccgtt atggtggtgc tgcaaacaag ctcatcttt 39

<210> 371
<211> 39
<212> DNA
<213> Homo sapiens

<400> 371
tgtgcccgtt atggtggtgc tacaaacaag ctcatcttt 39

<210> 372
<211> 39
<212> DNA
<213> Homo sapiens

<400> 372
tgtgctcgtt atggtggtgc tacaaacaag ctcatcttt 39

<210> 373
<211> 39
<212> DNA
<213> Homo sapiens

<400> 373
tgtgcccgct atggtggtgg tacaaacaag ctcattttt 39

<210> 374
<211> 39
<212> DNA
<213> Homo sapiens

<400> 374
tgtgcctccg agtccacggg agcccagaag ctggtattt 39

<210> 375
<211> 39
<212> DNA
<213> Homo sapiens

<400> 375
tgtgcctcct atggtggtgc tacaaacaag ctcatcttt 39

<210> 376
<211> 39
<212> DNA
<213> Homo sapiens

<400> 376
tgtgcctcgt atggtggtgc tacaaacaag ctcatcttt 39

<210> 377
<211> 39
<212> DNA
<213> Homo sapiens

<400> 377
tgtgcctctt atggtggtgc tacaaacaag ctcatcttt 39

<210> 378
<211> 39
<212> DNA
<213> Homo sapiens

<400> 378
tgtgcctcct atgttggtgc tacaaacaag ctcatcttt 39

<210> 379
<211> 30
<212> DNA
<213> Homo sapiens

<400> 379
tgtgccttcg acaaccgtaa gctgatttgg 30

<210> 380
<211> 30
<212> DNA
<213> Homo sapiens

<400> 380
tgtgcctttg acaaccgtaa gctgatttgg 30

<210> 381
<211> 36
<212> DNA
<213> Homo sapiens

<400> 381
tgtgccttta aggctgcagg caacaagcta actttt 36

<210> 382
<211> 36
<212> DNA
<213> Homo sapiens

<400> 382
tgtgcctttc gagctgcagg caacaagcta actttt 36

<210> 383
<211> 36
<212> DNA
<213> Homo sapiens

<400> 383
tgtgcctcag aaactggggc aaacaacctc ttcttt 36

<210> 384
<211> 36
<212> DNA
<213> Homo sapiens

<400> 384
tgtgcgacgc gccgtgcagg caacatgctc accttt 36

<210> 385
<211> 36
<212> DNA
<213> Homo sapiens

<400> 385
tgtgcctacg agggggcatc tgaaaagctg gtcttt 36

<210> 386
<211> 39
<212> DNA
<213> Homo sapiens

<400> 386
tgtgccgaat atggtggtgc tacaaacaag ctcatcttt 39

<210> 387
<211> 39
<212> DNA
<213> Homo sapiens

<400> 387
tgtgccttcg cttctggttc tgcaaggcaa ctgaccttt 39

<210> 388
<211> 39
<212> DNA
<213> Homo sapiens

<400> 388
tgtgcctccg agtccacggg agcccagaag ctggtattt 39

<210> 389
<211> 42
<212> DNA
<213> Homo sapiens

<400> 389
tgtgcctttt acccccctgg caacaaccgt aagctgattt gg 42

<210> 390
<211> 36
<212> DNA
<213> Homo sapiens

<400> 390
tgtgcattta aagctgcagg caacaagcta actttt 36

<210> 391
<211> 36
<212> DNA
<213> Homo sapiens

<400> 391
tgtgccttca aagctgcagg caacaagcta actttt 36

<210> 392
<211> 36
<212> DNA
<213> Homo sapiens

<400> 392
tgtgcctttta aagctgcagg caacaagcta actttt 36

<210> 393
<211> 36
<212> DNA
<213> Homo sapiens

<400> 393
tgtgccttta aagatgcagg caacaagcta actttt 36

<210> 394
<211> 36
<212> DNA
<213> Homo sapiens

<400> 394
tgtgcctttc ctaatgcagg caacatgctc accttt 36

<210> 395
<211> 36
<212> DNA
<213> Homo sapiens

<400> 395
tgtgccttta gggctgcagg caacaagcta actttt 36

<210> 396
<211> 36
<212> DNA
<213> Homo sapiens

<400> 396
tgtgcctttc ggaatgcagg caacatgctc accttt 36

<210> 397
<211> 36
<212> DNA
<213> Homo sapiens

<400> 397
tgtgctctaa aagctgcagg caacaagcta actttt 36

<210> 398
<211> 36
<212> DNA
<213> Homo sapiens

<400> 398
tgtgctctaa aagatgcagg caacaagcta actttt 36

<210> 399
<211> 36
<212> DNA
<213> Homo sapiens

<400> 399
tgtgccttaa aaaatgcagg caacatgctc accttt 36

<210> 400
<211> 36
<212> DNA
<213> Homo sapiens

<400> 400
tgtgccctca ataatgcagg caacatgctc accttt 36

<210> 401
<211> 36
<212> DNA
<213> Homo sapiens

<400> 401
tgtgccctgc ggaatgcagg caacatgctc accttt 36

<210> 402
<211> 36
<212> DNA
<213> Homo sapiens

<400> 402
tgtgcccccca aagctgcagg caacaagcta actttt 36

<210> 403
<211> 36
<212> DNA
<213> Homo sapiens

<400> 403
tgtgcccccca aagatgcagg caacaagcta actttt 36

<210> 404
<211> 36
<212> DNA
<213> Homo sapiens

<400> 404
tgtgcctcca agaatgcagg caacatgctc accttt 36

<210> 405
<211> 36
<212> DNA
<213> Homo sapiens

<400> 405
tgtgcctcca tgaatgcagg caacatgctc accttt 36

<210> 406
<211> 45
<212> DNA
<213> Homo sapiens

<400> 406
tgtgccttta agggaggtgg aggtagcaac tataaactga cattt 45

<210> 407
<211> 30
<212> DNA
<213> Homo sapiens

<400> 407
tgtgcctttg gcgactacaa gctcagcttt 30

<210> 408
<211> 36
<212> DNA
<213> Homo sapiens

<400> 408
tgtgcctttc acgctgcagg caacaagcta actttt 36

<210> 409
<211> 36
<212> DNA
<213> Homo sapiens

<400> 409
tgtgccttca aagctgcagg caacaagcta actttt 36

<210> 410
<211> 36
<212> DNA
<213> Homo sapiens

<400> 410
tgtgccttta agggaggagg aaacaaactc accttt 36

<210> 411
<211> 36
<212> DNA
<213> Homo sapiens

<400> 411
tgtgccttta acgctgcagg caacaagcta actttt 36

<210> 412
<211> 36
<212> DNA
<213> Homo sapiens

<400> 412
tgtgccttcc aggctgcagg caacaagcta actttt 36

<210> 413
<211> 36
<212> DNA
<213> Homo sapiens

<400> 413
tgtgccttta ggggaggagg aaacaaactc accttt 36

<210> 414
<211> 36
<212> DNA
<213> Homo sapiens

<400> 414
tgtgcctttc gaagagcagg caacatgctc accttt 36

<210> 415
<211> 36
<212> DNA
<213> Homo sapiens

<400> 415
tgtgcccaca aagctgcagg caacaagcta actttt 36

<210> 416
<211> 36
<212> DNA
<213> Homo sapiens

<400> 416
tgtgcccaca aaggagcagg caacaagcta actttt 36

<210> 417
<211> 39
<212> DNA
<213> Homo sapiens

<400> 417
tgtgccgaat atggtggtgc tacaaacaag ctcatcttt 39

<210> 418
<211> 39
<212> DNA
<213> Homo sapiens

<400> 418
tgtgccaact atggcggtgc tacaaacaag ctcatcttt 39

<210> 419
<211> 39
<212> DNA
<213> Homo sapiens

<400> 419
tgtgcccccg gggggggcgg atctgaaaag ctggtcttt 39

<210> 420
<211> 39
<212> DNA
<213> Homo sapiens

<400> 420
tgtgcccctt atggtggtgc tacaaacaag ctcatcttt 39

<210> 421
<211> 39
<212> DNA
<213> Homo sapiens

<400> 421
tgtgctcctt atggcggtgc tacaaacaag ctcatcttt 39

<210> 422
<211> 39
<212> DNA
<213> Homo sapiens

<400> 422
tgtgcctcct atggcggtgc tacaaacaag ctcatcttt 39

<210> 423
<211> 39
<212> DNA
<213> Homo sapiens

<400> 423
tgtgcctcct atggtggtgc tacaaacaag ctcatcttt 39

<210> 424
<211> 39
<212> DNA
<213> Homo sapiens

<400> 424
tgtgcctctt atggtggtgc tacaaacaag ctcatcttt 39

<210> 425
<211> 33
<212> DNA
<213> Homo sapiens

<400> 425
tgtggggggg ataacaatgc cagactcatg ttt 33

<210> 426
<211> 33
<212> DNA
<213> Homo sapiens

<400> 426
tgtggggggg ataacaatgc cagactcacg ttt 33

<210> 427
<211> 36
<212> DNA
<213> Homo sapiens

<400> 427
tgtgcctctc tctataacac cgacaagctc atcttt 36

<210> 428
<211> 39
<212> DNA
<213> Homo sapiens

<400> 428
tgtgccaact atggcggtgc tacaaacaag ctcatcttt 39

<210> 429
<211> 30
<212> DNA
<213> Homo sapiens

<400> 429
tgtgcccgtg acgaccgtaa gctgatttgg 30

<210> 430
<211> 30
<212> DNA
<213> Homo sapiens

<400> 430
tgtgctcgtg acgaccgtaa gctgatttgg 30

<210> 431
<211> 36
<212> DNA
<213> Homo sapiens

<400> 431
tgtgccttta cggctgcagg caacaagcta actttt 36

<210> 432
<211> 36
<212> DNA
<213> Homo sapiens

<400> 432
tgtgccctcg gtaatgcagg caacatgctc accttt 36

<210> 433
<211> 39
<212> DNA
<213> Homo sapiens

<400> 433
tgtgccttta tcccaggagg aagctacata cctacattt 39

<210> 434
<211> 39
<212> DNA
<213> Homo sapiens

<400> 434
tgtgcctcgt atggtggtgc tacaaacaag ctcatcttt 39

<210> 435
<211> 39
<212> DNA
<213> Homo sapiens

<400> 435
tgtgcctctt atggtggtgc tacaaacaag ctcatcttt 39

<210> 436
<211> 42
<212> DNA
<213> Homo sapiens

<400> 436
tgtgctctcg attcaggagg aggtgctgac ggactcacct tt 42

<210> 437
<211> 42
<212> DNA
<213> Homo sapiens

<400> 437
tgtgctctcg attcgggagg aggtgctgac ggactcacct tt 42

<210> 438
<211> 36
<212> DNA
<213> Homo sapiens

<400> 438
tgtgccttta aagctgcagg caacaagcta actttt 36

<210> 439
<211> 36
<212> DNA
<213> Homo sapiens

<400> 439
tgtgccaggc gtaatgcagg caacatgctc accttt 36

<210> 440
<211> 36
<212> DNA
<213> Homo sapiens

<400> 440
tgtgcctcta aagctgcagg caacaagcta actttt 36

<210> 441
<211> 36
<212> DNA
<213> Homo sapiens

<400> 441
tgtgctctca aagctgcagg caacaagcta actttt 36

<210> 442
<211> 36
<212> DNA
<213> Homo sapiens

<400> 442
tgtgctctca aagctgctgg caacaagcta actttt 36

<210> 443
<211> 36
<212> DNA
<213> Homo sapiens

<400> 443
tgtgccttga ggcaagcagg caacatgctc accttt 36

<210> 444
<211> 36
<212> DNA
<213> Homo sapiens

<400> 444
tgtgccttga ggcaagcggg caacatgctc accttt 36

<210> 445
<211> 33
<212> DNA
<213> Homo sapiens

<400> 445
tgtgcccctg ggggttacca gaaagttacc ttt 33

<210> 446
<211> 33
<212> DNA
<213> Homo sapiens

<400> 446
tgtgcctggg gttctgcaag gcaactgacc ttt 33

<210> 447
<211> 36
<212> DNA
<213> Homo sapiens

<400> 447
tgtgccttca aagctgcagg caacaagcta actttt 36

<210> 448
<211> 36
<212> DNA
<213> Homo sapiens

<400> 448
tgtgccttta aagctgcagg caacaagcta actttt 36

<210> 449
<211> 36
<212> DNA
<213> Homo sapiens

<400> 449
tgtgccttta acgctgcagg caacaagcta actttt 36

<210> 450
<211> 36
<212> DNA
<213> Homo sapiens

<400> 450
tgtgcctttg tagctgctgg tcagaatttt gtcttt 36

<210> 451
<211> 39
<212> DNA
<213> Homo sapiens

<400> 451
tgtgccgaat atggtggtgc tacaaacaag ctcatcttt 39

<210> 452
<211> 39
<212> DNA
<213> Homo sapiens

<400> 452
tgtgccaact atggcggtgc tacaaacaag ctcatcttt 39

<210> 453
<211> 39
<212> DNA
<213> Homo sapiens

<400> 453
tgtgcctcgt atggtggtgc tacaaacaag ctcatcttt 39

<210> 454
<211> 42
<212> DNA
<213> Homo sapiens

<400> 454
tgtgccttaa tgacaactga cagctggggg aaattgcagt tt 42

<210> 455
<211> 13
<212> PRT
<213> Homo sapiens

<400> 455

Cys Ala Ser Leu Gly Pro Leu Arg His Glu Gln Tyr Phe
1 5 10

<210> 456
<211> 14
<212> PRT
<213> Homo sapiens

<400> 456

Cys Ala Ser Lys Pro Leu Val Ser Thr Asp Thr Gln Tyr Phe
1 5 10

<210> 457
<211> 14
<212> PRT
<213> Homo sapiens

<400> 457

Cys Ala Ser Leu Glu Arg Thr Ser Gly Gly Glu Gln Phe Phe
1 5 10

<210> 458
<211> 14
<212> PRT
<213> Homo sapiens

<400> 458

Cys Ala Ser Thr Arg Asp Arg Thr Lys Asn Glu Gln Phe Phe
1 5 10

<210> 459
<211> 15
<212> PRT
<213> Homo sapiens

<400> 459

Cys Ala Ser Ser Ala Leu Ala Ser Gly Gly Asp Glu Gln Phe Phe
1 5 10 15

<210> 460
<211> 15
<212> PRT
<213> Homo sapiens

<400> 460

Cys Ala Ser Ser Ala Leu Ala Ser Gly Thr Asp Thr Gln Tyr Phe
1 5 10 15

<210> 461
<211> 15
<212> PRT
<213> Homo sapiens

<400> 461

Cys Ala Ser Ser Glu Leu Thr Ser Arg Thr Tyr Glu Gln Tyr Phe
1               5                   10                  15

<210> 462
<211> 15
<212> PRT
<213> Homo sapiens

<400> 462

Cys Ala Ser Ser Glu Arg Val Ser Gly Asn Gln Pro Gln His Phe
1               5                   10                  15

<210> 463
<211> 15
<212> PRT
<213> Homo sapiens

<400> 463

Cys Ala Ser Ser Pro Met Ala Ser Gly Gly Asp Glu Gln Phe Phe
1               5                   10                  15

<210> 464
<211> 15
<212> PRT
<213> Homo sapiens

<400> 464

Cys Ala Ser Ser Arg Arg Thr Ser Gly Thr Tyr Glu Gln Tyr Phe
1               5                   10                  15

<210> 465
<211> 15
<212> PRT
<213> Homo sapiens

<400> 465

Cys Ala Ser Ser Val Met Ala Ser Arg Gly Asn Glu Gln Phe Phe
1               5                   10                  15

<210> 466
<211> 16
<212> PRT
<213> Homo sapiens

<400> 466

Cys Ala Ser Gln Arg Gly Ala Arg Gly Gly Asn Gln Pro Gln His Phe
1               5                   10                  15

<210> 467
<211> 16
<212> PRT

<213>  Homo sapiens

<400>  467

Cys Ala Ser Arg Ala Arg Thr Gly Ala Thr Asn Glu Lys Leu Phe Phe
1               5                   10                  15

<210>  468
<211>  16
<212>  PRT
<213>  Homo sapiens

<400>  468

Cys Ala Ser Ser Ala Lys Thr Ser Gly Gly Ser Asp Thr Gln Tyr Phe
1               5                   10                  15

<210>  469
<211>  16
<212>  PRT
<213>  Homo sapiens

<400>  469

Cys Ala Ser Ser Ala Leu Ala Ser Gly Gly Arg Asp Thr Gln Tyr Phe
1               5                   10                  15

<210>  470
<211>  16
<212>  PRT
<213>  Homo sapiens

<400>  470

Cys Ala Ser Ser Ala Arg Thr Ser Gly Gly Leu Asp Glu Gln Phe Phe
1               5                   10                  15

<210>  471
<211>  16
<212>  PRT
<213>  Homo sapiens

<400>  471

Cys Ala Ser Ser Ala Arg Thr Ser Gly Gly Ser Asp Thr Gln Tyr Phe
1               5                   10                  15

<210>  472
<211>  16
<212>  PRT
<213>  Homo sapiens

<400>  472

Cys Ala Ser Ser Lys Arg Ala Ser Gly Gly Thr Asp Thr Gln Tyr Phe
1               5                   10                  15

<210> 473
<211> 16
<212> PRT
<213> Homo sapiens

<400> 473

Cys Ala Ser Ser Leu Arg Thr Ser Gly Gly Ser Asp Thr Gln Tyr Phe
1 5 10 15

<210> 474
<211> 16
<212> PRT
<213> Homo sapiens

<400> 474

Cys Ala Ser Ser Arg Leu Thr Ser Gly Gly Arg Asn Glu Gln Phe Phe
1 5 10 15

<210> 475
<211> 16
<212> PRT
<213> Homo sapiens

<400> 475

Cys Ala Ser Ser Ser Lys Thr Ser Gly Gly Thr Asp Thr Gln Tyr Phe
1 5 10 15

<210> 476
<211> 16
<212> PRT
<213> Homo sapiens

<400> 476

Cys Ala Ser Ser Ser Arg Thr Ser Gly Gly Gln Asp Glu Gln Phe Phe
1 5 10 15

<210> 477
<211> 16
<212> PRT
<213> Homo sapiens

<400> 477

Cys Ala Thr Ser Arg Gly Ala Arg Gly Ser Asn Gln Pro Gln His Phe
1 5 10 15

<210> 478
<211> 13
<212> PRT
<213> Homo sapiens

<400> 478

Cys Ala Ser Ala Arg Thr Gly Gly Val Gly Tyr Thr Phe

1 5 10

<210> 479
<211> 13
<212> PRT
<213> Homo sapiens

<400> 479

Cys Ala Ser Arg Thr Ser Gly Thr Tyr Glu Gln Tyr Phe
1 5 10

<210> 480
<211> 14
<212> PRT
<213> Homo sapiens

<400> 480

Cys Ala Ser Lys Ala Lys Thr Val Thr Tyr Lys Gln Tyr Phe
1 5 10

<210> 481
<211> 14
<212> PRT
<213> Homo sapiens

<400> 481

Cys Ala Ser Lys Pro Lys Ala Val Thr Tyr Glu Gln Tyr Phe
1 5 10

<210> 482
<211> 14
<212> PRT
<213> Homo sapiens

<400> 482

Cys Ala Ser Arg Gly Thr Ala Thr Gly Asn Thr Ile Tyr Phe
1 5 10

<210> 483
<211> 14
<212> PRT
<213> Homo sapiens

<400> 483

Cys Ala Ser Arg Pro Thr Ala Thr Asn Glu Lys Leu Phe Phe
1 5 10

<210> 484
<211> 14
<212> PRT
<213> Homo sapiens

<400> 484

Cys Ala Ser Ser Glu Tyr Ala Thr Ser Asn Glu Gln Phe Phe
1 5 10

<210> 485
<211> 14
<212> PRT
<213> Homo sapiens

<400> 485

Cys Ala Ser Ser Arg Gly Gln Arg His Arg Tyr Ala Val Phe
1 5 10

<210> 486
<211> 15
<212> PRT
<213> Homo sapiens

<400> 486

Cys Ala Ile Ser Arg Leu Ala Gly Gly Met Asp Glu Gln Tyr Phe
1 5 10 15

<210> 487
<211> 15
<212> PRT
<213> Homo sapiens

<400> 487

Cys Ala Ser Gly Arg Leu Ala Ser Gly Thr Asp Thr Gln Tyr Phe
1 5 10 15

<210> 488
<211> 15
<212> PRT
<213> Homo sapiens

<400> 488

Cys Ala Ser Arg Arg Gly Thr Ser Gly Thr Gly Glu Leu Phe Phe
1 5 10 15

<210> 489
<211> 15
<212> PRT
<213> Homo sapiens

<400> 489

Cys Ala Ser Ser Asp Gly Ala Ser Gly Val Gly Glu Gln Tyr Phe
1 5 10 15

<210> 490
<211> 15

<212> PRT
<213> Homo sapiens

<400> 490

Cys Ala Ser Ser Glu Ala Ala Arg Gly Asn Ser Pro Leu His Phe
1 5 10 15

<210> 491
<211> 15
<212> PRT
<213> Homo sapiens

<400> 491

Cys Ala Ser Ser Glu Gly Ala Ser Gly Leu Gly Glu Gln Tyr Phe
1 5 10 15

<210> 492
<211> 15
<212> PRT
<213> Homo sapiens

<400> 492

Cys Ala Ser Ser Glu Leu Ala Ser Gly Ile Ser Glu Gln Phe Phe
1 5 10 15

<210> 493
<211> 15
<212> PRT
<213> Homo sapiens

<400> 493

Cys Ala Ser Ser Glu Leu Ala Ser Gly Leu Ala Glu Gln Phe Phe
1 5 10 15

<210> 494
<211> 15
<212> PRT
<213> Homo sapiens

<400> 494

Cys Ala Ser Ser Glu Leu Ala Ser Gly Thr Gly Glu Gln Phe Phe
1 5 10 15

<210> 495
<211> 15
<212> PRT
<213> Homo sapiens

<400> 495

Cys Ala Ser Ser Glu Arg Ala Ser Gly Thr Asp Glu Gln Tyr Phe
1 5 10 15

<210> 496
<211> 15
<212> PRT
<213> Homo sapiens

<400> 496

Cys Ala Ser Ser Glu Arg Ala Ser Gly Val Gly Glu Leu Phe Phe
1 5 10 15

<210> 497
<211> 15
<212> PRT
<213> Homo sapiens

<400> 497

Cys Ala Ser Ser Gly Leu Ala Ser Gly Thr Asp Thr Gln Tyr Phe
1 5 10 15

<210> 498
<211> 15
<212> PRT
<213> Homo sapiens

<400> 498

Cys Ala Ser Ser Gly Leu Ala Ser Gly Thr Gly Glu Leu Phe Phe
1 5 10 15

<210> 499
<211> 15
<212> PRT
<213> Homo sapiens

<400> 499

Cys Ala Ser Ser Gly Met Thr Ser Arg Ser Tyr Glu Gln Tyr Phe
1 5 10 15

<210> 500
<211> 15
<212> PRT
<213> Homo sapiens

<400> 500

Cys Ala Ser Ser Pro Gly Leu Ala Gly Thr Gly Glu Leu Phe Phe
1 5 10 15

<210> 501
<211> 15
<212> PRT
<213> Homo sapiens

<400> 501

Cys Ala Ser Ser Pro Leu Thr Ser Gly Thr Asp Thr Gln Tyr Phe
1               5                   10                  15

<210> 502
<211> 15
<212> PRT
<213> Homo sapiens

<400> 502

Cys Ala Ser Ser Pro Arg Ala Arg Gly Asn Gln Pro Gln His Phe
1               5                   10                  15

<210> 503
<211> 15
<212> PRT
<213> Homo sapiens

<400> 503

Cys Ala Ser Ser Pro Arg Thr Ser Gly Pro Tyr Glu Gln Tyr Phe
1               5                   10                  15

<210> 504
<211> 15
<212> PRT
<213> Homo sapiens

<400> 504

Cys Ala Ser Ser Pro Arg Thr Ser Gly Ser Tyr Glu Gln Tyr Phe
1               5                   10                  15

<210> 505
<211> 15
<212> PRT
<213> Homo sapiens

<400> 505

Cys Ala Ser Ser Gln Gly Leu Ala Gly Thr Gly Glu Leu Phe Phe
1               5                   10                  15

<210> 506
<211> 15
<212> PRT
<213> Homo sapiens

<400> 506

Cys Ala Ser Ser Gln Leu Ala Arg Gly Thr Asp Thr Gln Tyr Phe
1               5                   10                  15

<210> 507
<211> 15
<212> PRT
<213> Homo sapiens

<400> 507

Cys Ala Ser Ser Gln Leu Val Ser Leu Arg Gly Glu Gln Tyr Phe
1 5 10 15

<210> 508
<211> 15
<212> PRT
<213> Homo sapiens

<400> 508

Cys Ala Ser Ser Gln Arg Thr Ser Gly Ser Asp Glu Gln Tyr Phe
1 5 10 15

<210> 509
<211> 15
<212> PRT
<213> Homo sapiens

<400> 509

Cys Ala Ser Ser Gln Val Ala Gly Gly Thr Ala Thr Gln Tyr Phe
1 5 10 15

<210> 510
<211> 15
<212> PRT
<213> Homo sapiens

<400> 510

Cys Ala Ser Ser Arg Gly Ala Arg Gly Asn Gln Pro Gln His Phe
1 5 10 15

<210> 511
<211> 15
<212> PRT
<213> Homo sapiens

<400> 511

Cys Ala Ser Ser Arg Leu Ala Gly Gly Leu Gly Glu Gln Phe Phe
1 5 10 15

<210> 512
<211> 15
<212> PRT
<213> Homo sapiens

<400> 512

Cys Ala Ser Ser Arg Leu Ala Gly Gly Met Asp Glu Gln Phe Phe
1 5 10 15

<210> 513

<211> 15
<212> PRT
<213> Homo sapiens

<400> 513

Cys Ala Ser Ser Arg Leu Ala Gly Gly Thr Asp Glu Gln Phe Phe
1 5 10 15

<210> 514
<211> 15
<212> PRT
<213> Homo sapiens

<400> 514

Cys Ala Ser Ser Arg Leu Thr Ser Gly Thr Asp Thr Gln Tyr Phe
1 5 10 15

<210> 515
<211> 15
<212> PRT
<213> Homo sapiens

<400> 515

Cys Ala Ser Ser Arg Val Thr Gly Gly Met Asp Glu Gln Phe Phe
1 5 10 15

<210> 516
<211> 15
<212> PRT
<213> Homo sapiens

<400> 516

Cys Ala Ser Thr Lys Leu Ala Gly Gly Thr Ser Glu Gln Phe Phe
1 5 10 15

<210> 517
<211> 15
<212> PRT
<213> Homo sapiens

<400> 517

Cys Ala Ser Thr Lys Leu Ala Trp Gly Thr Tyr Thr Gln Tyr Phe
1 5 10 15

<210> 518
<211> 15
<212> PRT
<213> Homo sapiens

<400> 518

Cys Ala Thr Thr Pro Gly Ala Ser Gly Ile Ser Glu Gln Phe Phe
1 5 10 15

<210> 519
<211> 16
<212> PRT
<213> Homo sapiens

<400> 519

Cys Ala Ser Ser Glu Arg Gly Gln Gly Ala Arg Tyr Glu Gln Tyr Phe
1 5 10 15

<210> 520
<211> 16
<212> PRT
<213> Homo sapiens

<400> 520

Cys Ala Ser Ser Arg Met Thr Gly Gly Gly Thr Asp Thr Gln Tyr Phe
1 5 10 15

<210> 521
<211> 16
<212> PRT
<213> Homo sapiens

<400> 521

Cys Ala Ser Ser Arg Arg Thr Ser Gly Gly Thr Asp Thr Gln Tyr Phe
1 5 10 15

<210> 522
<211> 13
<212> PRT
<213> Homo sapiens

<400> 522

Cys Ala Ser His Arg Thr Tyr Thr Asp Thr Gln Tyr Phe
1 5 10

<210> 523
<211> 13
<212> PRT
<213> Homo sapiens

<400> 523

Cys Ala Ser Ser Gly Gln Thr Asn Thr Glu Ala Phe Phe
1 5 10

<210> 524
<211> 14
<212> PRT
<213> Homo sapiens

<400> 524

Cys Ala Ser Arg Trp Thr Ala Thr Ser Tyr Gly Tyr Thr Phe
1 5 10

<210> 525
<211> 14
<212> PRT
<213> Homo sapiens

<400> 525

Cys Ala Ser Ser Pro Thr Thr Thr Gly Tyr Gly Tyr Thr Phe
1 5 10

<210> 526
<211> 15
<212> PRT
<213> Homo sapiens

<400> 526

Cys Ala Ser His Glu Gly Ala Gly Gly Phe Gly Glu Leu Phe Phe
1 5 10 15

<210> 527
<211> 15
<212> PRT
<213> Homo sapiens

<400> 527

Cys Ala Ser His Glu Gly Ala Gly Gly Tyr Gly Glu Leu Phe Phe
1 5 10 15

<210> 528
<211> 15
<212> PRT
<213> Homo sapiens

<400> 528

Cys Ala Ser Ser Ala Gly Thr Arg Gly Val Gly Glu Gln Phe Phe
1 5 10 15

<210> 529
<211> 15
<212> PRT
<213> Homo sapiens

<400> 529

Cys Ala Ser Ser Ala Leu Ala Ser Gly Thr Asp Thr Gln Tyr Phe
1 5 10 15

<210> 530
<211> 15
<212> PRT

<213> Homo sapiens

<400> 530

Cys Ala Ser Ser Asp Ala Ala Ser Gly Val Gly Glu Gln Tyr Phe
1 5 10 15

<210> 531
<211> 15
<212> PRT
<213> Homo sapiens

<400> 531

Cys Ala Ser Ser Asp Leu Ala Ser Gly Thr Asn Glu Gln Phe Phe
1 5 10 15

<210> 532
<211> 15
<212> PRT
<213> Homo sapiens

<400> 532

Cys Ala Ser Ser Asp Arg Ala Ser Gly Val Gly Glu Gln Phe Phe
1 5 10 15

<210> 533
<211> 15
<212> PRT
<213> Homo sapiens

<400> 533

Cys Ala Ser Ser Asp Arg Thr Ser Gly Pro His Glu Gln Phe Phe
1 5 10 15

<210> 534
<211> 15
<212> PRT
<213> Homo sapiens

<400> 534

Cys Ala Ser Ser Gly Leu Ala Gly Gly Met Asp Glu Gln Phe Phe
1 5 10 15

<210> 535
<211> 15
<212> PRT
<213> Homo sapiens

<400> 535

Cys Ala Ser Ser Pro Gly Ala Arg Gly Ile Asp Glu Gln Phe Phe
1 5 10 15

<210> 536
<211> 15
<212> PRT
<213> Homo sapiens

<400> 536

Cys Ala Ser Ser Pro Gly Thr Ser Gly Val Gly Glu Gln Phe Phe
1 5 10 15

<210> 537
<211> 15
<212> PRT
<213> Homo sapiens

<400> 537

Cys Ala Ser Ser Pro Gly Thr Ser Gly Val Gly Lys Gln Phe Phe
1 5 10 15

<210> 538
<211> 15
<212> PRT
<213> Homo sapiens

<400> 538

Cys Ala Ser Ser Pro Arg Thr Ser Gly Gly Gly Glu Gln Tyr Phe
1 5 10 15

<210> 539
<211> 15
<212> PRT
<213> Homo sapiens

<400> 539

Cys Ala Ser Ser Pro Ser Ala Arg Gly Asn Gln Pro Gln His Phe
1 5 10 15

<210> 540
<211> 15
<212> PRT
<213> Homo sapiens

<400> 540

Cys Ala Ser Ser Pro Thr Thr Ser Gly Arg Gly Glu Gln Tyr Phe
1 5 10 15

<210> 541
<211> 15
<212> PRT
<213> Homo sapiens

<400> 541

Cys Ala Ser Ser Ser Gly Thr Ser Gly Ala Gly Glu Gln Phe Phe

1 5 10 15

<210> 542
<211> 15
<212> PRT
<213> Homo sapiens

<400> 542

Cys Ala Ser Ser Ser Gly Thr Ser Gly Val Gly Glu Gln Phe Phe
1 5 10 15

<210> 543
<211> 15
<212> PRT
<213> Homo sapiens

<400> 543

Cys Ala Ser Ser Ser Gly Thr Ser Gly Val Gly Glu Gln Phe Phe
1 5 10 15

<210> 544
<211> 15
<212> PRT
<213> Homo sapiens

<400> 544

Cys Ala Ser Ser Val Gly Thr Ser Gly Val Gly Glu Gln Tyr Phe
1 5 10 15

<210> 545
<211> 15
<212> PRT
<213> Homo sapiens

<400> 545

Cys Ala Ser Ser Tyr Gly Ala Ser Gly Val Gly Glu Gln Phe Phe
1 5 10 15

<210> 546
<211> 15
<212> PRT
<213> Homo sapiens

<400> 546

Cys Ala Ser Ser Tyr Arg Thr Ser Gly Pro Arg Glu Gln Phe Phe
1 5 10 15

<210> 547
<211> 12
<212> PRT
<213> Homo sapiens

<400> 547

Cys Ala Ser Arg Lys Glu Gly Ser Arg Leu His Leu
1 5 10

<210> 548
<211> 13
<212> PRT
<213> Homo sapiens

<400> 548

Cys Ala Ser Ser Asp Arg Thr Thr Cys Gly Tyr Thr Phe
1 5 10

<210> 549
<211> 13
<212> PRT
<213> Homo sapiens

<400> 549

Cys Ala Ser Ser Glu Arg Arg Ile Tyr Gly Tyr Thr Phe
1 5 10

<210> 550
<211> 14
<212> PRT
<213> Homo sapiens

<400> 550

Cys Ala Ser Arg Ala Leu Ala Ser Gly Gly Glu Gln Phe Phe
1 5 10

<210> 551
<211> 14
<212> PRT
<213> Homo sapiens

<400> 551

Cys Ala Ser Ser Ala Leu Ala Ser Gly Asp Lys Gln Tyr Phe
1 5 10

<210> 552
<211> 14
<212> PRT
<213> Homo sapiens

<400> 552

Cys Ala Ser Ser Ala Leu Ala Ser Gly Asp Thr Gln Tyr Phe
1 5 10

<210> 553
<211> 14

<212> PRT
<213> Homo sapiens

<400> 553

Cys Ala Ser Ser Asp Asp Arg Val Gly Asp Glu Gln Phe Phe
1 5 10

<210> 554
<211> 14
<212> PRT
<213> Homo sapiens

<400> 554

Cys Ala Ser Ser Lys Leu Ala Ser Gly Asp Glu Gln Phe Phe
1 5 10

<210> 555
<211> 14
<212> PRT
<213> Homo sapiens

<400> 555

Cys Ala Ser Ser Val Leu Pro Gly Gly Asn Asp Pro Leu Phe
1 5 10

<210> 556
<211> 14
<212> PRT
<213> Homo sapiens

<400> 556

Cys Ala Ser Ser Val Leu Pro Gly Arg Asn Glu Pro Phe Phe
1 5 10

<210> 557
<211> 14
<212> PRT
<213> Homo sapiens

<400> 557

Cys Ala Ser Ser Val Leu Arg Gly Gly Asn Glu Gln Phe Phe
1 5 10

<210> 558
<211> 14
<212> PRT
<213> Homo sapiens

<400> 558

Cys Ala Ser Ser Val Leu Arg Gly Arg Asn Glu Pro Phe Phe
1 5 10

<210> 559
<211> 14
<212> PRT
<213> Homo sapiens

<400> 559

Cys Ala Ser Ser Val Leu Arg Gly Arg Asn Glu Gln Phe Phe
1               5                   10

<210> 560
<211> 14
<212> PRT
<213> Homo sapiens

<400> 560

Cys Ala Ser Ser Val Ser Arg Gly Gly Asn Lys Gln Phe Phe
1               5                   10

<210> 561
<211> 14
<212> PRT
<213> Homo sapiens

<400> 561

Cys Ala Ser Val Leu Met Arg Thr Asn Asn Glu Gln Phe Phe
1               5                   10

<210> 562
<211> 14
<212> PRT
<213> Homo sapiens

<400> 562

Cys Ser Ser Arg Ala Arg Gly Cys Ala Gly Lys Gln Tyr Phe
1               5                   10

<210> 563
<211> 15
<212> PRT
<213> Homo sapiens

<400> 563

Cys Ala Ser Ser Ala Leu Thr Ser Gly Gly Asp Glu Gln Phe Phe
1               5                   10                  15

<210> 564
<211> 15
<212> PRT
<213> Homo sapiens

<400> 564

Cys Ala Ser Ser Ala Arg Thr Ser Gly Gly Ser Glu Gln Phe Phe
1 5 10 15

<210> 565
<211> 15
<212> PRT
<213> Homo sapiens

<400> 565

Cys Ala Ser Ser Ala Arg Thr Ser Gly Ser Asp Glu Gln Tyr Phe
1 5 10 15

<210> 566
<211> 15
<212> PRT
<213> Homo sapiens

<400> 566

Cys Ala Ser Ser Asp Arg Ala Ser Gly Gly Asp Thr Gln Tyr Phe
1 5 10 15

<210> 567
<211> 15
<212> PRT
<213> Homo sapiens

<400> 567

Cys Ala Ser Ser Asp Arg Ala Ser Gly Gly Asp Thr Gln Tyr Phe
1 5 10 15

<210> 568
<211> 15
<212> PRT
<213> Homo sapiens

<400> 568

Cys Ala Ser Ser Asp Arg Ala Thr Gly Gly Asp Thr Gln Tyr Phe
1 5 10 15

<210> 569
<211> 15
<212> PRT
<213> Homo sapiens

<400> 569

Cys Ala Ser Ser Glu Lys Ala Ser Gly Gly Asp Thr Gln Tyr Phe
1 5 10 15

<210> 570
<211> 15
<212> PRT
<213> Homo sapiens

<400> 570

Cys Ala Ser Ser Glu Leu Ala Ser Gly Gly Asp Glu Gln Phe Phe
1 5 10 15

<210> 571
<211> 15
<212> PRT
<213> Homo sapiens

<400> 571

Cys Ala Ser Ser His Lys Ala Ser Gly Gly Asp Lys Gln Tyr Phe
1 5 10 15

<210> 572
<211> 15
<212> PRT
<213> Homo sapiens

<400> 572

Cys Ala Ser Ser His Met Ala Ser Gly Gly Asp Thr Gln Tyr Phe
1 5 10 15

<210> 573
<211> 15
<212> PRT
<213> Homo sapiens

<400> 573

Cys Ala Ser Ser His Arg Ala Ser Gly Gly Ala Thr Pro Tyr Phe
1 5 10 15

<210> 574
<211> 15
<212> PRT
<213> Homo sapiens

<400> 574

Cys Ala Ser Ser His Arg Ala Ser Gly Gly Asp Thr Pro His Phe
1 5 10 15

<210> 575
<211> 15
<212> PRT
<213> Homo sapiens

<400> 575

Cys Ala Ser Ser His Arg Ala Ser Gly Gly Asp Thr Gln Tyr Phe
1 5 10 15

<210> 576

<211> 15
<212> PRT
<213> Homo sapiens

<400> 576

Cys Ala Ser Ser Lys Leu Ala Ser Gly Ala Asp Glu Gln Tyr Phe
1 5 10 15

<210> 577
<211> 15
<212> PRT
<213> Homo sapiens

<400> 577

Cys Ala Ser Ser Lys Leu Thr Arg Gly Ala Asp Lys Gln Tyr Phe
1 5 10 15

<210> 578
<211> 15
<212> PRT
<213> Homo sapiens

<400> 578

Cys Ala Ser Ser Lys Arg Thr Ser Gly Thr Tyr Glu Gln Tyr Phe
1 5 10 15

<210> 579
<211> 15
<212> PRT
<213> Homo sapiens

<400> 579

Cys Ala Ser Ser Leu Arg Thr Ser Gly Ser Tyr Glu Gln Tyr Phe
1 5 10 15

<210> 580
<211> 15
<212> PRT
<213> Homo sapiens

<400> 580

Cys Ala Ser Ser Pro Arg Thr Ser Gly Thr Tyr Glu Gln Tyr Phe
1 5 10 15

<210> 581
<211> 15
<212> PRT
<213> Homo sapiens

<400> 581

Cys Ala Ser Ser Pro Arg Val Phe Ser Val Gly Glu Leu Phe Phe
1 5 10 15

<210> 582
<211> 15
<212> PRT
<213> Homo sapiens

<400> 582

Cys Ala Ser Ser Gln Arg Ala Ser Gly Gly Asp Glu Gln Phe Phe
1 5 10 15

<210> 583
<211> 15
<212> PRT
<213> Homo sapiens

<400> 583

Cys Ala Ser Ser Arg Arg Thr Ser Gly Thr Tyr Glu Gln Tyr Phe
1 5 10 15

<210> 584
<211> 15
<212> PRT
<213> Homo sapiens

<400> 584

Cys Ala Ser Ser Val Arg Thr Ser Gly Ser Tyr Glu Gln Tyr Phe
1 5 10 15

<210> 585
<211> 15
<212> PRT
<213> Homo sapiens

<400> 585

Cys Thr Ser Ser Gly Arg Thr Ser Gly Arg Asp Lys Gln Tyr Phe
1 5 10 15

<210> 586
<211> 15
<212> PRT
<213> Homo sapiens

<400> 586

Cys Thr Ser Ser His Arg Ala Ser Gly Gly Asp Thr Gln Tyr Phe
1 5 10 15

<210> 587
<211> 16
<212> PRT
<213> Homo sapiens

<400> 587

Cys Ala Ser Ser Ala Arg Ile Ser Gly Gly Leu Asn Glu Gln Tyr Phe
1 5 10 15

<210> 588
<211> 16
<212> PRT
<213> Homo sapiens

<400> 588

Cys Ala Ser Ser Ala Arg Thr Ser Gly Gly Ala Asp Thr Gln Tyr Phe
1 5 10 15

<210> 589
<211> 16
<212> PRT
<213> Homo sapiens

<400> 589

Cys Ala Ser Ser Ala Arg Thr Ser Gly Gly Leu Asp Glu Gln Tyr Phe
1 5 10 15

<210> 590
<211> 16
<212> PRT
<213> Homo sapiens

<400> 590

Cys Ala Ser Ser Ala Arg Thr Ser Gly Gly Ser Asp Thr Gln Tyr Phe
1 5 10 15

<210> 591
<211> 16
<212> PRT
<213> Homo sapiens

<400> 591

Cys Ala Ser Ser Lys Arg Thr Ser Gly Gly Ala Asp Thr Gln Tyr Phe
1 5 10 15

<210> 592
<211> 16
<212> PRT
<213> Homo sapiens

<400> 592

Cys Ala Ser Ser Leu Arg Thr Ser Gly Gly Thr Asp Thr Gln Tyr Phe
1 5 10 15

<210> 593
<211> 16
<212> PRT

<213> Homo sapiens

<400> 593

Cys Ala Ser Ser Pro Arg Thr Ser Gly Gly Thr Asp Thr Gln Tyr Phe
1 5 10 15

<210> 594
<211> 16
<212> PRT
<213> Homo sapiens

<400> 594

Cys Ala Ser Ser Arg Arg Ala Ser Gly Gly Thr Thr Pro His Tyr Phe
1 5 10 15

<210> 595
<211> 16
<212> PRT
<213> Homo sapiens

<400> 595

Cys Ala Ser Ser Arg Arg Thr Ser Gly Gly Thr Asp Thr Gln Tyr Phe
1 5 10 15

<210> 596
<211> 16
<212> PRT
<213> Homo sapiens

<400> 596

Cys Ala Ser Ser Arg Arg Thr Ser Gly Arg Ala Asp Thr Gln Tyr Phe
1 5 10 15

<210> 597
<211> 16
<212> PRT
<213> Homo sapiens

<400> 597

Cys Ala Ser Ser Arg Arg Thr Ser Gly Ser Leu Asp Thr Gln Tyr Phe
1 5 10 15

<210> 598
<211> 16
<212> PRT
<213> Homo sapiens

<400> 598

Cys Ala Ser Ser Thr Arg Ile Arg Gly Gly Thr Asp Lys Gln Tyr Phe
1 5 10 15

<210> 599
<211> 16
<212> PRT
<213> Homo sapiens

<400> 599

Cys Ala Ser Ser Val Arg Thr Ser Gly Gly Thr Asp Thr Gln Tyr Phe
1 5 10 15

<210> 600
<211> 12
<212> PRT
<213> Homo sapiens

<400> 600

Cys Ala Ser Arg Asp Ser Asn Tyr Gly Tyr Thr Phe
1 5 10

<210> 601
<211> 12
<212> PRT
<213> Homo sapiens

<400> 601

Cys Ala Ser Ser Arg Arg Thr Glu Thr Gln Tyr Phe
1 5 10

<210> 602
<211> 13
<212> PRT
<213> Homo sapiens

<400> 602

Cys Ala Ser Ser Glu Thr Arg Ala Asn Ile Gln Tyr Phe
1 5 10

<210> 603
<211> 13
<212> PRT
<213> Homo sapiens

<400> 603

Cys Ala Ser Ser Gly Leu Ala Ala Asn Glu Gln Phe Phe
1 5 10

<210> 604
<211> 14
<212> PRT
<213> Homo sapiens

<400> 604

Cys Ala Ser Arg Ala Gly Ser Val Ala Thr Glu Ala Phe Phe

1 5 10

<210> 605
<211> 14
<212> PRT
<213> Homo sapiens

<400> 605

Cys Ala Ser Ser Gly Arg Thr Ser Gly Asn Glu Gln Phe Phe
1 5 10

<210> 606
<211> 14
<212> PRT
<213> Homo sapiens

<400> 606

Cys Ala Ser Ser Val Val Gly Ser Tyr Asn Glu Gln Phe Phe
1 5 10

<210> 607
<211> 14
<212> PRT
<213> Homo sapiens

<400> 607

Cys Ala Ser Val Leu Met Arg Thr Asn Asn Glu Gln Phe Phe
1 5 10

<210> 608
<211> 15
<212> PRT
<213> Homo sapiens

<400> 608

Cys Ala Ser Arg Ala Gly Thr Ser Gly Thr Gly Glu Leu Phe Phe
1 5 10 15

<210> 609
<211> 15
<212> PRT
<213> Homo sapiens

<400> 609

Cys Ala Ser Arg Lys Gly Thr Ser Gly Ser Gly Lys Gln Tyr Phe
1 5 10 15

<210> 610
<211> 15
<212> PRT
<213> Homo sapiens

<400> 610

Cys Ala Ser Ser Glu Lys Ala Ser Gly Val Asp Glu Gln Phe Phe
1 5 10 15

<210> 611
<211> 15
<212> PRT
<213> Homo sapiens

<400> 611

Cys Ala Ser Ser Glu Arg Ala Ser Gly His Asp Thr Gln Tyr Phe
1 5 10 15

<210> 612
<211> 15
<212> PRT
<213> Homo sapiens

<400> 612

Cys Ala Ser Ser His Arg Ala Ser Gly Gly Asp Thr Gln Tyr Phe
1 5 10 15

<210> 613
<211> 15
<212> PRT
<213> Homo sapiens

<400> 613

Cys Ala Ser Ser Lys Leu Ala Ser Gly Ala Asp Glu Gln Tyr Phe
1 5 10 15

<210> 614
<211> 15
<212> PRT
<213> Homo sapiens

<400> 614

Cys Ala Ser Ser Lys Gln Ala Ser Gly Gly Asp Glu Gln Tyr Phe
1 5 10 15

<210> 615
<211> 14
<212> PRT
<213> Homo sapiens

<400> 615

Cys Ala Ser Arg Glu Tyr Ala Thr Ser Asn Glu Gln Tyr Phe
1 5 10

<210> 616
<211> 14

<212> PRT
<213> Homo sapiens

<400> 616

Cys Ala Ser Ser Glu Met Ala Thr Gly Leu Arg Tyr Thr Phe
1 5 10

<210> 617
<211> 14
<212> PRT
<213> Homo sapiens

<400> 617

Cys Ala Ser Ser Glu Thr Ala Thr Gly Leu Arg Tyr Thr Phe
1 5 10

<210> 618
<211> 14
<212> PRT
<213> Homo sapiens

<400> 618

Cys Ala Ser Thr Leu Thr Arg Val Asn Ser Pro Leu His Phe
1 5 10

<210> 619
<211> 15
<212> PRT
<213> Homo sapiens

<400> 619

Cys Ala Ser Asn Gln Arg Thr Ser Gly Pro Tyr Glu Gln Tyr Phe
1 5 10 15

<210> 620
<211> 15
<212> PRT
<213> Homo sapiens

<400> 620

Cys Ala Ser Ser Asp Leu Ala Ser Gly Thr Gly Glu Gln Phe Phe
1 5 10 15

<210> 621
<211> 15
<212> PRT
<213> Homo sapiens

<400> 621

Cys Ala Ser Ser Glu Ala Ala Gly Gly Tyr Gly Glu Gln Phe Phe
1 5 10 15

<210> 622
<211> 15
<212> PRT
<213> Homo sapiens

<400> 622

Cys Ala Ser Ser Glu Phe Ala Arg Gly Asn Gln Pro Gln His Phe
1 5 10 15

<210> 623
<211> 15
<212> PRT
<213> Homo sapiens

<400> 623

Cys Ala Ser Ser Glu Phe Val Arg Asp Asn Gln Pro Gln His Phe
1 5 10 15

<210> 624
<211> 15
<212> PRT
<213> Homo sapiens

<400> 624

Cys Ala Ser Ser Glu Phe Val Arg Gly Asn Gln Pro Gln His Phe
1 5 10 15

<210> 625
<211> 15
<212> PRT
<213> Homo sapiens

<400> 625

Cys Ala Ser Ser Glu Gly Ala Ala Gly Asn Gln Pro Gln His Phe
1 5 10 15

<210> 626
<211> 15
<212> PRT
<213> Homo sapiens

<400> 626

Cys Ala Ser Ser Glu Gly Ala Arg Gly Val Gly Glu Gln Phe Phe
1 5 10 15

<210> 627
<211> 15
<212> PRT
<213> Homo sapiens

<400> 627

Cys Ala Ser Ser Glu Gly Ala Ser Gly Thr Gly Ala Gln Tyr Phe
1               5                   10                  15

<210> 628
<211> 15
<212> PRT
<213> Homo sapiens

<400> 628

Cys Ala Ser Ser Glu Gly Ala Ser Gly Val Gly Glu Gln Phe Phe
1               5                   10                  15

<210> 629
<211> 15
<212> PRT
<213> Homo sapiens

<400> 629

Cys Ala Ser Ser Glu Gly Thr Ser Thr Phe Arg Glu Gln Phe Phe
1               5                   10                  15

<210> 630
<211> 15
<212> PRT
<213> Homo sapiens

<400> 630

Cys Ala Ser Ser Glu Leu Ala Ser Gly Thr Gly Glu Leu Phe Phe
1               5                   10                  15

<210> 631
<211> 15
<212> PRT
<213> Homo sapiens

<400> 631

Cys Ala Ser Ser Gly Ala Ala Arg Gly Asn Gln Pro Gln His Phe
1               5                   10                  15

<210> 632
<211> 15
<212> PRT
<213> Homo sapiens

<400> 632

Cys Ala Ser Ser Lys Leu Thr Ser Gly Gly Tyr Glu Gln Tyr Phe
1               5                   10                  15

<210> 633
<211> 15
<212> PRT
<213> Homo sapiens

<400> 633

Cys Ala Ser Ser Pro Gly Thr Ser Gly Val Gly Glu Gln Phe Phe
1 5 10 15

<210> 634
<211> 15
<212> PRT
<213> Homo sapiens

<400> 634

Cys Ala Ser Ser Arg Leu Ala Gly Gly Phe Asp Glu Gln Phe Phe
1 5 10 15

<210> 635
<211> 15
<212> PRT
<213> Homo sapiens

<400> 635

Cys Ala Ser Ser Arg Leu Ala Gly Gly Thr Asp Thr Gln Tyr Phe
1 5 10 15

<210> 636
<211> 15
<212> PRT
<213> Homo sapiens

<400> 636

Cys Ala Ser Ser Arg Leu Ala Ser Gly Thr Asp Thr Gln Tyr Phe
1 5 10 15

<210> 637
<211> 15
<212> PRT
<213> Homo sapiens

<400> 637

Cys Ala Ser Ser Val Leu Ala Ser Gly Leu Gly Glu Gln Tyr Phe
1 5 10 15

<210> 638
<211> 16
<212> PRT
<213> Homo sapiens

<400> 638

Cys Ala Ser Arg Gln Gly Ala Arg Gly Gly Asn Gln Pro Gln His Phe
1 5 10 15

<210> 639

<211> 16
<212> PRT
<213> Homo sapiens

<400> 639

Cys Ala Ser Ser Gln Gly Ala Arg Gly Gly Asn Gln Pro Gln His Phe
1 5 10 15

<210> 640
<211> 16
<212> PRT
<213> Homo sapiens

<400> 640

Cys Ala Ser Ser Arg Leu Ala Gly Gly Ser Ser Tyr Glu Gln Tyr Phe
1 5 10 15

<210> 641
<211> 16
<212> PRT
<213> Homo sapiens

<400> 641

Cys Ala Ser Ser Arg Leu Thr Ser Gly Gly Ala Asp Thr Gln Tyr Phe
1 5 10 15

<210> 642
<211> 16
<212> PRT
<213> Homo sapiens

<400> 642

Cys Ala Ser Ser Arg Arg Thr Ser Gly Gly Thr Asp Thr Gln Tyr Phe
1 5 10 15

<210> 643
<211> 14
<212> PRT
<213> Homo sapiens

<400> 643

Cys Ala Ser Ser Pro Arg Ala Ser Gly Gly Glu Gln Tyr Phe
1 5 10

<210> 644
<211> 14
<212> PRT
<213> Homo sapiens

<400> 644

Cys Ala Ser Ser Val Arg Arg Asn Asn Glu Lys Leu Phe Phe
1 5 10

<210> 645
<211> 15
<212> PRT
<213> Homo sapiens

<400> 645

Cys Ala Ser Asn Arg Arg Thr Ser Gly Thr Tyr Glu Gln Tyr Phe
1 5 10 15

<210> 646
<211> 15
<212> PRT
<213> Homo sapiens

<400> 646

Cys Ala Ser Ser Ala Leu Ala Ser Gly Gly Asp Thr Gln Tyr Phe
1 5 10 15

<210> 647
<211> 15
<212> PRT
<213> Homo sapiens

<400> 647

Cys Ala Ser Ser Ala Arg Ala Ser Gly Gly Asp Glu Gln Phe Phe
1 5 10 15

<210> 648
<211> 15
<212> PRT
<213> Homo sapiens

<400> 648

Cys Ala Ser Ser Ala Arg Thr Ser Gly Gly Gln Pro Gln His Phe
1 5 10 15

<210> 649
<211> 15
<212> PRT
<213> Homo sapiens

<400> 649

Cys Ala Ser Ser Ala Arg Thr Ser Gly Asn Gln Pro Gln His Phe
1 5 10 15

<210> 650
<211> 15
<212> PRT
<213> Homo sapiens

<400> 650

Cys Ala Ser Ser Glu Leu Ala Ser Gly Ile Asn Glu Gln Phe Phe
1               5                   10                  15

<210> 651
<211> 15
<212> PRT
<213> Homo sapiens

<400> 651

Cys Ala Ser Ser Glu Leu Thr Ser Gly Gly Asp Glu Gln Phe Phe
1               5                   10                  15

<210> 652
<211> 15
<212> PRT
<213> Homo sapiens

<400> 652

Cys Ala Ser Ser Lys Arg Thr Ser Gly Gly Asp Thr Gln Tyr Phe
1               5                   10                  15

<210> 653
<211> 15
<212> PRT
<213> Homo sapiens

<400> 653

Cys Ala Ser Ser Leu Arg Thr Ser Gly Gly Asp Glu Gln Tyr Phe
1               5                   10                  15

<210> 654
<211> 15
<212> PRT
<213> Homo sapiens

<400> 654

Cys Ala Ser Ser Pro Leu Thr Ser Ala Thr Asp Thr Gln Tyr Phe
1               5                   10                  15

<210> 655
<211> 15
<212> PRT
<213> Homo sapiens

<400> 655

Cys Ala Ser Ser Pro Arg Ala Ser Gly Gly Asp Glu Gln Phe Phe
1               5                   10                  15

<210> 656
<211> 15
<212> PRT

<213> Homo sapiens

<400> 656

Cys Ala Ser Ser Pro Arg Thr Ser Gly Gly Asp Glu Gln Tyr Phe
1 5 10 15

<210> 657
<211> 16
<212> PRT
<213> Homo sapiens

<400> 657

Cys Ala Ser Arg Leu Arg Thr Ser Gly Gly Thr Asp Thr Gln Tyr Phe
1 5 10 15

<210> 658
<211> 16
<212> PRT
<213> Homo sapiens

<400> 658

Cys Ala Ser Arg Arg Leu Ala Gly Phe Met Ala Asp Thr Gln Tyr Phe
1 5 10 15

<210> 659
<211> 16
<212> PRT
<213> Homo sapiens

<400> 659

Cys Ala Ser Ser Ala Arg Thr Ser Ala Gly Thr Asp Thr Gln Tyr Phe
1 5 10 15

<210> 660
<211> 16
<212> PRT
<213> Homo sapiens

<400> 660

Cys Ala Ser Ser Ala Arg Thr Ser Gly Gly Ala Asp Thr His Tyr Phe
1 5 10 15

<210> 661
<211> 16
<212> PRT
<213> Homo sapiens

<400> 661

Cys Ala Ser Ser Ala Arg Thr Ser Gly Gly Ala Asp Thr Gln Tyr Phe
1 5 10 15

<210> 662
<211> 16
<212> PRT
<213> Homo sapiens

<400> 662

Cys Ala Ser Ser Ala Arg Thr Ser Gly Gly Ser Asp Thr Gln Tyr Phe
1 5 10 15

<210> 663
<211> 16
<212> PRT
<213> Homo sapiens

<400> 663

Cys Ala Ser Ser Ala Arg Thr Ser Gly Gly Thr Asp Thr Gln Tyr Phe
1 5 10 15

<210> 664
<211> 16
<212> PRT
<213> Homo sapiens

<400> 664

Cys Ala Ser Ser Glu Arg Thr Ser Gly Gly Arg Asp Thr Gln Tyr Phe
1 5 10 15

<210> 665
<211> 16
<212> PRT
<213> Homo sapiens

<400> 665

Cys Ala Ser Ser Gly Arg Thr Ser Gly Gly Ser Asp Thr Gln Tyr Phe
1 5 10 15

<210> 666
<211> 16
<212> PRT
<213> Homo sapiens

<400> 666

Cys Ala Ser Ser Leu Arg Ala Ser Gly Gly Ser Asp Thr Gln Tyr Phe
1 5 10 15

<210> 667
<211> 16
<212> PRT
<213> Homo sapiens

<400> 667

Cys Ala Ser Ser Leu Arg Thr Ser Gly Gly Ala Asp Thr Gln Tyr Phe

1 5 10 15

<210> 668
<211> 16
<212> PRT
<213> Homo sapiens

<400> 668

Cys Ala Ser Ser Gln Arg Thr Ser Gly Gly Ala Asp Thr Gln Tyr Phe
1 5 10 15

<210> 669
<211> 16
<212> PRT
<213> Homo sapiens

<400> 669

Cys Ala Ser Ser Arg Leu Thr Ser Gly Gly Ser Asp Thr Gln Tyr Phe
1 5 10 15

<210> 670
<211> 16
<212> PRT
<213> Homo sapiens

<400> 670

Cys Ala Ser Ser Arg Arg Thr Ser Gly Gly Leu Asp Thr Gln Tyr Phe
1 5 10 15

<210> 671
<211> 16
<212> PRT
<213> Homo sapiens

<400> 671

Cys Ala Ser Ser Arg Arg Thr Ser Gly Gly Pro Asn Glu Gln Phe Phe
1 5 10 15

<210> 672
<211> 16
<212> PRT
<213> Homo sapiens

<400> 672

Cys Ala Ser Ser Arg Arg Thr Ser Gly Gly Thr Asp Thr Gln Tyr Phe
1 5 10 15

<210> 673
<211> 14
<212> PRT
<213> Homo sapiens

<400> 673

Cys Ala Ser Ser Glu Gly Trp Glu Pro Tyr Glu Gln Tyr Phe
1 5 10

<210> 674
<211> 15
<212> PRT
<213> Homo sapiens

<400> 674

Cys Ala Ser Ser Glu Leu Thr Ser Gly Gly Asp Glu Gln Phe Phe
1 5 10 15

<210> 675
<211> 15
<212> PRT
<213> Homo sapiens

<400> 675

Cys Ala Ser Ser Glu Leu Thr Ser Gly Gly Asp Glu Gln Leu Phe
1 5 10 15

<210> 676
<211> 16
<212> PRT
<213> Homo sapiens

<400> 676

Cys Ala Ser Ser Ala Arg Thr Ser Gly Gly Thr Asp Thr Gln Tyr Phe
1 5 10 15

<210> 677
<211> 18
<212> PRT
<213> Homo sapiens

<400> 677

Cys Ala Thr Thr Ala Ala Gly Thr Gly Val Asp Gly Asn Tyr Gly Tyr
1 5 10 15

Thr Phe

<210> 678
<211> 14
<212> PRT
<213> Homo sapiens

<400> 678

Cys Ala Ser Gln Arg Gly Asn Ser Tyr Asn Glu Gln Phe Phe
1 5 10

<210> 679
<211> 14
<212> PRT
<213> Homo sapiens

<400> 679

Cys Ala Ser Arg Ile Arg Thr Ser Gly Gly Glu Gln Tyr Phe
1 5 10

<210> 680
<211> 14
<212> PRT
<213> Homo sapiens

<400> 680

Cys Ala Ser Thr Arg Asp Arg Thr Lys Asn Glu Gln Phe Phe
1 5 10

<210> 681
<211> 15
<212> PRT
<213> Homo sapiens

<400> 681

Cys Ala Ser Ser Ala Leu Ala Ser Gly Gly Asp Glu Gln Phe Phe
1 5 10 15

<210> 682
<211> 15
<212> PRT
<213> Homo sapiens

<400> 682

Cys Ala Ser Ser Leu Arg Thr Ser Gly Gly Asp Glu Gln Phe Phe
1 5 10 15

<210> 683
<211> 15
<212> PRT
<213> Homo sapiens

<400> 683

Cys Ala Ser Ser Pro Met Ala Ser Gly Gly Asp Glu Gln Phe Phe
1 5 10 15

<210> 684
<211> 15
<212> PRT
<213> Homo sapiens

<400> 684

Cys Ala Ser Ser Pro Arg Thr Ser Gly Thr Asp Thr Gln Tyr Phe
1               5                   10                  15

<210> 685
<211> 15
<212> PRT
<213> Homo sapiens

<400> 685

Cys Ala Ser Ser Arg Arg Thr Ser Gly Thr Tyr Glu Gln Tyr Phe
1               5                   10                  15

<210> 686
<211> 15
<212> PRT
<213> Homo sapiens

<400> 686

Cys Ala Ser Ser Val Met Ala Ser Arg Gly Asn Glu Gln Phe Phe
1               5                   10                  15

<210> 687
<211> 15
<212> PRT
<213> Homo sapiens

<400> 687

Cys Val Ser Ser Ala Leu Ala Ser Gly Gly Asp Glu Gln Phe Phe
1               5                   10                  15

<210> 688
<211> 16
<212> PRT
<213> Homo sapiens

<400> 688

Cys Ala Ser Asn Lys Leu Thr Ser Gly Gly Arg Asp Thr Gln Tyr Phe
1               5                   10                  15

<210> 689
<211> 16
<212> PRT
<213> Homo sapiens

<400> 689

Cys Ala Ser Ser Ala Arg Thr Ser Gly Gly Ser Asp Thr Gln Tyr Phe
1               5                   10                  15

<210> 690
<211> 16
<212> PRT

<213> Homo sapiens

<400> 690

Cys Ala Ser Ser Lys Arg Thr Ser Gly Ala Thr Asp Glu Gln Phe Phe
1 5 10 15

<210> 691
<211> 16
<212> PRT
<213> Homo sapiens

<400> 691

Cys Ala Ser Ser Leu Leu Thr Ser Gly Gly Arg Glu Thr Gln Tyr Phe
1 5 10 15

<210> 692
<211> 16
<212> PRT
<213> Homo sapiens

<400> 692

Cys Ala Ser Ser Arg Leu Thr Ser Gly Gly Arg Asn Glu Gln Phe Phe
1 5 10 15

<210> 693
<211> 16
<212> PRT
<213> Homo sapiens

<400> 693

Cys Ala Ser Ser Ser Arg Thr Ser Gly Gly Thr Asp Thr Gln Tyr Phe
1 5 10 15

<210> 694
<211> 16
<212> PRT
<213> Homo sapiens

<400> 694

Cys Ala Thr Ser Arg Gly Ala Arg Gly Ser Asn Gln Pro Gln His Phe
1 5 10 15

<210> 695
<211> 12
<212> PRT
<213> Homo sapiens

<400> 695

Cys Ala Ser Arg Asp Gly Leu Gly Glu Leu Phe Phe
1 5 10

<210> 696
<211> 13
<212> PRT
<213> Homo sapiens

<400> 696

Cys Ala Ser Ser Gly Gln Thr Asn Thr Glu Ala Phe Phe
1 5 10

<210> 697
<211> 15
<212> PRT
<213> Homo sapiens

<400> 697

Cys Ala Ser Ser Ala Leu Ala Ser Gly Thr Asp Thr Gln Tyr Phe
1 5 10 15

<210> 698
<211> 15
<212> PRT
<213> Homo sapiens

<400> 698

Cys Ala Ser Ser Glu Leu Ala Ser Gly Gln Gly Ser Gln Tyr Phe
1 5 10 15

<210> 699
<211> 15
<212> PRT
<213> Homo sapiens

<400> 699

Cys Ala Ser Ser Glu Leu Ala Ser Gly Ser Tyr Glu Gln Tyr Phe
1 5 10 15

<210> 700
<211> 15
<212> PRT
<213> Homo sapiens

<400> 700

Cys Ala Ser Ser Glu Leu Ala Ser Gly Thr Gly Glu Gln Phe Phe
1 5 10 15

<210> 701
<211> 15
<212> PRT
<213> Homo sapiens

<400> 701

Cys Ala Ser Ser Pro Arg Thr Ser Gly Pro Tyr Glu Gln Tyr Phe

1 5 10 15

<210> 702
<211> 15
<212> PRT
<213> Homo sapiens

<400> 702

Cys Ala Ser Ser Gln Leu Thr Ser Gly Thr Asp Thr Gln Tyr Phe
1 5 10 15

<210> 703
<211> 15
<212> PRT
<213> Homo sapiens

<400> 703

Cys Ala Ser Ser Arg Leu Ala Gly Gly Phe Asp Glu Gln Phe Phe
1 5 10 15

<210> 704
<211> 15
<212> PRT
<213> Homo sapiens

<400> 704

Cys Ala Ser Ser Arg Leu Ala Ser Gly Thr Asp Thr Gln Tyr Phe
1 5 10 15

<210> 705
<211> 15
<212> PRT
<213> Homo sapiens

<400> 705

Cys Ala Ser Ser Arg Thr Val Ser Gly Asn Gln Pro Gln His Phe
1 5 10 15

<210> 706
<211> 15
<212> PRT
<213> Homo sapiens

<400> 706

Cys Ala Ser Ser Ser Leu Ala Ser Arg Pro Tyr Glu Gln Tyr Phe
1 5 10 15

<210> 707
<211> 15
<212> PRT
<213> Homo sapiens

<400>  707

Cys Ala Ser Thr Lys Gly Ala Ser Gly Ser Gly Glu Gln Phe Phe
1               5                   10                  15

<210>  708
<211>  15
<212>  PRT
<213>  Homo sapiens

<400>  708

Cys Ala Thr Thr Pro Gly Ala Ser Gly Ile Ser Glu Gln Phe Phe
1               5                   10                  15

<210>  709
<211>  16
<212>  PRT
<213>  Homo sapiens

<400>  709

Cys Ala Ser Ser Glu Arg Gly Gln Gly Ala Arg Tyr Glu Gln Tyr Phe
1               5                   10                  15

<210>  710
<211>  16
<212>  PRT
<213>  Homo sapiens

<400>  710

Cys Ala Ser Ser Arg Arg Thr Ser Gly Gly Thr Asp Thr Gln Tyr Phe
1               5                   10                  15

<210>  711
<211>  15
<212>  PRT
<213>  Homo sapiens

<400>  711

Cys Ala Ser Ala Arg Leu Ala Gly Gly Thr Asp Glu Gln Phe Phe
1               5                   10                  15

<210>  712
<211>  15
<212>  PRT
<213>  Homo sapiens

<400>  712

Cys Ala Ser Ser Ala Lys Ala Arg Gly Asn Gln Pro Gln His Phe
1               5                   10                  15

<210>  713
<211>  15

<212> PRT
<213> Homo sapiens

<400> 713

Cys Ala Ser Ser Ala Leu Ala Gly Gly Thr Asp Thr Gln Tyr Phe
1 5 10 15

<210> 714
<211> 15
<212> PRT
<213> Homo sapiens

<400> 714

Cys Ala Ser Ser Ala Leu Ala Ser Gly Thr Asp Thr Gln Tyr Phe
1 5 10 15

<210> 715
<211> 15
<212> PRT
<213> Homo sapiens

<400> 715

Cys Ala Ser Ser Asp Ala Ala Ser Gly Val Gly Glu Gln Tyr Phe
1 5 10 15

<210> 716
<211> 15
<212> PRT
<213> Homo sapiens

<400> 716

Cys Ala Ser Ser Gly Gln Ala Arg Gly Asn Gln Pro Gln His Phe
1 5 10 15

<210> 717
<211> 15
<212> PRT
<213> Homo sapiens

<400> 717

Cys Ala Ser Ser Pro Gly Thr Ser Gly Val Gly Glu Gln Phe Phe
1 5 10 15

<210> 718
<211> 15
<212> PRT
<213> Homo sapiens

<400> 718

Cys Ala Ser Ser Pro Ser Ala Arg Gly Asn Gln Pro Gln His Phe
1 5 10 15

<210> 719
<211> 15
<212> PRT
<213> Homo sapiens

<400> 719

Cys Ala Ser Ser Val Gly Thr Ser Gly Val Gly Glu Gln Tyr Phe
1 5 10 15

<210> 720
<211> 14
<212> PRT
<213> Homo sapiens

<400> 720

Cys Ala Ser Gln Ala Ser Gly Arg Ser Tyr Glu Gln Tyr Phe
1 5 10

<210> 721
<211> 14
<212> PRT
<213> Homo sapiens

<400> 721

Cys Ala Ser Ser Glu Phe Trp Gly Gln Glu Thr Gln Tyr Phe
1 5 10

<210> 722
<211> 14
<212> PRT
<213> Homo sapiens

<400> 722

Cys Ala Ser Ser Glu Leu Ala Ser Gly Asp Glu Gln Phe Phe
1 5 10

<210> 723
<211> 14
<212> PRT
<213> Homo sapiens

<400> 723

Cys Ala Ser Ser Val Ser Gln Gly Ser Asp Glu Gln Tyr Phe
1 5 10

<210> 724
<211> 15
<212> PRT
<213> Homo sapiens

<400> 724

Cys Ala Ser Cys Pro Met Ala Ser Arg Ser Tyr Glu Gln Tyr Phe
1               5                   10                  15

<210> 725
<211> 15
<212> PRT
<213> Homo sapiens

<400> 725

Cys Ala Ser Ile Ile Gly Ser Gln Gly Ala Tyr Gly Tyr Thr Phe
1               5                   10                  15

<210> 726
<211> 15
<212> PRT
<213> Homo sapiens

<400> 726

Cys Ala Ser Ser Ala Leu Ala Ser Gly Gly Asp Thr Gln Tyr Phe
1               5                   10                  15

<210> 727
<211> 15
<212> PRT
<213> Homo sapiens

<400> 727

Cys Ala Ser Ser Ala Arg Ala Ser Gly Gly Asp Glu Gln Phe Phe
1               5                   10                  15

<210> 728
<211> 15
<212> PRT
<213> Homo sapiens

<400> 728

Cys Ala Ser Ser Ala Arg Thr Ser Gly Asn Gln Pro Gln His Phe
1               5                   10                  15

<210> 729
<211> 15
<212> PRT
<213> Homo sapiens

<400> 729

Cys Ala Ser Ser Glu Arg Gly Thr Ala Asn Thr Glu Ala Phe Phe
1               5                   10                  15

<210> 730
<211> 15
<212> PRT
<213> Homo sapiens

<400> 730

Cys Ala Ser Ser Phe Arg Thr Ser Gly Gly Asp Thr Gln Tyr Phe
1 5 10 15

<210> 731
<211> 15
<212> PRT
<213> Homo sapiens

<400> 731

Cys Ala Ser Ser Lys Arg Ala Ser Gly Gly Asp Thr Gln Tyr Phe
1 5 10 15

<210> 732
<211> 15
<212> PRT
<213> Homo sapiens

<400> 732

Cys Ala Ser Ser Pro Arg Ala Ser Gly Gly Asp Glu Gln Phe Phe
1 5 10 15

<210> 733
<211> 15
<212> PRT
<213> Homo sapiens

<400> 733

Cys Ala Ser Ser Pro Arg Thr Ser Gly Gly Asp Glu Gln Tyr Phe
1 5 10 15

<210> 734
<211> 15
<212> PRT
<213> Homo sapiens

<400> 734

Cys Ala Ser Ser Pro Arg Thr Ser Gly Thr Tyr Glu Gln Tyr Phe
1 5 10 15

<210> 735
<211> 15
<212> PRT
<213> Homo sapiens

<400> 735

Cys Ala Ser Ser Pro Val Ala Arg Gly Pro Tyr Glu Gln Tyr Phe
1 5 10 15

<210> 736

<211>  15
<212>  PRT
<213>  Homo sapiens

<400>  736

Cys Ala Ser Ser Gln Leu Thr Ser Arg Thr Tyr Glu Gln Tyr Phe
1               5                   10                  15

<210>  737
<211>  16
<212>  PRT
<213>  Homo sapiens

<400>  737

Cys Ala Ser Ser Ala Arg Thr Ser Gly Gly Ser Asp Thr Gln Tyr Phe
1               5                   10                  15

<210>  738
<211>  16
<212>  PRT
<213>  Homo sapiens

<400>  738

Cys Ala Ser Ser Ala Arg Thr Ser Gly Gly Thr Asp Thr Gln Tyr Phe
1               5                   10                  15

<210>  739
<211>  16
<212>  PRT
<213>  Homo sapiens

<400>  739

Cys Ala Ser Ser Gly Arg Thr Ser Gly Gly Ser Asp Thr Gln Tyr Phe
1               5                   10                  15

<210>  740
<211>  16
<212>  PRT
<213>  Homo sapiens

<400>  740

Cys Ala Ser Ser Leu Arg Thr Ser Gly Gly Ala Asp Thr Gln Tyr Phe
1               5                   10                  15

<210>  741
<211>  16
<212>  PRT
<213>  Homo sapiens

<400>  741

Cys Ala Ser Ser Leu Arg Thr Ser Gly Gly Ser Asp Thr Gln Tyr Phe
1               5                   10                  15

<210> 742
<211> 16
<212> PRT
<213> Homo sapiens

<400> 742

Cys Ala Ser Ser Pro Leu Thr Ser Gly Gly Thr Asp Thr Gln Tyr Phe
1 5 10 15

<210> 743
<211> 16
<212> PRT
<213> Homo sapiens

<400> 743

Cys Ala Ser Ser Arg Arg Thr Ser Gly Ala Ser Asp Thr Gln Tyr Phe
1 5 10 15

<210> 744
<211> 16
<212> PRT
<213> Homo sapiens

<400> 744

Cys Ala Ser Ser Arg Arg Thr Ser Gly Gly Ala Asp Glu Gln Phe Phe
1 5 10 15

<210> 745
<211> 16
<212> PRT
<213> Homo sapiens

<400> 745

Cys Ala Ser Ser Arg Arg Thr Ser Gly Gly Thr Asp Thr Gln Tyr Phe
1 5 10 15

<210> 746
<211> 16
<212> PRT
<213> Homo sapiens

<400> 746

Cys Ala Ser Ser Arg Arg Thr Ser Gly Gly Tyr Asp Glu Gln Phe Phe
1 5 10 15

<210> 747
<211> 16
<212> PRT
<213> Homo sapiens

<400> 747

Cys Ala Ser Ser Ser Arg Thr Ser Gly Gly Ser Asp Thr Gln Tyr Phe
1               5                   10                  15

<210>  748
<211>  16
<212>  PRT
<213>  Homo sapiens

<400>  748

Cys Ala Ser Ser Val Arg Thr Ser Gly Gly Ser Asp Thr Gln Tyr Phe
1               5                   10                  15

<210>  749
<211>  17
<212>  PRT
<213>  Homo sapiens

<400>  749

Cys Ala Ser Ser Ile Ser Leu Pro Gly Thr Leu Tyr Tyr Glu Gln Tyr
1               5                   10                  15

Phe

<210>  750
<211>  39
<212>  DNA
<213>  Homo sapiens

<400>  750
tgtgccagct tagggcccct acggcacgag cagtacttc    39

<210>  751
<211>  42
<212>  DNA
<213>  Homo sapiens

<400>  751
tgtgccagca aaccactagt tagcacagat acgcagtatt tt    42

<210>  752
<211>  42
<212>  DNA
<213>  Homo sapiens

<400>  752
tgtgccagcc ttgaaaggac tagcgggggt gagcagttct tc    42

<210>  753
<211>  42
<212>  DNA
<213>  Homo sapiens

<400> 753
tgtgccagca cccgggacag gacaaagaat gagcagttct tc 42

<210> 754
<211> 45
<212> DNA
<213> Homo sapiens

<400> 754
tgtgccagca gtgcgttggc tagcgggga gatgagcagt tcttc 45

<210> 755
<211> 45
<212> DNA
<213> Homo sapiens

<400> 755
tgtgccagca gtgcgttagc tagcggtaca gatacgcagt atttt 45

<210> 756
<211> 45
<212> DNA
<213> Homo sapiens

<400> 756
tgtgccagca gtgaactgac ctctagaacc tacgagcagt acttc 45

<210> 757
<211> 45
<212> DNA
<213> Homo sapiens

<400> 757
tgtgccagca gtgaacgggt ttcgggcaat cagccccagc atttt 45

<210> 758
<211> 45
<212> DNA
<213> Homo sapiens

<400> 758
tgtgccagca gccctatggc tagcgggggg gatgagcagt tcttc 45

<210> 759
<211> 45
<212> DNA
<213> Homo sapiens

<400> 759
tgtgccagca gccgtaggac tagcgggact tacgagcagt acttc 45

<210> 760
<211> 45
<212> DNA
<213> Homo sapiens

<400> 760
tgtgccagca gtgtgatggc tagccgtggg aatgagcagt tcttc 45

<210> 761
<211> 48
<212> DNA
<213> Homo sapiens

<400> 761
tgtgccagcc aaagggggc tcgggggggc aatcagcccc agcatttt 48

<210> 762
<211> 48
<212> DNA
<213> Homo sapiens

<400> 762
tgtgccagca gggctcgaac aggggcaact aatgaaaaac tgtttttt 48

<210> 763
<211> 48
<212> DNA
<213> Homo sapiens

<400> 763
tgtgccagca gtgccaagac tagcgggggc tcagatacgc agtatttt 48

<210> 764
<211> 48
<212> DNA
<213> Homo sapiens

<400> 764
tgtgccagca gcgccctggc tagcgggggc cgggatacgc agtatttt 48

<210> 765
<211> 48
<212> DNA
<213> Homo sapiens

<400> 765
tgtgccagca gtgcccggac tagcggggga ctcgatgagc agttcttc 48

<210> 766
<211> 48
<212> DNA
<213> Homo sapiens

<400> 766
tgtgccagca gtgccaggac tagcgggggc tcggatacgc agtatttt 48

<210> 767
<211> 48
<212> DNA
<213> Homo sapiens

<400> 767
tgtgccagca gtaagagggc tagcgggggc acagatacgc agtatttt 48

<210> 768
<211> 48
<212> DNA
<213> Homo sapiens

<400> 768
tgtgccagca gcctaaggac tagcggggt tcagatacgc agtatttt 48

<210> 769
<211> 48
<212> DNA
<213> Homo sapiens

<400> 769
tgtgccagca gccgcttgac gagcgggggg cggaatgagc agttcttc 48

<210> 770
<211> 48
<212> DNA
<213> Homo sapiens

<400> 770
tgtgccagca gttctaagac tagcgggggc acagatacgc agtatttt 48

<210> 771
<211> 48
<212> DNA
<213> Homo sapiens

<400> 771
tgtgccagca gttcaaggac tagcgggggc caagatgagc agttcttc 48

<210> 772
<211> 48
<212> DNA
<213> Homo sapiens

<400> 772
tgtgccacct ccagaggagc gcggggaagc aatcagcccc agcatttt 48

<210> 773
<211> 39
<212> DNA
<213> Homo sapiens

<400> 773
tgtgccagcg ccaggacagg gggcgttggc tacaccttc 39

<210> 774
<211> 39
<212> DNA
<213> Homo sapiens

<400> 774
tgtgccagca ggactagcgg gacctacgag cagtacttc 39

<210> 775
<211> 42
<212> DNA
<213> Homo sapiens

<400> 775
tgtgccagca aagcaaaaac ggtaacctac aagcagtact tc 42

<210> 776
<211> 42
<212> DNA
<213> Homo sapiens

<400> 776
tgtgccagca aaccaaaagc ggtaacctac gagcagtact tc 42

<210> 777
<211> 42
<212> DNA
<213> Homo sapiens

<400> 777
tgtgccagca gagggacagc gactggaaac accatatatt tt 42

<210> 778
<211> 42
<212> DNA
<213> Homo sapiens

<400> 778
tgtgccagca gaccgacagc aactaatgaa aaactgtttt tt 42

<210> 779
<211> 42
<212> DNA
<213> Homo sapiens

<400> 779
tgtgccagca gtgaatatgc gactagcaat gagcagttct tc 42

<210> 780
<211> 42
<212> DNA
<213> Homo sapiens

<400> 780
tgtgccagca gtcgtggaca gcggcacaga tacgcagtat tt 42

<210> 781
<211> 45
<212> DNA
<213> Homo sapiens

<400> 781
tgtgccatct cgcgtctagc gggaggcatg gacgagcagt acttc 45

<210> 782
<211> 45
<212> DNA
<213> Homo sapiens

<400> 782
tgtgccagcg gccgactagc atcgggcaca gatacgcagt atttt 45

<210> 783
<211> 45
<212> DNA
<213> Homo sapiens

<400> 783
tgtgccagca ggagggggac tagcggcacc ggggagctgt ttttt 45

<210> 784
<211> 45
<212> DNA
<213> Homo sapiens

<400> 784
tgtgccagca gtgatggggc tagcggggtg ggagagcagt acttc 45

<210> 785
<211> 45
<212> DNA
<213> Homo sapiens

<400> 785
tgtgccagca gtgaagctgc caggggtaat tcacccctcc acttt 45

<210> 786
<211> 45
<212> DNA
<213> Homo sapiens

<400> 786
tgtgccagca gtgaaggggc tagcgggctc ggggagcagt acttc 45

<210> 787
<211> 45
<212> DNA
<213> Homo sapiens

<400> 787
tgtgccagca gtgaactggc tagcgggata agtgagcagt tcttc 45

<210> 788
<211> 45
<212> DNA
<213> Homo sapiens

<400> 788
tgtgccagca gtgaacttgc tagcgggctc gcagagcagt tcttc 45

<210> 789
<211> 45
<212> DNA
<213> Homo sapiens

<400> 789
tgtgccagca gtgaactggc tagcgggacg ggtgagcagt tcttc 45

<210> 790
<211> 45
<212> DNA
<213> Homo sapiens

<400> 790
tgtgccagca gtgaacgggc tagcgggacc gacgagcagt acttc 45

<210> 791
<211> 45
<212> DNA
<213> Homo sapiens

<400> 791
tgtgccagca gtgaaagggc tagcggggtc ggggagctgt ttttt 45

<210> 792
<211> 45
<212> DNA
<213> Homo sapiens

<400> 792
tgtgccagca gcggactggc tagcggcaca gatacgcagt atttt 45

<210> 793
<211> 45
<212> DNA
<213> Homo sapiens

<400> 793
tgtgccagtt cgggactagc gtcgggcacc ggggagctgt ttttt 45

<210> 794
<211> 45
<212> DNA
<213> Homo sapiens

<400> 794
tgtgccagca gcgggatgac tagccgatcc tacgagcagt acttc 45

<210> 795
<211> 45
<212> DNA
<213> Homo sapiens

<400> 795
tgtgccagca gcccgggact agccggcacc ggggagctgt ttttt 45

<210> 796
<211> 45
<212> DNA
<213> Homo sapiens

<400> 796
tgtgccagca gtccgttgac tagcggaaca gatacgcagt atttt 45

<210> 797
<211> 45
<212> DNA
<213> Homo sapiens

<400> 797
tgtgccagca gtcctcgggc caggggggaat cagccccagc atttt 45

<210> 798
<211> 45
<212> DNA
<213> Homo sapiens

<400> 798
tgtgccagca gccctcggac tagcggtccc tacgagcagt acttc 45

<210> 799
<211> 45
<212> DNA
<213> Homo sapiens

<400> 799
tgtgccagca gtcctcggac tagcgggagt tacgagcagt acttc 45

<210> 800
<211> 45
<212> DNA
<213> Homo sapiens

<400> 800
tgtgccagca gccaagggct agcgggcacc ggggagctgt ttttt 45

<210> 801
<211> 45
<212> DNA
<213> Homo sapiens

<400> 801
tgtgccagca gtcaattagc taggggcaca gatacgcagt atttt 45

<210> 802
<211> 45
<212> DNA
<213> Homo sapiens

<400> 802
tgtgccagca gtcaacttgt atcgctgagg ggggagcagt acttc 45

<210> 803
<211> 45
<212> DNA
<213> Homo sapiens

<400> 803
tgtgccagca gtcaacggac tagcgggagc gacgagcagt acttc 45

<210> 804
<211> 45
<212> DNA
<213> Homo sapiens

<400> 804
tgtgccagca gccaagttgc gggggtaca gctacgcagt atttt 45

<210> 805
<211> 45
<212> DNA
<213> Homo sapiens

<400> 805
tgtgccagct cccgggggc tcggggcaat cagccccagc atttt 45

<210> 806
<211> 45
<212> DNA
<213> Homo sapiens

<400> 806
tgtgccagca gccgactagc gggaggttta ggtgagcagt tcttc 45

<210> 807
<211> 45
<212> DNA
<213> Homo sapiens

<400> 807
tgtgccagca gccgactagc gggagggatg gatgagcagt tcttc 45

<210> 808
<211> 45
<212> DNA
<213> Homo sapiens

<400> 808
tgtgccagca gccgactagc gggagggacg gatgagcagt tcttc 45

<210> 809
<211> 45
<212> DNA
<213> Homo sapiens

<400> 809
tgtgccagca gtcgtctgac tagcggcaca gatacgcagt atttt 45

<210> 810
<211> 45
<212> DNA
<213> Homo sapiens

<400> 810
tgtgccagca gccgagtgac gggagggatg gatgagcagt tcttc 45

<210> 811
<211> 45
<212> DNA
<213> Homo sapiens

<400> 811
tgtgccagca ccaaactagc gggggtaca tctgagcagt tcttc 45

<210> 812
<211> 45
<212> DNA
<213> Homo sapiens

<400> 812
tgtgccagca ccaaactagc gtggggcaca tatacgcagt atttt 45

<210> 813
<211> 45
<212> DNA
<213> Homo sapiens

<400> 813
tgtgccacca cccccggggc tagcgggata agtgagcagt tcttc 45

<210> 814
<211> 45
<212> DNA
<213> Homo sapiens

<400> 814
tgtgccacca cccccggggc tagtgggata agtgagcagt tcttc 45

<210> 815
<211> 48
<212> DNA
<213> Homo sapiens

<400> 815
tgtgccagca gtgaaagggg acagggggcg cggtacgagc agtacttc 48

<210> 816
<211> 48
<212> DNA
<213> Homo sapiens

<400> 816
tgtgccagca gtcgtatgac tggcgggggc acagatacgc agtatttt 48

<210> 817
<211> 48
<212> DNA
<213> Homo sapiens

<400> 817
tgtgccagca gtcgtaggac tagcgggggc acagatacgc agtatttt 48

<210> 818
<211> 39
<212> DNA
<213> Homo sapiens

<400> 818
tgtgccagtc accggacata cacagatacg cagtatttt 39

<210> 819
<211> 39
<212> DNA
<213> Homo sapiens

<400> 819
tgtgccagct caggacagac gaacactgaa gctttcttt 39

<210> 820
<211> 42
<212> DNA
<213> Homo sapiens

<400> 820
tgtgccagca gatggacagc aacctcttat ggctacacct tc 42

<210> 821
<211> 42
<212> DNA
<213> Homo sapiens

<400> 821
tgtgccagca gtcccacaac gacagggtat ggctacacct tc 42

<210> 822
<211> 45
<212> DNA
<213> Homo sapiens

<400> 822
tgtgcctccc acgaaggggc cgggggcttc ggggagctgt ttttt 45

<210> 823
<211> 45
<212> DNA
<213> Homo sapiens

<400> 823
tgtgcctccc acgaaggggc cgggggctac ggggagctgt ttttt 45

<210> 824
<211> 45
<212> DNA
<213> Homo sapiens

<400> 824
tgtgccagca gtgccggaac tagaggggtg ggggagcagt tcttc 45

<210> 825
<211> 45
<212> DNA
<213> Homo sapiens

<400> 825
tgtgccagca gtgctttggc tagcggcaca gatacgcagt atttt 45

<210> 826
<211> 45
<212> DNA
<213> Homo sapiens

<400> 826
tgtgccagca gtgacgcggc tagcggtgtg ggcgagcagt acttc 45

<210> 827
<211> 45
<212> DNA
<213> Homo sapiens

<400> 827
tgtgccagca gtgatctggc tagcgggacg aatgagcagt tcttc 45

<210> 828
<211> 45
<212> DNA
<213> Homo sapiens

<400> 828
tgtgccagca gtgaccgggc tagcggggtc ggggagcagt tcttc 45

<210> 829
<211> 45
<212> DNA
<213> Homo sapiens

<400> 829
tgtgccagca gtgacaggac tagcggtccc catgagcagt tcttc 45

<210> 830
<211> 45
<212> DNA
<213> Homo sapiens

<400> 830
tgtgccagca gcggactagc gggaggaatg gatgagcagt tcttc 45

<210> 831
<211> 45
<212> DNA
<213> Homo sapiens

<400> 831
tgtgccagca gccccggggc gagaggaatt gatgagcagt tcttc 45

<210> 832
<211> 45
<212> DNA
<213> Homo sapiens

<400> 832
tgtgcaagca gccccgggac tagcggagtt ggtgagcagt tcttc 45

<210> 833
<211> 45
<212> DNA
<213> Homo sapiens

<400> 833
tgtgcaagca gccccgggac tagcggagtt ggtgagcagt ttttc 45

<210> 834
<211> 45
<212> DNA
<213> Homo sapiens

<400> 834
tgtgcaagca gccccgggac gagcggagtt ggtaagcagt ttttc 45

<210> 835
<211> 45
<212> DNA
<213> Homo sapiens

<400> 835
tgtgccagca gccccaggac tagcgggga ggcgagcagt acttc 45

<210> 836
<211> 45
<212> DNA
<213> Homo sapiens

<400> 836
tgtgccagca gtcccagtgc tcgcggcaat cagccccagc atttt 45

<210> 837
<211> 45
<212> DNA
<213> Homo sapiens

<400> 837
tgtgccagca gcccgacgac tagcgggaga ggcgagcagt acttc 45

<210> 838
<211> 45
<212> DNA
<213> Homo sapiens

<400> 838
tgtgccagca gttccgggac tagcggggcc ggggagcagt tcttc 45

<210> 839
<211> 45
<212> DNA
<213> Homo sapiens

<400> 839
tgtgccagca gttccgggac tagcggagtt ggtgagcagt tcttc 45

<210> 840
<211> 45
<212> DNA
<213> Homo sapiens

<400> 840
tgtgccagca gttccgggac tagcggggtc ggggagcagt tcttc 45

<210> 841
<211> 45
<212> DNA
<213> Homo sapiens

<400> 841
tgtgccagca gtgtcgggac tagcggggtg ggcgagcagt acttc 45

<210> 842
<211> 45
<212> DNA
<213> Homo sapiens

<400> 842
tgtgccagca gttacggggc tagcggggtg ggggagcagt tcttc 45

<210> 843
<211> 45
<212> DNA
<213> Homo sapiens

<400> 843
tgtgccagca gttacaggac tagcgggccc cgggagcagt tcttc 45

<210> 844
<211> 36
<212> DNA
<213> Homo sapiens

<400> 844
tgtgccagca ggaaagaagg atctaggcta cacctc 36

<210> 845
<211> 39
<212> DNA
<213> Homo sapiens

<400> 845
tgtgccagca gtgacagaac gacatgtggc tacaccttc 39

<210> 846
<211> 39
<212> DNA
<213> Homo sapiens

<400> 846
tgtgccagca gtgaaagaag gatctatggc tacaccttc 39

<210> 847
<211> 42
<212> DNA
<213> Homo sapiens

<400> 847
tgtgccagca gagccctagc gtcgggggt gagcagttct tc 42

<210> 848
<211> 42
<212> DNA
<213> Homo sapiens

<400> 848
tgtgccagca gtgctttagc tagcggagat aagcagtatt tt 42

<210> 849
<211> 42
<212> DNA
<213> Homo sapiens

<400> 849
tgtgccagca gtgctttggc tagcggagat acgcagtatt tt 42

<210> 850
<211> 42
<212> DNA
<213> Homo sapiens

<400> 850
tgtgccagca gtgacgacag ggtcggcgat gagcagttct tc 42

<210> 851
<211> 42
<212> DNA
<213> Homo sapiens

<400> 851
tgtgccagca gcaaactagc gtctggagat gagcagttct tc 42

<210> 852
<211> 42
<212> DNA
<213> Homo sapiens

<400> 852
tgtgccagca gtgttttacc tggaggcaat gatccgctct tc 42

<210> 853
<211> 42
<212> DNA
<213> Homo sapiens

<400> 853
tgtgccagca gtgttttacc tggtcgcaat gagccgttct tc 42

<210> 854
<211> 42
<212> DNA
<213> Homo sapiens

<400> 854
tgcgccagca gtgttttacg tggtggcaat gagcagtttt tc 42

<210> 855
<211> 42
<212> DNA
<213> Homo sapiens

<400> 855
tgtgccagca gtgttttacg tggtcgcaat gagccgttct tc 42

<210> 856
<211> 42
<212> DNA
<213> Homo sapiens

<400> 856
tgtgccagca gtgttttacg tggtcgcaat gagcagttct tc 42

<210> 857
<211> 42
<212> DNA
<213> Homo sapiens

<400> 857
tgtgccagca gtgtttcacg aggtggcaat aagcagtttt tc 42

<210> 858
<211> 42
<212> DNA
<213> Homo sapiens

<400> 858
tgtgccagtg tattaatgag gacgaacaat gagcagttct tc 42

<210> 859
<211> 42
<212> DNA
<213> Homo sapiens

<400> 859
tgctccagca gagctagagg gtgcgcgggt aagcagtatt tc 42

<210> 860
<211> 45
<212> DNA
<213> Homo sapiens

<400> 860
tgtgccagca gtgccctgac tagcgggggc gatgagcagt tcttc 45

<210> 861
<211> 45
<212> DNA
<213> Homo sapiens

<400> 861
tgtgccagca gtgcaaggac tagcggggga tccgagcagt tcttc 45

<210> 862
<211> 45
<212> DNA
<213> Homo sapiens

<400> 862
tgtgccagca gtgctaggac tagcgggagt gacgagcagt acttc 45

<210> 863
<211> 45
<212> DNA
<213> Homo sapiens

<400> 863
tgtgccagca gtgacagggc ctcaggcggg gatacgcagt atttt 45

<210> 864
<211> 45
<212> DNA
<213> Homo sapiens

<400> 864
tgtgccagca gtgatcgggc tagcgggggg gatacgcagt atttt 45

<210> 865
<211> 45
<212> DNA
<213> Homo sapiens

<400> 865
tgtgccagca gtgatcgggc tacagggggg gatacgcagt atttt 45

<210> 866
<211> 45
<212> DNA
<213> Homo sapiens

<400> 866
tgtgccagca gtgaaaaggc tagcgggggg gatacgcagt atttt 45

<210> 867
<211> 45
<212> DNA
<213> Homo sapiens

<400> 867
tgtgccagca gtgaattggc tagcgggggg gatgagcagt tcttc 45

<210> 868
<211> 45
<212> DNA
<213> Homo sapiens

<400> 868
tgtgccagca gtcacaaggc ttcagggggg gataagcagt atttt 45

<210> 869
<211> 45
<212> DNA
<213> Homo sapiens

<400> 869
tgtgccagct ctcacatggc ctcaggcggc gatacgcagt atttt 45

<210> 870
<211> 45
<212> DNA
<213> Homo sapiens

<400> 870
tgtgccagca gtcacagggc ctcaggcggg gctactccgt atttt 45

<210> 871
<211> 45
<212> DNA
<213> Homo sapiens

<400> 871
tgtgccagca gtcaccgggc ctcaggtggg gatactccgc atttt 45

<210> 872
<211> 45
<212> DNA
<213> Homo sapiens

<400> 872
tgtgccagca gtcacagggc ctcaggcggg gatacgcagt atttt 45

<210> 873
<211> 45
<212> DNA
<213> Homo sapiens

<400> 873
tgtgccagct caaaactggc tagcggggcc gacgagcagt acttc 45

<210> 874
<211> 45
<212> DNA
<213> Homo sapiens

<400> 874
tgtgccagct caaaactggc tagcggggcc gacgagcagt atttc 45

<210> 875
<211> 45
<212> DNA
<213> Homo sapiens

<400> 875
tgtgccagct caaaacttac taggggcgca gataagcagt atttc 45

<210> 876
<211> 45
<212> DNA
<213> Homo sapiens

<400> 876
tgtgccagca gtaagaggac tagcggtacc tacgagcagt acttc 45

<210> 877
<211> 45
<212> DNA
<213> Homo sapiens

<400> 877
tgtgccagca gcctccggac tagcggctcc tacgagcagt acttc 45

<210> 878
<211> 45
<212> DNA
<213> Homo sapiens

<400> 878
tgtgccagca gtccccggac tagcggtacc tacgagcagt acttc 45

<210> 879
<211> 45
<212> DNA
<213> Homo sapiens

<400> 879
tgtgccagct cgccgcgagt cttctctgtc ggggagctgt ttttt 45

<210> 880
<211> 45
<212> DNA
<213> Homo sapiens

<400> 880
tgtgccagca gccaaagggc tagcgggggg gacgagcagt tcttc 45

<210> 881
<211> 45
<212> DNA
<213> Homo sapiens

<400> 881
tgtgccagca gtagacggac tagcgggacc tacgagcagt acttc 45

<210> 882
<211> 45
<212> DNA
<213> Homo sapiens

<400> 882
tgtgccagca gcgtccggac tagcgggtcc tacgagcagt acttc 45

<210> 883
<211> 45
<212> DNA
<213> Homo sapiens

<400> 883
tgcaccagca gtggtaggac tagcgggagg gataagcagt atttc 45

<210> 884
<211> 45
<212> DNA
<213> Homo sapiens

<400> 884
tgtaccagca gtcacagggc ctcaggcggg gatacgcagt atttt 45

<210> 885
<211> 48
<212> DNA
<213> Homo sapiens

<400> 885
tgtgccagca gtgccaggat tagcgggggg ctcaacgagc agtacttc 48

<210> 886
<211> 48
<212> DNA
<213> Homo sapiens

<400> 886
tgtgccagca gtgcaaggac tagcgggggg gccgatacgc agtatttt 48

<210> 887
<211> 48
<212> DNA
<213> Homo sapiens

<400> 887
tgtgccagca gtgccaggac tagcgggggg cttgacgagc agtacttc 48

<210> 888
<211> 48
<212> DNA
<213> Homo sapiens

<400> 888
tgtgccagca gtgctcggac tagcgggggg tcagatacgc agtatttt 48

<210> 889
<211> 48
<212> DNA
<213> Homo sapiens

<400> 889
tgtgccagca gtaaaaggac tagcgggggg gccgatacgc agtatttt 48

<210> 890
<211> 48
<212> DNA
<213> Homo sapiens

<400> 890
tgtgccagca gtcttaggac tagcgggggc acagatacgc agtatttt 48

<210> 891
<211> 48
<212> DNA
<213> Homo sapiens

<400> 891
tgtgccagca gccccaggac tagcgggggc acagatacgc agtatttt 48

<210> 892
<211> 48
<212> DNA
<213> Homo sapiens

<400> 892
tgtgccagct cacgacgggc ttccgggggc actactccgc attatttt 48

<210> 893
<211> 48
<212> DNA
<213> Homo sapiens

<400> 893
tgtgccagca gtcgacggac tagcgggggc acagatacgc agtatttt 48

<210> 894
<211> 48
<212> DNA
<213> Homo sapiens

<400> 894
tgtgccagct cacgacggac tagcgggggc acagatacgc agtatttt 48

<210> 895
<211> 48
<212> DNA
<213> Homo sapiens

<400> 895
tgtgccagca gtcgtcggac tagcgggagg gcggatacgc agtatttt 48

<210> 896
<211> 48
<212> DNA
<213> Homo sapiens

<400> 896
tgtgccagca gtcgtaggac tagcgggagt ctagatacgc agtatttt 48

<210> 897
<211> 48
<212> DNA
<213> Homo sapiens

<400> 897
tgtgccagca gcaccaggat tagagggggc acagataagc agtatttt 48

<210> 898
<211> 48
<212> DNA
<213> Homo sapiens

<400> 898
tgtgccagca gtgttaggac tagcgggggc acagatacgc agtatttt 48

<210> 899
<211> 36
<212> DNA
<213> Homo sapiens

<400> 899
tgtgccagca gagacagtaa ctatggctac accttc 36

<210> 900
<211> 36
<212> DNA
<213> Homo sapiens

<400> 900
tgtgccagca gtcgacggac ggagacccag tacttc 36

<210> 901
<211> 39
<212> DNA
<213> Homo sapiens

<400> 901
tgtgccagca gtgaaaccag ggccaacatt cagtacttc 39

<210> 902
<211> 39
<212> DNA
<213> Homo sapiens

<400> 902
tgtgccagca gcggactagc ggcgaatgag cagttcttc 39

<210> 903
<211> 42
<212> DNA
<213> Homo sapiens

<400> 903
tgtgcctcaa gggcagggtc ggtggccact gaagctttct tt 42

<210> 904
<211> 42
<212> DNA
<213> Homo sapiens

<400> 904
tgtgccagca gtgggaggac tagcggcaat gagcagttct tc 42

<210> 905
<211> 42
<212> DNA
<213> Homo sapiens

<400> 905
tgtgccagca gtgtcgtggg cagttacaat gagcagttct tc 42

<210> 906
<211> 42
<212> DNA
<213> Homo sapiens

<400> 906
tgtgccagtg tattaatgag gacgaacaat gagcagttct tc 42

<210> 907
<211> 45
<212> DNA
<213> Homo sapiens

<400> 907
tgtgccagca gggccgggac ctcgggcacc ggggagctgt ttttt 45

<210> 908
<211> 45
<212> DNA
<213> Homo sapiens

<400> 908
tgtgccagca ggaaggggac tagcgggagt ggcaagcagt acttc 45

<210> 909
<211> 45
<212> DNA
<213> Homo sapiens

<400> 909
tgtgccagca gtgaaaaggc tagcggggtg gatgagcagt tcttc 45

<210> 910
<211> 45
<212> DNA
<213> Homo sapiens

<400> 910
tgtgccagca gtgaaagggc tagcgggcac gatacgcagt atttt 45

<210> 911
<211> 45
<212> DNA
<213> Homo sapiens

<400> 911
tgtgccagca gtcacagggc ctcaggcggg gatacgcagt atttt 45

<210> 912
<211> 45
<212> DNA
<213> Homo sapiens

<400> 912
tgtgccagct caaaactggc tagcggggcc gacgagcagt acttc 45

<210> 913
<211> 45
<212> DNA
<213> Homo sapiens

<400> 913
tgtgccagca gtaaacaggc tagcgggggg gacgagcagt acttc 45

<210> 914
<211> 42
<212> DNA
<213> Homo sapiens

<400> 914
tgtgccagca gagaatacgc gactagcaac gagcagtact tc 42

<210> 915
<211> 42
<212> DNA
<213> Homo sapiens

<400> 915
tgtgccagca gtgaaatggc gaccgggttg cgctacacct tc 42

<210> 916
<211> 42
<212> DNA
<213> Homo sapiens

<400> 916
tgtgccagca gtgaaacggc gacagggttg cgctacacct tc 42

<210> 917
<211> 42
<212> DNA
<213> Homo sapiens

<400> 917
tgtgccagca cgctaacacg ggttaattca cccctccact tt 42

<210> 918
<211> 45
<212> DNA
<213> Homo sapiens

<400> 918
tgtgccagca atcaacggac tagcgggcct tacgagcagt acttc 45

<210> 919
<211> 45
<212> DNA
<213> Homo sapiens

<400> 919
tgtgccagca gtgatttggc tagcggcaca ggggagcagt tcttc 45

<210> 920
<211> 45
<212> DNA
<213> Homo sapiens

<400> 920
tgtgccagca gtgaagctgc aggggctac ggtgagcagt tcttc 45

<210> 921
<211> 45
<212> DNA
<213> Homo sapiens

<400> 921
tgtgccagca gtgaatttgc caggggcaat cagccccagc atttt 45

<210> 922
<211> 45
<212> DNA
<213> Homo sapiens

<400> 922
tgtgccagca gtgaattcgt aagggacaat cagccccagc atttt 45

<210> 923
<211> 45
<212> DNA
<213> Homo sapiens

<400> 923
tgtgccagca gtgaattcgt aaggggcaat cagccccagc atttt 45

<210> 924
<211> 45
<212> DNA
<213> Homo sapiens

<400> 924
tgtgccagca gtgaaggggc ggctggcaat cagccccagc atttt 45

<210> 925
<211> 45
<212> DNA
<213> Homo sapiens

<400> 925
tgtgccagca gtgaaggagc taggggcgtg gggagcagt tcttc 45

<210> 926
<211> 45
<212> DNA
<213> Homo sapiens

<400> 926
tgtgccagca gtgaaggggc tagcggtacg ggggcccagt acttc 45

<210> 927
<211> 45
<212> DNA
<213> Homo sapiens

<400> 927
tgtgccagca gtgaaggcgc tagtggcgta ggggagcagt tcttc 45

<210> 928
<211> 45
<212> DNA
<213> Homo sapiens

<400> 928
tgtgccagca gtgagggtac tagcaccttt cgtgagcagt tcttc 45

<210> 929
<211> 45
<212> DNA
<213> Homo sapiens

<400> 929
tgtgccagca gtgaattagc gagcggcacc ggggagctgt ttttt 45

<210> 930
<211> 45
<212> DNA
<213> Homo sapiens

<400> 930
tgtgccagca gtggggcagc gaggggcaat cagccccagc atttt 45

<210> 931
<211> 45
<212> DNA
<213> Homo sapiens

<400> 931
tgtgccagca gcaaactgac tagcggggga tacgagcagt acttc 45

<210> 932
<211> 45
<212> DNA
<213> Homo sapiens

<400> 932
tgtgccagca gtcccgggac tagcggggtt ggtgagcagt tcttc 45

<210> 933
<211> 45
<212> DNA
<213> Homo sapiens

<400> 933
tgtgccagca gtcgtctagc gggggtttc gatgagcagt tcttc 45

<210> 934
<211> 45
<212> DNA
<213> Homo sapiens

<400> 934
tgtgccagca gtcgactagc gggggcaca gatacgcagt atttt 45

<210> 935
<211> 45
<212> DNA
<213> Homo sapiens

<400> 935
tgtgccagca gtagactagc gtctggcaca gatacgcagt atttt 45

<210> 936
<211> 45
<212> DNA
<213> Homo sapiens

<400> 936
tgtgccagca gtgtattggc tagcgggctg ggtgagcagt acttc 45

<210> 937
<211> 48
<212> DNA
<213> Homo sapiens

<400> 937
tgtgccagca gacagggggc gaggggaggc aatcagcccc agcatttt 48

<210> 938
<211> 48
<212> DNA
<213> Homo sapiens

<400> 938
tgtgccagct cacagggggc gcgggggga aatcagcccc agcatttt 48

<210> 939
<211> 48
<212> DNA
<213> Homo sapiens

<400> 939
tgtgccagca gtcgattagc gggggggagc tcctacgagc agtacttc 48

<210> 940
<211> 48
<212> DNA
<213> Homo sapiens

<400> 940
tgtgccagca gccgcctgac tagcgggggg gcagatacgc agtatttt 48

<210> 941
<211> 48
<212> DNA
<213> Homo sapiens

<400> 941
tgtgccagca gccgccggac tagcgggggc acagatacgc agtatttt 48

<210> 942
<211> 42
<212> DNA
<213> Homo sapiens

<400> 942
tgtgccagca gtcctcgggc ctcgggggga gagcagtact tc 42

<210> 943
<211> 42
<212> DNA
<213> Homo sapiens

<400> 943
tgtgccagca gtgtgcggcg gaataatgaa aaactgtttt tt 42

<210> 944
<211> 45
<212> DNA
<213> Homo sapiens

<400> 944
tgtgccagca accgaaggac tagcggaacc tacgagcagt acttc 45

<210> 945
<211> 45
<212> DNA
<213> Homo sapiens

<400> 945
tgtgccagca gcgcactagc gtccggggga gatacgcagt atttt 45

<210> 946
<211> 45
<212> DNA
<213> Homo sapiens

<400> 946
tgtgccagca gtgcacgggc tagcgggggg gatgagcagt tcttc 45

<210> 947
<211> 45
<212> DNA
<213> Homo sapiens

<400> 947
tgtgccagca gtgcacgggc tagcgggggg gatgagcagt ttttc 45

<210> 948
<211> 45
<212> DNA
<213> Homo sapiens

<400> 948
tgtgccagca gtgcccggac gagtgggggt cagccccagc atttt 45

<210> 949
<211> 45
<212> DNA
<213> Homo sapiens

<400> 949
tgtgccagca gcgcccggac atcgggcaat cagccccagc atttt 45

<210> 950
<211> 45
<212> DNA
<213> Homo sapiens

<400> 950
tgtgccagca gtgaactggc tagcgggatc aatgagcagt tcttc 45

<210> 951
<211> 45
<212> DNA
<213> Homo sapiens

<400> 951
tgtgccagca gtgagttgac tagcgggggg gatgagcagt tcttc 45

<210> 952
<211> 45
<212> DNA
<213> Homo sapiens

<400> 952
tgtgccagca gtaagaggac ctctggagga gatacgcagt atttt 45

<210> 953
<211> 45
<212> DNA
<213> Homo sapiens

<400> 953
tgtgccagca gcctaaggac tagcgggggg gatgagcagt acttc 45

<210> 954
<211> 45
<212> DNA
<213> Homo sapiens

<400> 954
tgtgccagca gccccctgac tagcgccaca gatacgcagt atttt 45

<210> 955
<211> 45
<212> DNA
<213> Homo sapiens

<400> 955
tgtgccagca gtcctcgggc tagcgggggc gatgagcagt tcttc 45

<210> 956
<211> 45
<212> DNA
<213> Homo sapiens

<400> 956
tgtgccagca gtccccggac tagcgggggc gacgagcagt acttc 45

<210> 957
<211> 48
<212> DNA
<213> Homo sapiens

<400> 957
tgtgccagca ggctccggac tagcggggt acagatacgc agtatttt 48

<210> 958
<211> 48
<212> DNA
<213> Homo sapiens

<400> 958
tgtgccagca gacgactagc ggggtttatg gcagatacgc agtatttt 48

<210> 959
<211> 48
<212> DNA
<213> Homo sapiens

<400> 959
tgtgccagca gtgcacggac tagcgcgggc acagatacgc agtatttt 48

<210> 960
<211> 48
<212> DNA
<213> Homo sapiens

<400> 960
tgtgccagca gtgcacggac tagcgctggc acagatacgc agtatttt 48

<210> 961
<211> 48
<212> DNA
<213> Homo sapiens

<400> 961
tgtgccagca gtgccaggac tagcgggggg gcagatacgc attatttt 48

<210> 962
<211> 48
<212> DNA
<213> Homo sapiens

<400> 962
tgtgccagca gtgccaggac tagcgggggg gcagatacgc agtatttt 48

<210> 963
<211> 48
<212> DNA
<213> Homo sapiens

<400> 963
tgtgccagca gtgctaggac tagcgggggg gcagatacgc agtatttt 48

<210> 964
<211> 48
<212> DNA
<213> Homo sapiens

<400> 964
tgtgccagca gcgcccggac tagcgggggg tcagatacgc agtatttt 48

<210> 965
<211> 48
<212> DNA
<213> Homo sapiens

<400> 965
tgtgccagca gtgccaggac tagcgggggg tcagatacgc agtatttt 48

<210> 966
<211> 48
<212> DNA
<213> Homo sapiens

<400> 966
tgtgccagca gtgccaggac tagcggaggc acagatacgc agtatttt 48

<210> 967
<211> 48
<212> DNA
<213> Homo sapiens

<400> 967
tgtgccagca gtgctaggac tagcgggggc acagatacgc agtatttt 48

<210> 968
<211> 48
<212> DNA
<213> Homo sapiens

<400> 968
tgtgccagca gtgaacggac tagcggggga cgcgatacgc agtatttt 48

<210> 969
<211> 48
<212> DNA
<213> Homo sapiens

<400> 969
tgtgccagca gcggtaggac tagcggggga tcggatacgc agtatttt 48

<210> 970
<211> 48
<212> DNA
<213> Homo sapiens

<400>  970
tgtgccagca gtcttagggc tagcgggggg tcagatacgc agtatttt    48

<210>  971
<211>  48
<212>  DNA
<213>  Homo sapiens

<400>  971
tgtgccagca gtctgaggac tagcgggggg gcagatacgc agtatttt    48

<210>  972
<211>  48
<212>  DNA
<213>  Homo sapiens

<400>  972
tgtgccagca gccaacggac tagcgggggg gcagatacgc agtatttt    48

<210>  973
<211>  48
<212>  DNA
<213>  Homo sapiens

<400>  973
tgtgccagca gccgactgac tagcgggggga tcagatacgc agtatttt    48

<210>  974
<211>  48
<212>  DNA
<213>  Homo sapiens

<400>  974
tgtgccagca gtcgccggac tagcgggggt ctagatacgc agtatttt    48

<210>  975
<211>  48
<212>  DNA
<213>  Homo sapiens

<400>  975
tgtgccagca gtcggcggac tagcgggggg cccaatgagc agttcttc    48

<210>  976
<211>  48
<212>  DNA
<213>  Homo sapiens

<400>  976
tgtgccagca gtcggaggac tagcgggggc acagatacgc agtatttt    48

<210>  977
<211>  42
<212>  DNA
<213>  Homo sapiens

<400> 977
tgtgccagca gtgaagggtg ggaaccctac gagcagtact tc 42

<210> 978
<211> 45
<212> DNA
<213> Homo sapiens

<400> 978
tgtgccagca gtgagttgac tagcgggggg gatgagcagt tcttc 45

<210> 979
<211> 45
<212> DNA
<213> Homo sapiens

<400> 979
tgtgccagca gtgagttgac tagcgggggg gatgagcagt tgttc 45

<210> 980
<211> 48
<212> DNA
<213> Homo sapiens

<400> 980
tgtgccagca gtgccaggac tagcggaggc acagatacgc agtatttt 48

<210> 981
<211> 54
<212> DNA
<213> Homo sapiens

<400> 981
tgtgccacca ccgccgccgg gacaggggta gacggaaact atggctacac cttc 54

<210> 982
<211> 42
<212> DNA
<213> Homo sapiens

<400> 982
tgtgccagcc agcggggaaa ttcctacaat gagcagttct tc 42

<210> 983
<211> 42
<212> DNA
<213> Homo sapiens

<400> 983
tgtgccagca gaatccggac tagcgggggg gagcagtact tc 42

<210> 984
<211> 42
<212> DNA
<213> Homo sapiens

<400> 984
tgtgccagca cccgggacag gacaaagaat gagcagttct tc 42

<210> 985
<211> 45
<212> DNA
<213> Homo sapiens

<400> 985
tgtgccagca gtgcgttggc tagcgggga gatgagcagt tcttc 45

<210> 986
<211> 45
<212> DNA
<213> Homo sapiens

<400> 986
tgtgccagca gtctccggac tagcgggga gatgagcagt tcttc 45

<210> 987
<211> 45
<212> DNA
<213> Homo sapiens

<400> 987
tgtgccagca gccctatggc tagcgggggg gatgagcagt tcttc 45

<210> 988
<211> 45
<212> DNA
<213> Homo sapiens

<400> 988
tgtgccagca gcccccggac tagcggcaca gatacgcagt atttt 45

<210> 989
<211> 45
<212> DNA
<213> Homo sapiens

<400> 989
tgtgccagca gccgtaggac tagcgggact tacgagcagt acttc 45

<210> 990
<211> 45
<212> DNA
<213> Homo sapiens

<400> 990
tgtgccagca gtgtgatggc tagccgtggg aatgagcagt tcttc 45

<210> 991
<211> 45
<212> DNA
<213> Homo sapiens

<400> 991
tgtgtcagca gtgcgttggc tagcggggga gatgagcagt tcttc 45

<210> 992
<211> 48
<212> DNA
<213> Homo sapiens

<400> 992
tgtgccagca ataaattgac tagcgggggc cgggatacgc agtatttt 48

<210> 993
<211> 48
<212> DNA
<213> Homo sapiens

<400> 993
tgtgccagca gtgccaggac tagcgggggc tcggatacgc agtatttt 48

<210> 994
<211> 48
<212> DNA
<213> Homo sapiens

<400> 994
tgtgccagca gcaaaaggac tagcggggcg actgatgagc agttcttc 48

<210> 995
<211> 48
<212> DNA
<213> Homo sapiens

<400> 995
tgtgccagca gtttgttgac tagcggggga cgggagaccc agtacttc 48

<210> 996
<211> 48
<212> DNA
<213> Homo sapiens

<400> 996
tgtgccagca gccgcttgac gagcgggggg cggaatgagc agttcttc 48

<210> 997
<211> 48
<212> DNA
<213> Homo sapiens

<400> 997
tgtgccagca gctccaggac tagcgggggc acagatacgc agtatttt 48

<210> 998
<211> 48
<212> DNA
<213> Homo sapiens

<400> 998
tgtgccacct ccagaggagc gcggggaagc aatcagcccc agcatttt 48

<210> 999
<211> 36
<212> DNA
<213> Homo sapiens

<400> 999
tgtgccagca gagacggcct cggggagctg tttttt 36

<210> 1000
<211> 39
<212> DNA
<213> Homo sapiens

<400> 1000
tgtgccagct caggacagac gaacactgaa gctttcttt 39

<210> 1001
<211> 45
<212> DNA
<213> Homo sapiens

<400> 1001
tgtgccagca gtgcactggc tagcggaaca gatacgcagt atttt 45

<210> 1002
<211> 45
<212> DNA
<213> Homo sapiens

<400> 1002
tgtgccagca gtgaattggc tagcgggcag ggatcccagt acttc 45

<210> 1003
<211> 45
<212> DNA
<213> Homo sapiens

<400> 1003
tgtgccagca gtgaactggc tagcgggagc tacgagcagt acttc 45

<210> 1004
<211> 45
<212> DNA
<213> Homo sapiens

<400> 1004
tgtgccagca gtgaactggc tagcgggacg ggtgagcagt tcttc 45

<210> 1005
<211> 45
<212> DNA
<213> Homo sapiens

<400> 1005
tgtgccagca gccctcggac tagcggtccc tacgagcagt acttc 45

<210> 1006
<211> 45
<212> DNA
<213> Homo sapiens

<400> 1006
tgtgccagca gccaattgac tagcggcaca gatacgcagt atttt 45

<210> 1007
<211> 45
<212> DNA
<213> Homo sapiens

<400> 1007
tgtgccagca gtcgactagc gggaggattt gatgagcagt tcttc 45

<210> 1008
<211> 45
<212> DNA
<213> Homo sapiens

<400> 1008
tgtgccagca gtcggttggc tagcggcaca gatacgcagt atttt 45

<210> 1009
<211> 45
<212> DNA
<213> Homo sapiens

<400> 1009
tgtgccagca gtcggacagt ctcgggcaat cagccccagc atttt 45

<210> 1010
<211> 45
<212> DNA
<213> Homo sapiens

<400> 1010
tgtgccagca gttcgttggc tagcagaccc tacgagcagt acttc 45

<210> 1011
<211> 45
<212> DNA
<213> Homo sapiens

<400> 1011
tgtgccagca cgaagggcgc tagcgggtcg ggtgagcagt tcttc 45

<210> 1012
<211> 45
<212> DNA
<213> Homo sapiens

<400> 1012
tgtgccacca cccccggggc tagcgggata agtgagcagt tcttc 45

<210> 1013
<211> 48
<212> DNA
<213> Homo sapiens

<400> 1013
tgtgccagca gtgaaagggg acaggggcg cggtacgagc agtacttc 48

<210> 1014
<211> 48
<212> DNA
<213> Homo sapiens

<400> 1014
tgtgccagca gccgaaggac tagcggaggc acagatacgc agtatttt 48

<210> 1015
<211> 48
<212> DNA
<213> Homo sapiens

<400> 1015
tgtgccagca gccgaaggac tagcggaggt acagatacgc agtatttt 48

<210> 1016
<211> 45
<212> DNA
<213> Homo sapiens

<400> 1016
tgtgccagcg cccgactagc gggaggtacc gatgagcagt tcttc 45

<210> 1017
<211> 45
<212> DNA
<213> Homo sapiens

<400> 1017
tgtgccagca gcgcgaaggc ccgcgggaat cagccccagc atttt 45

<210> 1018
<211> 45
<212> DNA
<213> Homo sapiens

<400> 1018
tgtgccagca gcgcactagc gggggaaca gatacgcagt atttt 45

<210> 1019
<211> 45
<212> DNA
<213> Homo sapiens

<400> 1019
tgtgccagca gtgctttggc tagcggcaca gatacgcagt atttt 45

<210> 1020
<211> 45
<212> DNA
<213> Homo sapiens

<400> 1020
tgtgccagca gtgacgcggc tagcggtgtg ggcgagcagt acttc 45

<210> 1021
<211> 45
<212> DNA
<213> Homo sapiens

<400> 1021
tgtgccagca gcggacaggc gaggggcaat cagccccagc atttt 45

<210> 1022
<211> 45
<212> DNA
<213> Homo sapiens

<400> 1022
tgtgcaagca gccccgggac tagcggagtt ggtgagcagt tcttc 45

<210> 1023
<211> 45
<212> DNA
<213> Homo sapiens

<400> 1023
tgtgccagca gtcccagtgc tcgcggcaat cagccccagc atttt 45

<210> 1024
<211> 45
<212> DNA
<213> Homo sapiens

<400> 1024
tgtgccagca gtgtcgggac tagcggggtg ggcgagcagt acttc 45

<210> 1025
<211> 42
<212> DNA
<213> Homo sapiens

<400> 1025
tgtgccagcc aggcaagcgg ccgatcctac gagcagtact tc 42

<210> 1026
<211> 42
<212> DNA
<213> Homo sapiens

<400> 1026
tgtgccagca gtgaattttg ggggcaagag acccagtact tc 42

<210> 1027
<211> 42
<212> DNA
<213> Homo sapiens

<400> 1027
tgtgccagca gtgagctggc tagcggggat gagcagttct tc 42

<210> 1028
<211> 42
<212> DNA
<213> Homo sapiens

<400> 1028
tgtgccagca gtgtatcgca ggggagcgac gagcagtact tc 42

<210> 1029
<211> 45
<212> DNA
<213> Homo sapiens

<400> 1029
tgtgccagct gtcccatggc tagccgatcc tacgagcagt acttc 45

<210> 1030
<211> 45
<212> DNA
<213> Homo sapiens

<400> 1030
tgtgccagca ttatcggttc ccaaggggcc tatggctaca ccttc 45

<210> 1031
<211> 45
<212> DNA
<213> Homo sapiens

<400> 1031
tgtgccagca gcgcactagc gtccggggga gatacgcagt atttt 45

<210> 1032
<211> 45
<212> DNA
<213> Homo sapiens

<400> 1032
tgtgccagca gtgcacgggc tagcgggggg gatgagcagt tcttc 45

<210> 1033
<211> 45
<212> DNA
<213> Homo sapiens

<400> 1033
tgtgccagca gcgcccggac atcgggcaat cagccccagc atttt 45

<210> 1034
<211> 45
<212> DNA
<213> Homo sapiens

<400> 1034
tgtgccagca gtgaacgcgg gacagccaac actgaagctt tcttt 45

<210> 1035
<211> 45
<212> DNA
<213> Homo sapiens

<400> 1035
tgtgccagca gcttcaggac tagcgggggt gatacgcagt atttt 45

<210> 1036
<211> 45
<212> DNA
<213> Homo sapiens

<400> 1036
tgtgccagca gtaagcgggc tagcgggggga gatacgcagt atttt 45

<210> 1037
<211> 45
<212> DNA
<213> Homo sapiens

<400> 1037
tgtgccagca gtcctcgggc tagcgggggc gatgagcagt tcttc 45

<210> 1038
<211> 45
<212> DNA
<213> Homo sapiens

<400> 1038
tgtgccagca gtccccggac tagcgggggc gacgagcagt acttc 45

<210> 1039
<211> 45
<212> DNA
<213> Homo sapiens

<400> 1039
tgtgccagca gccccaggac tagcgggacc tacgagcagt acttc 45

<210> 1040
<211> 45
<212> DNA
<213> Homo sapiens

<400>  1040
tgtgccagca gtcctcggac tagcgggacc tacgagcagt acttc        45

<210>  1041
<211>  45
<212>  DNA
<213>  Homo sapiens

<400>  1041
tgtgccagca gccccgtggc caggggggcct tacgagcagt acttc        45

<210>  1042
<211>  45
<212>  DNA
<213>  Homo sapiens

<400>  1042
tgtgccagca gtcaactgac tagcagaacc tacgagcagt acttc        45

<210>  1043
<211>  48
<212>  DNA
<213>  Homo sapiens

<400>  1043
tgtgccagca gtgccaggac tagcgggggg tcagatacgc agtatttt        48

<210>  1044
<211>  48
<212>  DNA
<213>  Homo sapiens

<400>  1044
tgtgccagca gcgcccggac tagcgggggc acagatacgc agtatttt        48

<210>  1045
<211>  48
<212>  DNA
<213>  Homo sapiens

<400>  1045
tgtgccagca gtgccaggac tagcggaggc acagatacgc agtatttt        48

<210>  1046
<211>  48
<212>  DNA
<213>  Homo sapiens

<400>  1046
tgtgccagca gcggtaggac tagcggggga tcggatacgc agtatttt        48

<210>  1047
<211>  48
<212>  DNA
<213>  Homo sapiens

<400> 1047
tgtgccagca gtctgaggac tagcgggggg gcagatacgc agtatttt 48

<210> 1048
<211> 48
<212> DNA
<213> Homo sapiens

<400> 1048
tgtgccagca gtctcaggac tagcgggggt tcagatacgc agtatttt 48

<210> 1049
<211> 48
<212> DNA
<213> Homo sapiens

<400> 1049
tgtgccagca gtcctctgac tagcgggggc acagatacgc agtatttt 48

<210> 1050
<211> 48
<212> DNA
<213> Homo sapiens

<400> 1050
tgtgccagca gtcggaggac tagcggggcc tcagatacgc agtatttt 48

<210> 1051
<211> 48
<212> DNA
<213> Homo sapiens

<400> 1051
tgtgccagca gtcgacgtac tagcgggggg gccgatgagc agttcttc 48

<210> 1052
<211> 48
<212> DNA
<213> Homo sapiens

<400> 1052
tgtgccagca gccgacggac tagcgggggg acggatacgc agtatttt 48

<210> 1053
<211> 48
<212> DNA
<213> Homo sapiens

<400> 1053
tgtgccagca gccggcggac tagcgggggc acagatacgc agtatttt 48

<210> 1054
<211> 48
<212> DNA
<213> Homo sapiens

<400> 1054
tgtgccagca gtagaaggac tagcggtggc acagatacgc agtatttt 48

<210> 1055
<211> 48
<212> DNA
<213> Homo sapiens

<400> 1055
tgtgccagca gtagacggac tagcgggggg acagatacgc agtatttt 48

<210> 1056
<211> 48
<212> DNA
<213> Homo sapiens

<400> 1056
tgtgccagca gcagacgaac tagcggggga tacgatgagc agttcttc 48

<210> 1057
<211> 48
<212> DNA
<213> Homo sapiens

<400> 1057
tgtgccagca gtagccggac tagcggggga tcagatacgc agtatttt 48

<210> 1058
<211> 48
<212> DNA
<213> Homo sapiens

<400> 1058
tgtgccagca gtgttcggac tagcgggggg tcagatacgc agtatttt 48

<210> 1059
<211> 51
<212> DNA
<213> Homo sapiens

<400> 1059
tgtgccagca gtataagttt acccgggaca ctttattacg agcagtactt c 51

<210> 1060
<211> 12
<212> PRT
<213> Homo sapiens

<400> 1060

Cys Ala Phe Lys Ala Ala Gly Asn Lys Leu Thr Phe
1 5 10

<210> 1061
<211> 12
<212> PRT

<213> Homo sapiens

<400> 1061

Cys Ala Phe Thr Ala Ala Gly Asn Lys Leu Thr Phe
1 5 10

<210> 1062
<211> 12
<212> PRT
<213> Homo sapiens

<400> 1062

Cys Ala Leu Glu Asn Ala Gly Asn Lys Leu Thr Phe
1 5 10

<210> 1063
<211> 13
<212> PRT
<213> Homo sapiens

<400> 1063

Cys Ala Ser Tyr Gly Gly Ala Thr Asn Lys Leu Ile Phe
1 5 10

<210> 1064
<211> 11
<212> PRT
<213> Homo sapiens

<400> 1064

Cys Ala Ser Gly Asn Thr Asp Lys Leu Ile Phe
1 5 10

<210> 1065
<211> 10
<212> PRT
<213> Homo sapiens

<400> 1065

Cys Ala Arg Asp Asp Arg Lys Leu Ile Trp
1 5 10

<210> 1066
<211> 12
<212> PRT
<213> Homo sapiens

<400> 1066

Cys Ala Phe Cys Asn Ala Gly Asn Met Leu Thr Phe
1 5 10

<210> 1067
<211> 12
<212> PRT
<213> Homo sapiens

<400> 1067

Cys Ala Leu Gly Asn Ala Gly Asn Met Leu Thr Phe
1 5 10

<210> 1068
<211> 13
<212> PRT
<213> Homo sapiens

<400> 1068

Cys Ala Ser Asp Gly Gly Ser Gln Gly Asn Leu Ile Phe
1 5 10

<210> 1069
<211> 12
<212> PRT
<213> Homo sapiens

<400> 1069

Cys Ser Pro Gln Gly Gly Ser Glu Lys Leu Val Phe
1 5 10

<210> 1070
<211> 13
<212> PRT
<213> Homo sapiens

<400> 1070

Cys Ala Phe Ile Pro Gly Gly Ser Tyr Ile Pro Thr Phe
1 5 10

<210> 1071
<211> 12
<212> PRT
<213> Homo sapiens

<400> 1071

Cys Ala Phe Lys Ala Ala Gly Asn Lys Leu Thr Phe
1 5 10

<210> 1072
<211> 12
<212> PRT
<213> Homo sapiens

<400> 1072

Cys Ala Phe Arg Ala Ala Gly Asn Lys Leu Thr Phe

1 5 10

<210> 1073
<211> 12
<212> PRT
<213> Homo sapiens

<400> 1073

Cys Ala Leu Lys Ala Ala Gly Asn Lys Leu Thr Phe
1 5 10

<210> 1074
<211> 12
<212> PRT
<213> Homo sapiens

<400> 1074

Cys Ala Met Lys Ala Ala Gly Asn Lys Leu Thr Phe
1 5 10

<210> 1075
<211> 12
<212> PRT
<213> Homo sapiens

<400> 1075

Cys Ala Asn Arg Ala Ala Gly Asn Lys Leu Thr Phe
1 5 10

<210> 1076
<211> 11
<212> PRT
<213> Homo sapiens

<400> 1076

Cys Ala Pro Lys Ser Arg Gln Gln Ala Asn Phe
1 5 10

<210> 1077
<211> 12
<212> PRT
<213> Homo sapiens

<400> 1077

Cys Ala Ser Lys Ala Ala Gly Asn Lys Leu Thr Phe
1 5 10

<210> 1078
<211> 12
<212> PRT
<213> Homo sapiens

<400> 1078

Cys Ala Tyr Lys Ala Ala Gly Asn Lys Leu Thr Phe
1 5 10

<210> 1079
<211> 12
<212> PRT
<213> Homo sapiens

<400> 1079

Cys Gly Phe Lys Ala Ala Gly Asn Lys Leu Thr Phe
1 5 10

<210> 1080
<211> 12
<212> PRT
<213> Homo sapiens

<400> 1080

Cys Gly Trp Lys Ala Ala Gly Asn Lys Leu Thr Phe
1 5 10

<210> 1081
<211> 12
<212> PRT
<213> Homo sapiens

<400> 1081

Cys Ser Leu Lys Ala Ala Gly Asn Lys Leu Thr Phe
1 5 10

<210> 1082
<211> 12
<212> PRT
<213> Homo sapiens

<400> 1082

Cys Ser Leu Arg Ala Ala Gly Asn Lys Leu Thr Phe
1 5 10

<210> 1083
<211> 12
<212> PRT
<213> Homo sapiens

<400> 1083

Cys Ser Arg Arg Ala Ala Gly Asn Lys Leu Thr Phe
1 5 10

<210> 1084
<211> 12

<212> PRT
<213> Homo sapiens

<400> 1084

Cys Ser Trp Lys Ala Ala Gly Asn Lys Leu Thr Phe
1 5 10

<210> 1085
<211> 12
<212> PRT
<213> Homo sapiens

<400> 1085

Cys Ser Trp Arg Ala Ala Gly Asn Lys Leu Thr Phe
1 5 10

<210> 1086
<211> 12
<212> PRT
<213> Homo sapiens

<400> 1086

Cys Thr Lys Lys Ala Ala Gly Asn Lys Leu Thr Phe
1 5 10

<210> 1087
<211> 12
<212> PRT
<213> Homo sapiens

<400> 1087

Cys Thr Leu Lys Ala Ala Gly Asn Lys Leu Thr Phe
1 5 10

<210> 1088
<211> 12
<212> PRT
<213> Homo sapiens

<400> 1088

Cys Thr Asn Lys Ala Ala Gly Asn Lys Leu Thr Phe
1 5 10

<210> 1089
<211> 12
<212> PRT
<213> Homo sapiens

<400> 1089

Cys Thr Arg Lys Ala Ala Gly Asn Lys Leu Thr Phe
1 5 10

<210> 1090
<211> 12
<212> PRT
<213> Homo sapiens

<400> 1090

Cys Ala Leu Lys Gln Ala Gly Asn Lys Leu Thr Phe
1 5 10

<210> 1091
<211> 12
<212> PRT
<213> Homo sapiens

<400> 1091

Cys Ala Leu Arg Gln Ala Gly Asn Met Leu Thr Phe
1 5 10

<210> 1092
<211> 12
<212> PRT
<213> Homo sapiens

<400> 1092

Cys Ala Leu Lys Ala Ala Gly Asn Lys Leu Thr Phe
1 5 10

<210> 1093
<211> 12
<212> PRT
<213> Homo sapiens

<400> 1093

Cys Ala Leu Arg Ala Ala Gly Asn Lys Leu Thr Phe
1 5 10

<210> 1094
<211> 12
<212> PRT
<213> Homo sapiens

<400> 1094

Cys Ala Leu Arg Ala Ala Gly Asn Met Leu Thr Phe
1 5 10

<210> 1095
<211> 12
<212> PRT
<213> Homo sapiens

<400> 1095

Cys Ala Ser Gln Ala Ala Gly Asn Lys Leu Thr Phe
1               5                   10

<210>  1096
<211>  12
<212>  PRT
<213>  Homo sapiens

<400>  1096

Cys Ser Arg Arg Ala Ala Gly Asn Lys Leu Thr Phe
1               5                   10

<210>  1097
<211>  12
<212>  PRT
<213>  Homo sapiens

<400>  1097

Cys Ala Leu Arg Gln Ala Gly Asn Met Leu Thr Phe
1               5                   10

<210>  1098
<211>  13
<212>  PRT
<213>  Homo sapiens

<400>  1098

Cys Ala Phe Asp Asn Gln Ala Ala Asn Asn Leu Ile Phe
1               5                   10

<210>  1099
<211>  13
<212>  PRT
<213>  Homo sapiens

<400>  1099

Cys Ala Phe Asp Asn Gln Ala Gly Thr Ala Leu Ile Phe
1               5                   10

<210>  1100
<211>  12
<212>  PRT
<213>  Homo sapiens

<400>  1100

Cys Ala Phe Lys Ala Ala Gly Asn Lys Leu Thr Phe
1               5                   10

<210>  1101
<211>  12
<212>  PRT
<213>  Homo sapiens

<400> 1101

Cys Ala Leu Lys Ala Ala Gly Asn Lys Leu Thr Phe
1 5 10

<210> 1102
<211> 12
<212> PRT
<213> Homo sapiens

<400> 1102

Cys Ala Ser Lys Ala Ala Gly Asn Lys Leu Thr Phe
1 5 10

<210> 1103
<211> 10
<212> PRT
<213> Homo sapiens

<400> 1103

Cys Ala Phe Asp Asn Ala Arg Leu Met Phe
1 5 10

<210> 1104
<211> 10
<212> PRT
<213> Homo sapiens

<400> 1104

Cys Ala Ser Tyr Gly Ala Ala Leu Met Phe
1 5 10

<210> 1105
<211> 13
<212> PRT
<213> Homo sapiens

<400> 1105

Cys Ala Cys Tyr Gly Gly Ala Thr Asn Lys Leu Ile Phe
1 5 10

<210> 1106
<211> 13
<212> PRT
<213> Homo sapiens

<400> 1106

Cys Ala Ser Tyr Gly Gly Ala Thr Asn Lys Leu Ile Phe
1 5 10

<210> 1107

<211> 11
<212> PRT
<213> Homo sapiens

<400> 1107

Cys Ala Phe Asp Asn Ala Asp Lys Leu Ile Phe
1 5 10

<210> 1108
<211> 11
<212> PRT
<213> Homo sapiens

<400> 1108

Cys Ala Phe Asp Asn Thr Asp Lys Leu Ile Phe
1 5 10

<210> 1109
<211> 10
<212> PRT
<213> Homo sapiens

<400> 1109

Cys Ala Tyr Gly Ala Asn Asn Leu Phe Phe
1 5 10

<210> 1110
<211> 12
<212> PRT
<213> Homo sapiens

<400> 1110

Cys Val Phe Gln Thr Gly Gly Asn Asn Leu Phe Phe
1 5 10

<210> 1111
<211> 12
<212> PRT
<213> Homo sapiens

<400> 1111

Cys Ala Phe Cys Asn Ala Gly Asn Met Leu Thr Phe
1 5 10

<210> 1112
<211> 12
<212> PRT
<213> Homo sapiens

<400> 1112

Cys Ala Leu Gly Asn Ala Gly Asn Met Leu Thr Phe
1 5 10

<210> 1113
<211> 14
<212> PRT
<213> Homo sapiens

<400> 1113

Cys Ala Ser Asp Tyr Gly Gly Ser Gln Gly Asn Leu Ile Phe
1 5 10

<210> 1114
<211> 14
<212> PRT
<213> Homo sapiens

<400> 1114

Cys Ala Ser Tyr Asp Gly Gly Thr Gln Gly Asn Leu Ile Val
1 5 10

<210> 1115
<211> 13
<212> PRT
<213> Homo sapiens

<400> 1115

Cys Ala Tyr Cys Gly Gly Ser Pro Asn Asn Leu Ile Phe
1 5 10

<210> 1116
<211> 12
<212> PRT
<213> Homo sapiens

<400> 1116

Cys Ser Pro Gln Gly Gly Ser Glu Lys Leu Val Phe
1 5 10

<210> 1117
<211> 12
<212> PRT
<213> Homo sapiens

<400> 1117

Cys Ala Phe Lys Gly Gly Gly Asn Lys Leu Thr Phe
1 5 10

<210> 1118
<211> 12
<212> PRT
<213> Homo sapiens

<400> 1118

Cys Ala Phe Arg Gly Gly Gly Asn Lys Leu Thr Phe
1               5                   10

<210> 1119
<211> 12
<212> PRT
<213> Homo sapiens

<400> 1119

Cys Ala Phe His Ala Ala Gly Asn Lys Leu Thr Phe
1               5                   10

<210> 1120
<211> 12
<212> PRT
<213> Homo sapiens

<400> 1120

Cys Ala Phe Lys Ala Ala Gly Asn Lys Leu Thr Phe
1               5                   10

<210> 1121
<211> 12
<212> PRT
<213> Homo sapiens

<400> 1121

Cys Ala Phe Asn Ala Ala Gly Asn Lys Leu Thr Phe
1               5                   10

<210> 1122
<211> 12
<212> PRT
<213> Homo sapiens

<400> 1122

Cys Ala Phe Gln Ala Ala Gly Asn Lys Leu Thr Phe
1               5                   10

<210> 1123
<211> 12
<212> PRT
<213> Homo sapiens

<400> 1123

Cys Ala His Lys Ala Ala Gly Asn Lys Leu Thr Phe
1               5                   10

<210> 1124
<211> 12
<212> PRT

<213> Homo sapiens

<400> 1124

Cys Ala His Lys Gly Ala Gly Asn Lys Leu Thr Phe
1 5 10

<210> 1125
<211> 10
<212> PRT
<213> Homo sapiens

<400> 1125

Cys Ala Phe Gly Asp Tyr Lys Leu Ser Phe
1 5 10

<210> 1126
<211> 13
<212> PRT
<213> Homo sapiens

<400> 1126

Cys Ala Asn Tyr Gly Gly Ala Thr Asn Lys Leu Ile Phe
1 5 10

<210> 1127
<211> 13
<212> PRT
<213> Homo sapiens

<400> 1127

Cys Ala Ser Tyr Gly Gly Ala Thr Asn Lys Leu Ile Phe
1 5 10

<210> 1128
<211> 13
<212> PRT
<213> Homo sapiens

<400> 1128

Cys Ala Glu Tyr Gly Gly Ala Thr Asn Lys Leu Ile Phe
1 5 10

<210> 1129
<211> 13
<212> PRT
<213> Homo sapiens

<400> 1129

Cys Ala Pro Tyr Gly Gly Ala Thr Asn Lys Leu Ile Phe
1 5 10

<210> 1130
<211> 12
<212> PRT
<213> Homo sapiens

<400> 1130

Cys Ala Phe Arg Arg Ala Gly Asn Met Leu Thr Phe
1 5 10

<210> 1131
<211> 13
<212> PRT
<213> Homo sapiens

<400> 1131

Cys Ala Pro Gly Gly Gly Gly Ser Glu Lys Leu Val Phe
1 5 10

<210> 1132
<211> 12
<212> PRT
<213> Homo sapiens

<400> 1132

Cys Ala Phe Lys Ala Ala Gly Asn Lys Leu Thr Phe
1 5 10

<210> 1133
<211> 12
<212> PRT
<213> Homo sapiens

<400> 1133

Cys Ser Phe Lys Ala Ala Gly Asn Lys Leu Thr Phe
1 5 10

<210> 1134
<211> 12
<212> PRT
<213> Homo sapiens

<400> 1134

Cys Ser Phe Lys Gly Ala Gly Asn Lys Leu Ile Phe
1 5 10

<210> 1135
<211> 13
<212> PRT
<213> Homo sapiens

<400> 1135

Cys Ala Leu Tyr Gly Gly Ala Thr Asn Lys Leu Ile Phe

1 5 10

<210> 1136
<211> 13
<212> PRT
<213> Homo sapiens

<400> 1136

Cys Ala Ser Tyr Gly Gly Ala Pro His Asp Leu Ile Phe
1 5 10

<210> 1137
<211> 13
<212> PRT
<213> Homo sapiens

<400> 1137

Cys Ala Ser Tyr Gly Gly Ala Thr Lys Ile Leu Phe Val
1 5 10

<210> 1138
<211> 13
<212> PRT
<213> Homo sapiens

<400> 1138

Cys Ala Ser Tyr Gly Gly Ala Thr Asn Lys Leu Ile Phe
1 5 10

<210> 1139
<211> 11
<212> PRT
<213> Homo sapiens

<400> 1139

Cys Ala Phe Asp Asn Thr Asp Lys Leu Ile Phe
1 5 10

<210> 1140
<211> 12
<212> PRT
<213> Homo sapiens

<400> 1140

Cys Ala Leu Gly Asn Ala Gly Asn Met Leu Thr Phe
1 5 10

<210> 1141
<211> 12
<212> PRT
<213> Homo sapiens

<400> 1141

Cys Ser Pro Gln Gly Gly Cys Glu Lys Leu Val Phe
1 5 10

<210> 1142
<211> 13
<212> PRT
<213> Homo sapiens

<400> 1142

Cys Ala Phe Ile Pro Gly Gly Ser Tyr Ile Pro Thr Phe
1 5 10

<210> 1143
<211> 16
<212> PRT
<213> Homo sapiens

<400> 1143

Cys Ala Ser Arg Ala Arg Thr Gly Ala Thr Asn Glu Lys Leu Phe Phe
1 5 10 15

<210> 1144
<211> 16
<212> PRT
<213> Homo sapiens

<400> 1144

Cys Ala Ser Gln Arg Gly Ala Arg Gly Gly Asn Gln Pro Gln His Phe
1 5 10 15

<210> 1145
<211> 15
<212> PRT
<213> Homo sapiens

<400> 1145

Cys Ala Ser Ser Glu Arg Val Ser Gly Asn Gln Pro Gln His Phe
1 5 10 15

<210> 1146
<211> 16
<212> PRT
<213> Homo sapiens

<400> 1146

Cys Ala Thr Ser Arg Gly Ala Arg Gly Ser Asn Gln Pro Gln His Phe
1 5 10 15

<210> 1147
<211> 14

<212> PRT
<213> Homo sapiens

<400> 1147

Cys Ala Ser Thr Arg Asp Arg Thr Lys Asn Glu Gln Phe Phe
1 5 10

<210> 1148
<211> 14
<212> PRT
<213> Homo sapiens

<400> 1148

Cys Ala Ser Leu Glu Arg Thr Ser Gly Gly Glu Gln Phe Phe
1 5 10

<210> 1149
<211> 15
<212> PRT
<213> Homo sapiens

<400> 1149

Cys Ala Ser Ser Ala Leu Ala Ser Gly Gly Asp Glu Gln Phe Phe
1 5 10 15

<210> 1150
<211> 16
<212> PRT
<213> Homo sapiens

<400> 1150

Cys Ala Ser Ser Ala Arg Thr Ser Gly Gly Leu Asp Glu Gln Phe Phe
1 5 10 15

<210> 1151
<211> 15
<212> PRT
<213> Homo sapiens

<400> 1151

Cys Ala Ser Ser Pro Met Ala Ser Gly Gly Asp Glu Gln Phe Phe
1 5 10 15

<210> 1152
<211> 16
<212> PRT
<213> Homo sapiens

<400> 1152

Cys Ala Ser Ser Arg Leu Thr Ser Gly Gly Arg Asn Glu Gln Phe Phe
1 5 10 15

<210>  1153
<211>  16
<212>  PRT
<213>  Homo sapiens

<400>  1153

Cys Ala Ser Ser Ser Arg Thr Ser Gly Gly Gln Asp Glu Gln Phe Phe
1               5                   10                  15

<210>  1154
<211>  15
<212>  PRT
<213>  Homo sapiens

<400>  1154

Cys Ala Ser Ser Val Met Ala Ser Arg Gly Asn Glu Gln Phe Phe
1               5                   10                  15

<210>  1155
<211>  14
<212>  PRT
<213>  Homo sapiens

<400>  1155

Cys Ala Ser Lys Pro Leu Val Ser Thr Asp Thr Gln Tyr Phe
1               5                   10

<210>  1156
<211>  16
<212>  PRT
<213>  Homo sapiens

<400>  1156

Cys Ala Ser Ser Ala Lys Thr Ser Gly Gly Ser Asp Thr Gln Tyr Phe
1               5                   10                  15

<210>  1157
<211>  16
<212>  PRT
<213>  Homo sapiens

<400>  1157

Cys Ala Ser Ser Ala Leu Ala Ser Gly Gly Arg Asp Thr Gln Tyr Phe
1               5                   10                  15

<210>  1158
<211>  15
<212>  PRT
<213>  Homo sapiens

<400>  1158

Cys Ala Ser Ser Ala Leu Ala Ser Gly Thr Asp Thr Gln Tyr Phe
1 5 10 15

<210> 1159
<211> 16
<212> PRT
<213> Homo sapiens

<400> 1159

Cys Ala Ser Ser Ala Arg Thr Ser Gly Gly Ser Asp Thr Gln Tyr Phe
1 5 10 15

<210> 1160
<211> 16
<212> PRT
<213> Homo sapiens

<400> 1160

Cys Ala Ser Ser Lys Arg Ala Ser Gly Gly Thr Asp Thr Gln Tyr Phe
1 5 10 15

<210> 1161
<211> 16
<212> PRT
<213> Homo sapiens

<400> 1161

Cys Ala Ser Ser Leu Arg Thr Ser Gly Gly Ser Asp Thr Gln Tyr Phe
1 5 10 15

<210> 1162
<211> 16
<212> PRT
<213> Homo sapiens

<400> 1162

Cys Ala Ser Ser Ser Lys Thr Ser Gly Gly Thr Asp Thr Gln Tyr Phe
1 5 10 15

<210> 1163
<211> 13
<212> PRT
<213> Homo sapiens

<400> 1163

Cys Ala Ser Leu Gly Pro Leu Arg His Glu Gln Tyr Phe
1 5 10

<210> 1164
<211> 15
<212> PRT
<213> Homo sapiens

<400> 1164

Cys Ala Ser Ser Glu Leu Thr Ser Arg Thr Tyr Glu Gln Tyr Phe
1 5 10 15

<210> 1165
<211> 15
<212> PRT
<213> Homo sapiens

<400> 1165

Cys Ala Ser Ser Arg Arg Thr Ser Gly Thr Tyr Glu Gln Tyr Phe
1 5 10 15

<210> 1166
<211> 14
<212> PRT
<213> Homo sapiens

<400> 1166

Cys Ala Ser Ile Pro Arg Thr Ser Gly Gly Leu Gln Phe Phe
1 5 10

<210> 1167
<211> 16
<212> PRT
<213> Homo sapiens

<400> 1167

Cys Ala Ser Ser Ala Arg Thr Ser Gly Gly Gln Asp Glu Gln Phe Phe
1 5 10 15

<210> 1168
<211> 15
<212> PRT
<213> Homo sapiens

<400> 1168

Cys Ala Ser Ser Pro Met Ala Ser Gly Gly Asp Glu Gln Phe Phe
1 5 10 15

<210> 1169
<211> 16
<212> PRT
<213> Homo sapiens

<400> 1169

Cys Ala Ser Ser Arg Leu Thr Ser Gly Gly Arg Asn Glu Gln Phe Phe
1 5 10 15

<210> 1170

<211> 15
<212> PRT
<213> Homo sapiens

<400> 1170

Cys Ala Ser Ser Arg Trp Ala Ser Gly Gly Asp Glu Gln Phe Phe
1 5 10 15

<210> 1171
<211> 15
<212> PRT
<213> Homo sapiens

<400> 1171

Cys Ala Ser Ser Val Met Ala Ser Arg Gly Asn Glu Gln Phe Phe
1 5 10 15

<210> 1172
<211> 15
<212> PRT
<213> Homo sapiens

<400> 1172

Cys Ala Ser Gln Leu Leu Val Gln His Thr Asp Thr Gln Tyr Phe
1 5 10 15

<210> 1173
<211> 14
<212> PRT
<213> Homo sapiens

<400> 1173

Cys Ala Ser Lys Ala Leu Val Ser Thr Asp Thr Gln Tyr Phe
1 5 10

<210> 1174
<211> 14
<212> PRT
<213> Homo sapiens

<400> 1174

Cys Ala Ser Lys Asp Arg Thr Ser Gly Asp Thr Gln Tyr Phe
1 5 10

<210> 1175
<211> 15
<212> PRT
<213> Homo sapiens

<400> 1175

Cys Ala Ser Ser Ala Leu Ala Ser Gly Thr Asp Thr Gln Tyr Phe
1 5 10 15

<210> 1176
<211> 16
<212> PRT
<213> Homo sapiens

<400> 1176

Cys Ala Ser Ser Ala Arg Thr Ser Gly Gly Gly Asp Thr Gln Tyr Phe
1 5 10 15

<210> 1177
<211> 16
<212> PRT
<213> Homo sapiens

<400> 1177

Cys Ala Ser Ser Ala Arg Thr Ser Gly Gly Arg Asp Thr Gln Tyr Phe
1 5 10 15

<210> 1178
<211> 16
<212> PRT
<213> Homo sapiens

<400> 1178

Cys Ala Ser Ser Ala Arg Thr Ser Gly Gly Ser Asp Thr Gln Tyr Phe
1 5 10 15

<210> 1179
<211> 14
<212> PRT
<213> Homo sapiens

<400> 1179

Cys Ala Ser Ser Glu Leu Val Ser Gly Gly Leu His Arg Tyr
1 5 10

<210> 1180
<211> 16
<212> PRT
<213> Homo sapiens

<400> 1180

Cys Ala Ser Ser Arg Arg Thr Ser Gly Gly Ser Asp Thr Gln Tyr Phe
1 5 10 15

<210> 1181
<211> 16
<212> PRT
<213> Homo sapiens

<400> 1181

Cys Ala Ser Ser Ser Lys Thr Ser Gly Gly Thr Asp Thr Gln Tyr Phe
1 5 10 15

<210> 1182
<211> 14
<212> PRT
<213> Homo sapiens

<400> 1182

Cys Ala Ser Ala Arg Glu Arg Thr Lys Asn Ile Gln Tyr Phe
1 5 10

<210> 1183
<211> 14
<212> PRT
<213> Homo sapiens

<400> 1183

Cys Ala Ser Arg Leu Leu Val Ser Gln Glu Thr Gln Tyr Phe
1 5 10

<210> 1184
<211> 16
<212> PRT
<213> Homo sapiens

<400> 1184

Cys Ala Ser Ser Leu Leu Thr Ser Gly Gly Arg Glu Thr Gln Tyr Phe
1 5 10 15

<210> 1185
<211> 14
<212> PRT
<213> Homo sapiens

<400> 1185

Cys Ala Ser Ser Pro Thr Val Asn Thr Tyr Glu Gln Tyr Phe
1 5 10

<210> 1186
<211> 14
<212> PRT
<213> Homo sapiens

<400> 1186

Cys Ala Cys Arg Ile Arg Thr Ser Gly Gly Glu Gln Tyr Phe
1 5 10

<210> 1187
<211> 14
<212> PRT

<213> Homo sapiens

<400> 1187

Cys Ala Ser Arg Ile Arg Thr Ser Gly Gly Glu Gln Tyr Phe
1 5 10

<210> 1188
<211> 14
<212> PRT
<213> Homo sapiens

<400> 1188

Cys Ala Ser Ser Ala Leu Ala Gly Val Asn Glu Gln Phe Phe
1 5 10

<210> 1189
<211> 15
<212> PRT
<213> Homo sapiens

<400> 1189

Cys Ala Ser Ser Pro Arg Thr Ser Gly Thr Tyr Glu Gln Tyr Phe
1 5 10 15

<210> 1190
<211> 15
<212> PRT
<213> Homo sapiens

<400> 1190

Cys Ala Ser Ser Pro Trp Ala Arg Gly Gly Asp Glu Gln Phe Phe
1 5 10 15

<210> 1191
<211> 13
<212> PRT
<213> Homo sapiens

<400> 1191

Cys Ala Ser Ser Gly Gln Thr Asn Thr Glu Ala Phe Phe
1 5 10

<210> 1192
<211> 14
<212> PRT
<213> Homo sapiens

<400> 1192

Cys Ala Ser Arg Trp Thr Ala Thr Ser Tyr Gly Tyr Thr Phe
1 5 10

<210> 1193
<211> 14
<212> PRT
<213> Homo sapiens

<400> 1193

Cys Ala Ser Ser Pro Thr Thr Thr Gly Tyr Gly Tyr Thr Phe
1 5 10

<210> 1194
<211> 15
<212> PRT
<213> Homo sapiens

<400> 1194

Cys Ala Ser Ser Pro Ser Ala Arg Gly Asn Gln Pro Gln His Phe
1 5 10 15

<210> 1195
<211> 15
<212> PRT
<213> Homo sapiens

<400> 1195

Cys Ala Ser Ser Ala Gly Thr Arg Gly Val Gly Glu Gln Phe Phe
1 5 10 15

<210> 1196
<211> 15
<212> PRT
<213> Homo sapiens

<400> 1196

Cys Ala Ser Ser Pro Gly Ala Arg Gly Ile Asp Glu Gln Phe Phe
1 5 10 15

<210> 1197
<211> 15
<212> PRT
<213> Homo sapiens

<400> 1197

Cys Ala Ser Ser Pro Gly Thr Ser Gly Val Gly Lys Gln Phe Phe
1 5 10 15

<210> 1198
<211> 15
<212> PRT
<213> Homo sapiens

<400> 1198

Cys Ala Ser Ser Asp Arg Ala Ser Gly Val Gly Glu Gln Phe Phe

1 5 10 15

<210> 1199
<211> 15
<212> PRT
<213> Homo sapiens

<400> 1199

Cys Ala Ser Ser Asp Arg Thr Ser Gly Pro His Glu Gln Phe Phe
1 5 10 15

<210> 1200
<211> 15
<212> PRT
<213> Homo sapiens

<400> 1200

Cys Ala Ser Ser Pro Gly Thr Ser Gly Val Gly Glu Gln Phe Phe
1 5 10 15

<210> 1201
<211> 15
<212> PRT
<213> Homo sapiens

<400> 1201

Cys Ala Ser Ser Ser Gly Thr Ser Gly Ala Gly Glu Gln Phe Phe
1 5 10 15

<210> 1202
<211> 15
<212> PRT
<213> Homo sapiens

<400> 1202

Cys Ala Ser Ser Ser Gly Thr Ser Gly Val Gly Glu Gln Phe Phe
1 5 10 15

<210> 1203
<211> 15
<212> PRT
<213> Homo sapiens

<400> 1203

Cys Ala Ser Ser Tyr Arg Thr Ser Gly Pro Arg Glu Gln Phe Phe
1 5 10 15

<210> 1204
<211> 15
<212> PRT
<213> Homo sapiens

<400> 1204

Cys Ala Ser Ser Asp Leu Ala Ser Gly Thr Asn Glu Gln Phe Phe
1 5 10 15

<210> 1205
<211> 15
<212> PRT
<213> Homo sapiens

<400> 1205

Cys Ala Ser Ser Gly Leu Ala Gly Gly Met Asp Glu Gln Phe Phe
1 5 10 15

<210> 1206
<211> 15
<212> PRT
<213> Homo sapiens

<400> 1206

Cys Ala Ser His Glu Gly Ala Gly Gly Phe Gly Glu Leu Phe Phe
1 5 10 15

<210> 1207
<211> 15
<212> PRT
<213> Homo sapiens

<400> 1207

Cys Ala Ser His Glu Gly Ala Gly Gly Tyr Gly Glu Leu Phe Phe
1 5 10 15

<210> 1208
<211> 13
<212> PRT
<213> Homo sapiens

<400> 1208

Cys Ala Ser His Arg Thr Tyr Thr Asp Thr Gln Tyr Phe
1 5 10

<210> 1209
<211> 15
<212> PRT
<213> Homo sapiens

<400> 1209

Cys Ala Ser Ser Ala Leu Ala Ser Gly Thr Asp Thr Gln Tyr Phe
1 5 10 15

<210> 1210
<211> 15

<212> PRT
<213> Homo sapiens

<400> 1210

Cys Ala Ser Ser Asp Ala Ala Ser Gly Val Gly Glu Gln Tyr Phe
1 5 10 15

<210> 1211
<211> 15
<212> PRT
<213> Homo sapiens

<400> 1211

Cys Ala Ser Ser Pro Arg Thr Ser Gly Gly Gly Glu Gln Tyr Phe
1 5 10 15

<210> 1212
<211> 15
<212> PRT
<213> Homo sapiens

<400> 1212

Cys Ala Ser Ser Val Gly Thr Ser Gly Val Gly Glu Gln Tyr Phe
1 5 10 15

<210> 1213
<211> 15
<212> PRT
<213> Homo sapiens

<400> 1213

Cys Ala Ser Ser Tyr Gly Ala Ser Gly Val Gly Glu Gln Phe Phe
1 5 10 15

<210> 1214
<211> 15
<212> PRT
<213> Homo sapiens

<400> 1214

Cys Ala Ser Ser Pro Thr Thr Ser Gly Arg Gly Glu Gln Tyr Phe
1 5 10 15

<210> 1215
<211> 13
<212> PRT
<213> Homo sapiens

<400> 1215

Cys Ala Ser Ser Gly Gln Thr Asn Thr Glu Ala Phe Phe
1 5 10

<210> 1216
<211> 15
<212> PRT
<213> Homo sapiens

<400> 1216

Cys Ala Ser Ser Pro Ser Ala Arg Gly Asn Gln Pro Gln His Phe
1 5 10 15

<210> 1217
<211> 15
<212> PRT
<213> Homo sapiens

<400> 1217

Cys Ala Ser Ser Pro Gly Thr Ser Gly Val Gly Glu Gln Phe Phe
1 5 10 15

<210> 1218
<211> 15
<212> PRT
<213> Homo sapiens

<400> 1218

Cys Ala Ser Ala Arg Leu Ala Gly Gly Thr Asp Glu Gln Phe Phe
1 5 10 15

<210> 1219
<211> 15
<212> PRT
<213> Homo sapiens

<400> 1219

Cys Ala Ser Ser Ala Met Ala Ser Gly Ser Asp Thr Gln Tyr Phe
1 5 10 15

<210> 1220
<211> 15
<212> PRT
<213> Homo sapiens

<400> 1220

Cys Ala Ser Ser Glu Leu Ala Ser Gly Thr Asp Thr Gln Tyr Phe
1 5 10 15

<210> 1221
<211> 15
<212> PRT
<213> Homo sapiens

<400> 1221

Cys Ala Ser Ser Val Gly Thr Ser Gly Val Gly Glu Gln Tyr Phe
1               5                   10                  15

<210> 1222
<211> 15
<212> PRT
<213> Homo sapiens

<400> 1222

Cys Ala Ser Ser Arg Leu Ala Gly Gly Met Asp Glu Gln Phe Phe
1               5                   10                  15

<210> 1223
<211> 15
<212> PRT
<213> Homo sapiens

<400> 1223

Cys Ala Ser Ser Ala Leu Ala Ser Gly Gly Asp Thr Gln Tyr Phe
1               5                   10                  15

<210> 1224
<211> 16
<212> PRT
<213> Homo sapiens

<400> 1224

Cys Ala Ser Ser Arg Arg Thr Ser Gly Gly Thr Asp Thr Gln Tyr Phe
1               5                   10                  15

<210> 1225
<211> 14
<212> PRT
<213> Homo sapiens

<400> 1225

Cys Ala Ser Ser Val Arg Arg Asn Asn Glu Lys Leu Phe Phe
1               5                   10

<210> 1226
<211> 15
<212> PRT
<213> Homo sapiens

<400> 1226

Cys Ala Ser Ser Ala Arg Thr Ser Gly Gly Gln Pro Gln His Phe
1               5                   10                  15

<210> 1227
<211> 15
<212> PRT
<213> Homo sapiens

<400>  1227

Cys Ala Ser Ser Ala Arg Thr Ser Gly Asn Gln Pro Gln His Phe
1               5                   10                  15

<210>  1228
<211>  15
<212>  PRT
<213>  Homo sapiens

<400>  1228

Cys Ala Ser Ser Ala Arg Ala Ser Gly Gly Asp Glu Gln Phe Phe
1               5                   10                  15

<210>  1229
<211>  15
<212>  PRT
<213>  Homo sapiens

<400>  1229

Cys Ala Ser Ser Glu Leu Thr Ser Gly Gly Asp Glu Gln Phe Phe
1               5                   10                  15

<210>  1230
<211>  15
<212>  PRT
<213>  Homo sapiens

<400>  1230

Cys Ala Ser Ser Pro Arg Ala Ser Gly Gly Asp Glu Gln Phe Phe
1               5                   10                  15

<210>  1231
<211>  16
<212>  PRT
<213>  Homo sapiens

<400>  1231

Cys Ala Ser Ser Arg Arg Thr Ser Gly Gly Pro Asn Glu Gln Phe Phe
1               5                   10                  15

<210>  1232
<211>  15
<212>  PRT
<213>  Homo sapiens

<400>  1232

Cys Ala Ser Ser Glu Leu Ala Ser Gly Ile Asn Glu Gln Phe Phe
1               5                   10                  15

<210>  1233

<211>  16
<212>  PRT
<213>  Homo sapiens

<400>  1233

Cys Ala Ser Arg Leu Arg Thr Ser Gly Gly Thr Asp Thr Gln Tyr Phe
1               5                   10                  15

<210>  1234
<211>  16
<212>  PRT
<213>  Homo sapiens

<400>  1234

Cys Ala Ser Arg Arg Leu Ala Gly Phe Met Ala Asp Thr Gln Tyr Phe
1               5                   10                  15

<210>  1235
<211>  15
<212>  PRT
<213>  Homo sapiens

<400>  1235

Cys Ala Ser Ser Ala Leu Ala Ser Gly Gly Asp Thr Gln Tyr Phe
1               5                   10                  15

<210>  1236
<211>  16
<212>  PRT
<213>  Homo sapiens

<400>  1236

Cys Ala Ser Ser Ala Arg Thr Ser Ala Gly Thr Asp Thr Gln Tyr Phe
1               5                   10                  15

<210>  1237
<211>  16
<212>  PRT
<213>  Homo sapiens

<400>  1237

Cys Ala Ser Ser Ala Arg Thr Ser Gly Gly Ala Asp Thr His Tyr Phe
1               5                   10                  15

<210>  1238
<211>  16
<212>  PRT
<213>  Homo sapiens

<400>  1238

Cys Ala Ser Ser Ala Arg Thr Ser Gly Gly Ala Asp Thr Gln Tyr Phe
1               5                   10                  15

<210> 1239
<211> 16
<212> PRT
<213> Homo sapiens

<400> 1239

Cys Ala Ser Ser Ala Arg Thr Ser Gly Gly Ser Asp Thr Gln Tyr Phe
1 5 10 15

<210> 1240
<211> 16
<212> PRT
<213> Homo sapiens

<400> 1240

Cys Ala Ser Ser Ala Arg Thr Ser Gly Gly Thr Asp Thr Gln Tyr Phe
1 5 10 15

<210> 1241
<211> 16
<212> PRT
<213> Homo sapiens

<400> 1241

Cys Ala Ser Ser Glu Arg Thr Ser Gly Gly Arg Asp Thr Gln Tyr Phe
1 5 10 15

<210> 1242
<211> 16
<212> PRT
<213> Homo sapiens

<400> 1242

Cys Ala Ser Ser Gly Arg Thr Ser Gly Gly Ser Asp Thr Gln Tyr Phe
1 5 10 15

<210> 1243
<211> 16
<212> PRT
<213> Homo sapiens

<400> 1243

Cys Ala Ser Ser Leu Arg Ala Ser Gly Gly Ser Asp Thr Gln Tyr Phe
1 5 10 15

<210> 1244
<211> 16
<212> PRT
<213> Homo sapiens

<400> 1244

Cys Ala Ser Ser Leu Arg Thr Ser Gly Gly Ala Asp Thr Gln Tyr Phe
1 5 10 15

<210> 1245
<211> 15
<212> PRT
<213> Homo sapiens

<400> 1245

Cys Ala Ser Ser Pro Leu Thr Ser Ala Thr Asp Thr Gln Tyr Phe
1 5 10 15

<210> 1246
<211> 16
<212> PRT
<213> Homo sapiens

<400> 1246

Cys Ala Ser Ser Gln Arg Thr Ser Gly Gly Ala Asp Thr Gln Tyr Phe
1 5 10 15

<210> 1247
<211> 16
<212> PRT
<213> Homo sapiens

<400> 1247

Cys Ala Ser Ser Arg Leu Thr Ser Gly Gly Ser Asp Thr Gln Tyr Phe
1 5 10 15

<210> 1248
<211> 16
<212> PRT
<213> Homo sapiens

<400> 1248

Cys Ala Ser Ser Arg Arg Thr Ser Gly Gly Leu Asp Thr Gln Tyr Phe
1 5 10 15

<210> 1249
<211> 16
<212> PRT
<213> Homo sapiens

<400> 1249

Cys Ala Ser Ser Arg Arg Thr Ser Gly Gly Thr Asp Thr Gln Tyr Phe
1 5 10 15

<210> 1250
<211> 15
<212> PRT

<213>  Homo sapiens

<400>  1250

Cys Ala Ser Ser Lys Arg Thr Ser Gly Gly Asp Thr Gln Tyr Phe
1               5                   10                  15

<210>  1251
<211>  15
<212>  PRT
<213>  Homo sapiens

<400>  1251

Cys Ala Ser Asn Arg Arg Thr Ser Gly Thr Tyr Glu Gln Tyr Phe
1               5                   10                  15

<210>  1252
<211>  15
<212>  PRT
<213>  Homo sapiens

<400>  1252

Cys Ala Ser Ser Leu Arg Thr Ser Gly Gly Asp Glu Gln Tyr Phe
1               5                   10                  15

<210>  1253
<211>  14
<212>  PRT
<213>  Homo sapiens

<400>  1253

Cys Ala Ser Ser Pro Arg Ala Ser Gly Gly Glu Gln Tyr Phe
1               5                   10

<210>  1254
<211>  15
<212>  PRT
<213>  Homo sapiens

<400>  1254

Cys Ala Ser Ser Pro Arg Thr Ser Gly Gly Asp Glu Gln Tyr Phe
1               5                   10                  15

<210>  1255
<211>  12
<212>  PRT
<213>  Homo sapiens

<400>  1255

Cys Ala Phe Lys Ala Ala Gly Asn Lys Leu Thr Phe
1               5                   10

<210> 1256
<211> 12
<212> PRT
<213> Homo sapiens

<400> 1256

Cys Ala Phe Lys Ala Ala Gly Asn Lys Leu Thr Phe
1 5 10

<210> 1257
<211> 12
<212> PRT
<213> Homo sapiens

<400> 1257

Cys Ala Phe Lys Ala Ala Gly Asn Lys Leu Thr Phe
1 5 10

<210> 1258
<211> 12
<212> PRT
<213> Homo sapiens

<400> 1258

Cys Ala Ser Lys Ala Ala Gly Asn Lys Leu Thr Phe
1 5 10

<210> 1259
<211> 12
<212> PRT
<213> Homo sapiens

<400> 1259

Cys Ala Ser Lys Ala Ala Gly Asn Lys Leu Thr Phe
1 5 10

<210> 1260
<211> 12
<212> PRT
<213> Homo sapiens

<400> 1260

Cys Ser Arg Arg Ala Ala Gly Asn Lys Leu Thr Phe
1 5 10

<210> 1261
<211> 12
<212> PRT
<213> Homo sapiens

<400> 1261

Cys Ala Phe Lys Ala Ala Gly Asn Lys Leu Thr Phe

1 5 10

<210> 1262
<211> 12
<212> PRT
<213> Homo sapiens

<400> 1262

Cys Ala Phe Lys Ala Ala Gly Asn Lys Leu Thr Phe
1 5 10

<210> 1263
<211> 14
<212> PRT
<213> Homo sapiens

<400> 1263

Cys Ala Ser Asp Tyr Gly Gly Ser Gln Gly Asn Leu Ile Phe
1 5 10

<210> 1264
<211> 10
<212> PRT
<213> Homo sapiens

<400> 1264

Cys Ala Tyr Gly Ala Asn Asn Leu Phe Phe
1 5 10

<210> 1265
<211> 14
<212> PRT
<213> Homo sapiens

<400> 1265

Cys Ala Ser Ser Arg Leu Ala Gly Gly Met Asp Glu Gln Phe
1 5 10

<210> 1266
<211> 14
<212> PRT
<213> Homo sapiens

<400> 1266

Cys Ala Thr Thr Pro Gly Ala Ser Gly Ile Ser Glu Gln Phe
1 5 10

<210> 1267
<211> 14
<212> PRT
<213> Homo sapiens

<400> 1267

Cys Ala Ser Ser Gly Leu Ala Gly Gly Met Asp Glu Gln Phe
1 5 10

<210> 1268
<211> 14
<212> PRT
<213> Homo sapiens

<400> 1268

Cys Ala Ser Ser Arg Leu Ala Gly Gly Met Asp Glu Gln Phe
1 5 10

<210> 1269
<211> 14
<212> PRT
<213> Homo sapiens

<400> 1269

Cys Ala Thr Thr Pro Gly Ala Ser Gly Ile Ser Glu Gln Phe
1 5 10

<210> 1270
<211> 14
<212> PRT
<213> Homo sapiens

<400> 1270

Cys Ala Ser Ser Gly Leu Ala Gly Gly Met Asp Glu Gln Phe
1 5 10

<210> 1271
<211> 15
<212> PRT
<213> Homo sapiens

<400> 1271

Cys Ala Ser Ser Pro Gly Thr Ser Gly Val Gly Glu Gln Phe Phe
1 5 10 15

<210> 1272
<211> 16
<212> PRT
<213> Homo sapiens

<400> 1272

Cys Ala Ser Ser Arg Arg Thr Ser Gly Gly Thr Asp Thr Gln Tyr Phe
1 5 10 15

<210> 1273
<211> 13

<212> PRT
<213> Homo sapiens

<400> 1273

Cys Ala Ser Ser Glu Phe Arg Thr Arg Gly Tyr Thr Phe
1 5 10

<210> 1274
<211> 14
<212> PRT
<213> Homo sapiens

<400> 1274

Cys Ala Ser Arg Pro Leu His Ser Val Tyr Glu Gln Tyr Phe
1 5 10